# EUROPEAN PATENT APPLICATION

(11) **EP 4 782 452 A1**
(43) Date of publication of application: **29.07.2026**
(21) Application number: 24867606.6
(22) Date of filing: 23.09.2024
(51) Int. Cl.: C07D 487/04, C07D 471/10, C07D 403/12, C07D 401/14, A61K 31/513, A61P 35/00, A61P 35/02, A61P 25/08

(54) **IRAK DEGRADATION AGENT AND USE THEREOF**

(30) Priority: 21.09.2023 CN 202311229384; 24.04.2024 CN 202410504738
(71) Applicant: Increland, Sichuan 610000 (CN)
(72) Inventor: ZHANG, Dengyou, Chengdu, Sichuan 610000 (CN); ZHANG, Yangxue, Chengdu, Sichuan 610000 (CN); LI, Ping, Chengdu, Sichuan 610000 (CN); ZHOU, Ziyang, Chengdu, Sichuan 610000 (CN)
(74) Representative: Goodwin Procter (UK) LLP
(86) International application number: PCT/CN2024/120460
(87) International publication number: WO 2025/061203

(57) **Abstract**

A compound represented by formula (I) and a use thereof in the preparation of a drug. The invention relates to a novel compound having an IRAK4 degradation effect, which can effectively degrade IRAK4 or otherwise inhibit IRAK4 activity. The invention has very good prospects for application in IRAK4-mediated diseases, including immune diseases, tumors, Alzheimer's disease, and fibrotic diseases, and provides a new choice for clinical screening and/or preparation of drugs related to IRAK4 activity.

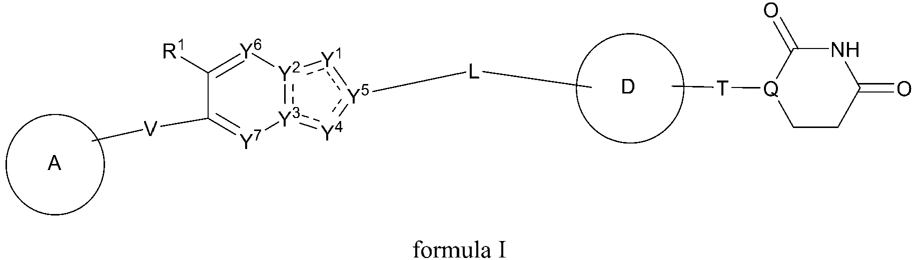

## Description

The present application claims priority to Chinese patent application with the application number of 202311229384X filed with the China National Intellectual Property Administration on 21 September 2023 and entitled "IRAK DEGRADATION AGENT AND USE THEREOF", Chinese patent application with the application number of 202410504738.5 filed with the China National Intellectual Property Administration on 24 April 2024 and entitled "IRAK DEGRADATION AGENT AND USE THEREOF", which are incorporated herein by reference in their entirety.

### Technical Field

The present invention belongs to the field of medicine, and specifically relates to a class of compounds with IRAK-degrading activity and their use in the preparation of drugs.

### Background Art

Protein degradation is a highly regulated and essential process for maintaining cellular homeostasis. Ubiquitin-proteasome pathway (UPP) functions in vivo to selectively identify and remove excess proteins, and degrade misfolded or abnormal proteins. Ubiquitin molecules are covalently linked to terminal lysine residues by E3 ubiquitin ligases, thereby marking proteins for degradation by proteasomes into small peptides, and finally digesting the small peptides into their constituent amino acids, which are then used as building blocks for new proteins. The UPP plays a central role in multiple cellular processes and, if defective or unbalanced, causes the pathogenesis of a variety of diseases. The UPP is central to regulating almost all cellular processes, including antigen processing, apoptosis, organelle biogenesis, cell cycle, DNA transcription and repair, differentiation and development, immune response and inflammation, neural and muscle degeneration, morphogenesis of neural networks, regulation of cell surface receptors, ion channels and secretory pathways, responses to stress and extracellular regulators, ribosome biogenesis and viral infection. Defective proteasomal degradation is implicated in a variety of clinical conditions, including, e.g., Alzheimer's disease, Parkinson's disease, Huntington's disease, myodystrophy, cardiovascular disease, and cancer.

Protein degradation targeted chimera (PROTAC) is an effective means for degrading pathogenic proteins. Small molecules capable of binding to target proteins are linked to small molecules capable of binding to E3 ligases including, e.g., CRBN, VHL, MDM2, and DRAF to form heterobifunctional molecules, and the target protein is ubiquitinated by simulating the ubiquitin-proteasome pathway (UPP), thereby degrading the target protein by the proteasome. Compared with small molecule inhibitors, a potential advantage of the protein degradation targeted chimera is that it can disable all functions of pathogenic proteins.

At present, more than 600 E3 ubiquitin ligases have been found to promote in vivo ubiquitination of different proteins, which can be classified into four families: HECT domain E3 family, U-box E3 family, monomeric RING E3 family, and multi-subunit E3 family. Cereblon (CRBN) ligase is a most widely used E3 ligase of PROTAC technology. As a 442-amino acid protein, the Cereblon belongs to a Cullin RING E3 ubiquitin ligase, forms a Cullin-4-RING E3 ubiquitin ligase (CRL4) complex, and interacts with adaptor protein damaged DNA binding protein 1 (DDB1). In a CRL4 complex, CRBN acts as a substrate-specific receptor. Known CRBN ligands include thalidomide and other derived immunomodulatory imide drugs. After the CRBN binds to the ligand, E3 ubiquitin ligase activity of the CRBN is re-regulated, thereby increasing recruitment of transcription factors Ikaros and Aiolos, and triggering subsequent ubiquitination and proteasomal degradation. At present, the CRBN, as an E3 ligase in PROTAC, has been successfully used to target more than 30 different proteins, including proteins implicated in various cancers (Sun X. et al., 2019), proteins implicated in immune dysfunction (Bassi et al., 2018), proteins implicated in neurodegenerative disease (Silva et al., 2019), and hepatitis C virus proteins (de Wispelaere et al., 2019). Most CRBN-targeted PROTACs employ pomalidomide, 4-hydroxythalidomide, alkyl-linked thalidomide derivatives, or lenalidomide derivatives. However, it is possible to develop better CRBN ligands. These new CRBN ligands will provide more options for the development of the PROTAC technology.

IRAK4 belongs to a serine/threonine kinase and is a key protein in mediating Toll-like receptor (TLR) signals of interleukin-1 (IL-1) receptor family (IL-1, IL-18, and IL-33 receptors) and pathogen recognition. Studies show that when foreign pathogens and inflammatory stress are recognized, under the action of extracellular ligands, interleukin-1 receptors or TLR receptors recruit the adapter protein myeloid differentiation primary response protein (Myd88), which then forms a complex with the IRAK4, thereby activating NF-κB light chain enhancer and activator protein 1 (AP-1), producing various inflammatory factors by cells, such as tumor necrosis factor α (TNFα) and IL-6, and inducing various immune diseases, such as psoriasis, hidradenitis suppurativa, atopic dermatitis, rheumatoid arthritis, and systemic lupus erythematosus. In addition, the IRAK4 has been verified to be implicated in lymphocytic leukemia and lymphoma, Alzheimer's disease, and fibrotic disease. Therefore, the IRAK4 is an attractive target for drug development.

At present, major pharmaceutical companies, including, e.g., Pfizer, Gilead, Bayer, and Curie, have successively promoted the entry of IRAK4 small molecule inhibitors into clinical trials for, e.g., hematological tumor, psoriasis, rheumatoid arthritis, enteritis, and systemic lupus erythematosus. The IRAK4 inhibitor PF-06650833 studied by Pfizer has entered phase II clinical trial. Early clinical results show that PF-06650833 has good safety, and its efficacy shows that PF-06650833 can inhibit IRAK4-mediated inflammatory pathway. Such clinical data fully demonstrates that the IRAK4 is a clinically proven drug target and has the potential to treat a variety of diseases.

Recent studies show that not only does the kinase activity of IRAK4 mediate the inflammatory signaling pathways, but also the IRAK4 protein skeleton can activate some inflammatory signaling pathways. In human skin fibroblasts, inhibition of IRAK4 by ATP-competitive small molecules cannot effectively inhibit the release of IL-6 and TNF-α stimulated by IL-1β. This means that although ATP-competitive inhibitors can inhibit the kinase activity of IRAK4, they may not be able to completely disrupt its protein backbone function, thus affecting the inhibitory effect on IL-6 and TNF-α release. In addition, knockout of the IRAK4 can effectively eliminate inflammatory responses mediated by IL-1, IL-8, and TLR ligands. Therefore, ATP-competitive small molecule inhibitors cannot completely eliminate the inflammatory signaling pathways mediated by IRAK4 proteins. Thus it can be seen that targeting IRAK4 by small molecule inhibitors has its therapeutic limitations.

Protein degradation targeted chimera (PROTAC) is an effective means for degrading pathogenic proteins. Small molecules capable of binding to target proteins are linked to small molecules capable of binding to E3 ligases including, e.g., CRBN, VHL, MDM2, and DRAF to form heterobifunctional molecules, and the target protein is ubiquitinated by simulating the ubiquitin-proteasome pathway (UPP), thereby degrading the target protein by the proteasome. Compared with small molecule inhibitors, a potential advantage of the protein degradation targeted chimera is that it can disable all functions of pathogenic proteins. In addition, GSK scientists have demonstrated that the IRAK4 proteins can be degraded by binding IRAK4 small molecule inhibitors with ligands of E3 ligases CRBN and VHL through linker fragments to form the protein degradation targeting chimera (PROTAC). At the same time, Kymera and Avinas have designed corresponding PROTAC for the IRAK4. These emerging technologies provide a new therapeutic means for targeting the IRAK4.

### Summary of the Invention

The present invention first provides a compound represented by formula I, a stereoisomer thereof, a deuterated compound thereof, or a pharmaceutically acceptable salt thereof: 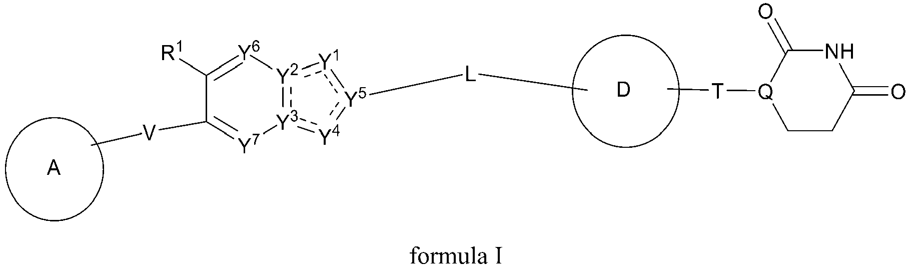 wherein
V is selected from -C(O)NH- or -NHC(O)-;
T is selected from -NH- or a chemical bond;
-̅ -̅ -̅ -̅ -̅ -̅ represents a single bond or a double bond;
Y² and Y³ are each independently selected from C or N;
Y¹ and Y⁴ are each independently selected from CR^{Y}, CR^{Y}R^{Y}, N, NR^{Y}, O, S, C(O), S(O), or S(O)₂;
Y⁵ is selected from C or N;
Y⁶ and Y⁷ are each independently selected from CR⁴ or N;
each R^{Y} is selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl;
Q is selected from CR^{Q} or N;
R^{Q} is selected from hydrogen, deuterium, halogen, cyano, C₁₋₆ alkyl, C₁₋₆ alkoxy, halogen-substituted C₁₋₆ alkyl, halogen-substituted C₁₋₆ alkoxy;
ring A is selected from 5- to 10-membered heteroaromatic rings; wherein the heteroaromatic ring is optionally substituted with 1, 2 or 3 R^{A1} groups:
   each R^{A1} is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{A2}, -C₀₋₂ alkylene-NR^{A2}R^{A3}, -C₀₋₂ alkylene-3- to 10-membered carbocyclyl, -C₀₋₂ alkylene-4- to 10-membered heterocyclyl;
   R^{A2} and R^{A3} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
   R¹ and R⁴ are each independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR¹¹, -C₀₋₂ alkylene-NR¹¹R¹², -C₀₋₂ alkylene-NR¹¹C(O)R¹², -C₀₋₂ alkylene-C(O)R¹¹, -C₀₋₂ alkylene-C(O)NR¹¹R¹², -C₀₋₂ alkylene-3- to 10-membered carbocyclyl, -C₀₋₂ alkylene-4- to 10-membered heterocyclyl, -C₀₋₂ alkylene-4- to 10-membered bridged rings, -C₀₋₂ alkylene-4- to 10-membered bridged heterocycles, -C₀₋₂ alkylene-5- to 12-membered spiro rings, -C₀₋₂ alkylene-5- to 12-membered spiro heterocycles, -C₀₋₂ alkylene-6- to 10-membered aromatic rings, -C₀₋₂ alkylene-5- to 10-membered heteroaromatic ring; wherein the carbocyclyl, heterocyclyl, bridged ring, bridged heterocycle, spiro ring, spiro heterocycle, aromatic ring and heteroaromatic ring are optionally substituted with 1, 2 or 3 R¹³ groups;
   R¹¹ and R¹² are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
   each R¹³ is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR¹⁴, -C₀₋₂ alkylene--NR¹⁴R¹⁵, -C₀₋₂ alkylene-NR¹⁴C(O)R¹⁵, -C₀₋₂ alkylene-C(O)R¹⁴, -C₀₋₂ alkylene-C(O)NR¹⁴R¹⁵;
   R¹⁴ and R¹⁵ are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
   ring D is selected from 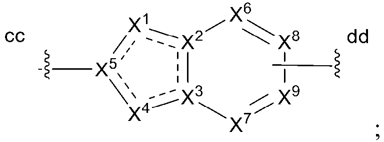 where the cc end in is attached to L and the dd end is attached to Q;
   -̅ -̅ -̅ -̅ -̅ -̅ represents a single bond or a double bond;
   X² and X³ are each independently selected from C or N;
   X¹ and X⁴ are each independently selected from CR^{X}, CR^{X}R^{X}, N, NR^{X}, O, S, C(O), S(O), or S(O)₂;
   X⁵ is selected from C or N;
   X⁶ and X⁷ are each independently selected from CR² or N;
   X⁸ and X⁹ are each independently selected from C, CR² or N;
   each R^{X} is selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl;
   each R² is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR²¹, -C₀₋₂ alkylene-NR²¹R²², -C₀₋₂ alkylene-3- to 10-membered carbocyclyl, -C₀₋₂ alkylene-4- to 10-membered heterocyclyl;
   R²¹ and R²² are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
   L is 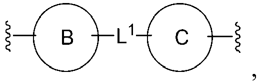 where ring B is attached to the nitrogen atom of the heteroaromatic ring at the R¹ end, and ring C is attached to ring D;
   ring B is selected from 3- to 10-membered carbocyclyl, 4- to 10-membered heterocyclyl, 4- to 10-membered bridged rings, 4- to 10-membered bridged heterocycles, 5- to 12-membered spiro rings, 5- to 12-membered spiro heterocycles, 6- to 10-membered aromatic rings, and 5- to 10-membered heteroaromatic rings; wherein the carbocyclyl, heterocyclyl, bridged ring, bridged heterocycle, spiro ring, spiro heterocycle, aromatic ring and heteroaromatic ring are optionally substituted with 1, 2 or 3 R^{B} groups;
   preferably, ring B is selected from 4-membered carbocyclyl, 5-membered carbocyclyl, 6-membered carbocyclyl, 7-membered carbocyclyl, 4-membered heterocyclyl, 5-membered heterocyclyl, 6-membered heterocyclyl, 7-membered heterocyclyl, 8-membered heterocyclyl, 5-membered bridged rings, 6-membered bridged rings, 7-membered bridged rings, 8-membered bridged rings, 5-membered bridged heterocycles, 6-membered bridged heterocycles, 7-membered bridged heterocycles, 8-membered bridged heterocycles, 7-membered spiro rings, 8-membered spiro rings, 9-membered spiro rings, 10-membered spiro rings, 11-membered spiro rings, 7-membered spiro heterocycles, 8-membered spiro heterocycles, 9-membered spiro heterocycles, 10-membered spiro heterocycles, and 11-membered spiro heterocycles; wherein the carbocyclyl, heterocyclyl, bridged ring, bridged heterocycle, spiro ring and spiro heterocycle are optionally substituted with 1, 2 or 3 R^{B} groups;
   ring C is selected from 3- to 10-membered carbocyclyl, 4- to 10-membered heterocyclyl, 4- to 10-membered bridged rings, 4- to 10-membered bridged heterocycles, 5- to 12-membered spiro rings, 5- to 12-membered spiro heterocycles, 6- to 10-membered aromatic rings, and 5- to 10-membered heteroaromatic rings; wherein the carbocyclyl, heterocyclyl, bridged ring, bridged heterocycle, spiro ring, spiro heterocycle, aromatic ring and heteroaromatic ring are optionally substituted with 1, 2 or 3 R^{C} groups;
   preferably, ring C is selected from 4-membered carbocyclyl, 5-membered carbocyclyl, 6-membered carbocyclyl, 7-membered carbocyclyl, 4-membered heterocyclyl, 5-membered heterocyclyl, 6-membered heterocyclyl, 7-membered heterocyclyl, 8-membered heterocyclyl, 5-membered bridged rings, 6-membered bridged rings, 7-membered bridged rings, 8-membered bridged rings, 5-membered bridged heterocycles, 6-membered bridged heterocycles, 7-membered bridged heterocycles, 8-membered bridged heterocycles, 7-membered spiro rings, 8-membered spiro rings, 9-membered spiro rings, 10-membered spiro rings, 11-membered spiro rings, 7-membered spiro heterocycles, 8-membered spiro heterocycles, 9-membered spiro heterocycles, 10-membered spiro heterocycles, and 11-membered spiro heterocycles; wherein the carbocyclyl, heterocyclyl, bridged ring, bridged heterocycle, spiro ring and spiro heterocycle are optionally substituted with 1, 2 or 3 R^{C} groups;
   R^{B} and R^{C} are each independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{B1}, -C₀₋₂ alkylene-NR^{B1}R^{B2};
   R^{B1} and R^{B2} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
   L¹ is selected from a chemical bond, C₁₋₆ alkylene, C₂₋₆ alkenylene, and C₂₋₆ alkynylene; wherein the carbon atom in the alkylene, alkenylene, or alkynylene is optionally substituted with 1, 2, or 3 heteroatoms, and the alkylene, alkenylene, or alkynylene is optionally substituted with 1, 2, or 3 R^{L1} groups;
   each R^{L1} is independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl.

In some preferred embodiments of the present invention,
Y¹ is selected from N, Y² is selected from C, Y³ is selected from C, Y⁴ is selected from CH, Y⁵ is selected from N, Y⁶ is selected from CH, Y⁷ is selected from CH; or Y¹ is selected from N, Y² is selected from C, Y³ is selected from N, Y⁴ is selected from CH, Y⁵ is selected from C, Y⁶ is selected from CH, Y⁷ is selected from CH; or Y¹ is selected from N, Y² is selected from C, Y³ is selected from N, Y⁴ is selected from N, Y⁵ is selected from C, Y⁶ is selected from CH, Y⁷ is selected from CH; or Y¹ is selected from N, Y² is selected from C, Y³ is selected from N, Y⁴ is selected from CH, Y⁵ is selected from C, Y⁶ is selected from CH, Y⁷ is selected from N;
or Y¹ is selected from N, Y² is selected from C, Y³ is selected from N, Y⁴ is selected from CH, Y⁵ is selected from C, Y⁶ is selected from N, Y⁷ is selected from CH;

In some preferred embodiments of the present invention,
ring D is selected from

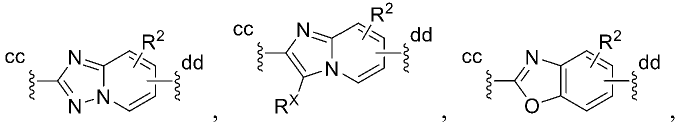

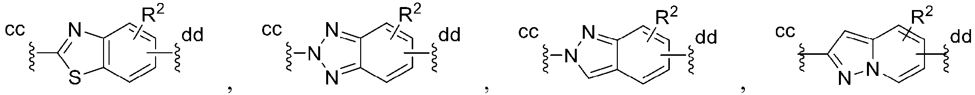

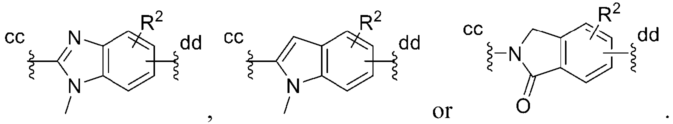

In some embodiments of the present invention, preferably, the compound of formula I is shown as follows: 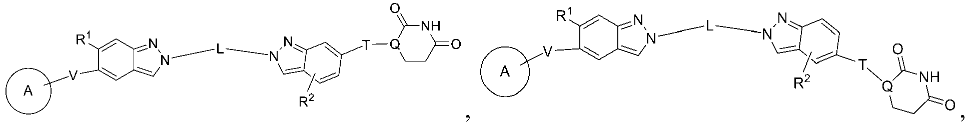 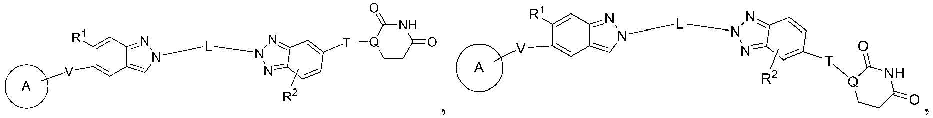 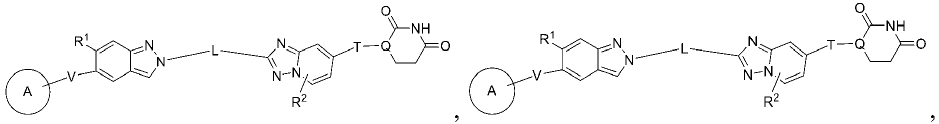 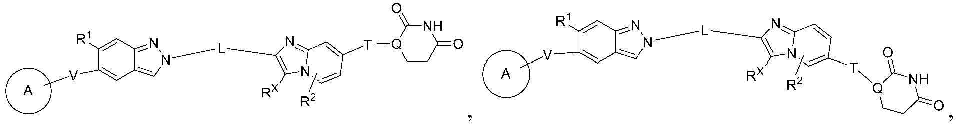 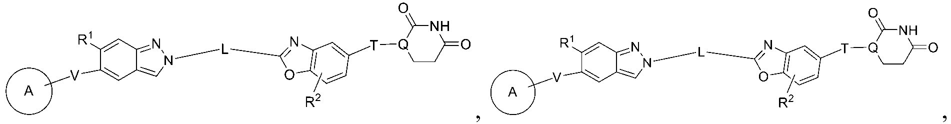 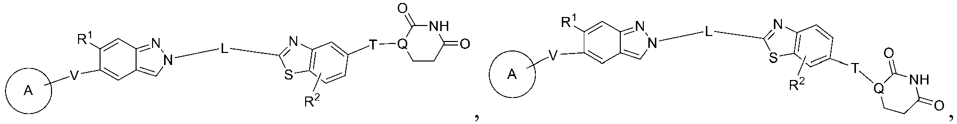 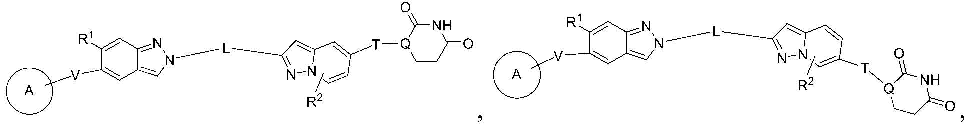 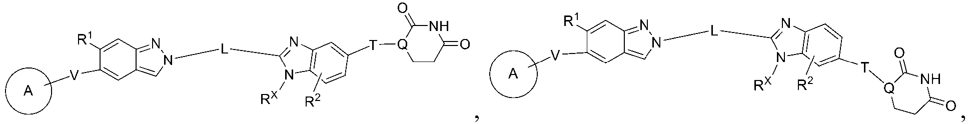 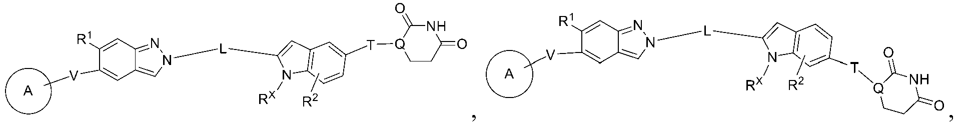 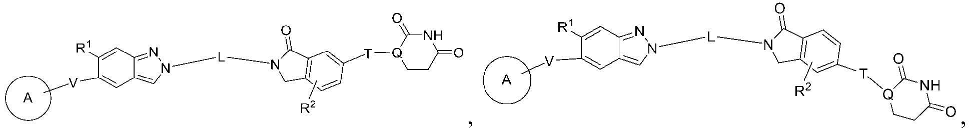 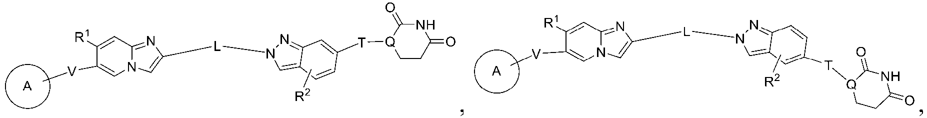 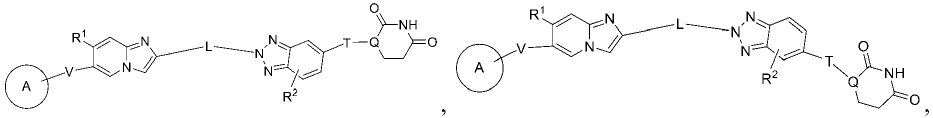 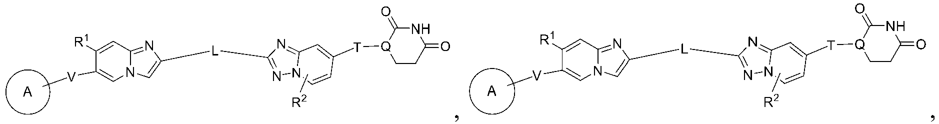 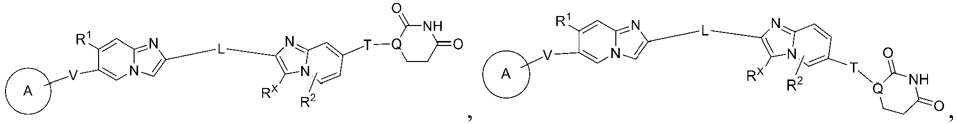 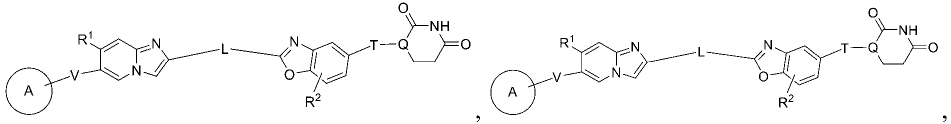 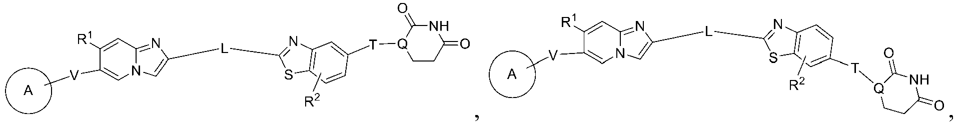 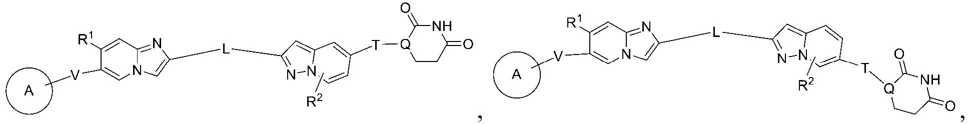 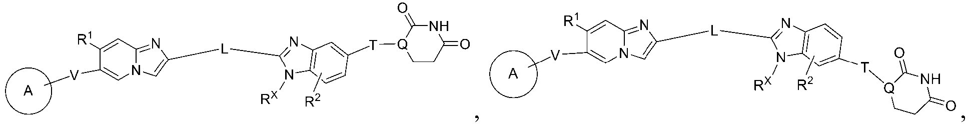 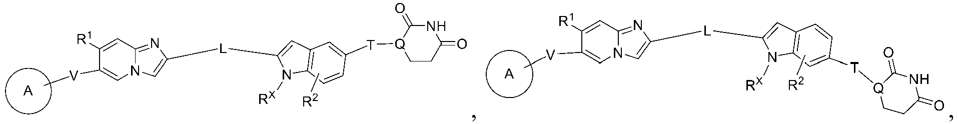 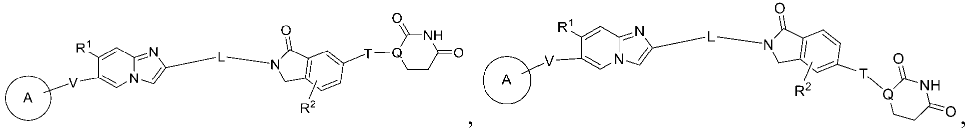 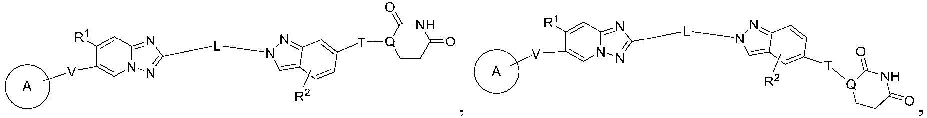 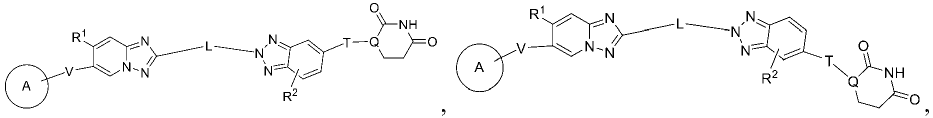 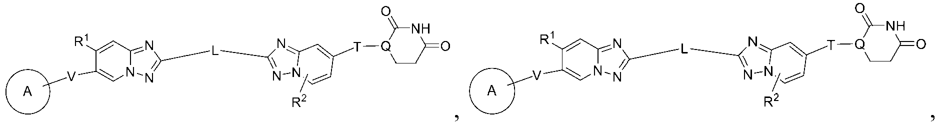 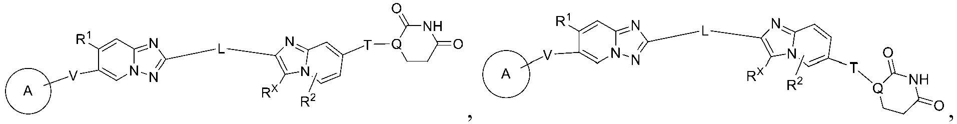 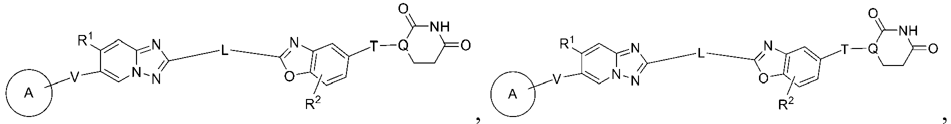 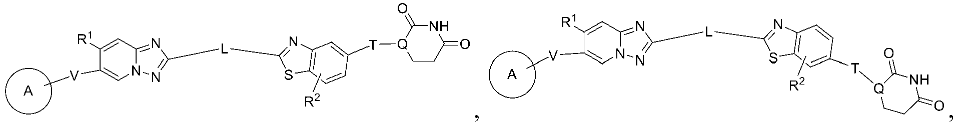 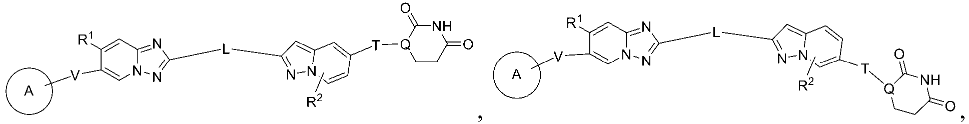 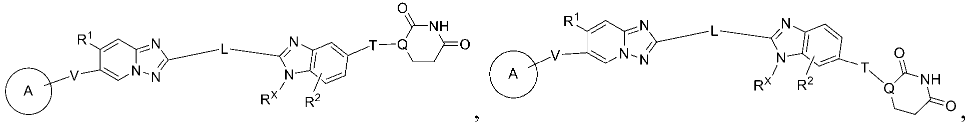 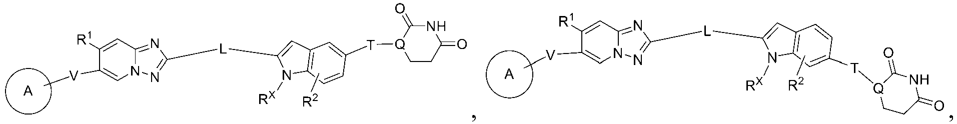 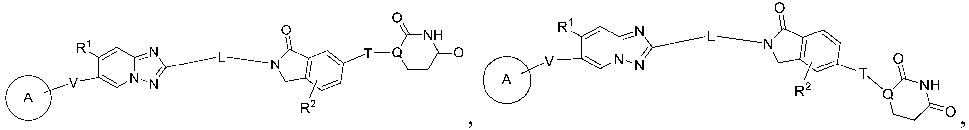 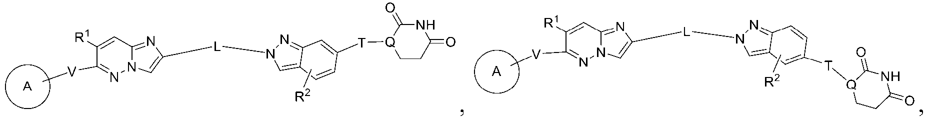 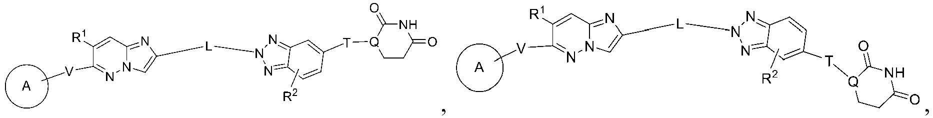 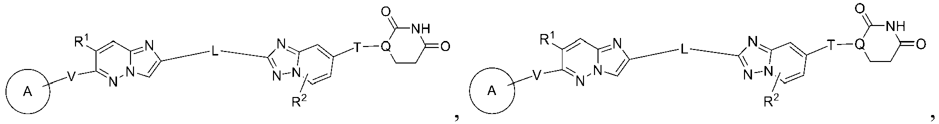 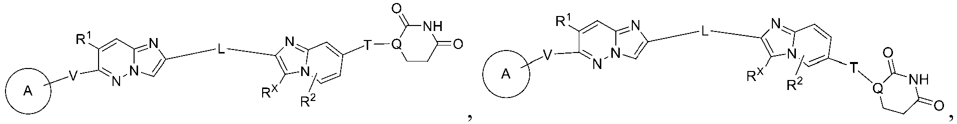 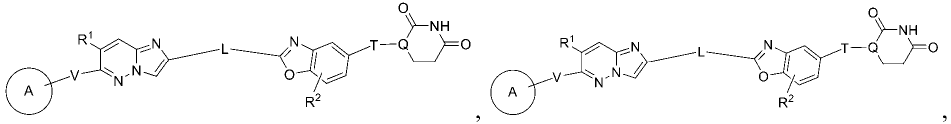 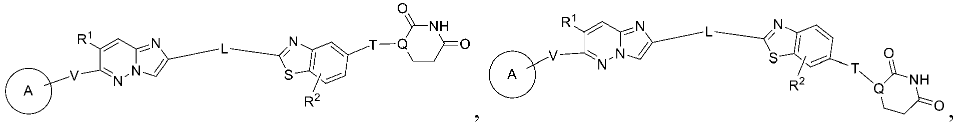 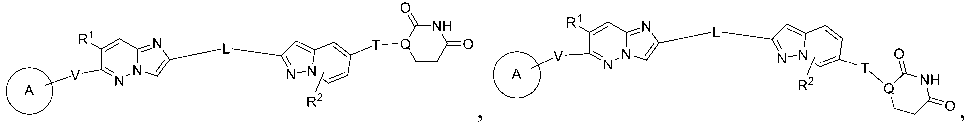 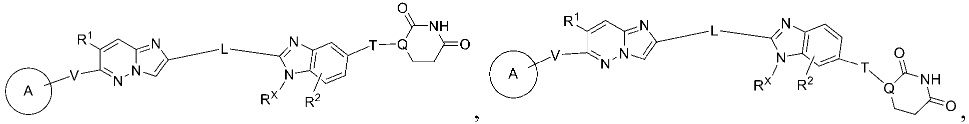 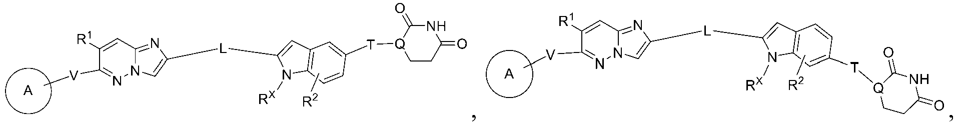 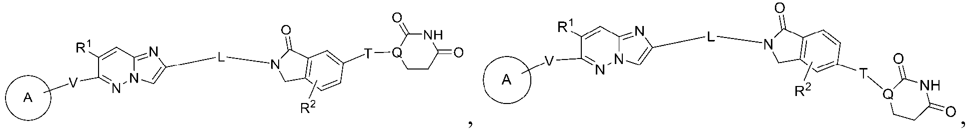 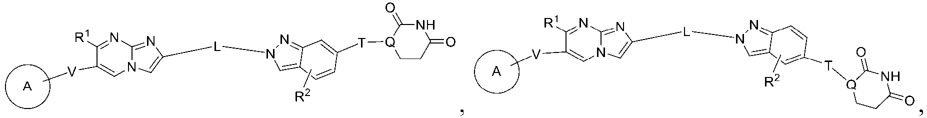 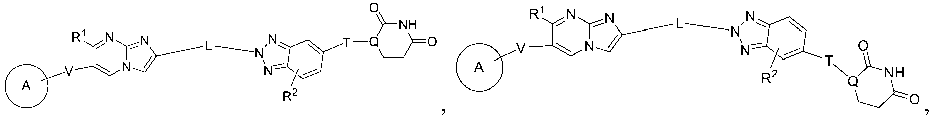 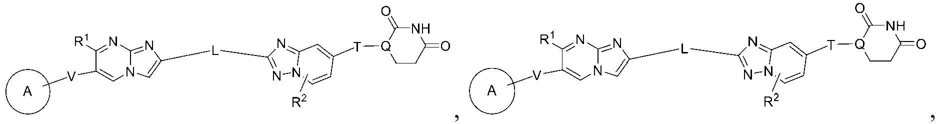 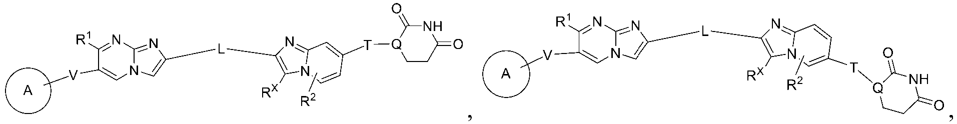 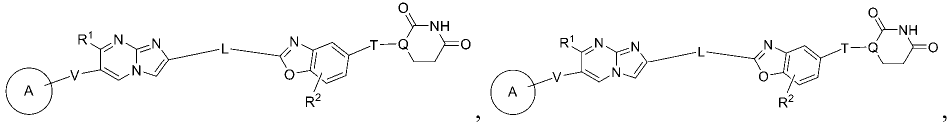 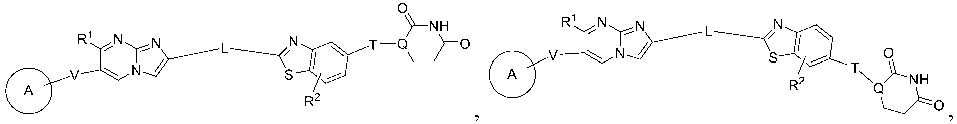 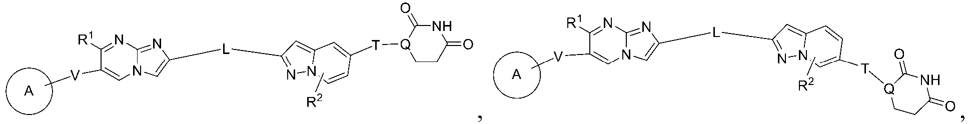 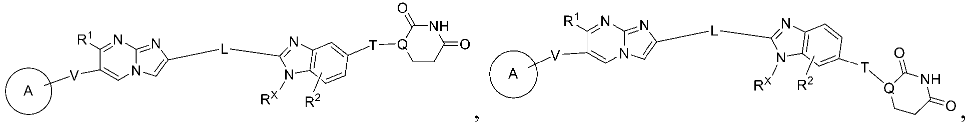 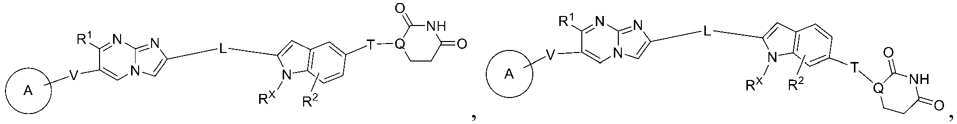 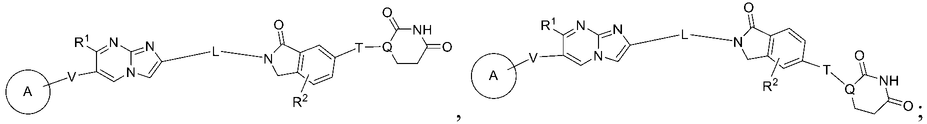
wherein V, T, Q, ring A, R¹, R², and L are as defined above. 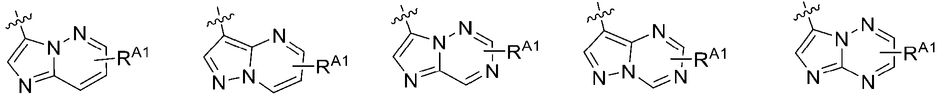 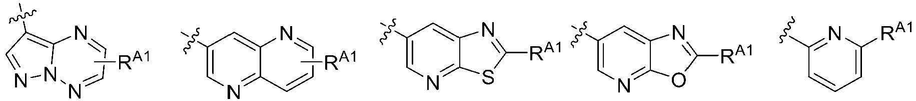 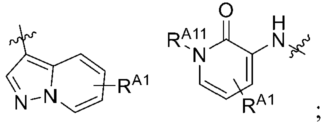
R^{A1} is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, monomethylamino or dimethylamino.
R^{A11} is selected from methyl, ethyl, propyl, cyclopropyl, and halogen-substituted methyl, ethyl, propyl, and cyclopropyl.

In some preferred embodiments of the present invention,
R¹ is selected from hydrogen, deuterium, halogen, cyano, methyl, ethyl, propyl, cyclopropyl, cyclobutyl, cyclopentyl, hydroxymethyl, methoxy, ethoxy, trifluoromethyl, difluoromethyl, monofluoromethyl, methoxymethyl, ethoxymethyl, monomethylamino, dimethylamino, deuterated monomethylamino, and deuterated dimethylamino;
or, R¹ is selected from rings below:

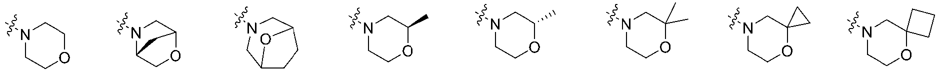

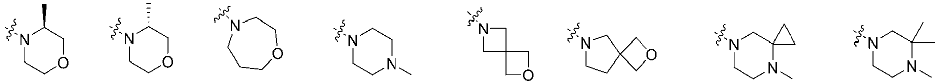

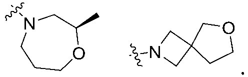

In some preferred embodiments of the present invention,
R² is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, monomethylamino or dimethylamino.

In some embodiments of the present invention, Q is selected from CH or N.

In some preferred embodiments of the present invention,
ring B is selected from 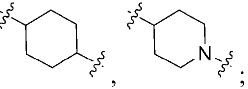 where q is 0, 1, 2 or 3;
each R^{B} is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{B1}, -C₀₋₂ alkylene-NR^{B1}R^{B2};
R^{B1} and R^{B2} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
ring C is selected from 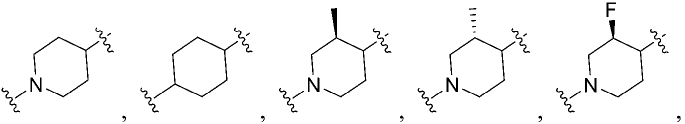 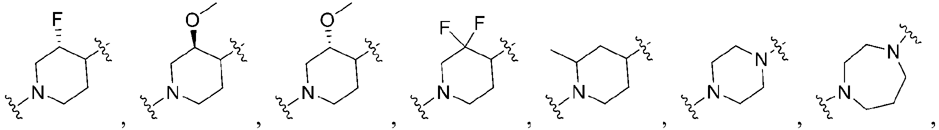 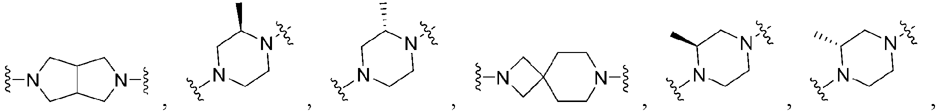 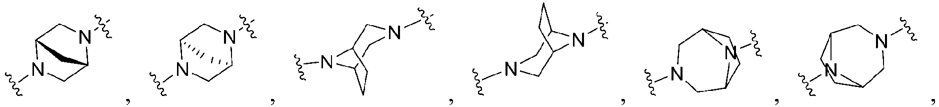 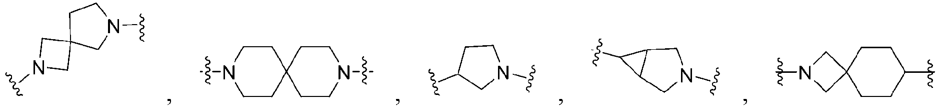 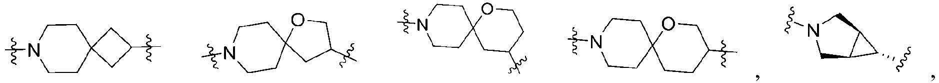 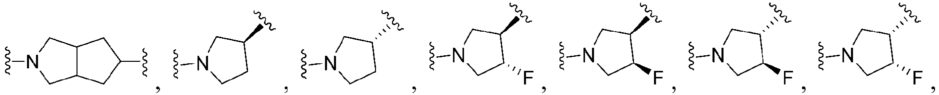 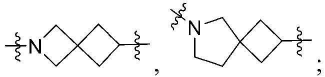
L¹ is selected from a chemical bond, methylene, and ethylene.

In some embodiments of the present invention, preferably, L is selected from structures below: 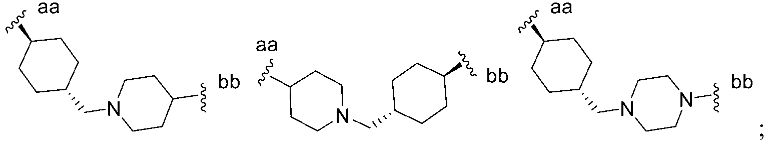 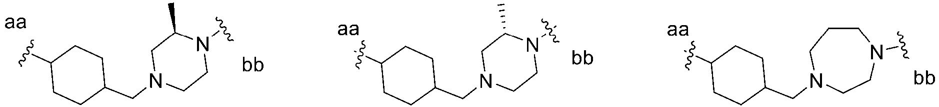 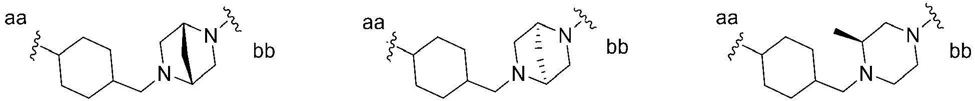 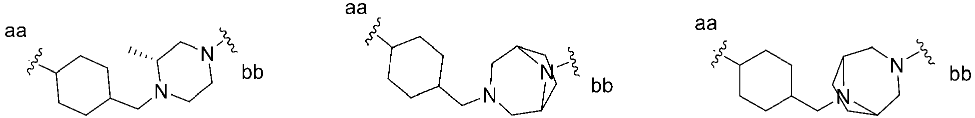 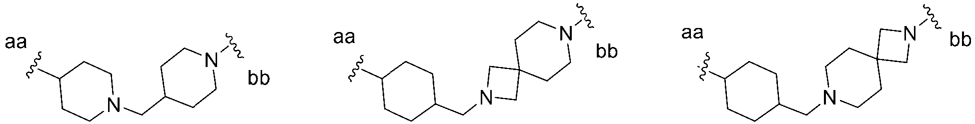 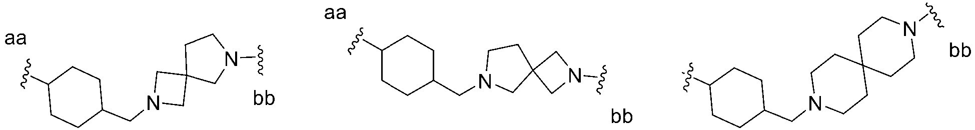 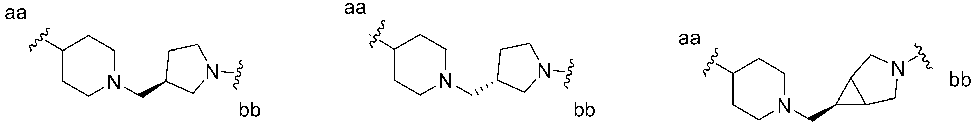 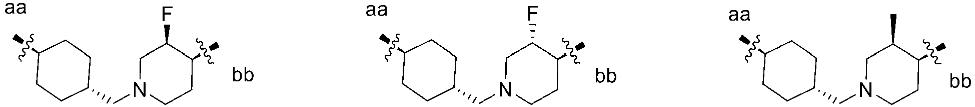 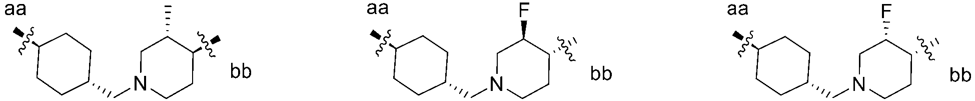 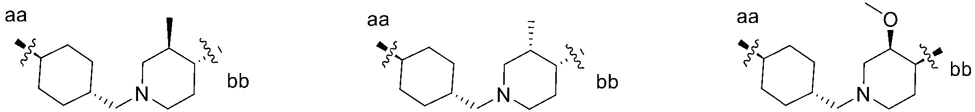 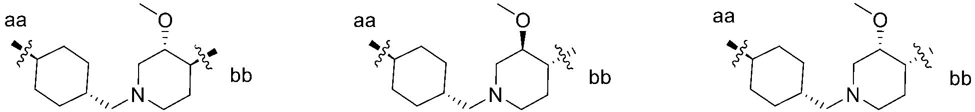 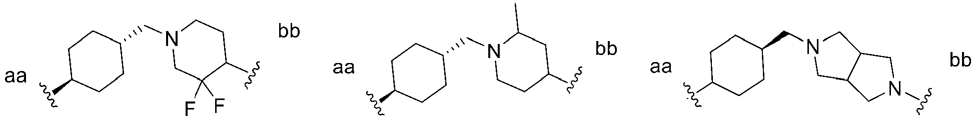 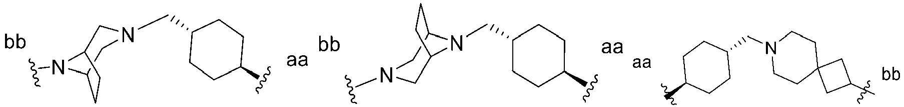 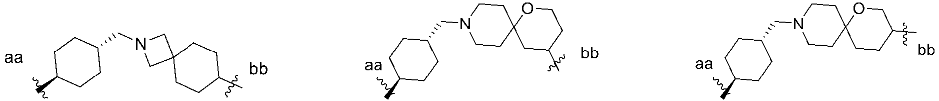 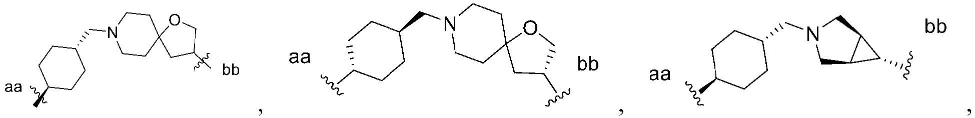 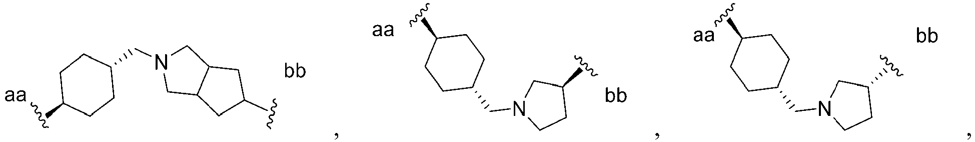 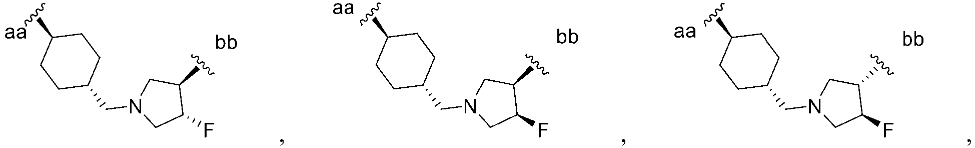 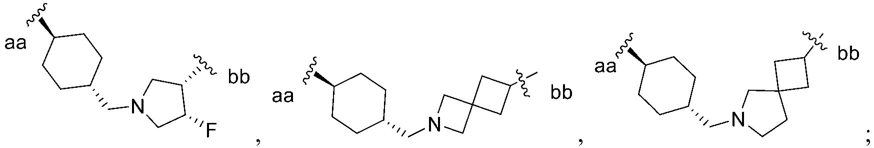 where the aa-end is attached to the nitrogen atom of the heteroaromatic ring at the R¹-end, and the bb-end is attached to ring D.

In some specific embodiments of the present invention, the compound represented by formula I specifically is:

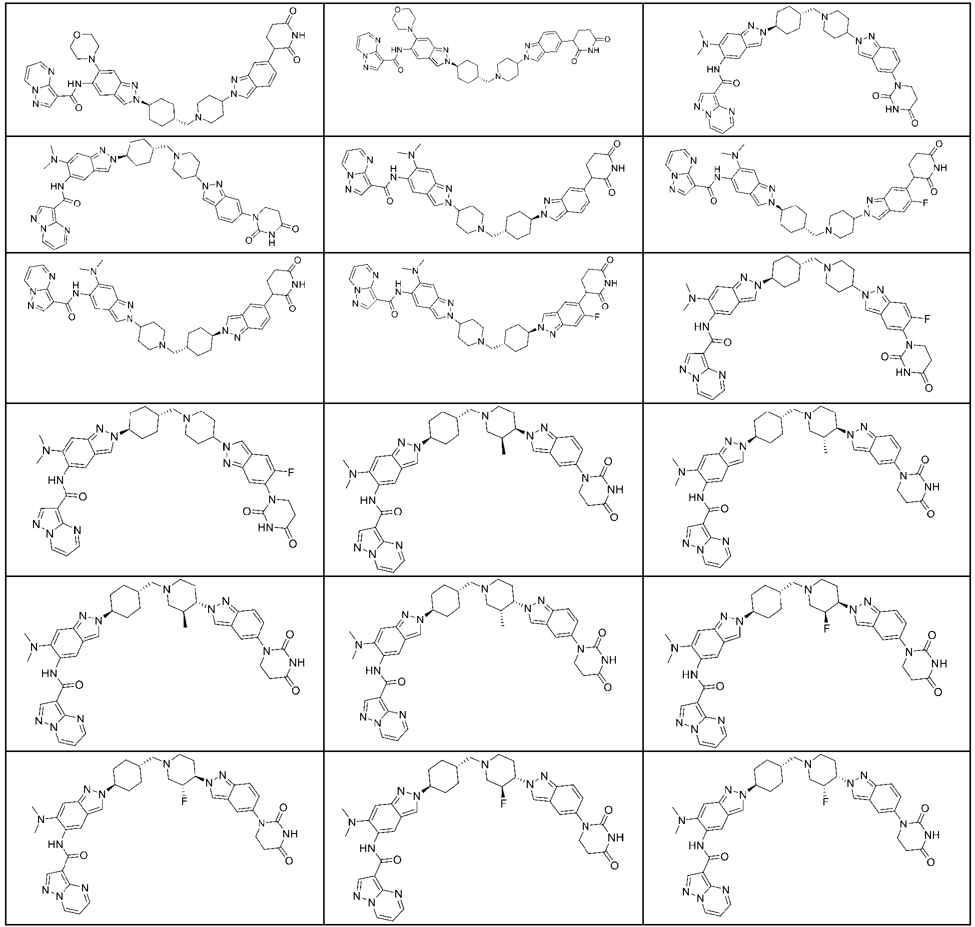

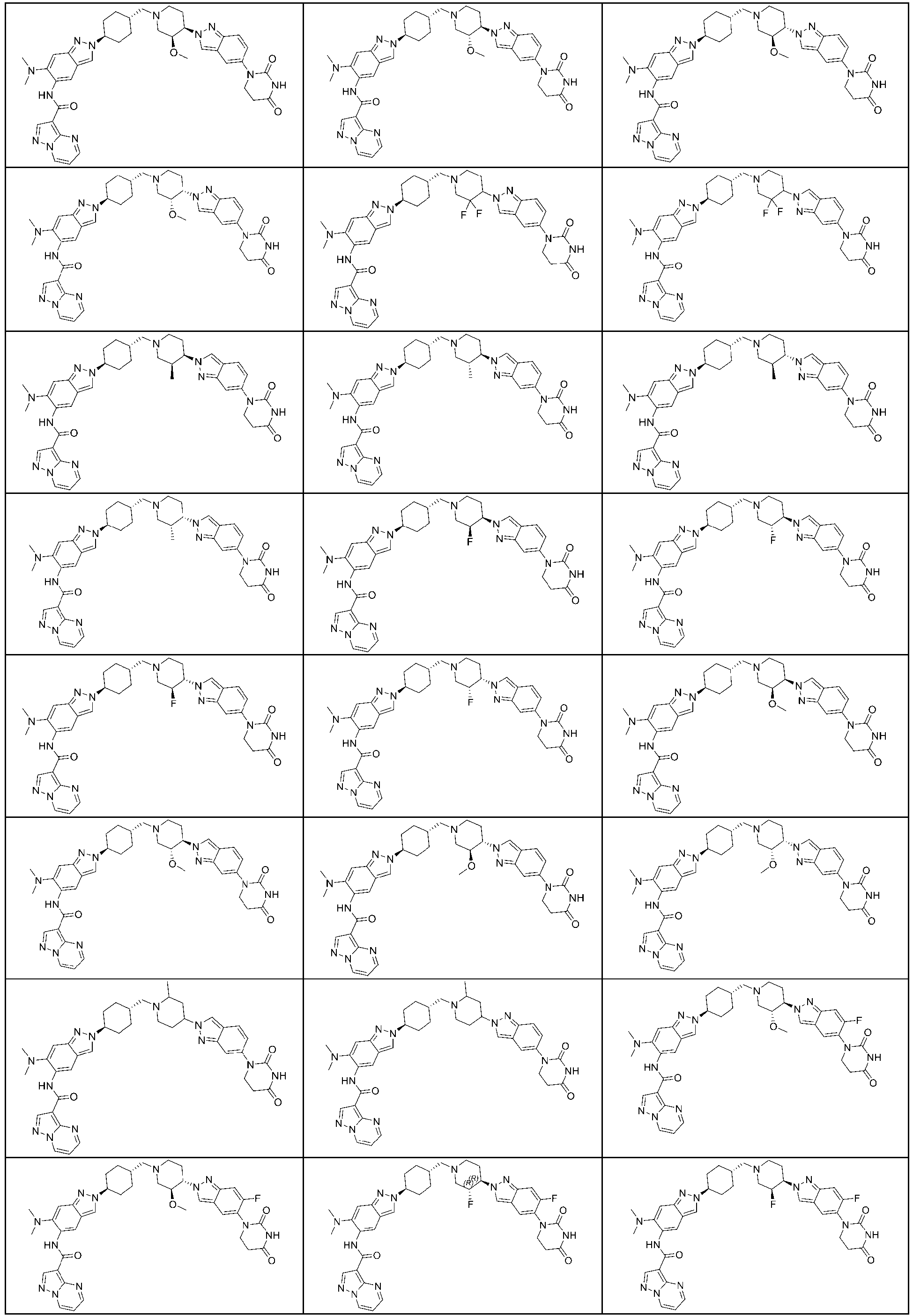

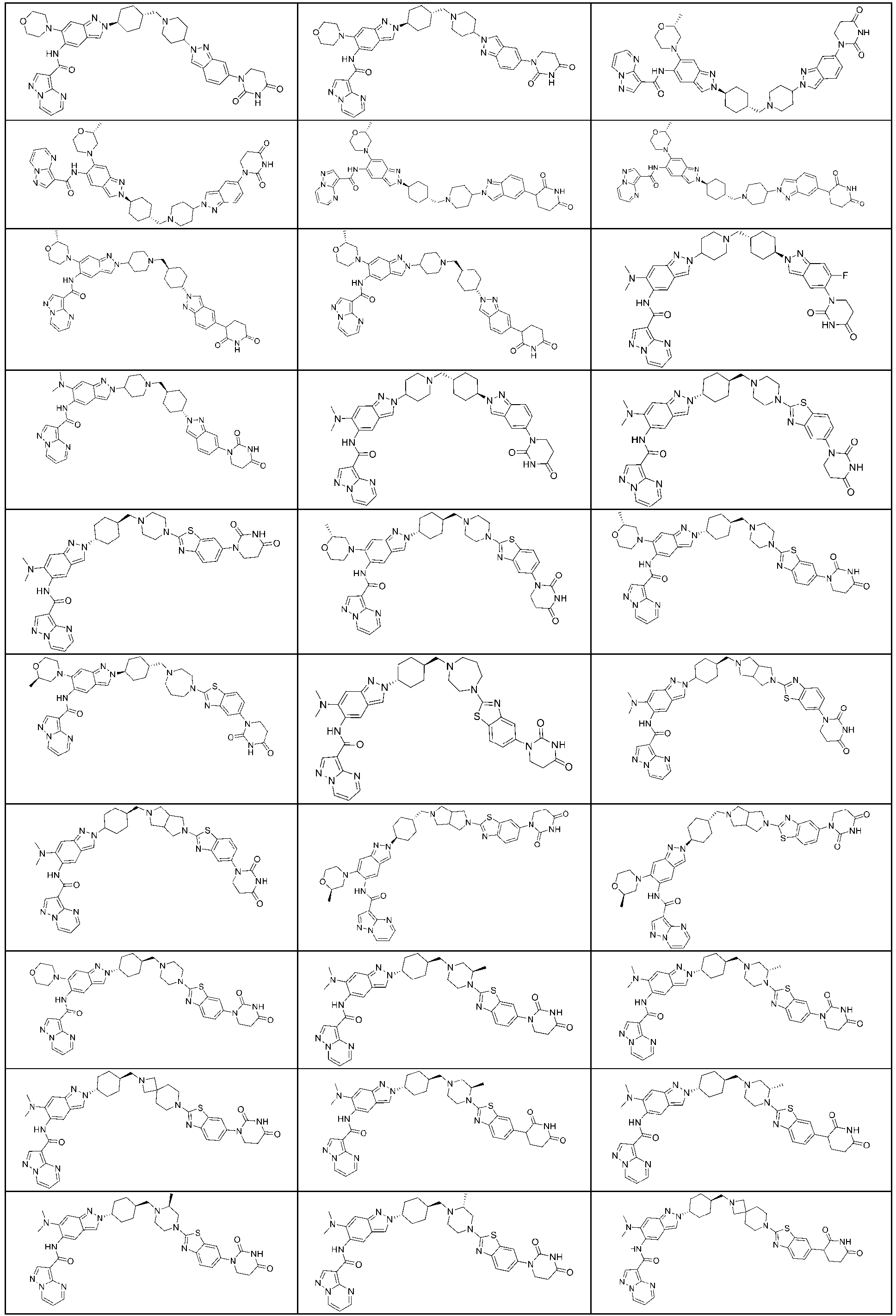

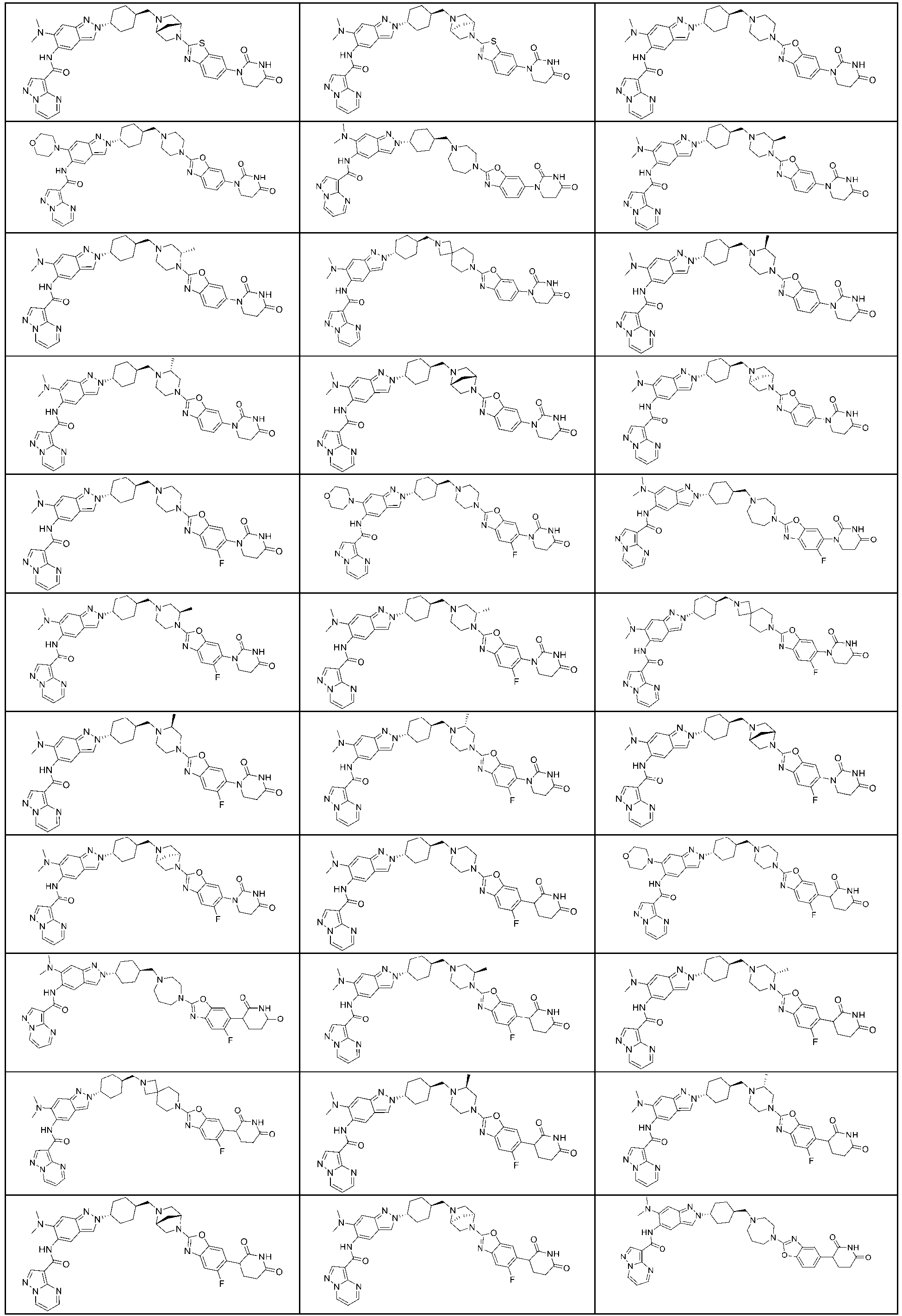

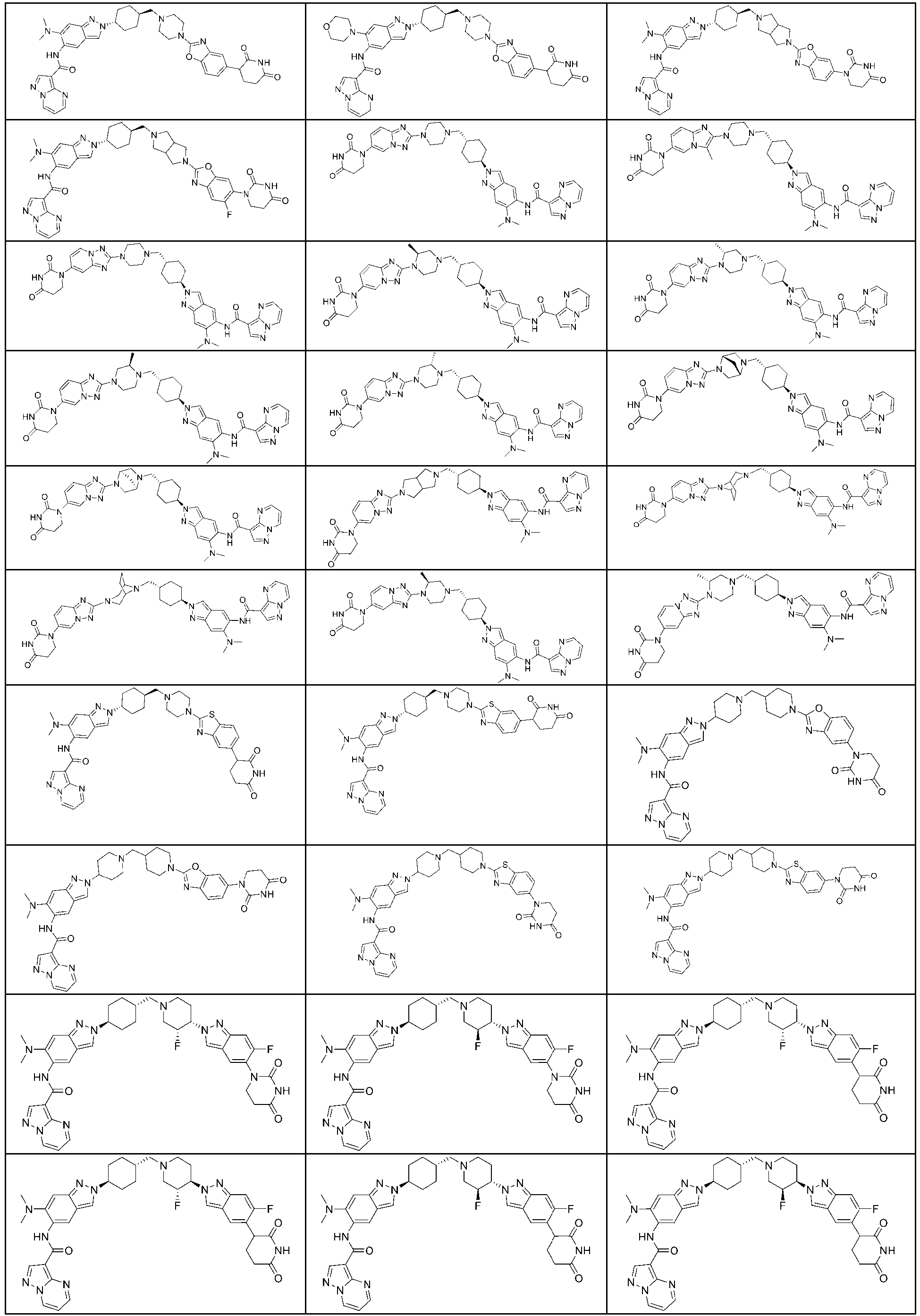

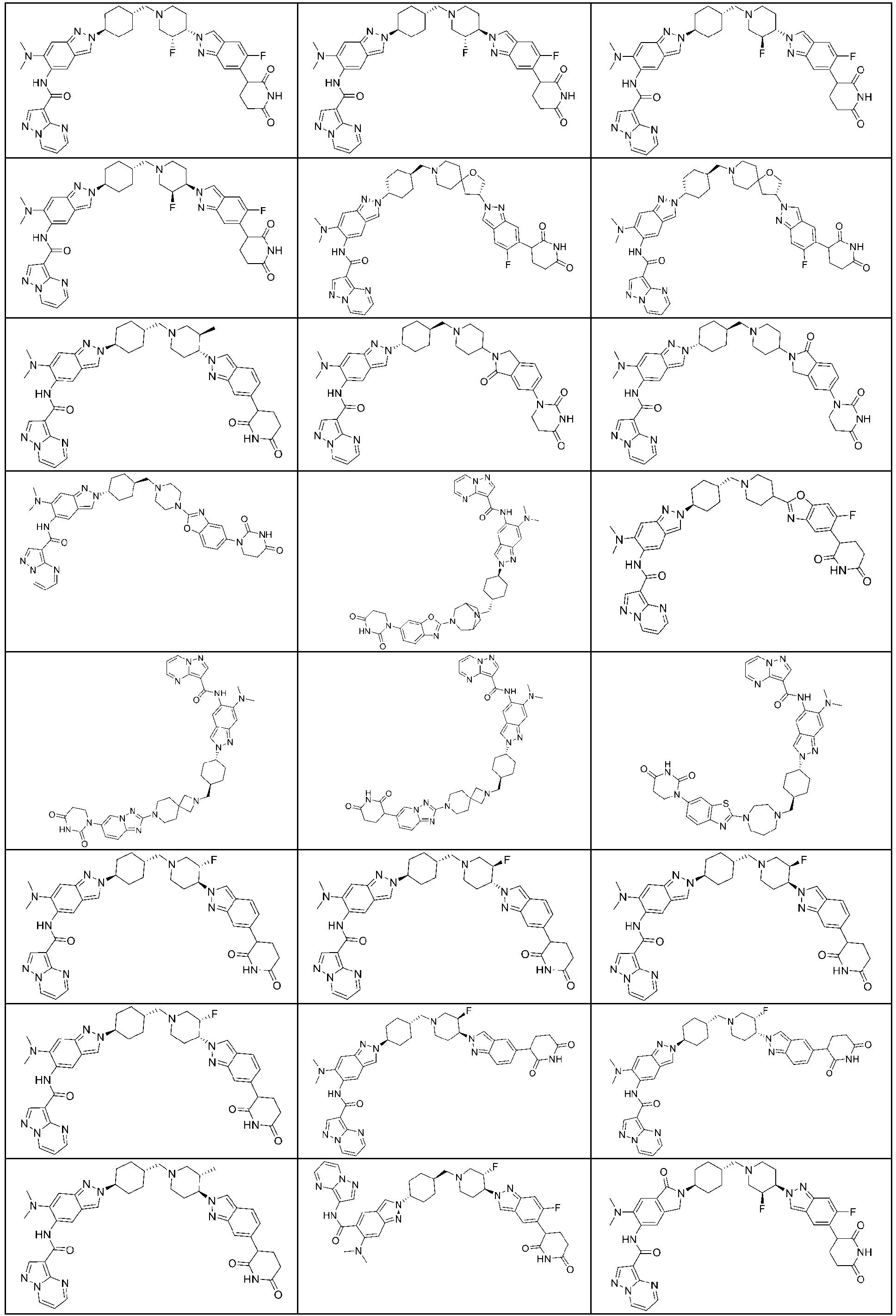

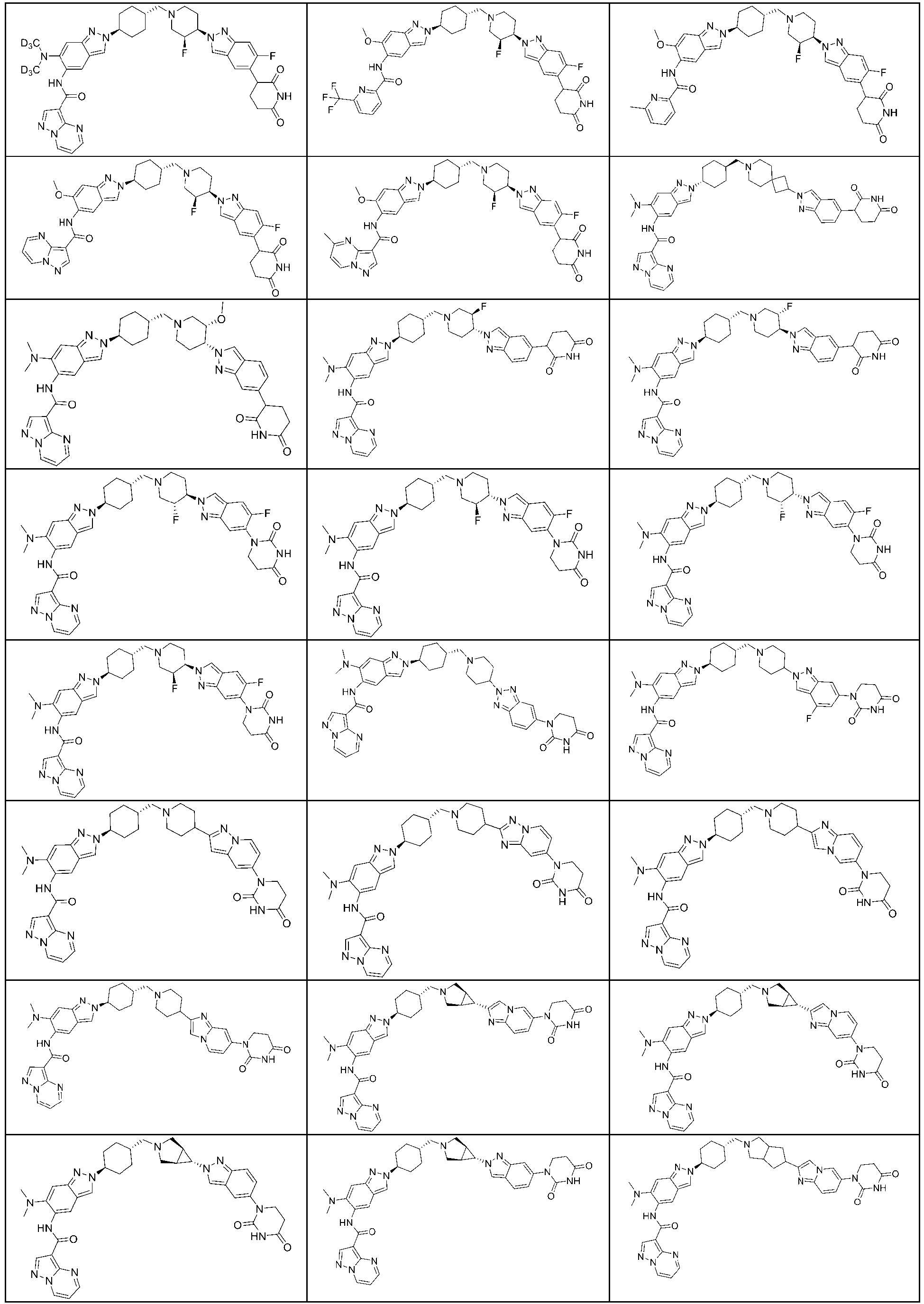

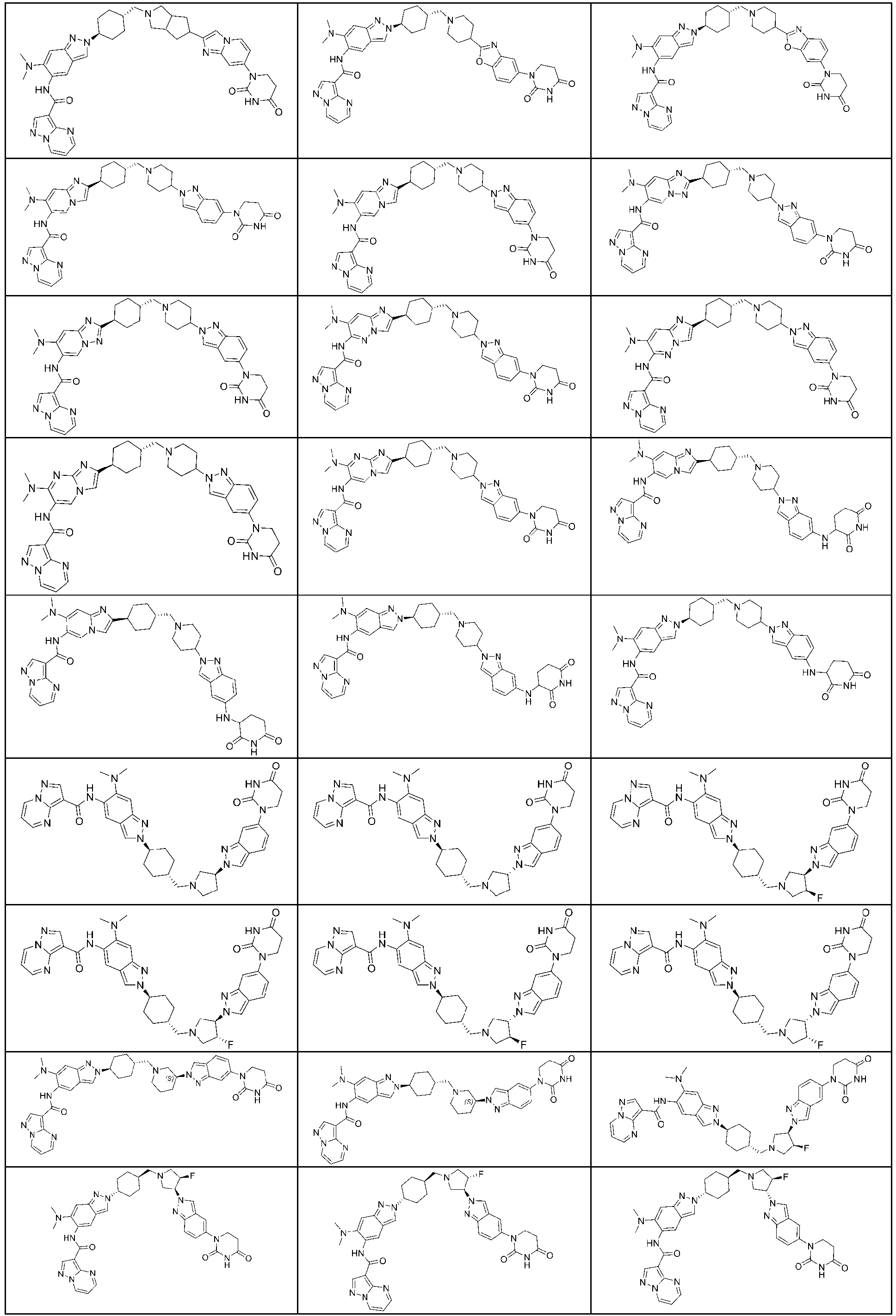

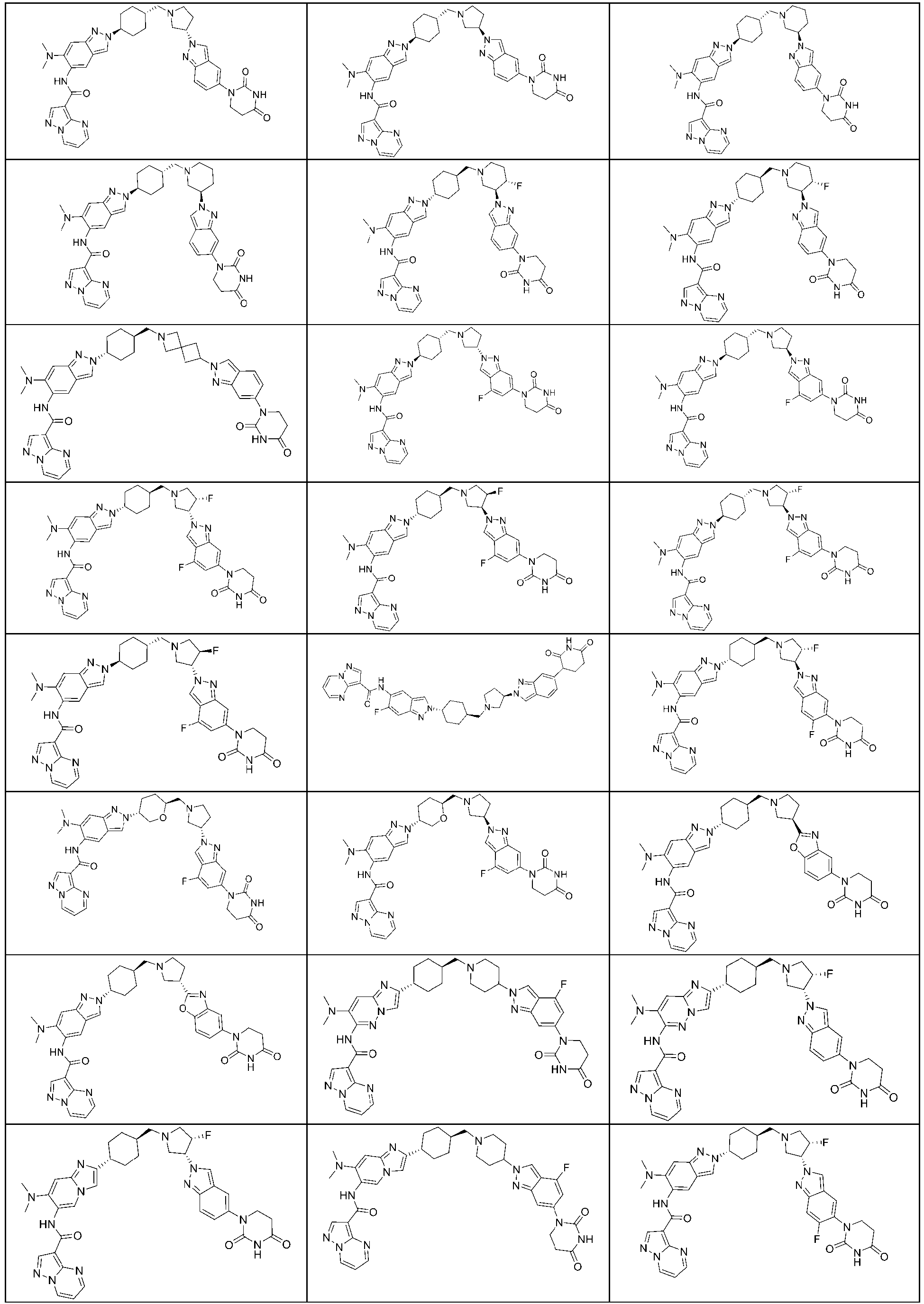

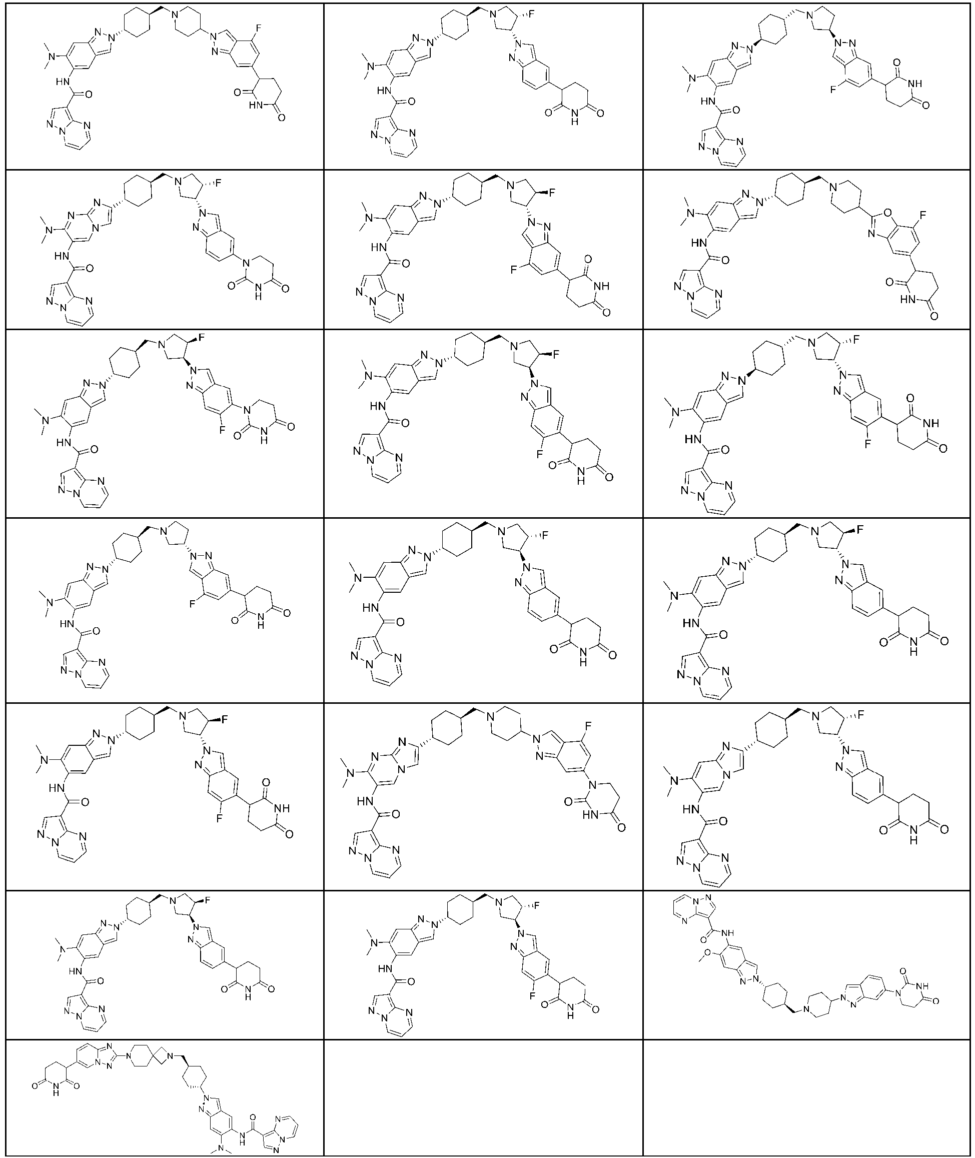

The present invention further provides use of any compound described above, the stereoisomer thereof, the deuterated compound thereof, or the pharmaceutically acceptable salt thereof, in the preparation of a drug for the treatment and prevention of diseases associated with or mediated by one or more of the interleukin-1 receptor-associated kinase 4 (IRAK4) signaling pathway, interleukin-6 (IL-6) receptor, and tumor necrosis factor-α (TNFα).

Furthermore, the disease includes a cancer, a neurodegenerative disease, a viral disease, an autoimmune disease, an inflammatory disease, a genetic disease, a hormone-related disease, a metabolic disorder, an organ transplantation-related disease, an immunodeficiency disease, an osteoclastic disease, a proliferative disease, an infectious disease, thrombin-induced platelet aggregation, a liver disease, a lesion caused by T cell activation, and a cardiovascular disease.

The present invention further provides a pharmaceutical composition, which is a preparation prepared from any compound described above, the stereoisomer thereof, the deuterated compound thereof, or the pharmaceutically acceptable salt thereof, together with a pharmaceutically acceptable excipient.

The present invention further provides a therapeutic method, which comprises administering the aforementioned pharmaceutical composition to a patient.

The modes of administration include oral administration, topical application and/or injection.

The compounds and derivatives provided in the present invention can be named according to the nomenclature system of the IUPAC (International Union of Pure and Applied Chemistry) or the CAS (Chemical Abstracts Service, CoLumbus, OH).

Definitions of terms used in the present invention: Unless otherwise stated, an initial definition provided for a group or term herein is applicable to the group or term throughout the specification; and for terms that are not specifically defined herein, meanings that can be given to them by a person skilled in the art should be provided based on the disclosure and context.

The "substitution" refers to the replacement of a hydrogen atom in a molecule with another different atom or molecule.

The "optionally substituted" means that the "substitution" may, but does not have to, occur, and the description includes occurrence or non-occurrence.

Minimum and maximum contents of carbon atoms in a hydrocarbon group are indicated by prefixes, for example, the prefix C_{a-b} alkyl indicates any alkyl containing "a" to "b" carbon atoms. Therefore, for example, "C₁₋₄ alkyl" refers to alkyl containing 1-4 carbon atoms.

The "alkyl" mentioned in the present invention refers to a saturated hydrocarbon chain having a specified number of member atoms. For example, C₁₋₆ alkyl refers to an alkyl group having 1 to 6 member atoms, such as 1 to 4 member atoms. The alkyl group may be linear or branched. A representative branched alkyl group has one, two, or three branched chains. The alkyl group may be optionally substituted with one or more substituents as defined herein. The alkyl includes methyl, ethyl, propyl (n-propyl and isopropyl), butyl (n-butyl, isobutyl, and tert-butyl), pentyl (n-pentyl, isopentyl, and neopentyl), and hexyl. The alkyl group may also be a moiety of an additional group, wherein the additional group is, for example, C₁₋₆ alkoxy.

The "alkylene" mentioned in the present invention refers to a divalent saturated aliphatic hydrocarbon group having a specified number of carbon atoms. "Cₐ-b alkylene" refers to an alkylene group having a to b carbon atoms. The alkylene group includes a branched and linear hydrocarbon group. For example, "C₁₋₆ alkylene" is intended to include, e.g., methylene, ethylene, propylidene, 2-methylpropylidene, dimethylethylidene, and pentylidene. Therefore, the term "propylidene" may be a structure as illustrated below: 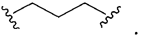 Similarly, the term "dimethylbutylidene" may be any one of structures as illustrated below: 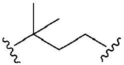 or 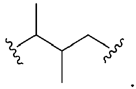 For another example, "C₀ alkylene" is intended to indicate that this position is a chemical bond, with the two moieties linked directly through the chemical bond.

The "alkenyl" mentioned in the present invention refers to a linear or branched hydrocarbon group having a specified number of carbon atoms and at least 1 unsaturated ethenyl site (>C=C<). For example, C_{a-b} alkenyl refers to an alkenyl group having a to b carbon atoms, and is intended to include, for example, ethenyl, propenyl, isopropenyl, 1,3-butadienyl, and the like.

The "alkynyl" mentioned in the present invention refers to a linear monovalent hydrocarbon group or a branched monovalent hydrocarbon group comprising at least one triple bond. The term "alkynyl" is further intended to include those hydrocarbon groups each having one triple bond and one double bond. For example, C₂₋₆ alkynyl is intended to include, e.g., ethynyl and propynyl.

The "chemical bond" mentioned in the present invention refers to a direct connection at that position via a single chemical bond;
The "halogen" mentioned in the present invention is fluorine, chlorine, bromine, or iodine.

The "haloalkyl" and "halogen-substituted alkyl" mentioned in the present invention refer to alkyl groups in which one or more hydrogen atoms are replaced by halogen atoms. For example, C₁₋₄ haloalkyl refers to alkyl having hydrogen atoms substituted with one or more halogen atoms and comprising 1-4 carbon atoms. Furthermore, for example, it includes trifluoromethyl, difluoromethyl, and the like.

The substituents such as "=O" and "=S" mentioned in the present invention mean that an oxygen atom or a sulfur atom replaces two hydrogen atoms to form a double bond, or replaces a lone pair of electrons to form a double bond.

The "-OR," "-NRR," or the like mentioned in the present invention means that th group R is linked to an oxygen atom or a nitrogen atom by a single bond.

In the "-C(O)R," "-S(O)₂R," or the like mentioned in the present invention, an oxygen atom is linked to a carbon atom, a sulfur atom, or a phosphorus atom by a double bond, and an R is linked to a carbon atom or a sulfur atom by a single bond. In the "-C(O)NRR," "-S(O)₂NRR," or the like mentioned in the present invention, an oxygen atom is linked to a carbon atom or a sulfur atom by a double bond, a nitrogen atom is linked to a carbon atom or a sulfur atom by a single bond, and an R is linked to a nitrogen atom by a single bond. In the "-NRC(O)R," "-NRS(O)₂R," or the like mentioned in the present invention, an R is linked to a nitrogen atom by a single bond, another R is linked to a carbon atom or a sulfur atom by a single bond, a nitrogen atom is linked to a carbon atom or a sulfur atom by a single bond, and an oxygen atom is linked to a carbon atom or a sulfur atom by a double bond.

The "carbocycle" and "carbocyclyl" mentioned in the present invention refer to saturated or partially saturated cyclic groups that contain multiple carbon atoms, have no ring heteroatoms, and consist of a single ring or multiple (fused) rings. Among others, carbon atoms include their oxidation states, such as C(O). For a polycyclic system having aromatic and nonaromatic rings without ring heteroatoms, the term "carbocycle" and "carbocyclyl" (e.g., 5,6,7,8-tetrahydronaphthalen-5-yl) are applicable when the point of attachment is at a nonaromatic carbon atom. The terms "carbocycle" and "carbocyclyl" include a cycloalkenyl group, such as cyclohexenyl. Examples of carbocyclyl include, for example, cyclopropyl, cyclobutyl, cyclohexyl, cyclopentyl, cyclooctyl, cyclopentenyl, and cyclohexenyl. Examples of carbocyclyl containing polybicycloalkyl ring systems include dicyclohexyl, dicyclopentyl, dicyclooctyl, and the like. Two such bicycloalkyl polycyclic structures are illustrated and named below: 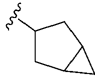 bicyclohexyl and 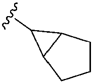 bicyclohexyl.

The "bridged ring" mentioned in the present invention means a saturated or partially saturated cyclic group which is formed by the bridging of multiple rings having multiple carbon atoms and no ring heteroatoms. The term "bridged ring" also includes the adamantane system; adamantyl includes, but is not limited to, the following structure:

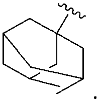

The "heterocycle" and "heterocyclyl" mentioned in the present invention refer to saturated rings or non-aromatic unsaturated rings that contain at least one heteroatom and consist of a single ring or multiple (fused) rings; wherein the heteroatoms refer to nitrogen atoms, oxygen atoms, sulfur atoms and the like. Among others, , carbon atoms and heteroatoms include their respective oxidation states, such as C(O), S(O), S(O)₂ and the like. The terms "heterocycle" and "heterocyclyl" shall also apply to polycyclic systems having aromatic and non-aromatic rings containing ring heteroatoms, for example 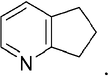 This typically represents a saturated or partially unsaturated monocyclic or bicyclic ring system with multiple ring atoms. Examples of monocyclic saturated heterocyclyl are oxetanyl, azetidinyl, pyrrolidinyl, 2-oxo-pyrrolidin-3-yl, tetrahydrofuranyl, tetrahydro-thienyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples of bicyclic saturated heterocyclyl are 8-aza-bicyclo[3.2.1]octyl, quinuclidinyl, 8-oxa-3-aza-bicyclo[3.2.1]octyl, 9-aza-bicyclo[3.3.1]nonyl, and examples of partially unsaturated heterocyclyl are dihydrofuranyl, imidazolinyl, tetrahydro-pyridyl, or dihydropyranyl.

The "bridged heterocycle" mentioned in the present invention means a saturated or partially saturated cyclic group which is formed by the bridging of multiple rings comprising at least one heteroatom.

The "aromatic ring" or the "aryl" mentioned in the present invention refers to an aromatic hydrocarbon group having a plurality of carbon atoms. The aryl generally includes a monocyclic, bicyclic, or tricyclic aryl. Further, the term "aryl" as used herein refers to an aromatic substituent that may be a monoaromatic ring or fused polyaromatic rings. Non-limiting examples include phenyl, naphthyl, or tetrahydronaphthyl.

The "heteroaromatic ring" or the "heteroaryl" mentioned in the present invention refers to an unsaturated aromatic ring comprising at least one heteroatom; wherein the heteroatom refers to, e.g., a nitrogen atom, an oxygen atom, or a sulfur atom. It generally includes an aromatic monocyclic or bicyclic hydrocarbon that comprises a plurality of ring atoms, with one or more ring atoms thereof selected from O, N, and S. Preferably, it has one to three heteroatoms. Representative heteroaryl includes, for example: pyridyl, indolyl, quinoxalinyl, quinolinyl, isoquinolinyl, benzothienyl, benzofuranyl, benzothienyl, benzopyranyl, benzothiapyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, and benzoxazolyl.

The "stereoisomers" include enantiomers and diastereomers, as well as their racemic or partially racemic mixtures;

The term "pharmaceutically acceptable" means that a carrier, a vehicle, a diluent, an adjuvant, and/or a formed salt is generally chemically or physically compatible with other components constituting a pharmaceutical dosage form, and is physiologically compatible with a receptor.

The terms "salt" and "pharmaceutically acceptable salt" refer to an acidic and/or basic salt of the above compound or a stereoisomer thereof formed with an inorganic and/or organic acid and base, further include a zwitterionic salt (inner salt), and still further include a quaternary ammonium salt, such as an alkylammonium salt. These salts may be obtained directly from final isolation and purification of the compound, They may also be obtained by mixing the above compound or the stereoisomer thereof with a certain amount of an acid or base appropriately (for example, equivalent). These salts may be obtained by precipitation in a solution and collection by filtration, or may be obtained by recycling after solvent evaporation, or may be obtained by reaction in an aqueous medium and then lyophilization. The salt mentioned in the present invention may be hydrochloride, sulfate, citrate, besylate, hydrobromide, hydrofluoride, phosphate, acetate, propionate, succinate, oxalate, malate, succinate, fumarate, maleate, tartrate, or trifluoroacetate of the compound.

In the present invention, "multiple" refers to two or more. The "and/or" describes the relationship between related objects, indicating that there can be three relationships. For example, A and/or B can represent three situations: A exists alone, A and B exist simultaneously, and B exists alone. The character "/" generally indicates that the objects before and after it are in an "or" relationship.

In some embodiments, one or more compounds of the present invention may be used in combination with each other. The compounds of the present invention are optionally used in combination with any other active agent for the manufacture of a drug or a pharmaceutical composition for regulating a cell function or treating a disease. If a group of compounds are used, these compounds may be administered to a subject concurrently, separately, or sequentially.

Obviously, based on the above contents of the present invention, according to the common technical knowledge and customary means in the art, without departing from the above basic technical idea of the present invention, various other forms of modifications, replacements, or alterations may be further made.

### Description of Drawings

Fig. 1 shows the therapeutic effect of the compound TM-174 of the present invention on the IMQ psoriasis model.
Fig. 2 shows the therapeutic effect of the compound TM-205 of the present invention on the IMQ psoriasis model.
Fig. 3 is a statistical chart showing the therapeutic effect of the compound TM-4 of the present invention on the DSS-induced enteritis model.
Fig. 4 is a statistical chart showing the therapeutic effect of the compound TM-174 of the present invention on the DSS-induced enteritis model.
Fig. 5 is a statistical chart showing the therapeutic effect of the compound TM-205 of the present invention on the DSS-induced enteritis model.
Fig. 6 is a statistical chart showing the therapeutic effect of the compound TM-221 of the present invention on the DSS-induced enteritis model.
Fig. 7 is a statistical chart showing the therapeutic effect of the compound TM-135 of the present invention on the CIA-induced rheumatoid arthritis model.
Fig. 8 is a statistical chart showing the therapeutic results of the compounds of the present invention on the MC903-induced atopic inflammation model on the 14th day.

### Detailed Description

The present invention is further illustrated by the following examples, which are not intended to limit the invention to the scope of the described examples. Experimental methods in the following examples without clear indication of specific conditions are carried out based on conventional methods and conditions, or are selected based on to product specifications.

Raw materials and devices employed in the Detailed Description of the present invention are all known products, and are obtained by purchasing commercially available products.

Unless otherwise specified in the examples, the reaction temperature is room temperature, which refers to 20-25°C. All temperatures are expressed in °C (Celsius).

The overnight is 14±1 h.

High performance liquid chromatography (HPLC) conditions: Waters high performance liquid chromatograph (e2695/e2487). Analytical high performance liquid chromatography conditions: C18 column (3.5 µm, 4.6×75 mm), UV detection wavebands: 220 and 254 nm, elution conditions: gradient elution with 5-95% acetonitrile (containing 0.1% V/V TFA) for 10 min.

GiLson GX-281 reversed-phase preparative chromatograph or the Biotage IsoLera One flash purification system is used for reversed-phase purification.

Bruker Avance III 400 or 600 NMR spectrometer is used for NMR measurements with NMR shifts (δ) given in a unit of 10⁻⁶ (ppm). The solvents are, e.g., deuterated dimethyl sulfoxide (DMSO-d₆), deuterated chloroform (CDCl₃), and deuterated methanol (CD₃OD), and the internal standard is tetramethylsilane (TMS).

Known starting materials, reagents, and solvents of the present invention may be synthesized using or according to known methods in the art, or may be purchased from, e.g., Chengdu Jinshan Chemical Reagent Co., Ltd., Shanghai Bide Technology Co., Ltd., and Shanghai Titan Technology Co., Ltd.

Known starting materials, reagents, and solvents of the present invention may be synthesized using or according to known methods in the art, or may be purchased from, e.g., Chengdu Jinshan Chemical Reagent Co., Ltd., Shanghai Bide Technology Co., Ltd., and Shanghai Titan Technology Co., Ltd.

### Examples

### Synthesis of intermediate IM-1

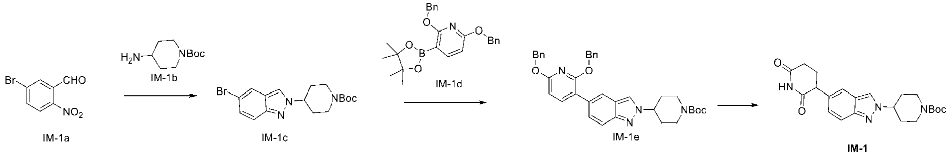

### Step 1: Synthesis of IM-1c

IM-1a (876 mg) was dissolved in 16 mL of isopropanol, followed by the addition of IM-1b (919.60 mg). The mixture was heated under reflux at 80°C for 4 h and monitored by thin-layer chromatography. After a new spot was detected, the reaction mixture was cooled to room temperature. Finally, tributylphosphine (1.62 g) was added, and the resulting mixture was heated under reflux at 80°C overnight. LC-MS detection showed that the raw material reaction was completed and the target product was produced. The reaction mixture was concentrated under reduced pressure, then diluted with water and extracted with EA. The organic phase was collected, washed with saturated brine, dried over sodium sulfate, and concentrated under reduced pressure to give the crude product. The crude product was purified by column chromatography (PE/EA = 20:1-10:1) to give 1.23 g of the product. LCMS (ESI) m/z: [M+1]=380.09.

### Step 2: Synthesis of IM-1e

IM-1c (2 g) and M-1d (2.19 g) were dissolved in 20 mL of dioxane/H₂O (4/1). Pd(PPh₃)₄ (300 mg) and potassium phosphate (2.23 g) were subsequently added successively. The mixture was purged with nitrogen, and heated under reflux with stirring at 90°C overnight. LC-MS detection showed that the raw material reaction was completed and the target product was produced. The reaction mixture was extracted with EA. The organic phase was collected, washed with saturated brine, dried over sodium sulfate, and concentrated under reduced pressure to give the crude product. The crude product was purified by column chromatography (PE/EA = 5:1-1:1) to give 2.53 g of the product. LCMS (ESI) m/z: [M+1]=591.4.

### Step 3: Synthesis of IM-1

IM-1e (4.1 g) was dissolved in a mixed solvent of MeOH (164 mL ) and DCM (82 mL), and Pd(OH)₂/C (1.23 g ) was added. The reaction system was purged with hydrogen, followed by hydrogenation at room temperature under a hydrogen balloon for 4 h. The reaction was completed as detected by LCMS. The mixture was filtered through celite, and the filter cake was washed with dichloromethane/methanol = 1/1 (100 ml * 2). The filtrates were combined and spin dried. The crude product (2.8 g) was purified by high-pressure reversed-phase preparative chromatography to afford IM-1 (1.5 g). LCMS (ESI) m/z: [M+1]=413.3.

Synthesis of intermediates IM-2 to IM-8: preparation methods for the intermediates IM-2 to IM-8 are similar to the preparation of the intermediate IM-1, as shown in Table 1.

**Table 1 Compound No. and structure**

| Compound No. | Structure | Compound No. | Structure |
|---|---|---|---|
| IM-2 | 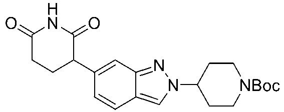 | IM-6 | 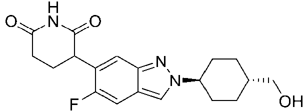 |
| IM-3 | 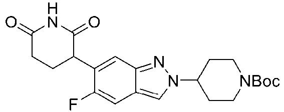 | IM-7 | 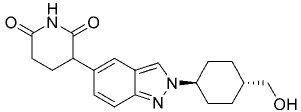 |
| IM-4 | 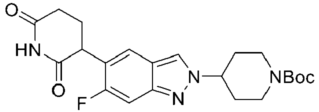 | IM-8 | 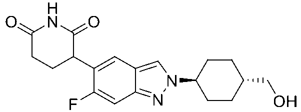 |
| IM-5 | 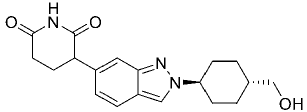 | | |

### Synthesis of intermediate IM-9

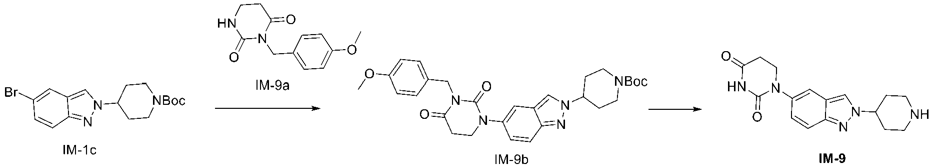

Synthesis of intermediates IM-9 to IM-39: preparation methods for the intermediates IM-9 to IM-39 are similar to the preparation of the intermediate IM-9, as shown in Table 2.

**Table 2 Compound No. and structure**

| Compound No. | Structure | Compound No. | Structure |
|---|---|---|---|
| IM-9 | 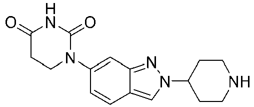 | IM-25 | 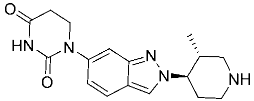 |
| IM-10 | 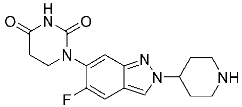 | IM-26 | 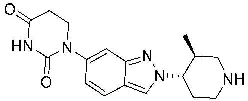 |
| IM-11 | 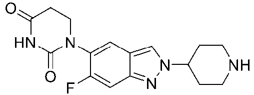 | IM-27 | 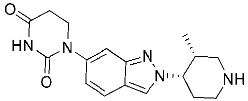 |
| IM-12 | 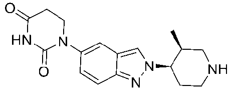 | IM-28 | 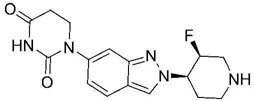 |
| IM-13 | 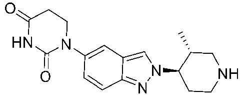 | IM-29 | 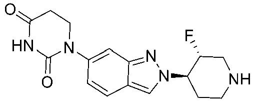 |
| IM-14 | 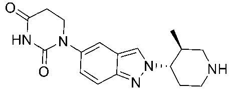 | IM-30 | 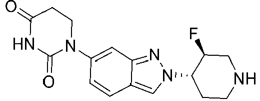 |
| IM-15 | 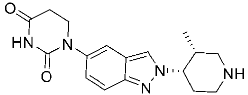 | IM-31 | 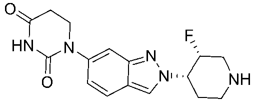 |
| IM-16 | 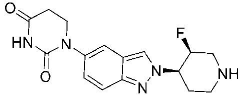 | IM-32 | 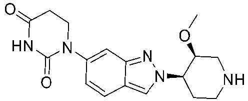 |
| IM-17 | 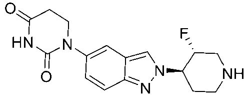 | IM-33 | 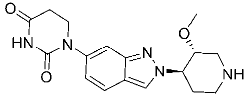 |
| IM-18 | 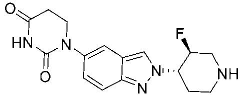 | IM-34 | 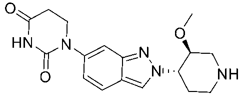 |
| IM-19 | 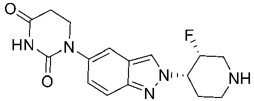 | IM-35 | 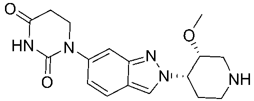 |
| IM-20 | 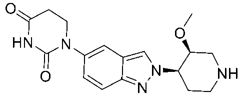 | IM-36 | 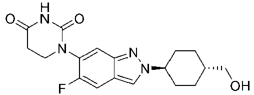 |
| IM-21 | 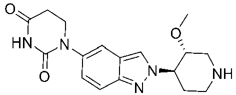 | IM-37 | 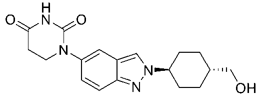 |
| IM-22 | 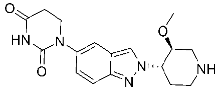 | IM-38 | 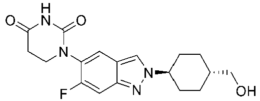 |
| IM-23 | 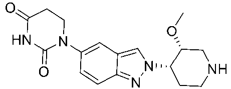 | IM-39 | 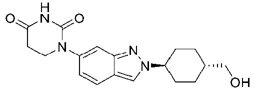 |
| IM-24 | 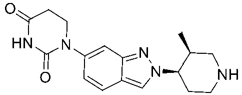 | | |

### Synthesis of intermediate IM-40

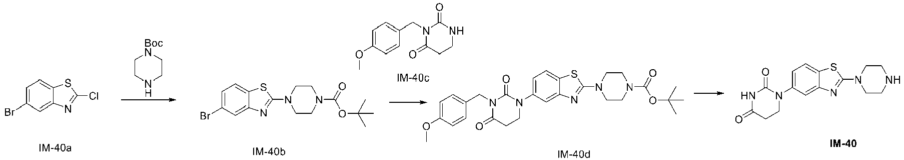

### Step 1: Synthesis of IM-40b

Reactants IM-40a (1 g), Boc piperazine (0.79 g), and Na₂CO₃ (0.51 g) and solvent DMF (10 mL) were added to a 25 mL flask and stirred overnight at 100°C. The mixture was diluted with 4-5 volumes of H₂O, extracted three times with EA, and the organic phases were collected. The organic phase was washed twice with H₂O and then twice with saturated brine, dried over anhydrous sodium sulfate, filtered and concentrated. The crude product was purified by column chromatography (PE/EA=3:1) to give the target product (1.44 g). LCMS (ESI) m/z: [M+1]=398.10.

### Step 2: Synthesis of IM-40c

IM-40b (300 mg) and IM-40c (194 mg) were dissolved in dioxane (2 mL). Cs₂CO₃ (613.49 mg) and Xantphos (43.58 mg) were added, followed by three times of N₂ purging. Under the protection of N₂, Pd(OAc)₂ (8.45 mg) was added, and three additional times of N2 purging were performed. The resulting reaction system was stirred overnight at 100°C. The reaction mixture was filtered through celite, and concentrated. The crude product was purified by column chromatography (PE/EA=1:1) to give the target product (145 mg). LCMS (ESI) m/z: [M+1]=552.49.

### Step 3: Synthesis of IM-40

IM-40d (140 mg, 0.254 mmol) was dissolved in TFA (1 mL), methanesulfonic acid (0.5 mL, 0.009 mmol) was added, and the reaction mixture was stirred at 70°C for 4 h. The reaction mixture was concentrated directly, and its pH was adjusted to neutral with saturated NaOH solution, followed by direct lyophilization. After lyophilization, the resultant solid was redissolved in DCM/MeOH=10:1, then filtered and concentrated. It was directly subjected to the next reaction step without purification. LCMS (ESI) m/z: [M+1]=332.29.

Synthesis of intermediates IM-41 to IM-110: preparation methods for the intermediates IM-41 to IM-110 are similar to the preparation for the intermediate IM-40, as shown in Table 3.

**Table 3 Compound No. and structure**

| Compound No. | Structure | Compound No. | Structure |
|---|---|---|---|
| IM-41 | 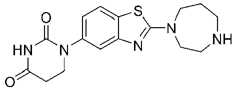 | IM-76 | 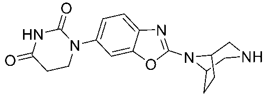 |
| IM-42 | 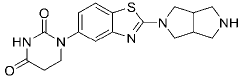 | IM-77 | 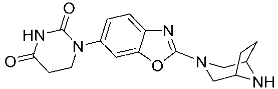 |
| IM-43 | 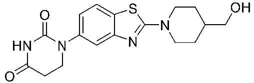 | IM-78 | 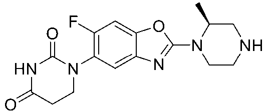 |
| IM-44 | 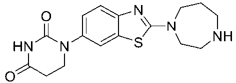 | IM-79 | 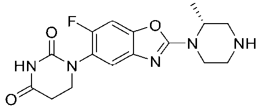 |
| IM-45 | 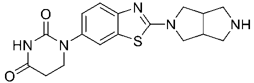 | IM-80 | 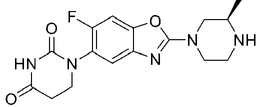 |
| IM-46 | 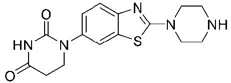 | IM-81 | 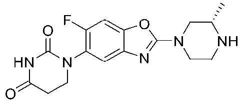 |
| IM-47 | 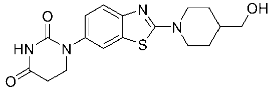 | IM-82 | 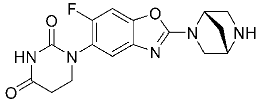 |
| IM-48 | 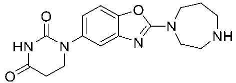 | IM-83 | 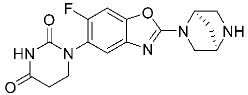 |
| IM-49 | 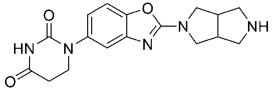 | IM-84 | 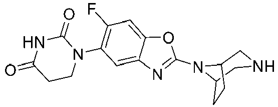 |
| IM-50 | 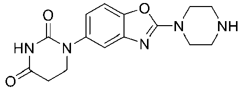 | IM-85 | 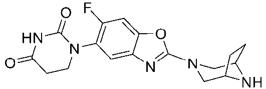 |
| IM-51 | 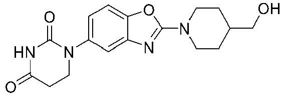 | IM-86 | 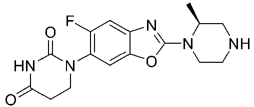 |
| IM-52 | 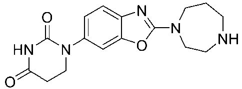 | IM-87 | 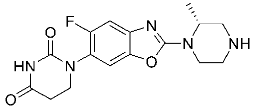 |
| IM-53 | 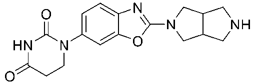 | IM-88 | 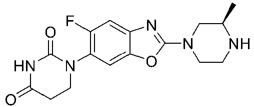 |
| IM-54 | 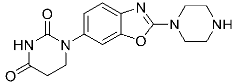 | IM-89 | 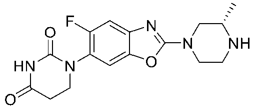 |
| IM-55 | 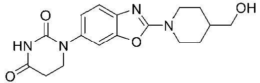 | IM-90 | 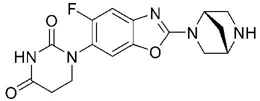 |
| IM-56 | 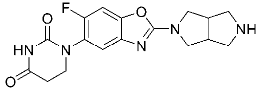 | IM-91 | 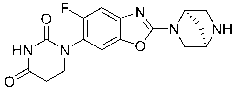 |
| IM-57 | 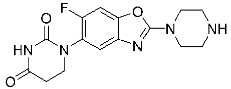 | IM-92 | 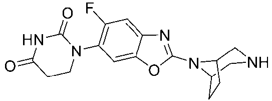 |
| IM-58 | 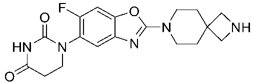 | IM-93 | 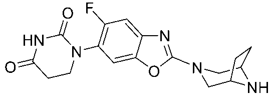 |
| IM-59 | 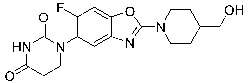 | IM-94 | 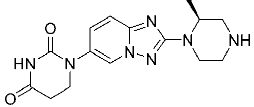 |
| IM-60 | 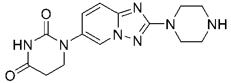 | IM-95 | 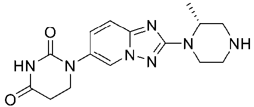 |
| IM-61 | 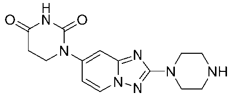 | IM-96 | 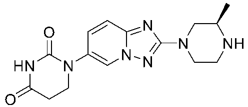 |
| IM-62 | 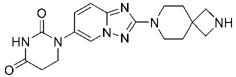 | IM-97 | 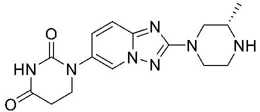 |
| IM-63 | 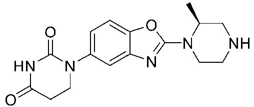 | IM-98 | 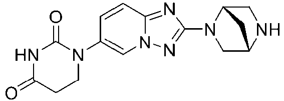 |
| IM-64 | 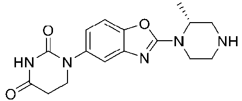 | IM-99 | 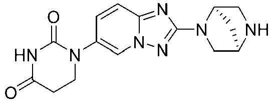 |
| IM-65 | 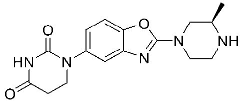 | IM-100 | 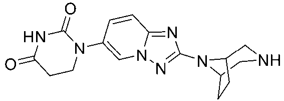 |
| IM-66 | 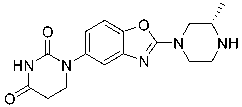 | IM-101 | 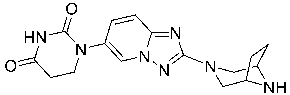 |
| IM-67 | 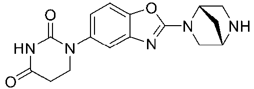 | IM-102 | 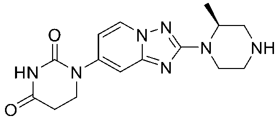 |
| IM-68 | 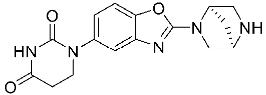 | IM-103 | 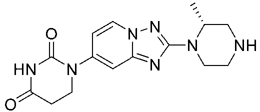 |
| IM-69 | 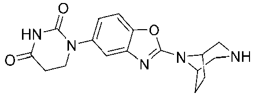 | IM-104 | 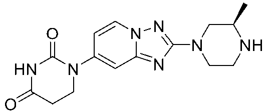 |
| IM-70 | 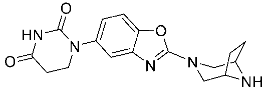 | IM-105 | 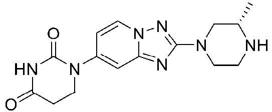 |
| IM-71 | 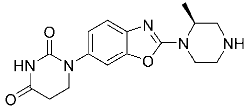 | IM-106 | 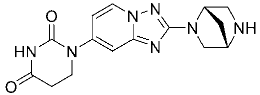 |
| IM-72 | 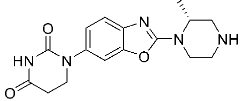 | IM-107 | 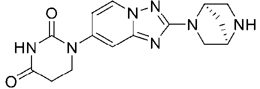 |
| IM-73 | 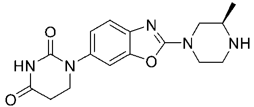 | IM-108 | 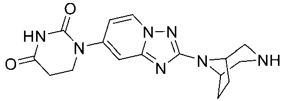 |
| IM-74 | 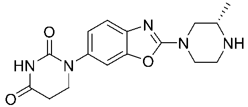 | IM-109 | 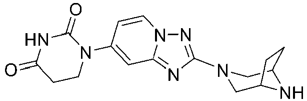 |
| IM-75 | 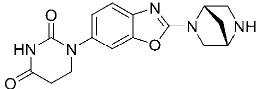 | IM-110 | 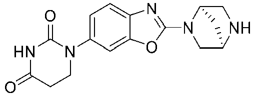 |

### Synthesis of intermediate IM-111

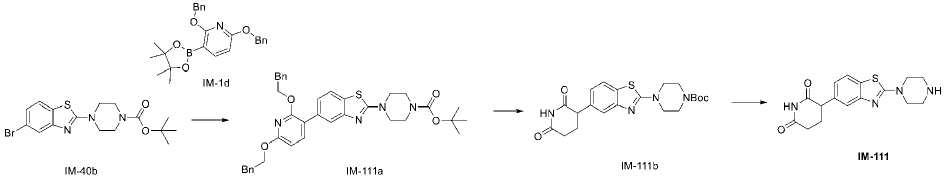

### Step 1: Synthesis of IM-111a

IM-40b (1 g) and M-1d (1.15 g) were dissolved in 20 mL of dioxane/H₂O (4/1). Pd(PPh₃)₄ (145 mg) and potassium phosphate (1.07 g) were subsequently added successively. The mixture was purged with nitrogen, and heated under reflux with stirring at 90°C overnight. LC-MS detection showed that the raw material reaction was completed and the target product was produced. The reaction mixture was extracted with EA. The organic phase was collected, washed with saturated brine, dried over sodium sulfate, and concentrated under reduced pressure to give the crude product. The crude product was purified by column chromatography to give 1.2 g of the product. LCMS (ESI) m/z: [M+1]=637.3.

### Step 2: Synthesis of IM-111b

IM-111a (500 mg) was dissolved in 20 mL of methanol, followed by the addition of 100 mg of Pd/C. The reaction system was purged 3 times with hydrogen, and the reaction was carried out overnight at room temperature. The mixture was subjected to suction filtration, and the filtrate was concentrated to afford 190 mg of the crude product, which was directly used in the subsequent reaction step without purification. LCMS (ESI) m/z: [M+1]=431.1.

### Step 3: Synthesis of IM-111

IM-111b (120 mg) was dissolved in 2 mL of dichloromethane, cooled in an ice bath, and 0.5 mL of trifluoroacetic acid was added. The mixture was allowed to react at room temperature for 1 h and concentrated to obtain the crude product, which was used directly in the next step without purification. LCMS (ESI) m/z: [M+1]=331.1.

Synthesis of intermediates IM-112 to IM-180: preparation methods for the intermediates IM-112 to IM-180 are similar to the preparation of the intermediate IM-111, as shown in Table 4.

**Table 4 Compound No. and structure**

| Compound No. | Structure | Compound No. | Structure |
|---|---|---|---|
| IM-112 | 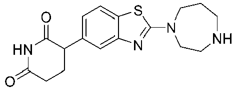 | IM-147 | 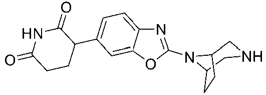 |
| IM-113 | 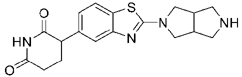 | IM-148 | 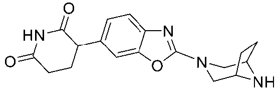 |
| IM-114 | 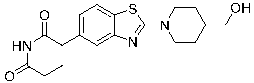 | IM-149 | 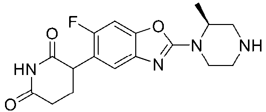 |
| IM-115 | 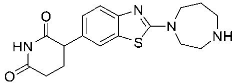 | IM-150 | 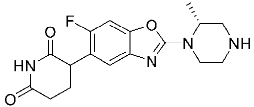 |
| IM-116 | 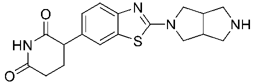 | IM-151 | 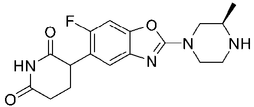 |
| IM-117 | 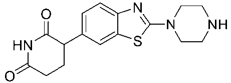 | IM-152 | 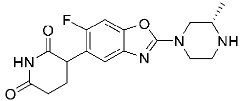 |
| IM-118 | 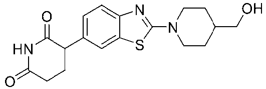 | IM-153 | 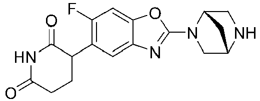 |
| IM-119 | 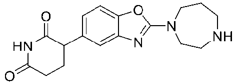 | IM-154 | 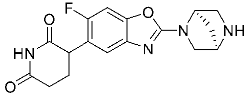 |
| IM-120 | 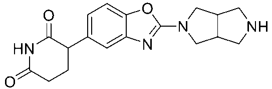 | IM-155 | 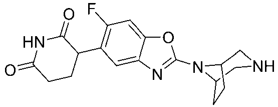 |
| IM-121 | 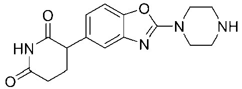 | IM-155 | 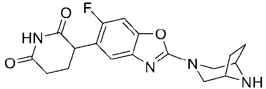 |
| IM-122 | 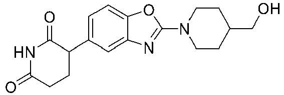 | IM-156 | 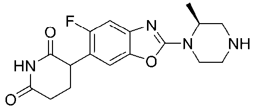 |
| IM-123 | 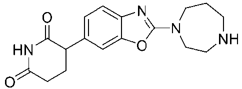 | IM-157 | 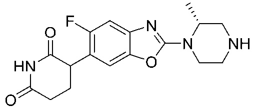 |
| IM-124 | 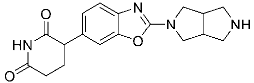 | IM-158 | 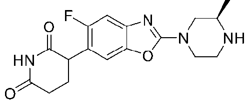 |
| IM-125 | 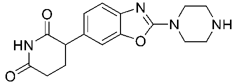 | IM-159 | 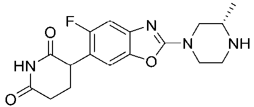 |
| IM-126 | 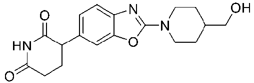 | IM-160 | 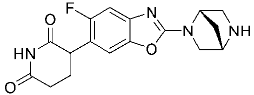 |
| IM-127 | 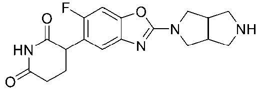 | IM-161 | 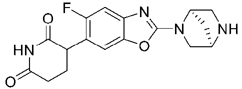 |
| IM-128 | 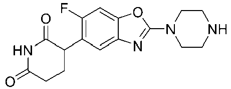 | IM-162 | 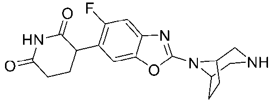 |
| IM-129 | 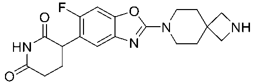 | IM-163 | 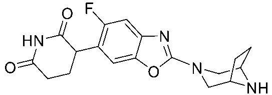 |
| IM-130 | 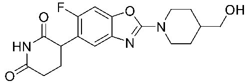 | IM-164 | 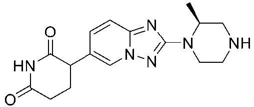 |
| IM-131 | 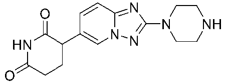 | IM-165 | 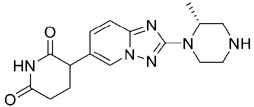 |
| IM-132 | 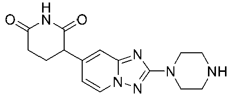 | IM-166 | 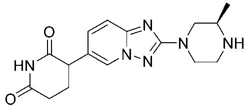 |
| IM-133 | 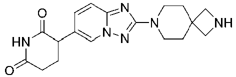 | IM-167 | 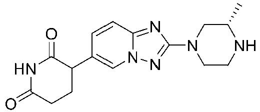 |
| IM-134 | 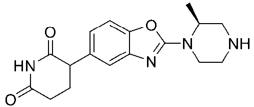 | IM-168 | 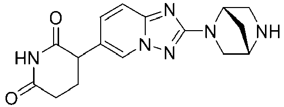 |
| IM-135 | 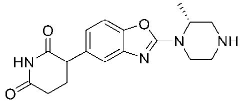 | IM-169 | 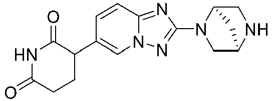 |
| IM-136 | 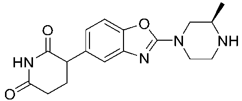 | IM-170 | 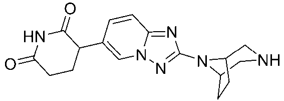 |
| IM-137 | 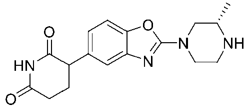 | IM-171 | 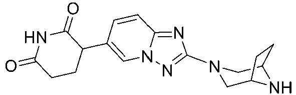 |
| IM-138 | 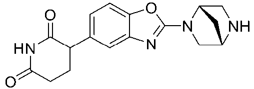 | IM-172 | 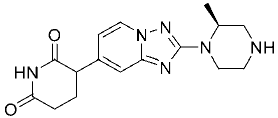 |
| IM-139 | 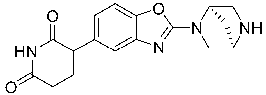 | IM-173 | 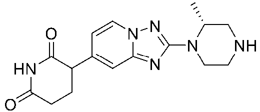 |
| IM-140 | 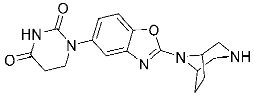 | IM-174 | 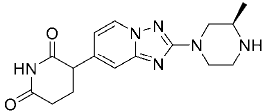 |
| IM-141 | 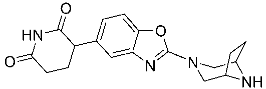 | IM-175 | 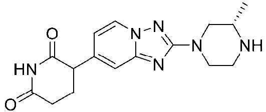 |
| IM-142 | 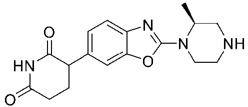 | IM-176 | 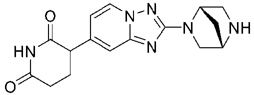 |
| IM-143 | 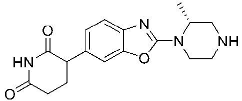 | IM-177 | 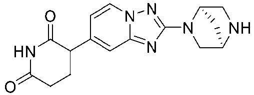 |
| IM-144 | 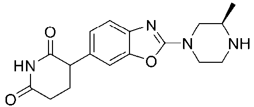 | IM-178 | 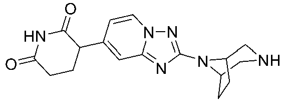 |
| IM-145 | 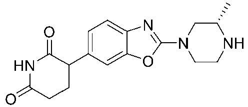 | IM-179 | 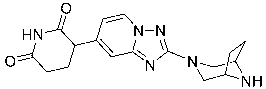 |
| IM-146 | 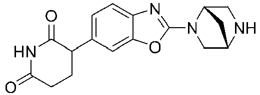 | IM-180 | 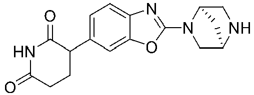 |

### Synthesis of intermediate IM-181

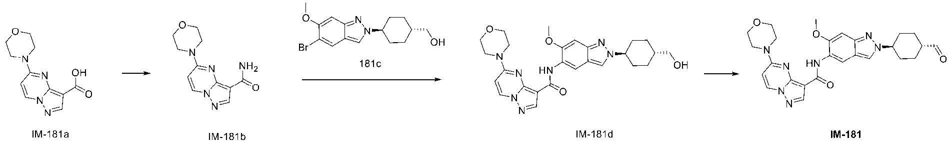

### Step 1: Synthesis of intermediate IM-181b

IM-181a (2 g), pyridine (1.3 g), and ammonium bicarbonate (1.3 g) were dispersed in 15 mL of dioxane, and Boc₂O (2.6 g) was added. After the addition was completed, the reaction mixture was heated to 60°C for reaction for 3 h. The reaction mixture was diluted with 45 mL of water, and suction-filtered. The filter cake was washed with a little dioxane to collect a solid, which was dried to give 1.85 g of a white solid. LCMS (ESI) m/z: [M+1]=248.1.

### Step 2: Synthesis of intermediate IM-181d

IM-181c (1.56 g, preparation method derived from WO2020/264499), IM-181b (1.25 g), Cs₂CO₃ (3.60 g), and Xantphos (532 mg) were dispersed in 20 mL of dioxane. Under nitrogen protection, Pd₂(dba)₃ (421 mg) was added, air in the reaction system was replaced with nitrogen, and the mixture was kept under nitrogen protection at 80°C for reaction for 72 h. 20 mL of dioxane was added to the reaction mixture. After full ultrasonic dispersion, the reaction mixture was suction-filtered through celite, the filtrate was concentrated, and the residue was subjected to column chromatography to give 320 mg of the target product. LCMS (ESI) m/z: [M+1]=506.2.

### Step 3: Synthesis of intermediate IM-181

The compound IM-181d (100 mg) was dissolved in 3 mL of DCM, DMP (1.3 eq) was added in an ice-water bath, and the mixture was stirred for 3 h. After the reaction was completed, the mixture was adjusted with saturated sodium bicarbonate to a PH of 7, filtered, layered, dried over anhydrous sodium sulfate, concentrated, and passed through a chromatographic column to give compound IM-181 (80 mg). LCMS (ESI) m/z: [M+1]=504.2.

Synthesis of intermediates IM-182 to IM-185: preparation methods for the intermediates IM-182 to IM-185 are similar to the preparation of the intermediate IM-181, as shown in Table 5.

**Table 5 Compound No. and structure**

| Compound No. | Structure | Compound No. | Structure |
|---|---|---|---|
| IM-182 | 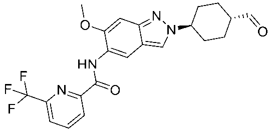 | IM-184 | 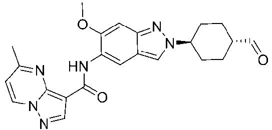 |
| IM-183 | 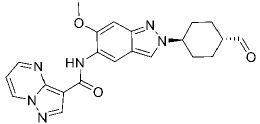 | IM-185 | 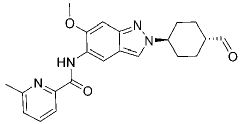 |

### Synthesis of intermediate IM-186

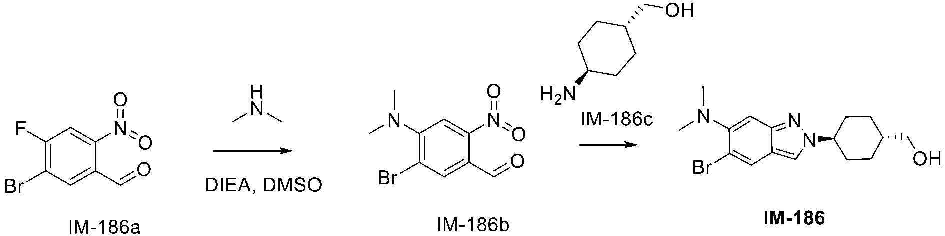

### Step 1: Synthesis of IM-186b

5-bromo-4-fluoro-2-nitrobenzaldehyde (15 g) and dimethylamine hydrochloride (9.86 g) were dissolved in 150 mL of DMSO, diisopropylethylamine (15.63 g) was added, and the reaction mixture was kept at 90°C for reaction overnight. After the reaction was completed, the mixture was diluted with water (600 mL), and extracted with EA (100 mL*3). The organic phase was washed with saturated brine, dried over anhydrous sodium sulfate, concentrated, dried, and purified by column chromatography (PE/DCM=5/1), to give 7.53 g of the target product. LCMS (ESI) m/z: [M+1]=272.98.

### Step 2: Synthesis of IM-186

IM-186b (2.5 g) was dissolved in 30 mL of isopropanol, and then IM-186c (1.43 g) was added. The mixture was kept under nitrogen protection at 80°C for reaction for four h, and then naturally cooled to 25°C. Tributylphosphine (5.56 g) was then added to the reaction mixture, and the mixture was kept under nitrogen protection at 80°C for reaction for 16 h. After the reaction was completed, the organic solvent was rotarily evaporated. The residue was diluted with water (100 mL), and extracted with EA (50 mL*3). The organic phase was washed with saturated brine, dried over anhydrous sodium sulfate, filtered, concentrated, and purified by column chromatography (PE/EA=1:1), to give 4.5 g of the product. LCMS (ESI) m/z: [M+1]=352.1.

Synthesis of intermediates IM-187 to IM-196: preparation methods for the intermediates IM-187 to IM-196 are similar to the preparation of the intermediate IM-186, as shown in Table 6.

**Table 6 Compound No. and structure**

| Compound No. | Structure | Compound No. | Structure |
|---|---|---|---|
| IM-187 | 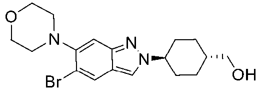 | IM-192 | 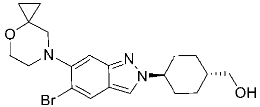 |
| IM-188 | 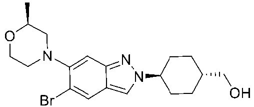 | IM-193 | 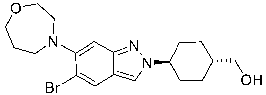 |
| IM-189 | 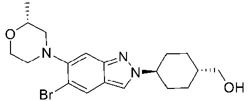 | IM-194 | 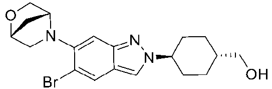 |
| IM-190 | 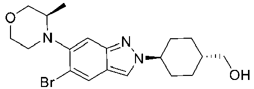 | IM-195 | 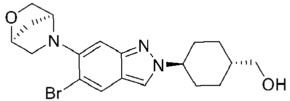 |
| IM-191 | 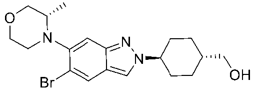 | IM-196 | 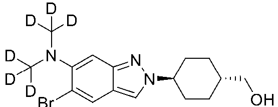 |

### Synthesis of intermediate IM-197

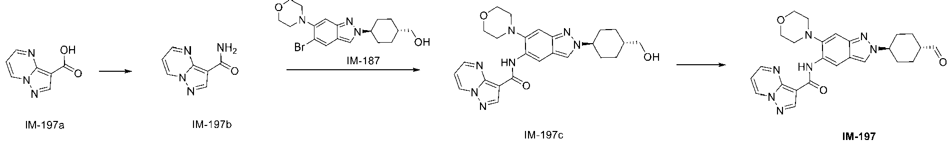

### Step 1: Synthesis of IM-197b

Pyrazolo[1,5-a]pyrimidine-3-carboxylic acid (1.5 g, 9.20 mmol) was dissolved in 15 mL of 1,4-dioxane, stirred until the mixture became clear. Pyridine (0.727 g, 9.19 mmol), BOC anhydride (3.01 g, 13.79 mmol), and NH₄HCO₃ (1.45 g, 18.34 mmol) were added. The reaction mixture was stirred in a water bath at room temperature for 12 h. After the reaction was completed, the mixture was filtered through celite, washed with water, washed with 1,4-dioxane, and dried in a vacuum drying oven to constant weight to give the target product, with a yield of 67%. LCMS (ESI) m/z: [M+1]=163.19.

### Step 2: Synthesis of IM-197c

IM-197b (1 g, 6.17 mmol), IM-175 (2.21 g, 5.60 mmol), Cs₂CO₃ (4.38 g, 13.44 mmol), and Xantphos (0.649 g, 1.12 mmol) were dissolved in 19 mL of 1,4-dioxane. Finally, Pd₂(dba)₃ (0.513 g, 0.56 mmol) was rapidly added. The mixture was purged with nitrogen 5 times and allowed to react at 100°C for 16 h. After the reaction was completed, the mixture was filtered through celite, spin dried under vacuum, and purified by column chromatography (DCM/MeOH=100/1 to 20/1) to give 1.168 g of the target product. The yield was 61.47%. LCMS (ESI) m/z: [M+1]=476.49.

### Step 3: Synthesis of IM-197

Oxalyl chloride (0.434 g, 3.42 mmol) was added to DCM (18 mL). In a dry-ice/ethanol bath at -70°C, DMSO (0.544 g, 6.96 mmol)/DCM (1 mL) solution was added dropwise, the mixture was stirred for 0.5 h, IM-47c (1.1 g, 2.28 mmol)/DCM (7 mL) solution was added, the mixture was stirred for 30 min, and DIPEA (1.47 g, 11.37 mmol)/DCM (2 mL) was added. The mixture was stirred for 15-30 min, and analyzed with acetonitrile by LCMS. After the reaction was completed, water (20 mL) was added, and the mixture was extracted with DCM (30 mL*3). The organic layer was washed with saturated brine, dried over anhydrous sodium sulfate, filtered, and concentrated to give the target product. 1 g of the product was obtained, with a yield of 93.46%. LCMS (ESI) m/z: [M+1]=476.49.

Synthesis of intermediates IM-198 to IM-207: preparation methods for the intermediates IM-198 to IM-207 are similar to the preparation of the intermediate IM-197, as shown in Table 7.

**Table 7 Compound No. and structure**

| Compound No. | Structure | Compound No. | Structure |
|---|---|---|---|
| IM-198 | 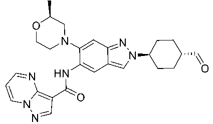 | IM-203 | 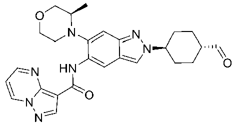 |
| IM-199 | 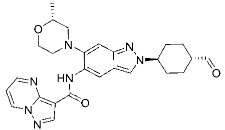 | IM-204 | 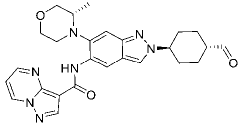 |
| IM-200 | 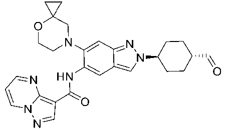 | IM-205 | 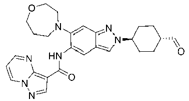 |
| IM-201 | 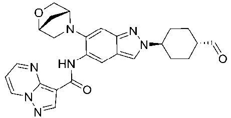 | IM-206 | 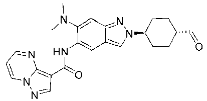 |
| IM-202 | 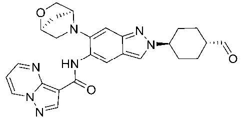 | IM-207 | 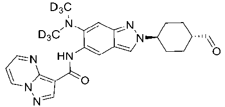 |

### Synthesis of intermediate IM-208

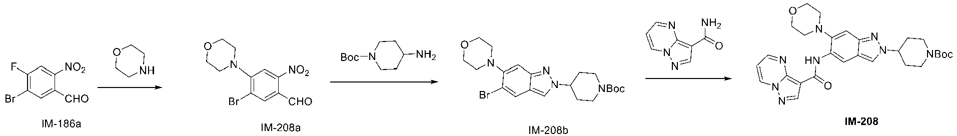

### Step 1: Synthesis of IM-208a

5-bromo-4-fluoro-2-nitrobenzaldehyde (35.6 g, 143.43 mmol) and morpholine (50.1 g, 575.06 mmol) were dissolved in 445 mL of DMSO, and the reaction mixture was kept for reaction at 80°C for 1 h. After the reaction was completed, the mixture was diluted with water (890 mL), and extracted with EA (900 mL*3). The organic phase was washed with saturated brine, dried over anhydrous sodium sulfate, concentrated, dried, and purified by column chromatography (PE/EA=10/1 to 1/4), to give 35 g of the product, with a yield of 77.6%. LCMS (ESI) m/z: [M+1]=316.12.

### Step 2: Synthesis of IM-208b

IM-208a (7 g, 22.22 mmol) was dissolved in 231 mL of isopropanol, and then tert-butyl 4-aminopiperidine-1-carboxylate (5.34 g, 26.66 mmol) was added. The mixture was kept under nitrogen protection at 80°C for reaction for four hours, and then naturally cooled to 25°C. Then, tributylphosphine (13.48 g, 66.63 mmol) was added to the reaction mixture, and the mixture was kept under nitrogen protection at 80°C for reaction for 16 h. After the reaction was completed, the organic solvent was rotarily evaporated. The residue was diluted with water (200 mL), and extracted with EA (200 mL*3). The organic phase was washed with saturated brine, dried over anhydrous sodium sulfate, filtered, and concentrated. It was purified by column chromatography (PE/EA=10:1 to PE/EA=5/1), to give 10 g of the product, with a yield of 96.7%. LCMS (ESI) m/z: [M+1]=467.29.

### Step 3: Synthesis of IM-208

IM-208b (2 g, 4.30 mmol), pyrazolo[1,5-a]pyrimidine-3-carboxamide (0.77 g, 4.75 mmol), Cs₂CO₃ (3.36 g, 10.31 mmol), and Xantphos (0.50 g, 0.864 mmol) were dissolved in 20 mL of 1,4-dioxane. Finally, Pd₂(dba)₃ (0.39 g, 0.426 mmol) was added rapidly. The reaction mixture was purged with nitrogen for 5 times and was then allowed to react at 100°C for 16 h. After the reaction was completed, the mixture was filtered through celite, spin dried, and purified by column chromatography (DCM/MeOH=100:1 to DCM/MeOH=40:1), to give 156 mg of the product, with a yield of 6.8%. LCMS (ESI) m/z: [M+1]=547.63.

Synthesis of intermediates IM-209 to IM-212: preparation methods for the intermediates IM-209 to 212 are similar to the preparation of the intermediate IM-208, as shown in Table 8.

**Table 8 Compound No. and structure**

| Compound No. | Structure | Compound No. | Structure |
|---|---|---|---|
| IM-209 | 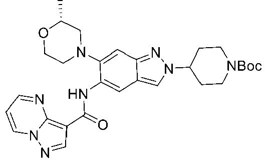 | IM-211 | 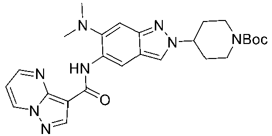 |
| IM-210 | 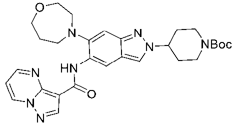 | IM-212 | 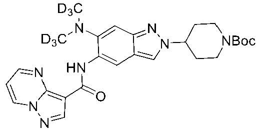 |

### Synthesis of intermediate IM-213

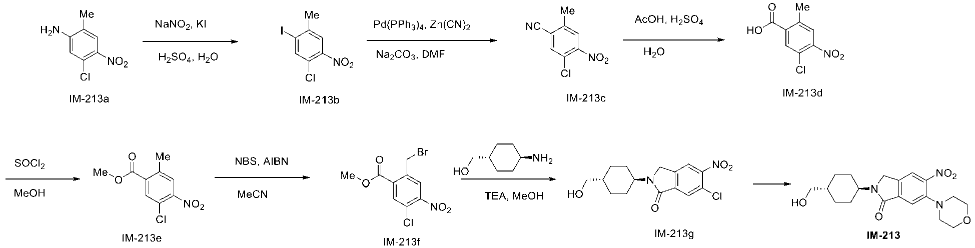

### Step 1: Synthesis of intermediate IM-213b

A solution of 5-chloro-2-methyl-4-nitroaniline (10 g, 53.6 mmol) in sulfuric acid (3 M, 100 mL) was placed in an ice bath at 0°C, and a solution of sodium nitrite (3.70 g, 53.6 mmol) in water (10 mL) was slowly added dropwise (in approximately 1 h). After the dropwise addition was completed, the mixture was stirred in an ice bath for 10 min, and an aqueous solution of potassium iodide (10.7 g, 64.3 mmol) was added. The mixture was kept in an ice bath at a reaction temperature of 0°C and stirred for 1 h, then slowly warmed to room temperature of 25°C, and further stirred at room temperature of 25°C for additional 1 h. TLC detection showed that the reaction was completed. The reaction mixture was diluted with water (500 mL), and extracted with ethyl acetate three times (3×200 mL). The organic layers were combined, washed with a saturated sodium chloride solution, dried over anhydrous sodium sulfate, filtered, concentrated under reduced pressure, and purified by silica gel column chromatography (petroleum ether:ethyl acetate=1:0 to 100:1) to give 1-chloro-5-iodo-4-methyl-2-nitrobenzene (11.6 g, 72% yield) as a yellow solid. ¹H NMR (400 MHz, DMSO-*d6*) δ 8.24 (s, 1H), 8.04 (s, 1H), 2.43 (s, 3H).

### Step 2: Synthesis of intermediate IM-213c

1-chloro-5-iodo-4-methyl-2-nitrobenzene (11.6 g, 39.2 mmol) was dissolved in a DMF solution (80 mL), tetrakis(triphenylphosphine)palladium (4.53 g, 3.92 mmol) and sodium carbonate (8.31 g, 78.4 mmol) were added, and then zinc cyanide solid (2.76 g, 23.5 mmol) was added. The mixture was vacuumized, and purged with nitrogen three times. The mixture was stirred under nitrogen protection at 50°C for 24 h. TLC monitoring showed that the reaction was completed. The reaction mixture was diluted with water (500 mL), and extracted with ethyl acetate three times (3×300 mL). The organic layers were combined, washed with a saturated sodium chloride solution, dried over anhydrous sodium sulfate, filtered, concentrated under reduced pressure, and purified by silica gel column chromatography (petroleum ether:ethyl acetate=1:0 to 80:1) to give 5-chloro-2-methyl-4-nitrobenzonitrile (5.99 g, 78% yield) as a yellow solid. ¹H NMR (400 MHz, CDCl₃) δ 7.82-7.79 (m, 2H), 2.64 (s, 3H).

### Step 3: Synthesis of intermediate IM-213d

5-chloro-2-methyl-4-nitrobenzonitrile (1 g, 5 mmol) was dissolved in a glacial acetic acid solution (10 mL), water (10 mL) and concentrated sulfuric acid (10 mL) were added, and the mixture was heated to 120°C for reaction overnight. The reaction was stopped after TLC monitoring showed that the reaction was completed. After cooled to room temperature, the reaction mixture was diluted with water (100 mL), to precipitate a solid, which was suction-filtered and dried to give 5-chloro-2-methyl-4-nitrobenzoic acid (0.86 g, yield 80%) as a gray white solid. ¹H NMR (400MHz, CDCl₃) δ 8.22 (1H), 7.76 (s, 1H), 2.70 (s, 3H).

### Step 4: Synthesis of intermediate IM-213e

5-chloro-2-methyl-4-nitrobenzoic acid (0.90 g, 4.15 mmol) was dissolved in a methanol solution (10 mL), and a thionyl chloride solution (0.3 mL, 4.15 mmol) was slowly added dropwise (in approximately 1 h). The mixture was then heated to 80°C and refluxed overnight. The reaction was stopped after TLC monitoring showed that the reaction was completed. The reaction mixture was distilled under reduced pressure until the solvent was evaporated to dryness, then dichloromethane (10 mL) and a saturated sodium bicarbonate solution (20 mL) were added, and the mixture was extracted with dichloromethane three times (3×50 mL). The organic layers were combined, washed with a saturated sodium chloride solution, dried over anhydrous sodium sulfate, filtered, concentrated under reduced pressure, and purified by silica gel column chromatography to give methyl 5-chloro-2-methyl-4-nitrobenzoate (0.84 g, yield 87%) as a light white solid. ¹H NMR (400 MHz, CDCI₃): δ 8.08 (s, 1H), 7.73 (s, 1H), 3.95 (s, 3H), 2.64 (s, 3H).

### Step 5: Synthesis of intermediate IM-213f

Methyl 5-chloro-2-methyl-4-nitrobenzoate (3.1 g, 13.5 mmol) was dissolved in an acetonitrile solution (60 mL), and NBS (2.88 g, 16.2 mmol) and AIBN (0.11 g, 0.68 mmol) were added. The mixture was then heated to 70°C under nitrogen protection, and refluxed for 16 h. The reaction was stopped after TLC monitoring showed that the reaction was completed. The reaction mixture was distilled under reduced pressure until the solvent was evaporated to dryness, ethyl acetate (100 mL) and water (100 mL) were added, and the mixture was extracted with ethyl acetate three times (3×100 mL). The organic layers were combined, washed with a saturated sodium chloride solution, dried over anhydrous sodium sulfate, filtered, concentrated under reduced pressure, and purified by silica gel column chromatography to give methyl 2-bromomethyl-5-chloro-4-nitrobenzoate (3.80 g, yield 91%) as a yellow solid. ¹H NMR (400MHz, CDCl₃) δ 8.15 (s, 1H), 7.99 (s, 1H), 4.92 (s, 2H), 4.01 (s, 3H).

### Step 6: Synthesis of intermediate IM-213g

Methyl 2-bromomethyl-5-chloro-4-nitrobenzoate (3.1 g, 13.5 mmol) was dissolved in a methanol solution (40 mL), (4-aminocyclohexyl)carbinol (2.01 g, 15.6 mmol) was added, and then a triethylamine solution (3.61 mL, 25.9 mmol) was added. The mixture was heated to 80°C under nitrogen protection and refluxed for 16 h. The reaction was stopped after TLC monitoring showed that the reaction was completed. The reaction mixture was distilled under reduced pressure until the solvent was evaporated to dryness, and then the residue was purified by silica gel column chromatography to give a yellow solid (2.40 g, yield 57%). ¹H NMR (400 MHz, CDCl₃) δ 8.01 (s, 1H), 7.91 (s, 1H), 4.44 (s, 2H), 4.25 (tt, *J=*3.6*,* 12.1 Hz, 1H), 3.53 (d, *J*=6.2 Hz, 2H), 2.01-1.93 (m, 4H), 1.62-1.55 (m, 2H), 1.55-1.49 (m, 1H), 1.46 (s, 1H), 1.28-1.17 (m, 2H).

### Step 7: Synthesis of intermediate IM-213

IM-213g (4.5 g) and diisopropylethylamine (7.2 mL) were dissolved in 45 mL of DMSO, morpholine (2.42 mL) was added to the reaction mixture, and the mixture was heat to 90°C for reaction for 12 h. The reaction mixture was diluted with 150 mL of water, and extracted with ethyl acetate. The organic layer was collected, successively washed with water and saturated sodium chloride, dried over anhydrous sodium sulfate, and concentrated under reduced pressure. The crude product was subjected to column chromatography (PE/EA=1/1) to give 3.3 g. LCMS (ESI) m/z: [M+1]=376.2.

Synthesis of intermediates IM-214 to IM-220: preparation methods for the intermediates IM-214 to 220 are similar to the preparation for the intermediate IM-213, as shown in Table 9.

**Table 9 Compound No. and structure**

| Compound No. | Structure | Compound No. | Structure |
|---|---|---|---|
| IM-214 | 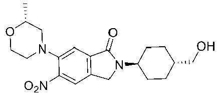 | IM-218 | 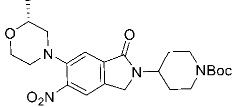 |
| IM-215 | 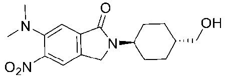 | IM-219 | 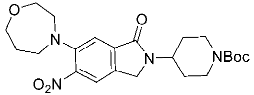 |
| IM-216 | 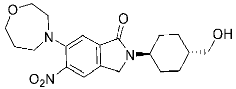 | IM-220 | 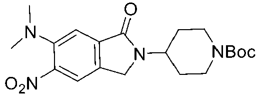 |
| IM-217 | 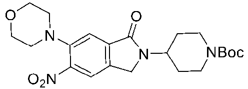 | | |

### Synthesis of intermediate IM-221

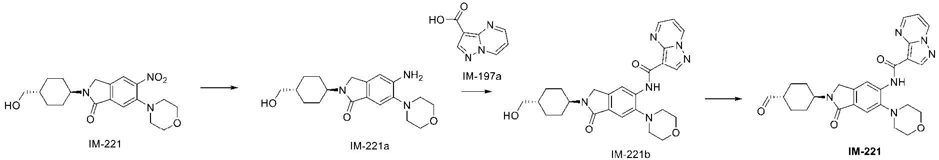

### Step 1: Synthesis of intermediate IM-221a

IM-221 (5 g) was dissolved in 50 mL of MeOH/H₂O (4/1), iron powder (7.4 g) and ammonium chloride (7.1 g) were successively added, and the mixture was heated under reflux at 70°C for reaction for 4 h. LC-MS detection showed that the raw material reaction was completed. The reaction mixture was filtered through celite, and the filter cake was washed with DCM/MeOH (10/1) solution. The filtrate was collected, and concentrated under reduced pressure. The residue was extracted with DCM. The organic layer was collected, washed with saturated brine, dried over sodium sulfate, and concentrated under reduced pressure to give 4 g of the crude product. It was used directly in the next step without purification. LCMS (ESI) m/z: [M+1]=346.2.

### Step 2: Synthesis of intermediate IM-221b

IM-197a (2.36 g) was dissolved in 30 mL of acetonitrile, N-methylimidazole (3.57 g) was added, the mixture was cooled to 0°C, TCFH (4.26 g) was added, the mixture was stirred for 10 min, and the IM-209a (5.00 g) was added. After the addition was completed, the mixture was kept at room temperature for reaction for 2 h, and water was added to quench the reaction. The mixture was evaporated to remove the organic solvent and extracted with ethyl acetate. The organic layer was collected, dried over anhydrous sodium sulfate, and concentrated under reduced pressure. The residue was separated by column chromatography to give the target compound IM-221b (700 mg). LCMS (ESI) m/z: [M+1]=491.24.

### Step 3: Synthesis of intermediate IM-221

Preparation method for IM-221 is similar to the step 3 of the preparation for the intermediate IM-197. LCMS (ESI) m/z: [M+1]=489.24.

Synthesis of intermediates IM-222 to IM-224: preparation methods for the intermediates IM-222 to 224 are similar to the preparation of the intermediate IM-221, as shown in Table 10.

**Table 10 Compound No. and structure**

| Compound No. | Structure | Compound No. | Structure |
|---|---|---|---|
| IM-222 | 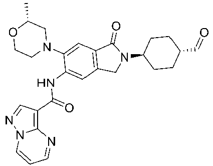 | IM-224 | 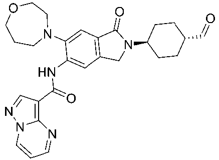 |
| IM-223 | 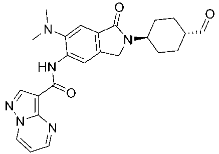 | | |

### Synthesis of intermediate IM-225

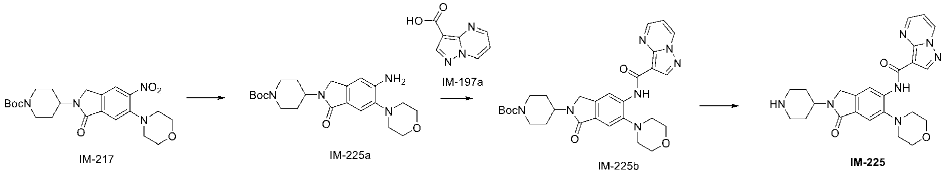

### Step 1: Synthesis of intermediate 225a

IM-217 (3.5 g) was dissolved in 50 mL of MeOH/H₂O (4/1), iron powder (4.4 g) and ammonium chloride (4.2 g) were successively added, and the mixture was heated under reflux at 70^{°}C for reaction overnight. LC-MS detection showed that the raw material reaction was completed. The reaction mixture was filtered through celite, and the filter cake was washed with DCM/MeOH (10/1) solution. The filtrate was collected, and concentrated under reduced pressure. The residue was extracted with DCM. The organic layer was collected, washed with saturated brine, dried over sodium sulfate, and concentrated under reduced pressure to give 2.68 g of the crude product. It was used directly in the next step without purification. LCMS (ESI) m/z: [M+1]=417.2.

### Step 2: Synthesis of intermediate 225b

IM-197a (1.1 g) was dissolved in 30 mL of acetonitrile, N-methylimidazole (2.04 g) was added, the mixture was cooled to 0°C, TCFH (2.27 g) was added, the mixture was stirred for 10 min, and IM-225a (2.6 g) was added. After the addition was completed, the mixture was kept at 40°C for reaction for 2 h, and suction-filtered. The filter cake was successively washed with water, washed with acetonitrile, and dried to give 2.6 g of the product. LCMS (ESI) m/z: [M+1]=562.2.

### Step 3: Synthesis of intermediate 225

IM-225b (200 mg) was dissolved in 4 mL of DCM, the mixture was cooled to 0°C, and 1 mL TFA was added. The mixture was warmed, and stirred for 2 h. LC-MS detection showed that the reaction was completed. The mixture was concentrated, and the residue was directly used in the next step. LCMS (ESI) m/z: [M+1]=462.2.

Synthesis of intermediates IM-226 to IM-228: preparation methods for the intermediates IM-226 to 228 are similar to the preparation of the intermediate IM-225, as shown in Table 11.

**Table 11 Compound No. and structure**

| Compound No. | Structure | Compound No. | Structure |
|---|---|---|---|
| IM-226 | 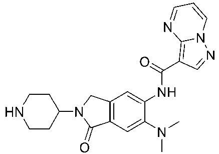 | IM-228 | 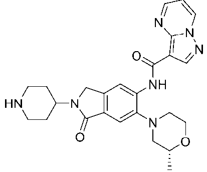 |
| IM-227 | 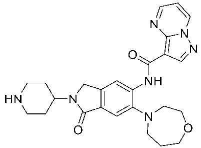 | | |

### Synthesis of intermediate IM-229

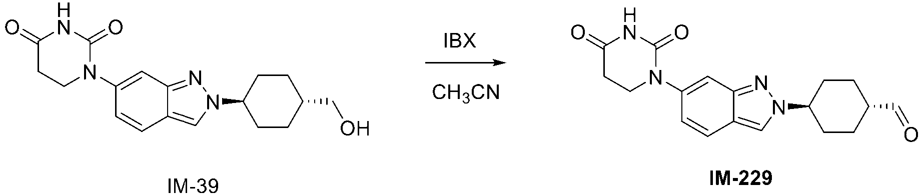

IM-39 (170 mg) was dissolved in 2 mL of acetonitrile, IBX (208 mg) was added, the mixture was heated to 60°C and allowed to react for 2 h. LC-MS showed that the reaction was complete. The reaction system was cooled to room temperature and filtered through a pad of silica gel to yield the crude product, which was directly used in the subsequent reaction step without purification. LCMS (ESI) m/z: [M+1]=341.15.

Synthesis of intermediates IM-230 to IM-244: preparation methods for the intermediates IM-230 to 244 are similar to the preparation of the intermediate IM-229, as shown in Table 12.

**Table 12 Compound No. and structure**

| Compound No. | Structure | Compound No. | Structure |
|---|---|---|---|
| IM-230 | 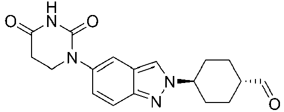 | IM-238 | 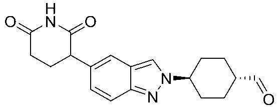 |
| IM-231 | 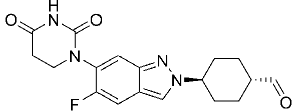 | IM-239 | 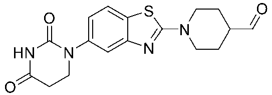 |
| IM-232 | 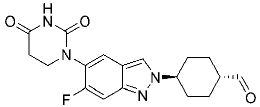 | IM-240 | 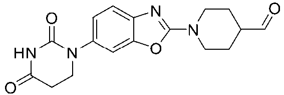 |
| IM-233 | 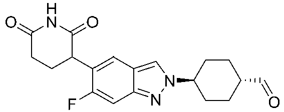 | IM-241 | 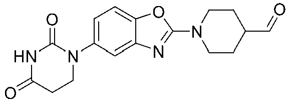 |
| IM-234 | 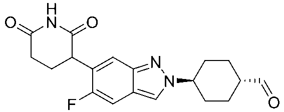 | IM-242 | 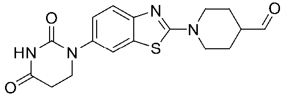 |
| IM-235 | 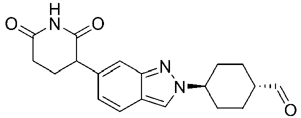 | IM-243 | 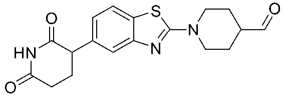 |
| IM-236 | 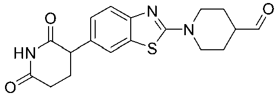 | IM-244 | 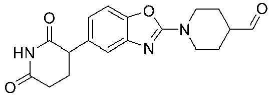 |
| IM-237 | 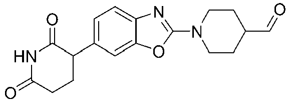 | | |

### Synthesis of intermediate IM-245

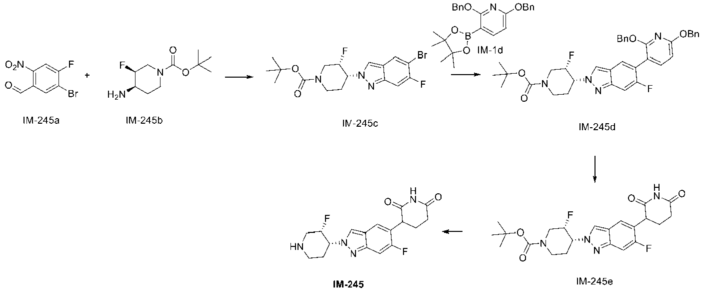

### Step 1: Synthesis of intermediate IM-245c

IM-245a (3 g) was dissolved in iPrOH (30 mL), IM-245b (3.17 g) was added, and the mixture was stirred at 80°C for 1 h, with the reaction monitored by thin-layer chromatography. After cooling the reaction system to room temperature, nBu₃P (9 mL, 36.289 mmol) was added, and the mixture was further allowed to react at 80°C for 2 h. After concentrating to remove iPrOH, MTBT was added and the temperature was raised to 50°C for slurrying. After stirring for 30 min, the mixture was stirred for another 30 min in an ice-cold ethanol bath and then filtered to give the product (3 g). LCMS (ESI) m/z: [M-55]=360.66.

### Step 2: Synthesis of intermediate IM-245d

Reactants IM-245c (615 mg), IM-1d (616.54 mg), K₃PO₄ (627.19 mg) and solvent dioxane/H₂O (7 mL, v/v = 4/1) were placed in a 50 mL eggplant-shaped flask. Pd(PPh₃)₄ (85.36 mg) was then added. The reaction mixture was purged with nitrogen three times and was stirred at 90°C to react for 12 h. The LCMS showed that the raw material was completely consumed, and the mass value of the target product was detected at t=1.96 min. The reaction solution was diluted with H₂O (15 mL), extracted with ethyl acetate (5mL*3), and the organic phases were collected. The organic phase was washed twice with saturated brine, then dried over anhydrous sodium sulfate, filtered, concentrated and weighed. The crude product was purified by column chromatography (PE/EA=3/1-1/1) to give the product (665 mg). LCMS (ESI) m/z: [M+H]=627.56.

### Step 3: Synthesis of intermediate IM-245e

IM-245d (100 mg) was dissolved in DCM/EtOH = 1:1 (100 mL), palladium acetate (10 mg) and activated carbon (100 mg) were added, followed by three times of H₂ purging. The reaction was carried out at 40°C for 12 h. The LCMS showed that the raw material was consumed, and the mass-56 value of the target product was detected at t=1.35 min. The reaction mixture was filtered through celite, repeatedly rinsed with DCM/MeOH at a ratio of 10:1, and the filtrate was collected and concentrated. Without purification, it was subjected to the next reaction step based on the theoretical yield. LCMS (ESI) m/z: [M+H]=449.49.

### Step 4: Synthesis of intermediate IM-245

IM-245e (70 mg) was dissolved in DCM (1 mL ), TFA (0.5 mL) was added under an ice-water bath, and the reaction mixture was stirred for half an hour. The reaction mixture was concentrated and used directly in the next step without purification. LCMS (ESI) m/z: [M+H]=349.29.

Synthesis of intermediates IM-246 to IM-265: preparation methods for the intermediates IM-246 to 265 are similar to the preparation of the intermediate IM-245, as shown in Table 13.

**Table 13 Compound No. and structure**

| Compound No. | Structure | Compound No. | Structure |
|---|---|---|---|
| IM-247 | 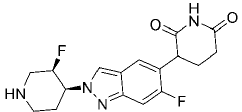 | IM-257 | 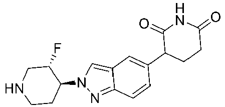 |
| IM-248 | 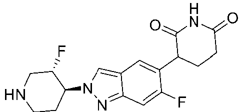 | IM-258 | 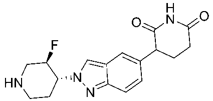 |
| IM-249 | 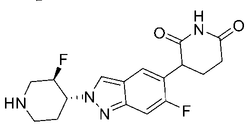 | IM-259 | 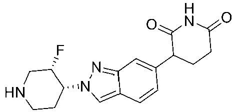 |
| IM-250 | 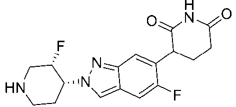 | IM-260 | 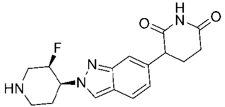 |
| IM-251 | 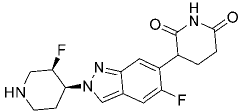 | IM-261 | 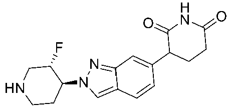 |
| IM-252 | 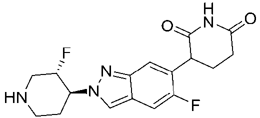 | IM-262 | 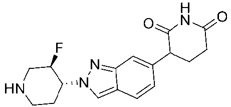 |
| IM-253 | 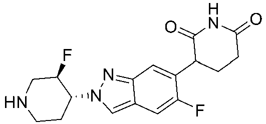 | IM-263 | 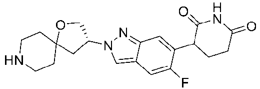 |
| IM-254 | 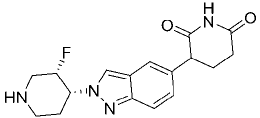 | IM-264 | 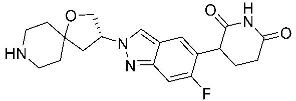 |
| IM-255 | 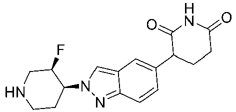 | IM-265 | 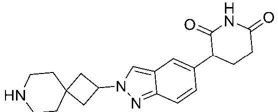 |

### Synthesis of intermediate IM-266

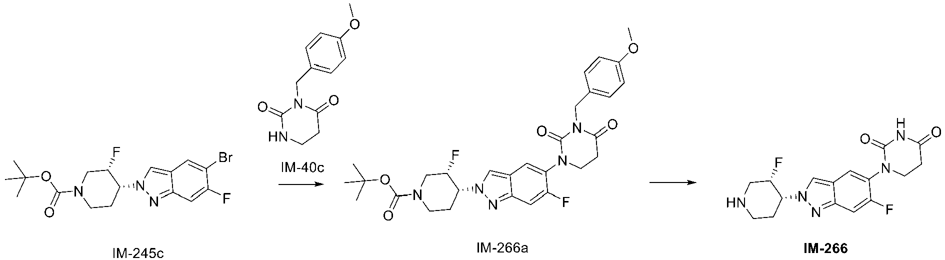

### Step 1: Synthesis of intermediate IM-266a

IM-245c (1.9 g), 4A molecular sieve (1 g), K₃PO₄ (1.94 g ), and IM-40c (0.13 g ) were dispersed in dioxane (20 mL), and CuI (0.17 g, 0.913 mmol) was added. The mixture was purged with nitrogen three times and was heated to 120°C and allowed to react for 48 h. The reaction mixture was filtered with celite, washed with dioxane, and spin dried to give the crude product. The crude product was purified by column chromatography (PE/EA=3:1-0:1) to give the product (1.5 g). LCMS (ESI) m/z: [M+23]=592.36.

### Step 2: Synthesis of intermediate IM-266

To IM-266a (1.5 g) were added TFA (27 mL) and TfOH (2.7 mL) in sequence, then the mixture was heated to 70°C and stirred for 12 h. The reaction mixture was directly concentrated under vacuum to give a residue. The PH was adjusted to 7-8 with TEA at 0°C, and the mixture was concentrated to give the crude product. The crude product was then pulped with EA, stirred for 0.5 h, and filtered. The filter cake was the product. LCMS (ESI) m/z: [M+H]=350.29.

Synthesis of intermediates IM-267 to IM-273: preparation methods for the intermediates IM-267 to 273 are similar to the preparation of the intermediate IM-266, as shown in Table 14.

**Table 14 Compound No. and structure**

| Compound No. | Structure | Compound No. | Structure |
|---|---|---|---|
| IM-267 | 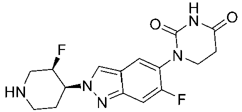 | IM-271 | 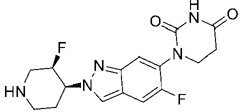 |
| IM-268 | 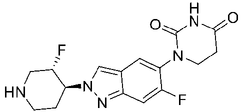 | IM-272 | 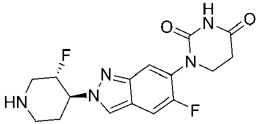 |
| IM-269 | 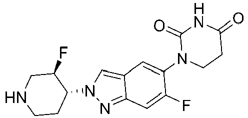 | IM-273 | 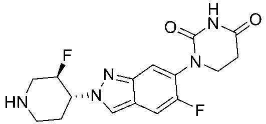 |
| IM-270 | 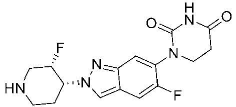 | | |

### Synthesis of intermediate IM-274

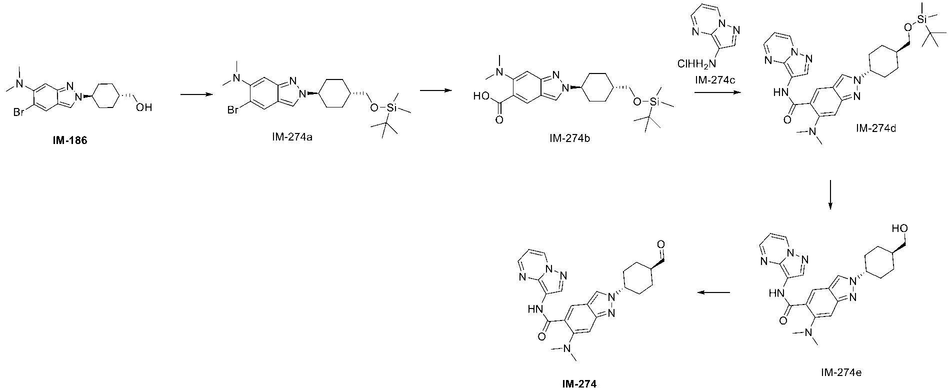

### Step 1: Synthesis of intermediate IM-274a

IM-186 (534 g) was dissolved in DMF (3000 mL), imidazole (154.80 g) was added, and TBSC1 (239.89 g) was added under an ice bath. The mixture was stirred overnight at room temperature. The mixture was diluted with water and extract with EA. The organic phases were collected, washed twice with water and twice with saturated brine, dried over anhydrous sodium sulfate, and concentrated and dried under reduced pressure to give the crude product. The crude product was purified by column chromatography (PE/EA=10:1-5:1) to give the product (510.67 g). LCMS (ESI) m/z: [M+H]=466.34.

### Step 2: Synthesis of intermediate IM-274b

Reactant IM-24a (1.71 g, 2.876 mmol) and solvent anhydrous THF (20 mL) were added to a 100 mL three-necked flask and cooled to -75°C. n-BuLi (5.573 mL, 13.932 mmol) was added dropwise using a syringe. After the addition was completed, the mixture was stirred for 1 h. Crushed dry ice was added under nitrogen protection. Upon complete addition, the system was gradually warmed back to room temperature and stirred overnight. The reaction was quenched with saturated ammonium chloride solution, followed by extraction with EA. The organic phases were collected, washed twice with water and twice with saturated brine, dried over anhydrous sodium sulfate, and concentrated and dried under reduced pressure to give the crude product. LCMS (ESI) m/z: [M+H]=432.18.

### Step 3: Synthesis of intermediate IM-274d

IM-274b (300 mg) was dissolved in DMF (3 mL), DIEA (0.233 mL) was added, and HATU (244.57 mg) was added at 0°C. After the addition was completed, the reaction was carried out for 5-10 min and then monitored by LCMS. After activation, IM-274c (91.44 mg) was added, and the entire system was allowed to react at room temperature overnight. The reaction mixture was diluted with water and extracted with EA. The organic phase was collected, washed with saturated brine, dried over anhydrous sodium sulfate and filtered to give the crude product. Purification: The crude product was loaded by the wet method and purified via column chromatography (PE/EA=10:1-5:1) to give 150 mg of the target product. LCMS (ESI) m/z: [M+H]=548.18.

### Step 4: Synthesis of intermediate IM-274e

IM-274d (1 g) was dissolved in CH₃CN (14 mL), and HF.Py (0.164 mL) was added under an ice bath. The mixture was allowed to return to room temperature to react for 4 h. LCMS (N230736-441 1.17 min 435.24) showed that the reactants reacted completely. Saturated Na₂CO₃ solution was added, and the mixture was extracted with EA. The organic phases were collected, washed with saturated brine, dried over anhydrous sodium sulfate and filtered to afford the crude product. The crude product was purified by column chromatography (DCM:MeOH=60:1-20:1), to give 400 mg of the product. LCMS (ESI) m/z: [M+H]=434.66.

### Step 5: Synthesis of intermediate IM-274

IM-274e (30 mg, 0.069 mmol) was dissolved in DCM (1 mL), and DMP (38.16 mg, 0.090 mmol) was added under an ice-water bath. After natural re-warming and stirring for 3 h, the test was conducted. The mixture was diluted with water and extract with DCM. The organic phases were collected, washed twice with saturated brine, dried over anhydrous sodium sulfate, and concentrated and dried under reduced pressure to give the crude product. The crude product was purified by column chromatography (DCM:MeOH = 80:1-60:1), to give 11 mg of the product. LCMS (ESI) m/z: [M+H]=432.2.

### Synthesis of intermediate IM-275

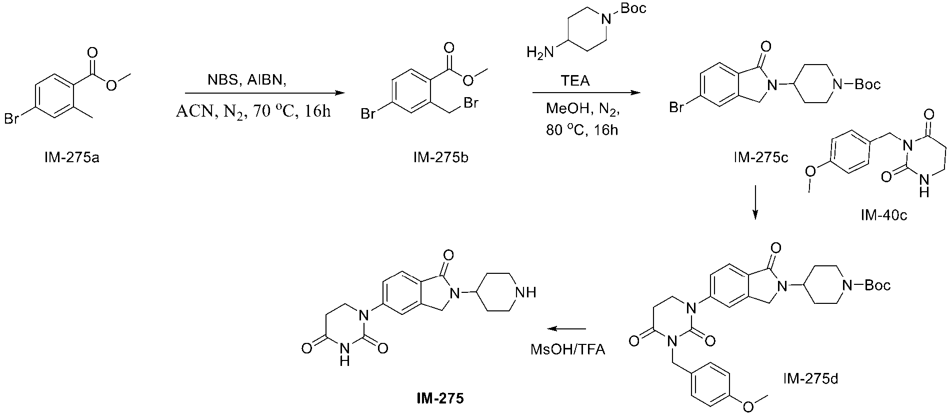

### Step 1: Synthesis of intermediate IM-275b

IM-275a (5 g) was added to a reaction flask containing ACN, then NBS (5 g) and AIBN (358 mg) were added in sequence, and the mixture was allowed to react at 70°C for 16 h under nitrogen protection. After the reaction was completed, the mixture was cooled to room temperature, extracted with EA, washed with saturated brine, dried over anhydrous Na₂SO₄, filtered, spin dried, and passed through a silica gel column (PE-PE:EA 50:1) to give 3 g of the target product.

### Step 2: Synthesis of intermediate IM-275c

IM-275b (2 g) was added to a reaction flask, methanol solution was added, 1-Boc-4-aminopiperidine (1.56 g) was added, and TEA (1.81 mL) was added dropwise while stirring. The mixture was allowed to react at 80°C for 16 h under nitrogen protection. After the reaction was completed, the mixture was cooled to room temperature, extracted with EA, washed with saturated brine, dried over anhydrous Na₂SO₄, filtered, spin dried, and passed through a silica gel column (PE:EA = 6:1-3:1) to give 2.2 g of the product. LCMS (ESI) m/z: [M+H]=395.10.

### Step 3: Synthesis of IM-275d

IM-275c (1 g) and IM-40c (0.65 g) were dissolved in dioxane (10 mL). Cs₂CO₃ (2.06 g) and Xantphos (0.15 g) were added, the mixture was purged three times with N₂. Under the protection of N₂, Pd(OAc)₂ (0.03 g) was added, and three additional times of N₂ purging were performed. The reaction system was stirred overnight at 100°C. The reaction mixture was filtered through celite, and concentrated. The crude product was purified by column chromatography (pure EA) to give 1.34 g of white solid. LCMS (ESI) m/z: [M+H]=549.53.

### Step 4: Synthesis of IM-275

IM-275d (100 mg) was dissolved in TFA (1 mL), methanesulfonic acid (0.5 mL) was added, and the reaction mixture was stirred at 70°C for 4 h. LCMS monitoring showed that the raw materials were completely consumed. After removing some of the solvent, the pH was adjusted to neutral with saturated sodium hydroxide solution, and the mixture was lyophilized. Then, it was washed with DCM/MeOH = 10:1, filtered, and the filtrate was concentrated. This gave the crude product, which was used directly in the next step without purification. LCMS (ESI) m/z: [M+H]=329.36.

### Synthesis of intermediate IM-276

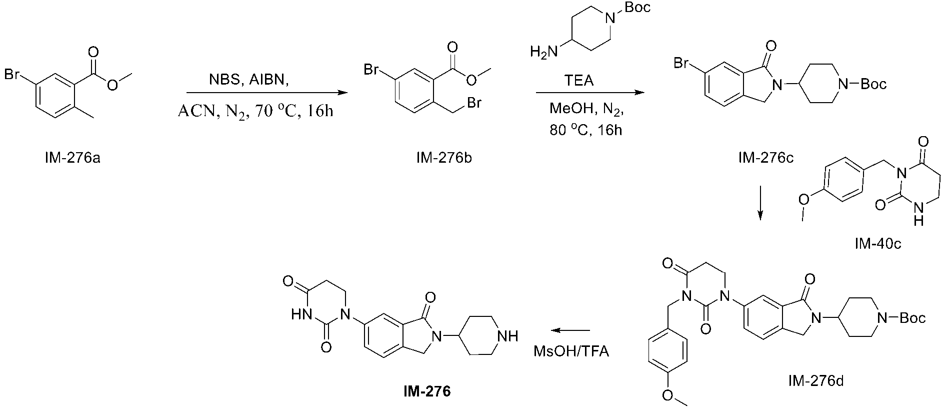

Preparation method for the intermediate IM-276 is similar to the preparation method for the intermediate IM-275. LCMS (ESI) m/z: [M+H]=329.37.

### Synthesis of intermediate IM-277

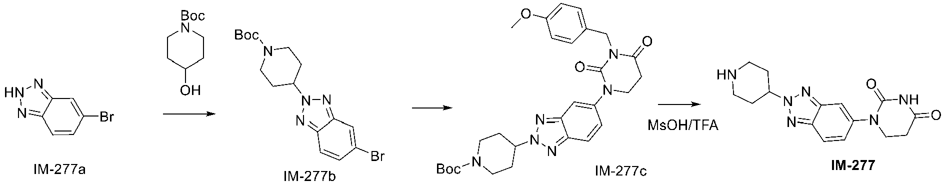

### Step 1: Synthesis of intermediate IM-277b

IM-277a (5 g), N-Boc-4-hydroxypiperidine (5.08 g), and triphenylphosphine (6.62 g) were dissolved in 50 mL of anhydrous tetrahydrofuran, cooled in an ice bath, DIAD (5 mL) was added dropwise, and thereafter the mixture was stirred overnight at room temperature. The sample was concentrated, mixed, and subjected to column chromatography (PE/EA = 1/1) to give 4 g of the target compound. LCMS (ESI) m/z: [M+H]=381.06.

### Step 2: Synthesis of intermediate IM-277c

IM-276c was replaced with IM-277b. The preparation method of IM-277c is the same as that of IM-276d. LCMS (ESI) m/z: [M+H]=535.15.

### Step 3: Synthesis of intermediate IM-277

IM-276d was replaced with IM-277c. The preparation method of IM-277 is the same as that of IM-276. LCMS (ESI) m/z: [M+H]=315.08.

### Synthesis of intermediate IM-278

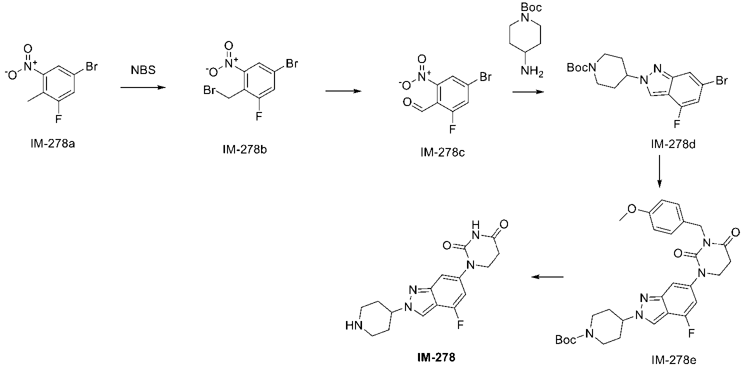

### Step 1: Synthesis of intermediate IM-278b

Reactants IM-288a (10 g), NBS (9.13 g) and solvent CH₃CN (100 mL) were placed in a 250 mL eggplant-shaped flask, followed by the addition of benzoyl peroxide (0.69 g). The mixture was refluxed for 6 h, followed by the supplementary addition of 0.6 eq NBS. Upon complete addition, the reaction was carried out overnight. The mixture was concentrated, slurried with dichloromethane and filtered. The filtrate was concentrated and subjected to column chromatography (0-20% ethyl acetate in petroleum ether) to afford the target product (10 g).

### Step 2: Synthesis of intermediate IM-278c

Reactants IM-288b (5 g), 4A molecular sieves and solvent CH₃CN (50 mL) were placed in a 100 mL eggplant-shaped flask, followed by the addition of N-methylmorpholine N-oxide (3.74 g). The mixture was stirred at room temperature for 3 h and extracted with EA. The organic phases were combined, washed with dilute hydrochloric acid and saturated brine, and dried over sodium sulfate. The mixture was filtered and concentrated, then subjected to column chromatography (0-10% EA in PE) to afford the target product (3.2 g).

### Step 3: Synthesis of intermediate IM-278d

IM-278c (2 g) was dissolved in 20 mL of isopropanol, 1-Boc-4-aminopiperidine (1.94 g) was added, the mixture was heated under reflux at 80°C for 2 h, tributylphosphine (4.08 g) was added, and the mixture was heated under reflux at 80°C overnight. LC-MS detection showed that the raw material reaction was completed and the target product was produced. The reaction mixture was concentrated under reduced pressure, then diluted with water and extracted with EA. The organic phase was collected, washed with saturated brine, dried over sodium sulfate, and concentrated under reduced pressure to give the crude product. The crude product was purified by column chromatography (PE/EA = 20:1-10:1) to give 2.5 g of the product. LCMS (ESI) m/z: [M+1]=398.10.

### Step 4: Synthesis of intermediate IM-278e

IM-277b was replaced with IM-278d. The preparation method of IM-278e is the same as that of IM-276d. LCMS (ESI) m/z: [M+H]=552.14.

### Step 5: Synthesis of intermediate IM-278

IM-277c was replaced with IM-278e. The preparation method of IM-278 is the same as that of IM-276. LCMS (ESI) m/z: [M+H]=332.16.

### Synthesis of intermediate IM-279

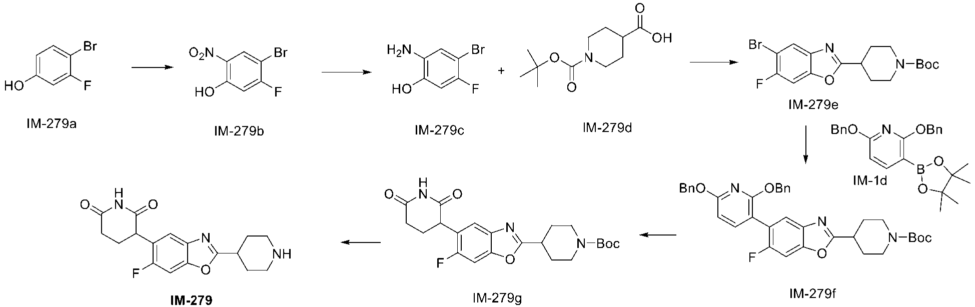

### Step 1: Synthesis of intermediate IM-279b

IM-279a (10 g) was dissolved in DCM and the solution was stirred at 0°C for 10 min. Then, 68% HNO₃ (6.93 mL) was added dropwise. The solution changed from pale yellow to clear orange-red. The reaction was carried out at rt for 4 h. After the reaction was completed, the mixture was extracted with DCM, dried over anhydrous sodium sulfate, filtered, spin dried, and passed through a column (PE-PE:EA = 30:1). The product was obtained as a yellow solid.

### Step 2: Synthesis of intermediate IM-279c

IM-279b (5 g) was dissolved in DMF, 4,4'-bipyridine (0.17 g) was added, and B₂(OH)₄ (5.7 g) was added in portions. There was a violent exothermic phenomenon, and the reaction mixture changed from yellow to purple-red. The reaction was carried out at room temperature for 20 min. After the reaction was completed, the solution was diluted with water, extracted with EA, washed with saturated brine, dried over anhydrous sodium sulfate, suction filtered, and spin dried to give the product. The product was a black oily substance. It was used directly in the next step without purification. LCMS (ESI) m/z: [M+H]=207.

### Step 3: Synthesis of intermediate IM-279e

IM-279c (4 g) and IM-279d (4.45 g) were added to a reaction flask, polyphosphoric acid and toluene (1:1) were added, the mixture was allowed to react at 190°C for 6 h, then the PH was adjusted to 9 with NaOH solution, di-tert-butyl dicarbonate (6.36 g) was added and the mixture was allowed to react for 3 h. The reaction mixture was extracted with EA, dried over anhydrous sodium sulfate, suction filtered, and purified by column chromatography (PE:EA10:1- 6: 1), to give 5 g of the target product. LCMS (ESI) m/z: [M+H]=399.06.

### Step 4: Synthesis of intermediate IM-279f

Reactants IM-279e (190 mg), IM-1d (198.59 mg) and K₃PO₄ (202.02 mg), along with solvent dioxane - water =4:1 (3 mL), were placed in a 25 mL eggplant-shaped flask. Finally, Pd(PPh₃)₄ (27.50 mg) was added. The reaction was carried out at 90°C. The reaction mixture was purged with nitrogen three times and was stirred for 12 h under N2 protection. The LCMS showed that the raw material was completely consumed, and the mass value of the target product was detected at t = 2.10 min. The reaction solution was diluted with H₂O (15 mL), extracted with ethyl acetate (5mL*3), and the organic phases were collected. The organic phase was washed twice with saturated brine, then dried over anhydrous sodium sulfate, filtered, concentrated and weighed. The crude product was purified by column chromatography (PE/EA=10/1-3/1) to give the product (145 mg). LCMS (ESI) m/z: [M+H]=610.3.

### Step 5: Synthesis of intermediate IM-279g

IM-279f (145 mg, 0.238 mmol) was dissolved in DCM/EtOH=1:1 (100 mL), Pd(OAc)2 (15 mg, 0.067 mmol) and activated carbon (145 mg, 0.033 mmol) were added. The reaction system was purged three times with H₂, and the reaction was carried out at 40°C for 12 h. The reaction mixture was filtered through celite, repeatedly rinsed with DCM/EtOH at a ratio of 1:1, and the filtrate was collected and concentrated. This gave the crude product, which was used directly in the next step. LCMS (ESI) m/z: [M+Na]=454.25.

### Step 6: Synthesis of intermediate IM-279

IM-279g (40 mg) was dissolved in DCM (1 mL ), TFA (0.5 mL) was added under an ice-water bath, and the reaction mixture was stirred for half an hour. The reaction mixture was concentrated and used directly in the next step without purification. LCMS (ESI) m/z: [M+H]=332.09.

### Synthesis of intermediate IM-280

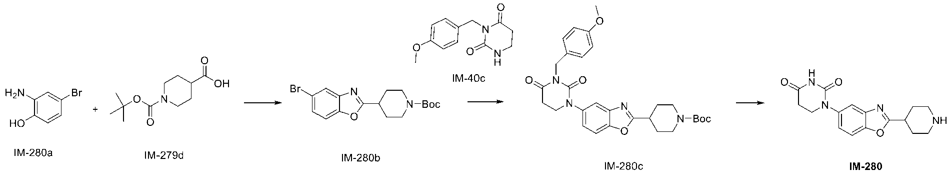

### Step 1: Synthesis of intermediate IM-280b

IM-279c was replaced with IM-280a. The preparation method of IM-280b is the same as that of IM-279e. LCMS (ESI) m/z: [M+H]=381.01.

### Step 2: Synthesis of intermediate IM-280c

IM-280b (1 g) and IM-40c (0.74 g) were dissolved in dioxane (10 mL). K₃PO₄ (1.11 g), CuI (100 mg), (1R,2R)-(-)-N,N'-dimethyl-1,2-cyclohexanediamine (CAS: 68737-65-5, 70 mg) and 4A molecular sieves (500 mg) were then added. Upon complete addition, the reaction system was purged with N₂, and stirred at 100°C overnight. The reaction mixture was filtered through celite, and concentrated. The crude product was purified by column chromatography to give the product (700 mg). LCMS (ESI) m/z: [M+H]=535.14.

### Step 3: Synthesis of intermediate IM-280

IM-280c (500 mg) was dissolved in TFA (5 mL) and TfOH (0.5 mL), then the mixture was heated to 70°C and stirred for 12 h. The reaction mixture was directly concentrated under vacuum to give a residue. The PH was adjusted to 7-8 with TEA at 0°C, and the mixture was concentrated to give the crude product. The crude product was then pulped with EA, and filtered to give the product. LCMS (ESI) m/z: [M+H]=315.10.

### Synthesis of intermediate IM-281

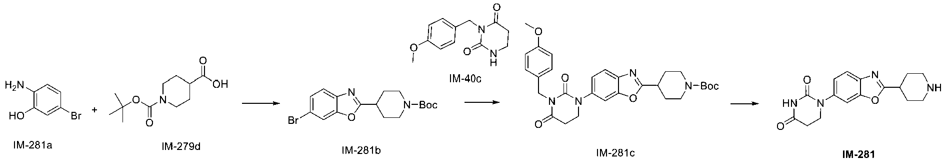

IM-280a was replaced with IM-281a. The preparation method of IM-281 is the same as that of IM-280. LCMS (ESI) m/z: [M+H]=315.13.

### Synthesis of intermediates IM-282 and IM-283

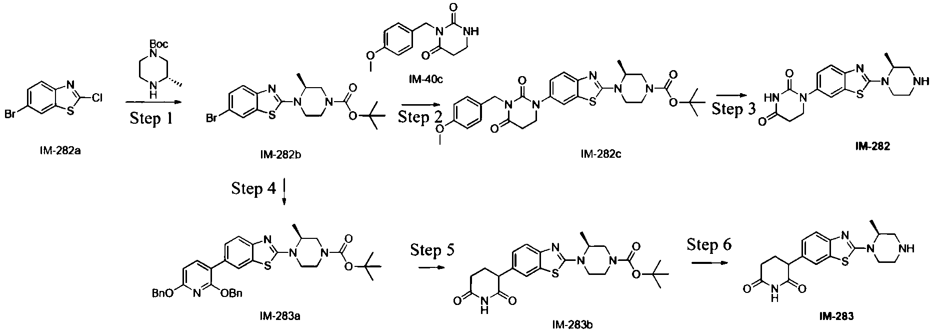

### Step 1: Synthesis of intermediate IM-282b

IM-282a (1 g) and (S)-4-N-tert-butyloxycarbonyl-2-methylpiperazine (0.85 g) were dissolved in 10 mL of DMF, K₂CO₃ (0.67 g) was added, and the mixture was stirred overnight at 100°C. Water (50 mL) was slowly added to the reaction system, and the mixture was extracted three times with EtOAc. The mixture was washed with water, dried over anhydrous sodium sulfate, and concentrated and spin dried to give the crude product. The crude product was subjected to column chromatography (PE:EA=1:0 to PE:EA=10:1) to give 830 mg of the product. LCMS (ESI) m/z: [M+H]=412.13.

### Step 2: Synthesis of intermediate IM-282c

IM-280b was replaced with IM-282c. The preparation method of IM-282c is the same as that of IM-280c. LCMS (ESI) m/z: [M+H]=566.52.

### Step 3: Synthesis of intermediate IM-282

IM-282c (80 mg) was dissolved in 2 mL of TFA:MsOH (1:2) solution and stirred overnight at 70°C. The reaction mixture was concentrated and adjusted to pH 7-8 with aqueous NaOH solution, lyophilized, and a DCM:MeOH = 10:1 solution was prepared. The sample was dissolved, filtered, and the filtrate was concentrated to give the product. LCMS (ESI) m/z: [M+H]=346.33.

### Steps 4 to 6: Synthesis of intermediate IM-283

IM-279e was replaced with IM-282b. The preparation method of IM-283 is the same as that of IM-279. LCMS (ESI) m/z: [M+H]=345.14.

Synthesis of intermediates IM-284 to IM-289: The preparation methods of intermediates IM-284 to 287 are similar to that of intermediate IM-282, and the preparation methods of intermediates IM-288 to 289 are similar to that of intermediate IM-283, as shown in Table 15.

**Table 15 Compound No. and structure**

| Compound No. | Structure | Compound No. | Structure |
|---|---|---|---|
| IM-284 | 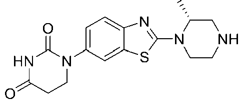 | IM-287 | 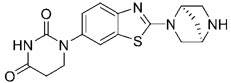 |
| IM-285 | 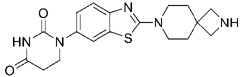 | IM-288 | 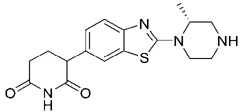 |
| IM-286 | 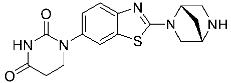 | IM-289 | 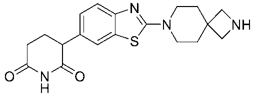 |

### Synthesis of intermediate IM-290

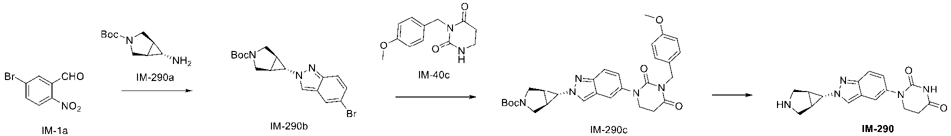

### Step 1: Synthesis of intermediate IM-290b

IM-1a (2 g) was dissolved in isopropanol (20 mL), followed by the addition of IM-290a (2.07 g). The reaction system was heated to 80°C and allowed to react for 2 h. After cooling, tributylphosphine (6.515 mL) was added, and the mixture was heated to 80°C and stirred overnight. The reaction mixture was concentrated under reduced pressure, slurried with MTBE, and filtered. The filter cake was collected to afford 2 g of the product. LCMS (ESI) m/z: [M+H]=378.29.

### Steps 2 to 3: Synthesis of intermediate IM-290

IM-280b was replaced with IM-290b. The preparation method of IM-290 is the same as that of IM-280. LCMS (ESI) m/z: [M+H]=312.34.

Synthesis of intermediates IM-291 to IM-299: preparation methods for the intermediates IM-281 to 299 are similar to the preparation of the intermediate IM-290, as shown in Table 16.

**Table 16 Compound No. and structure**

| Compound No. | Structure | Compound No. | Structure |
|---|---|---|---|
| IM-291 | 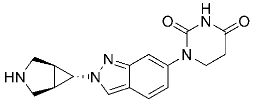 | IM-296 | 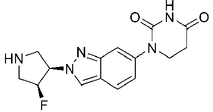 |
| IM-292 | 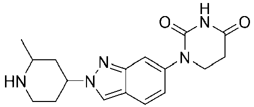 | IM-297 | 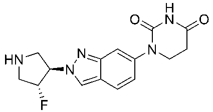 |
| IM-293 | 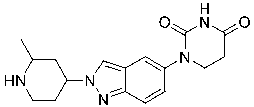 | IM-298 | 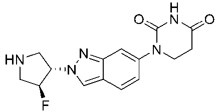 |
| IM-294 | 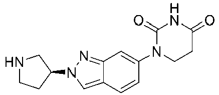 | IM-299 | 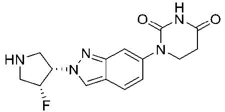 |
| IM-295 | 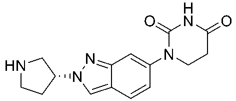 | | |

### Synthesis of intermediate IM-300

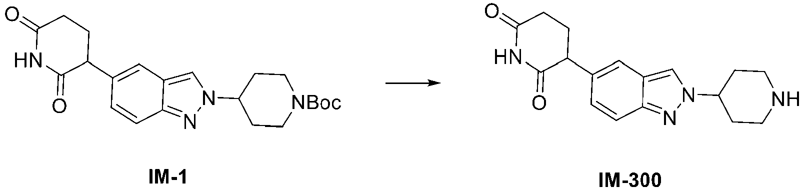

IM-1 (70 mg) was dissolved in DCM (3 mL), TFA (1 mL) was added at 0°C, and allowed to react at room temperature for two hours.

The mixture was repeatedly redissolved in DCM and spin dried. The resulting product was directly used in the next reaction step without further purification. LCMS (ESI) m/z: [M+H]=313.41.

Synthesis of intermediates IM-301 to IM-308: preparation methods for the intermediates IM-301 to 308 are similar to the preparation of the intermediate IM-300, as shown in Table 17.

**Table 17 Compound No. and structure**

| Compound No. | Structure | Compound No. | Structure |
|---|---|---|---|
| IM-301 | 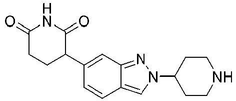 | IM-305 | 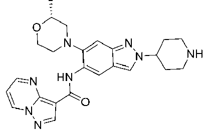 |
| IM-302 | 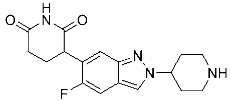 | IM-306 | 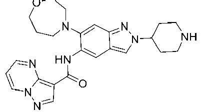 |
| IM-303 | 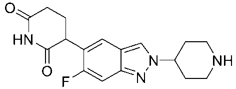 | IM-307 | 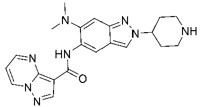 |
| IM-304 | 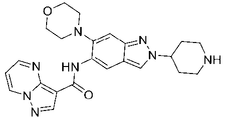 | IM-308 | 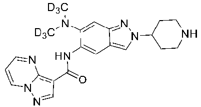 |

### Synthesis of intermediate IM-309

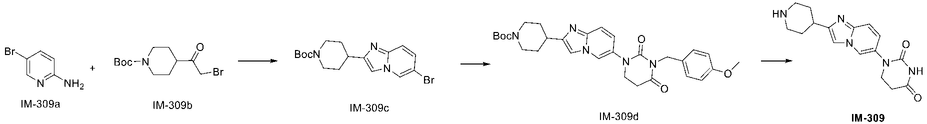

### Step 1: Synthesis of intermediate IM-309c

IM-309b (500 mg) and IM-309a (282.5 mg) were dissolved in EtOH (5 mL). The mixture as purged with nitrogen three times, and then heated to 60°C to react overnight. The mixture was concentrated, and saturated NaHCO₃ solution was added. The mixture was extracted with EA, the organic layer was dried over sodium sulfate, concentrated, and subjected to column chromatography (25% EA in PE) to give 496 mg of the product. LCMS (ESI) m/z: [M+H]=380.10.

### Steps 2 to 3: Synthesis of intermediate IM309

IM-280b was replaced with IM-309c. The preparation method of IM-309 is the same as that of IM-280. LCMS (ESI) m/z: [M+H]=314.14.

Synthesis of intermediates IM-310 to IM-314: preparation methods for the intermediates IM-310 to 314 are similar to the preparation of the intermediate IM-309, as shown in Table 18.

**Table 18 Compound No. and structure**

| Compound No. | Structure | Compound No. | Structure |
|---|---|---|---|
| IM-310 | 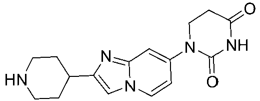 | IM-313 | 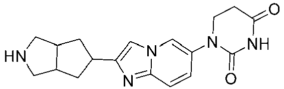 |
| IM-311 | 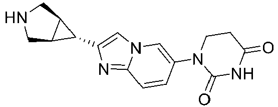 | IM-314 | 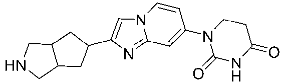 |
| IM-312 | 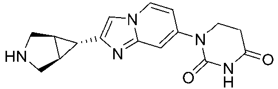 | | |

### Synthesis of intermediate IM-315

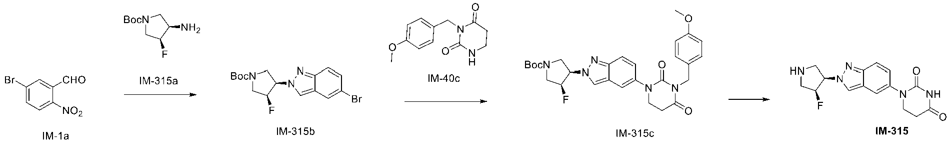

### Step 1: Synthesis of intermediate IM-315b

IM-1a (2.5 g) was dissolved in iPrOH (25 mL), IM-315a (2.66 g) was added, and the mixture was stirred at 80°C for 2 h, with the reaction monitored by thin-layer chromatography. After cooling the reaction system to room temperature, nBu₃P (8.14 mL) was added, and the mixture was further allowed to react at 80°C for 16 h. iPrOH (25 mL) was removed by concentration, and the residue was diluted with H₂O (30 mL). The mixture was extracted with EA (20mL*3), and the organic phases were collected and washed with saturated brine, dried over anhydrous sodium sulfate, concentrated and weighed to afford the crude product. The crude product was purified by column chromatography (PE/EA=20:1-PE/EA=5:1) to give 4 g of the product. LCMS (ESI) m/z: [M+H]=383.98, 386.42.

### Step 2: Synthesis of intermediate IM-315c

IM-315b (430 mg) and IM-40c (288.35 mg) were dissolved in dioxane (5 mL), Cs₂CO₃ (437.54 mg), Xantphos (64.75 mg), and Pd(OAc)₂ (12.56 mg) were added. The mixture was purged with nitrogen three times and allowed to react overnight at 100°C. The reaction mixture was filtered with celite, washed with 1,4-dioxane, and spin dried to give the crude product. The crude product was purified by column chromatography (PE/EA=1:1-0:1) to give the product (100 mg). LCMS (ESI) m/z: [M+H]=538.59.

### Step 3: Synthesis of intermediate IM-315

To IM-315c (200 mg) were added TFA (2 mL) and TfOH (0.2 mL) in sequence, then the mixture was heated to 70°C and stirred for 3 h. The reaction mixture was directly concentrated under vacuum to give a residue. The PH was adjusted to 7-8 with TEA at 0°C, and the mixture was concentrated to give the crude product. It was used directly in the next step without purification. LCMS (ESI) m/z: [M+H]=318.26.

Synthesis of intermediates IM-316 to IM-337: preparation methods for the intermediates IM-316 to 337 are similar to the preparation for the intermediate IM-315, as shown in Table 19.

**Table 19 Compound No. and structure**

| Compound No. | Structure | Compound No. | Structure |
|---|---|---|---|
| IM-316 | 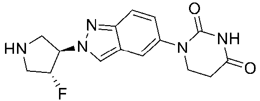 | IM-327 | 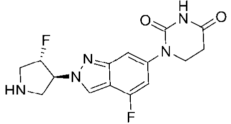 |
| IM-317 | 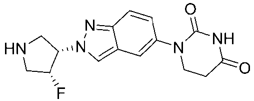 | IM-328 | 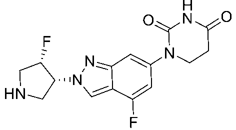 |
| IM-318 | 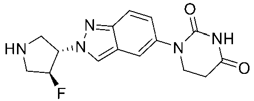 | IM-329 | 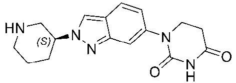 |
| IM-319 | 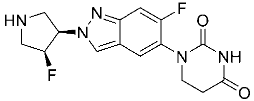 | IM-330 | 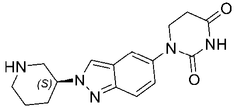 |
| IM-320 | 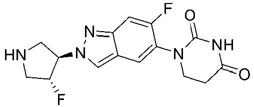 | IM-331 | 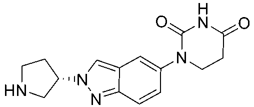 |
| IM-321 | 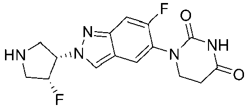 | IM-332 | 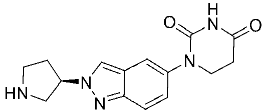 |
| IM-322 | 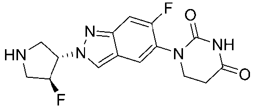 | IM-333 | 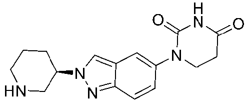 |
| IM-323 | 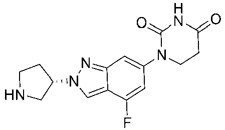 | IM-334 | 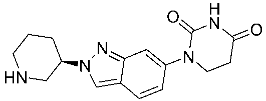 |
| IM-324 | 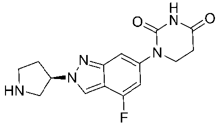 | IM-335 | 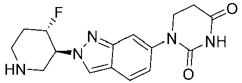 |
| IM-325 | 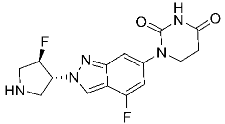 | IM-336 | 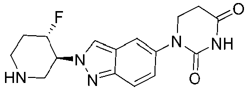 |
| IM-326 | 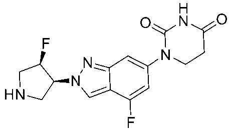 | IM-337 | 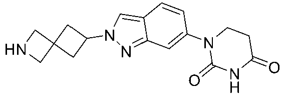 |

### Synthesis of intermediate 338

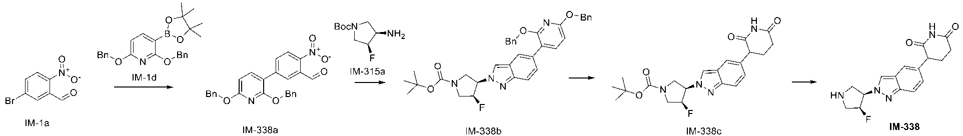

### Step 1: Synthesis of intermediate IM-338a

IM-1a (3 g) and IM-1d (6.53 g) were dissolved in dioxane:H₂O=4:1 (30 mL), K₃PO₄ (5.54 g) and Pd(PPh₃)₄ (0.75 g) were added, and the mixture was purged with nitrogen 3 times and then heated to 90°C for 12 h. The reaction mixture was diluted with water and extracted with EA. The organic phase was collected, washed with saturated brine, dried over anhydrous sodium sulfate, and filtered to give the crude product. The crude product was purified by column chromatography *(PE*/*EA=10:1-5:1)* to give the product (5.78 g). LCMS (ESI) m/z: [M+H]=441.04.

### Step 2: Synthesis of intermediate IM-338b

IM-338a (2.5 g) was dissolved in isopropanol (40 mL), IM-315a (1.28 g) was added, and the mixture was refluxed at 80°C for 30 min. Then, tri-n-butylphosphine (15.107 mL, 60.481 mmol) was added, and the mixture was allowed to react overnight at 80°C. The reaction mixture was poured into water and extracted three times with ethyl acetate (40 mL). The organic phases were combined, washed with saturated brine, dried over anhydrous sodium sulfate, filtered and spin dried to afford the crude product. The crude product was purified by column chromatography (PE/EA=5:1-1:1), to give 1.24 g of the product. LCMS (ESI) m/z: [M+H]=595.63.

### Step 3: Synthesis of intermediate IM-338c

IM-338b (500 mg) was dissolved in DCM:EtOH (500 mL), activated carbon (20 mg) and palladium acetate (50 mg) were added, and the mixture was purged with hydrogen three times and then heated to 40°C for 48 h. The mixture was filtered through celite, and spin dried to afford the product. LCMS (ESI) m/z: [M+H]=417.57.

### Step 4: Synthesis of intermediate IM-338

In a 25 mL round-bottom flask, IM-338c (170 mg) was dissolved in DCM (2 mL), and TFA (1 mL, 13.059 mmol) was added at 0-10°C. The reaction was allowed to proceed for 1 h. The reaction mixture was directly concentrated under vacuum and spin dried, then treated with DCM for 3 times, and directly added to the next step assuming a 100% yield. LCMS (ESI) m/z: [M+H]=317.18.

Synthesis of intermediates IM-339 to IM-345: preparation methods for the intermediates IM-339 to IM-345 are similar to the preparation of the intermediate IM-338, as shown in Table 20.

| Compound No. | Structure | Compound No. | Structure |
|---|---|---|---|
| IM-339 | 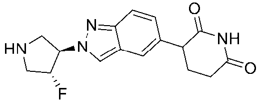 | IM-343 | 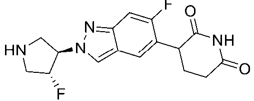 |
| IM-340 | 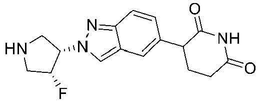 | IM-344 | 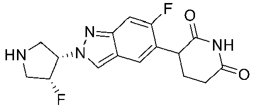 |
| IM-341 | 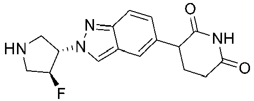 | IM-345 | 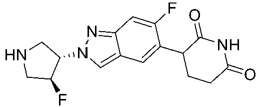 |
| IM-342 | 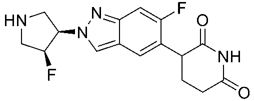 | | |

Synthesis of intermediates IM-346 to IM-348: preparation methods for the intermediates IM-346 to IM-348 are similar to the preparation of the intermediate IM-280, as shown in Table 21.

| Compound No. | Structure | Compound No. | Structure |
|---|---|---|---|
| IM-346 | 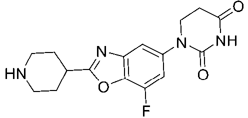 | IM-348 | 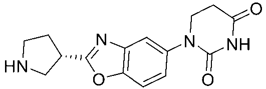 |
| IM-347 | 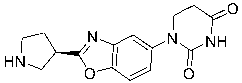 | | |

### Synthesis of intermediate IM-349

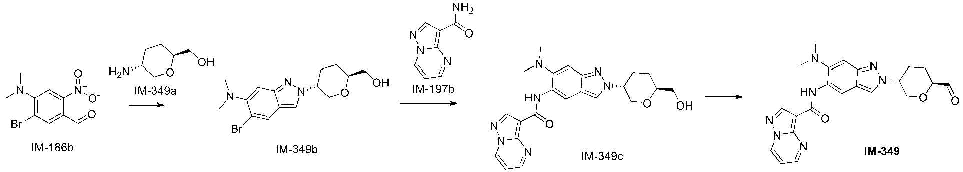

### Step 1: Synthesis of intermediate IM-349b

IM-186b (5.0 g) was dissolved in isopropanol (60 mL), IM-349a (2.88 g) was added, and the mixture was refluxed at 80°C for 4 h. Then, tri-n-butylphosphine (13.72mL) was added, and the mixture was allowed to react overnight at 80°C. A portion of the organic solvent was removed by rotary evaporation, the reaction mixture was poured into water and extracted three times with ethyl acetate (40 mL). The organic phases were combined, washed with saturated brine, dried over anhydrous sodium sulfate, filtered and spin dried to afford the crude product. The crude product was purified by column chromatography (PE/EA=5:1-0:1), to give 4 g of the product. LCMS (ESI) m/z: [M+H]=355.13.

### Steps 2 to 3: Synthesis of intermediate IM-349

IM-187 was replaced with IM-349b. The preparation of IM-349 is the same as that of IM-197. LCMS (ESI) m/z: [M+H]=434.49.

### Synthesis of intermediate IM-350

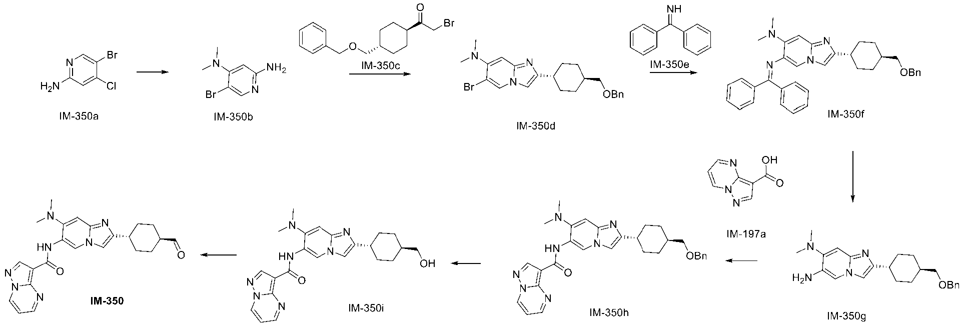

### Step 1: Synthesis of intermediate IM-350b

IM-350a (4.8 g) was dissolved in DMSO (48 mL), an aqueous solution of dimethylamine (48 mL) was added, and DIEA (4.030 mL) was added. The mixture was allowed to react at 100°C for 48 h. The reaction mixture was diluted with water, then extracted three times with ethyl acetate and tetrahydrofuran at a ratio of 4:1. The organic phases were collected, dried over anhydrous sodium sulfate, filtered and concentrated. The crude product was purified by column chromatography with dry loading (PE/EA=10:1-PE/EA=1:1), to afford the product (1.76 g). LCMS (ESI) m/z: [M+H]=216.17.

### Step 2: Synthesis of intermediate IM-350d

IM-350b (670 mg) was dissolved in EtOH (10 mL), IM-350c (1008.50 mg) and tetrahydropyrrole (0.026 mL) were added, and the mixture was heated to 80°C and refluxed overnight. The reaction mixture was directly subjected to column chromatography (PE/EA=10/1-1/1) to afford the target product. LCMS (ESI) m/z: [M+H]=442.34.

### Step 3: Synthesis of intermediate IM-350f

IM-350d (757 mg) and IM-350e (0.316 mL) were dissolved in toluene (8 mL), and sodium tert-butoxide (246.66 mg), XantPhos (99.01 mg), and palladium acetate (19.21 mg) were added. The mixture was then purged with nitrogen three times and heated to 110°C overnight under reflux. The mixture was filtered through celite, concentrated, and then subjected to column chromatography (DCM/MeOH=50/1-20/1) to afford the product (0.92 g). LCMS (ESI) m/z: [M+H]=543.77.

### Step 4: Synthesis of intermediate IM-350g

IM-350f (920 mg) was dissolved in THF/3N HCl = 1/1 (10 mL) and stirred overnight at room temperature. The reaction mixture was concentrated to remove THF, extracted with ethyl acetate, and the aqueous phase was collected. The pH was adjusted to neutral using NaOH solution, and extraction was continued using DCM/MeOH = 10/1. The organic phase was collected, dried over anhydrous sodium sulfate, filtered, and concentrated to give the crude product. The crude product was subjected to column chromatography (DCM/50/1-20/1) to give 640 mg of the product. LCMS (ESI) m/z: [M+H]=379.33.

### Step 5: Synthesis of intermediate IM-350h

IM-350g (640 mg, 1.691 mmol) was dissolved in DCM (8 mL), IM-197a (689.59 mg) and pyridine (0.684 mL) were added, and phosphorus oxychloride (0.394 mL, 4.227 mmol) was added under an ice-salt bath, and the mixture was stirred overnight. The reaction mixture was quenched with water, and extracted three times with DCM. The organic phases were collected, dried over anhydrous sodium sulfate, filtered and concentrated. The crude product was purified by column chromatography (DCM/MeOH=100/1-50/1), to give 246 mg of the product. LCMS (ESI) m/z: [M+H]=524.44.

### Step 6: Synthesis of intermediate IM-350i

IM-350h (246 mg) was dissolved in DCM (3 mL), and boron trichloride (1.719 mL) was added at 0°C and the mixture was stirred overnight. The reaction mixture was quenched with sodium bicarbonate solution and extracted with DCM/MeOH = 10/1. Due to incomplete extraction, the mixture was extracted three more times with EA/THF = 4/1, dried over anhydrous sodium sulfate, filtered, and concentrated. The crude product was subjected to column chromatography (DCM/MeOH=50/1-20/1) to give 190 mg of the product. LCMS (ESI) m/z: [M+H]=434.41.

### Step 7: Synthesis of intermediate IM-350

IM-350i (190 mg) was dissolved in DCM (3 mL), and DMP (241.66 mg) was added at 0°C. The reaction was carried out for three hours.

The reaction mixture was washed with saturated sodium bicarbonate solution, then extracted three times with DCM. The organic phases were collected, further washed with saturated brine, dried over anhydrous sodium sulfate and concentrated. The mixture was subjected to column chromatography (DCM/MeOH=50/1-30/1) to give 100 mg of the product. LCMS (ESI) m/z: [M+H]=432.47.

### Synthesis of intermediate IM-351

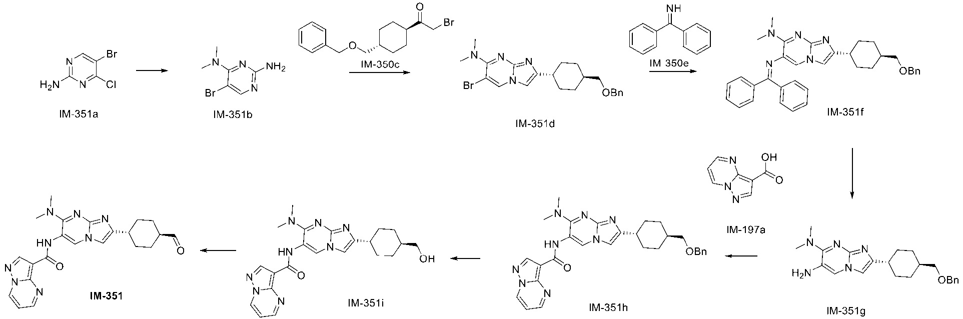

### Step 1: Synthesis of intermediate IM-351b

IM-351a (3 g) was dissolved in EtOH (30 mL), and dimethylamine hydrochloride (1.76 g) and DIEA (3.761 mL) were added. The reaction system was heated to 60°C and refluxed overnight. The reaction mixture was then concentrated. The crude product was subjected to column chromatography (PE/EA=10/1-1/1) to give 1.6 g of the product. LCMS (ESI) m/z: [M+H]=216.80, 218.80.

### Steps 2 to 7: Synthesis of intermediate IM-351

IM-350b was replaced with IM-350b. The preparation method of IM-351 is the same as that of IM-350. LCMS (ESI) m/z: [M+H] = 433.34.

### Synthesis of intermediate IM-352

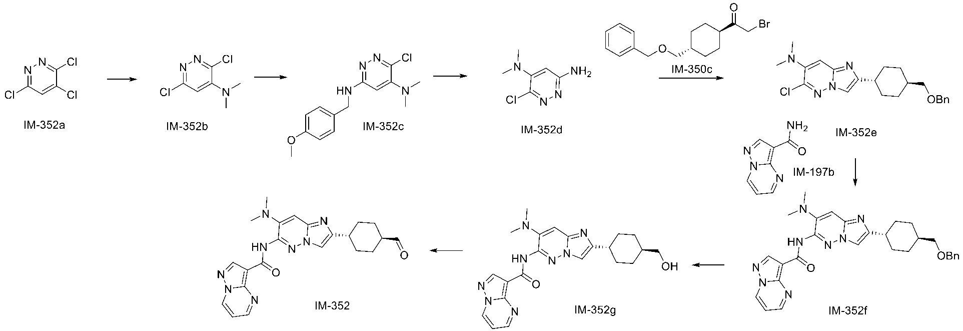

### Step 1: Synthesis of intermediate IM-352b

IM-352a (25 g) was dissolved in DCM (250 mL), and dimethylamine hydrochloride (12.23 g) and DIEA (47.484 mL) were added at 0°C. The mixture was allowed to react overnight at room temperature. The reaction mixture was poured into water and extracted three times with DCM (10 mL). The organic phases were combined, washed with saturated brine, dried over anhydrous sodium sulfate, filtered and spin dried to afford the crude product (26 g). The crude product was used directly in the next step without purification. LCMS (ESI) m/z: [M+H]=193.53.

### Step 2: Synthesis of intermediate IM-352c

IM-352b (26 g) and 4-methoxybenzylamine (26.5 mL) were dissolved in DMSO (400 mL), DIEA (67.3 mL) was added, the mixture was purged with nitrogen three times, and the reaction was carried out overnight at 120°C. The reaction mixture was poured into water and extracted three times with ethyl acetate. The organic phases were washed with saturated brine, dried over anhydrous sodium sulfate, filtered and spin dried. The crude product was purified by column chromatography (PE/EA=10:1-2:1), to give 15.6 g of the product. LCMS (ESI) m/z: [M+H]=293.96.

### Step 3: Synthesis of intermediate IM-352d

IM-352c (14 g) was dissolved in TFA (11.770 mL) and allowed to react overnight at 100°C. The reaction mixture was poured into water, adjusted to have neutral PH with TEA, and extracted three times with ethyl acetate (10 mL). The organic phases were washed with saturated brine, dried over anhydrous sodium sulfate, filtered and spin dried. The crude product was purified by normal-phase column chromatography. The crude product was treated with pure EA to give a pale yellow solid. LCMS (ESI) m/z: [M+H]=173.64.

### Step 4: Synthesis of intermediate IM-352e

IM-352d (1.5 g) and IM-350c (3.11 g) were dissolved in ethanol (15 mL), and tetrahydropyrrole (0.073 mL, 0.869 mmol) and NaHCO₃ (2.19 g) were added. The mixture was purged with nitrogen three times, and the reaction was carried out overnight at 80°C. The reaction mixture was poured into water and extracted three times with ethyl acetate (10 mL). The organic phases were washed with saturated brine, dried over anhydrous sodium sulfate, filtered and spin dried. The crude product was purified by normal-phase column chromatography. The crude product was treated with PE/EA=1:1 to give a pale yellow oily substance (1.1 g). LCMS (ESI) m/z: [M+H]=399.2.

### Step 5: Synthesis of intermediate IM-352f

IM-352e (1.6 g), IM-197b (0.72 g), sodium tert-butoxide (0.54 g), BINAP (0.50 g), and Pd₂(dba)₃ (0.37 g) were dissolved in dioxane (20 mL), the mixture was purged with nitrogen three times, and then heated to 110°C to react for 12 h. The reaction mixture was filtered with celite, washed with 1,4-dioxane, and spin dried to give the crude product. The crude product was purified by column chromatography purification (PE/EA=2:1-1:1 to DCM:MEoh=80:1-40:1). This gave the product as a yellow solid IM-352f (170 mg, 0.324 mmol, 8.08%). LCMS (ESI) m/z: [M+H]=525.48.

### Steps 6 to 7: Synthesis of intermediate IM-352

IM-350b was replaced with IM-352f. The preparation method of IM-352 is the same as that of IM-350. LCMS (ESI) m/z: [M+H] = 433.21.

**The synthesis of the compounds of the present invention is provided below.**

### Example 1: Synthesis of TM-1

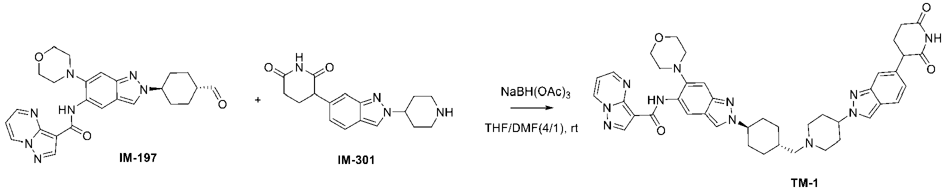

IM-197 (106 mg) and IM-301 (70 mg) were dissolved in 5 mL (THF/DMF = 4/1), and 100 mg of molecular sieve, DIPEA (43 mg), and NaBH(OAc)₃ (71 mg) were added successively under an ice-salt bath. The system was gradually warmed back to room temperature and allowed to react overnight. Water was added to the solution to precipitate a solid, which was then filtered. The filter cake was dissolved in DCM/MEOH(10:1) solution, and the resulting solution was filtered through celite. The filtrate was concentrated. The crude product was purified by preparative separation to afford 48.8 mg of a white solid. LCMS (ESI) m/z: [M+1]=770.95. ¹H NMR (600 MHz, DMSO-*d*₆) δ 10.84 (s, 1H), 10.67 (s, 1H), 9.39 (dd, *J =* 7.0, 1.6 Hz, 1H), 8.98 (dd, *J* = 4.2*,* 1.6 Hz, 1H), 8.75 (s, 1H), 8.72 (s, 1H), 8.40 (d, *J* = 1.0 Hz, 1H), 8.33 (d, *J* = 0.9 Hz, 1H), 7.63 (d, *J* = 8.6 Hz, 1H), 7.45 (s, 1H), 7.42 (s, 1H), 7.36 (dd, *J* = 7.0, 4.2 Hz, 1H), 6.88 (dd, *J* = 8.6, 1.4 Hz, 1H), 4.58 - 4.27 (m, 2H), 4.03 - 3.69 (m, 5H), 3.29 (s, 2H), 3.01 (br, 2H), 2.97 - 2.82 (m, 4H), 2.72-2.65 (m, 1H), 2.53-2.50 (m, 1H), 2.34 - 2.20 (m, 3H), 2.20 - 2.03 (m, 7H), 2.03 - 1.87 (m, 4H), 1.70-1.59 (m, 1H), 1.19 - 1.09 (m, 2H).

Synthesis of compounds TM-2 to TM-251: The preparation methods of compounds TM-2 to TM-251 are similar to that of product TM-1, as shown in Table 22.

**Table 22 Compound No. and structure**

| Example No. | Compound No. | Structure | Example No. | Compound No. | Structure |
|---|---|---|---|---|---|
| Example 2 | TM-2 | 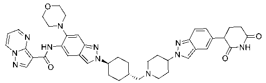 | Example 3 | TM-3 | 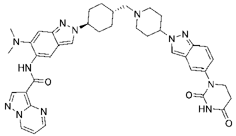 |
| Characterization | | | Characterization | | |
| LCMS (ESI) m/z: [M+1]=770.88. ¹H NMR (600 MHz, DMSO-*d*₆) δ 10.83 (s, 1H), 10.67 (s, 1H), 9.39 (dd, *J* = 6.9, 1.7 Hz, 1H), 8.97 (dd, *J* = 4.2, 1.7 Hz, 1H), 8.76 (s, 1H), 8.72 (s, 1H), 8.39 (s, 1H), 8.33 (s, 1H), 7.56 (d, *J* = 8.9 Hz, 1H), 7.49 (s, 1H), 7.45 (s, 1H), 7.36 (dd, *J* = 7.0, 4.2 Hz, 1H), 7.08 (dd, *J* = 9.0*,* 1.7 Hz, 1H), 4.60 - 4.31 (m, 2H), 3.94-3.84 (m, 5H), 3.29 (s, 2H), 3.09 - 2.97 (m, 2H), 2.96-2.87 (m, 4H), 2.71-2.63 (m, 1H), 2.33 - 2.04 (m, 11H), 2.02 - 1.88 (m, 4H), 1.72-1.61(m, 1H), 1.20-1.09 (m, 2H). | | | LCMS (ESI) m/z: [M+1]=729.73. ¹H NMR (400 MHz, DMSO-*d*₆) δ 10.85 (s, 1H), 10.33 (s, 1H), 9.36 (dd, *J* = 7.0, 1.7 Hz, 1H), 8.95 (dd, *J* = 4.2, 1.7 Hz, 1H), 8.72 (s, 1H), 8.70 (s, 1H), 8.45 (s, 1H), 8.29 (s, 1H), 7.63 - 7.53 (m, 2H), 7.37 (s, 1H), 7.33 (dd, *J* = 7.0, 4.2 Hz, 1H), 7.19 (dd, *J* = 9.1, 2.0 Hz, 1H), 4.52 - 4.30 (m, 2H), 3.79 (t, *J* = 6.7 Hz, 2H), 3.07-2.96 (m, 2H), 2.77 (s, 6H), 2.73 (t, J = 6.7 Hz, 2H), 2.33 - 2.05 (m, 10H), 2.04 - 1.83 (m, 4H), 1.72-1.60 (m, 1H), 1.20 - 1.07 (m, 2H). | | |

| Example No. | Compound No. | Structure | Example No. | Compound No. | Structure |
|---|---|---|---|---|---|
| Example 4 | TM-4 | 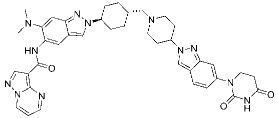 | Example 5 | TM-5 | 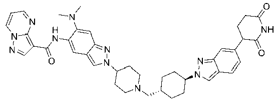 |
| Characterization | | | Characterization | | |
| LCMS (ESI) m/z: [M+1]=729.43. ¹H NMR (400 MHz, DMSO-*d*₆) δ 10.85 (s, 1H), 10.35 (s, 1H), 9.36 (dd, *J* = 7.0, 1.6 Hz, 1H), 8.95 (dd, *J* = 4.2, 1.7 Hz, 1H), 8.72 (s, 1H), 8.70 (s, 1H), 8.44 (d, *J* = 0.9 Hz, 1H), 8.29 (d, *J* = 1.0 Hz, 1H), 7.72 - 7.60 (m, 1H), 7.53 - 7.46 (m, 1H), 7.37 (s, 1H), 7.33 (dd, *J* = 7.1, 4.2 Hz, 1H), 7.02 (dd, *J* = 8.9, 1.8 Hz, 1H), 4.53-4.33 (m, 2H) , 3.83 (t, *J* = 6.7 Hz, 2H), 3.01 (s, 2H), 2.83-2.70 (m, 8H), 2.26-2.06 (m, 10H), 2.03-1.86 (m, 4H), 1.73-1.58(m, 1H), 1.20-1.05 (m, 2H). | | | LCMS (ESI) m/z: [M+1]=728.2. ¹H NMR (400 MHz, DMSO-*d*₆) δ 10.86 (s, 1H), 10.85 (s, 1H), 9.37 (dd, J = 7.0, 1.7 Hz, 1H), 8.95 (dd, J = 4.1, 1.7 Hz, 1H), 8.73 (s, 1H), 8.71 (s, 1H), 8.35 (s, 1H), 8.34 (s, 1H), 7.63 (d, J = 8.6 Hz, 1H), 7.42 (s, 1H), 7.38 (s, 1H), 7.33 (dd, J = 7.0, 4.2 Hz, 1H), 6.88 (dd, J = 8.6, 1.4 Hz, 1H), 4.51 - 4.33 (m, 2H), 3.93 (dd, J = 11.4, 4.9 Hz, 1H), 2.99 (br, 2H), 2.77 (s, 6H), 2.74-2.64 (m, 1H), 2.54 (t, J = 4.1 Hz, 1H), 2.34 - 1.87 (m, 16H), 1.72-1.57 (m, 1H), 1.21 - 1.03 (m, 2H). | | |
| Example 6 | TM-6 | 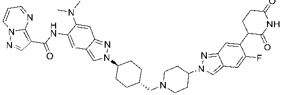 | Example 7 | TM-7 | 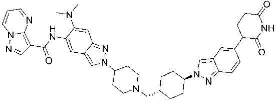 |
| Characterization | | | Characterization | | |
| LCMS (ESI) m/z: [M+1]=746.27. ¹H NMR (400 MHz, DMSO-*d*₆) δ 10.88 (s, 1H), 10.86 (s, 1H), 9.36 (dd, *J* = 7.0, 1.6 Hz, 1H), 8.95 (dd, *J* = 4.2, 1.7 Hz, 1H), 8.72 (s, 1H), 8.70 (s, 1H), 8.40 (d, *J* = 0.9 Hz, 1H), 8.29 (d, *J* = 0.9 Hz, 1H), 7.57 (d, *J* = 6.4 Hz, 1H), 7.42 (d, *J =* 11.1 Hz, 1H), 7.37 (s, 1H), 7.33 (dd, *J* = 7.0, 4.2 Hz, 1H), 4.57 - 4.31 (m, 2H), 4.08 (dd, *J* = 12.6, 4.9 Hz, 1H), 3.05-2.94 (m, 2H), 2.83-2.69 (m, 7H), 2.62 - 2.53 (m, 2H), 2.35 - 2.01 (m, 11H), 2.01-1.85 (m, 4H), 1.72-1.58 (m, 1H), 1.09-1.07 (m, 2H). | | | LCMS (ESI) m/z: [M+1] = 728.2. ¹H NMR (400 MHz, DMSO-*d*₆) δ 10.86 (s, 1H), 10.84 (s, 1H), 9.37 (dd, J = 7.0, 1.7 Hz, 1H), 8.95 (dd, J = 4.2, 1.7 Hz, 1H), 8.73 (s, 1H), 8.71 (s, 1H), 8.35 (s, 1H), 8.34 (s, 1H), 7.56 (d, J = 8.9 Hz, 1H), 7.49 (s, 1H), 7.38 (s, 1H), 7.33 (dd, J = 7.0, 4.2 Hz, 1H), 7.08 (dd, J = 8.9, 1.7 Hz, 1H), 4.51 - 4.35 (m, 2H), 3.90 (dd, J = 11.3, 4.9 Hz, 1H), 3.00 (br, 2H), 2.77 (s, 6H), 2.73-2.61(m, 1H), 2.54 (d, J = 2.9 Hz, 1H), 2.30 - 1.84 (m, 16H), 1.73-1.58 (m, 1H), 1.25 - 1.05 (m, 2H). | | |

| Example No. | Compound No. | Structure | Example No. | Compound No. | Structure |
|---|---|---|---|---|---|
| Example 8 | TM-8 | 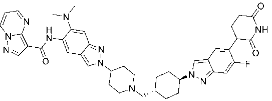 | Example 9 | TM-9 | 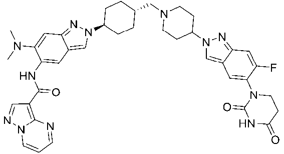 |
| Characterization | | | Characterization | | |
| LCMS (ESI) m/z: [M+1]=746.24. ¹H NMR (400 MHz, DMSO-*d*₆) δ 10.85 (s, 2H), 9.36 (d, *J* = 7.0 Hz, 1H), 8.95 (d, *J* = 4.1 Hz, 1H), 8.71 (s, 1H), 8.70 (s, 1H), 8.46 (s, 1H), 8.29 (s, 1H), 7.63 (d, *J* = 7.3 Hz, 1H), 7.44 - 7.16 (m, 3H), 4.54-4.31 (m, 2H), 4.09 - 3.98 (m, 1H), 3.04-2.94 (br, 3H), 2.83-2.68 (m, 7H), 2.61-2.52 (m, 2H), 2.35 - 1.81 (m, 14H), 1.70-1.60 (m, 1H), 1.20 - 1.00 (m, 2H). | | | LCMS (ESI) m/z: [M+1]=747.45. ¹H NMR (400 MHz, DMSO-*d*₆) δ 10.85 (s, 1H), 10.45 (s, 1H), 9.36 (dd, *J* = 7.0, 1.7 Hz, 1H), 8.95 (dd, *J* = 4.1, 1.7 Hz, 1H), 8.72 (s, 1H), 8.70 (s, 1H), 8.54 (s, 1H), 8.29 (s, 1H), 7.78 (d, *J* = 7.7 Hz, 1H), 7.46 (d, *J* = 11.3 Hz, 1H), 7.37 (s, 1H), 7.33 (dd, *J* = 7.0, 4.2 Hz, 1H), 4.66 - 4.11 (m, 2H), 3.72 (t, *J* = 6.6 Hz, 2H), 3.00 (br, 2H), 2.80-2.68 (m, 8H), 2.28-1.82 (m, 14H), 1.73-1.58 (m, 1H), 1.22-1.06 (m, 2H). | | |

| Example No. | Compound No. | Structure | Example No. | Compound No. | Structure |
|---|---|---|---|---|---|
| Example 10 | TM-10 | 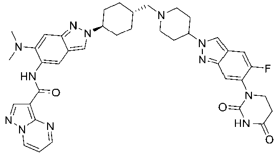 | Example 11 | TM-11 | 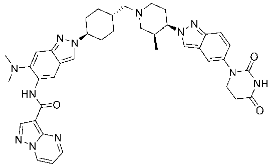 |
| Characterization | | | Characterization | | |
| LCMS (ESI) m/z: [M+1]=747.40. ¹H NMR (400 MHz, DMSO-*d*₆) δ 10.85 (s, 1H), 10.48 (s, 1H), 9.37 (dd, *J* = 7.0, 1.7 Hz, 1H), 8.95 (dd, *J* = 4.2, 1.7 Hz, 1H), 8.72 (s, 1H), 8.70 (s, 1H), 8.46 (s, 1H), 8.29 (s, 1H), 7.72 (d, *J* = 6.8 Hz, 1H), 7.53 (d, *J* = 10.6 Hz, 1H), 7.37 (s, 1H), 7.33 (dd, *J* = 7.0, 4.1 Hz, 1H), 4.56-4.33 (m, 2H), 3.76 (t, *J* = 6.7 Hz, 2H), 3.00 (br, 2H), 2.80-2.72(m, 8H), 2.23 (d, *J* = 7.1 Hz, 2H), 2.19-2.06 (m, 8H), 2.04-1.84 (m, 4H), 1.71-1.61 (m, 1H), 1.21-1.08 (m, 2H). | | | LCMS (ESI) m/z: [M+1]=743.38. | | |

| Example No. | Compound No. | Structure | Example No. | Compound No. | Structure |
|---|---|---|---|---|---|
| Example 12 | TM-12 | 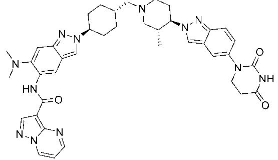 | Example 13 | TM-13 | 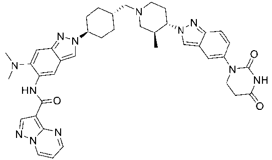 |
| Characterization | | | Characterization | | |
| LCMS (ESI) m/z: [M+1]=743.40. | | | LCMS (ESI) m/z: [M+1]=743.34. | | |
| Example 14 | TM-14 | 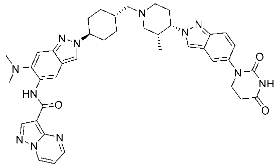 | Example 15 | TM-15 | 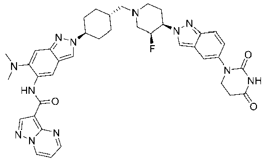 |
| Characterization | | | Characterization | | |
| LCMS (ESI) m/z: [M+1]=743.37. | | | LCMS (ESI) m/z: [M+1]=747.36. ¹H NMR (400 MHz, DMSO-*d*₆) δ 10.85 (s, 1H), 10.33 (s, 1H), 9.36 (dd, *J* = 7.0, 1.7 Hz, 1H), 8.95 (dd, *J* = 4.2, 1.7 Hz, 1H), 8.72 (s, 1H), 8.70 (s, 1H), 8.45 (s, 1H), 8.29 (s, 1H), 7.70 - 7.51 (m, 2H), 7.37 (s, 1H), 7.33 (dd, *J* = 7.0, 4.1 Hz, 1H), 7.22 (dd, *J* = 9.4*,* 1.8 Hz, 1H), 5.12 (d, *J* = 49.9 Hz, 1H), 4.76 (dd, *J* = 30.3, 12.7 Hz, 1H), 4.52 - 4.30 (m, 1H), 3.80 (t, *J* = 6.7 Hz, 2H), 3.26 - 3.15 (m, 1H), 3.08 (d, *J* = 11.0 Hz, 1H), 2.77 (s, 6H), 2.73 (t, *J* = 6.7 Hz, 2H), 2.61-2.45 (m, 1H), 2.38-2.20 (m, 4H), 2.19-2.09 (m, 3H), 2.04 - 1.84 (m, 4H), 1.74-1.59 (m, 1H), 1.19-1.07 (m, 2H). | | |

| Example No. | Compound No. | Structure | Example No. | Compound No. | Structure |
|---|---|---|---|---|---|
| Example 16 | TM-16 | 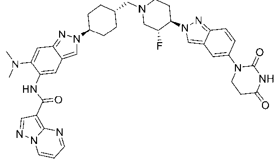 | Example 17 | TM-17 | 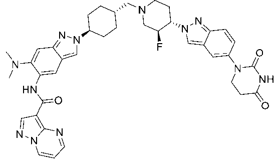 |
| Characterization | | | Characterization | | |
| LCMS (ESI) m/z: [M+1]=747.32. ¹H NMR (400 MHz, DMSO-*d*₆) δ 10.85 (s, 1H), 10.33 (s, 1H), 9.36 (dd, *J* = 7.0, 1.7 Hz, 1H), 8.95 (dd, *J* = 4.2, 1.6 Hz, 1H), 8.72 (s, 1H), 8.70 (s, 1H), 8.52 (s, 1H), 8.29 (s, 1H), 7.69 - 7.53 (m, 2H), 7.37 (s, 1H), 7.33 (dd, *J* = 7.0, 4.2 Hz, 1H), 7.22 (dd, *J* = 9.0, 2.1 Hz, 1H), 5.27 - 4.93 (m, 1H), 4.82 - 4.56 (m, 1H), 4.44-4.32 (m, 1H), 3.80 (t, *J* = 6.7 Hz, 2H), 2.97 (d*, J* = 10.3 Hz, 1H), 2.77 (s, 6H), 2.73 (t*, J* = 6.7 Hz, 2H), 2.39 - 1.86 (m, 13H), 1.71-1.59 (m, 1H), 1.19-1.07 (m, 2H). | | | LCMS (ESI) m/z: [M+1]=747.39. ¹H NMR (400 MHz, DMSO-*d*₆) δ 10.85 (s, 1H), 10.33 (s, 1H), 9.36 (dd, *J* = 7.0, 1.7 Hz, 1H), 8.95 (dd, *J* = 4.2, 1.7 Hz, 1H), 8.72 (s, 1H), 8.70 (s, 1H), 8.52 (s, 1H), 8.29 (s, 1H), 7.66 - 7.49 (m, 2H), 7.37 (s, 1H), 7.33 (dd, *J* = 7.0, 4.2 Hz, 1H), 7.26 - 7.17 (m, 1H), 5.20-4.97 (m , 1H), 4.78-4.58 (m, 1H), 4.48-4.33 (m, 1H), 3.80 (t, *J* = 6.7 Hz, 2H), 3.02-2.91 (m, 1H), 2.77 (s, 6H), 2.73 (t, *J* = 6.7 Hz, 2H), 2.41 - 1.87 (m, 13H), 1.73-1.60 (m, 1H), 1.21-1.08 (m, 2H). | | |

| Example No. | Compound No. | Structure | Example No. | Compound No. | Structure |
|---|---|---|---|---|---|
| Example 18 | TM-18 | 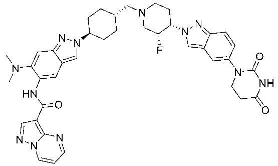 | Example 19 | TM-19 | 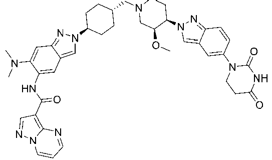 |
| Characterization | | | Characterization | | |
| LCMS (ESI) m/z: [M+1]=747.35. ¹H NMR (400 MHz, DMSO-*d*₆) δ 10.85 (s, 1H), 10.33 (s, 1H), 9.37 (dd, *J* = 7.0, 1.7 Hz, 1H), 8.95 (dd, *J* = 4.2, 1.7 Hz, 1H), 8.72 (s, 1H), 8.45 (s, 1H), 8.70 (s, 1H), 8.30 (s, 1H), 7.64-7.57 (m, 2H), 7.37 (s, 1H), 7.33 (dd, *J* = 7.0, 4.2 Hz, 1H), 7.22 (d, *J* = 9.3 Hz, 1H), 5.12 (d, *J* = 50.0 Hz, 1H), 4.89 - 4.61 (m, 1H), 4.47-4.32 (m, 1H), 3.80 (t, *J =* 6.7 Hz, 2H), 3.48-3.24 (m, 1H), 3.09 (d, *J* = 11.2 Hz, 1H), 2.77 (s, 6H), 2.73 (t, *J* = 6.7 Hz, 2H), 2.62-2.54 (m, 1H), 2.38 - 2.23 (m, 4H), 2.21-2.09 (m, 3H), 2.06 - 1.85 (m, 4H), 1.73-1.60 (m, 1H), 1.21-1.10 (m, 2H). | | | LCMS (ESI) m/z: [M+1]=759.38. | | |
| Example 20 | TM-20 | 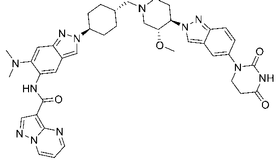 | Example 21 | TM-21 | 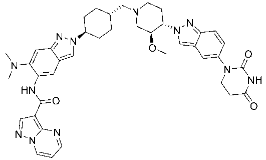 |
| Characterization | | | Characterization | | |
| LCMS (ESI) m/z: [M+1]=759.39. | | | LCMS (ESI) m/z: [M+1]=759.35. | | |

| Example No. | Compound No. | Structure | Example No. | Compound No. | Structure |
|---|---|---|---|---|---|
| Example 22 | TM-22 | 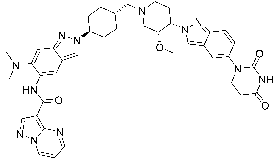 | Example 23 | TM-23 | 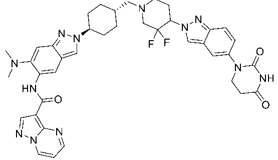 |
| Characterization | | | Characterization | | |
| LCMS (ESI) m/z: [M+1]=759.36. | | | LCMS (ESI) m/z: [M+1]=765.35. | | |

| Example No. | Compound No. | Structure | Example No. | Compound No. | Structure |
|---|---|---|---|---|---|
| Example 24 | TM-24 | 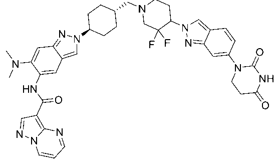 | Example 25 | TM-25 | 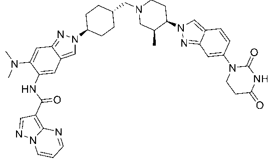 |
| Characterization | | | Characterization | | |
| LCMS (ESI) m/z: [M+1]=765.34. | | | LCMS (ESI) m/z: [M+1]=743.38. | | |

| Example No. | Compound No. | Structure | Example No. | Compound No. | Structure |
|---|---|---|---|---|---|
| Example 26 | TM-26 | 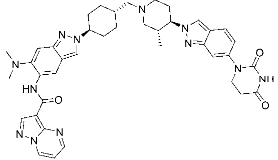 | Example 27 | TM-27 | 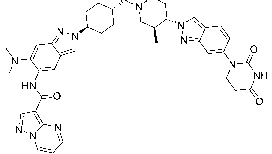 |
| Characterization | | | Characterization | | |
| LCMS (ESI) m/z: [M+1]=743.34. | | | LCMS (ESI) m/z: [M+1]=743.30. ¹H NMR (400 MHz, DMSO-*d*₆) δ 10.85 (s, 1H), 10.35 (s, 1H), 9.36 (dd, *J* = 7.0, 1.7 Hz, 1H), 8.95 (dd, *J* = 4.2, 1.7 Hz, 1H), 8.72 (s, 1H), 8.70 (s, 1H), 8.44 (s, 1H), 8.29 (s, 1H), 7.67 (d, *J* = 8.9 Hz, 1H), 7.50 (s, 1H), 7.37 (s, 1H), 7.33 (dd, *J* = 7.0, 4.2 Hz, 1H), 7.02 (dd, *J* = 8.9, 1.8 Hz, 1H), 4.45-4.32 (m, 1H), 4.15-4.04 (td, *J* = 11.3, 4.1 Hz, 1H), 3.83 (t, *J* = 6.7 Hz, 2H), 3.01 (t, *J* = 10.8 Hz, 2H), 2.82 - 2.65 (m, 8H), 2.38 - 1.55 (m, 14H), 1.18 - 1.06 (m, 2H), 0.58 (d, *J* = 6.4 Hz, 3H). | | |

| Example No. | Compound No. | Structure | Example No. | Compound No. | Structure |
|---|---|---|---|---|---|
| Example 28 | TM-28 | 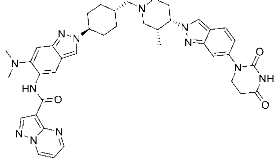 | Example 29 | TM-29 | 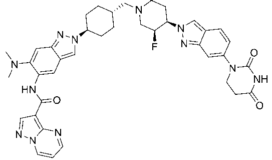 |
| Characterization | | | Characterization | | |
| LCMS (ESI) m/z: [M+1]=743.36. | | | LCMS (ESI) m/z: [M+1]=747.35. ¹H NMR (400 MHz, DMSO-*d*₆) δ 10.85 (s, 1H), 10.36 (s, 1H), 9.36 (d, *J* = 7.2 Hz, 1H), 8.94 (s, 1H), 8.72 (s, 1H), 8.70 (s, 1H), 8.44 (s, 1H), 8.29 (s, 1H), 7.69 (d, *J* = 8.9 Hz, 1H), 7.51 (s, 1H), 7.37 (s, 1H), 7.04 (d, *J* = 9.4 Hz, 1H), 5.12 (d, *J* = 50.0 Hz, 1H),4.85-4.69 (m, 1H), 4.46-4.31 (m, 1H), 3.84 (t, *J* = 6.6 Hz, 2H), 3.11-3.01 (m, 1H), 2.80-2.71 (m, 8H), 2.30 - 1.87 (m, 11H), 1.75-1.61 (m, 1H), 1.21-1.05 (m, 2H). | | |

| Example No. | Compound No. | Structure | Example No. | Compound No. | Structure |
|---|---|---|---|---|---|
| Example 30 | TM-30 | 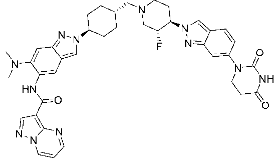 | Example 31 | TM-31 | 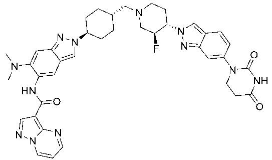 |
| Characterization | | | Characterization | | |
| LCMS (ESI) m/z: [M+1]=747.34. ¹H NMR (400 MHz, DMSO-*d*₆) δ 10.85 (s, 1H), 10.36 (s, 1H), 9.36 (dd, *J* = 7.0, 1.6 Hz, 1H), 8.95 (dd, *J* = 4.2, 1.7 Hz, 1H), 8.72 (s, 1H), 8.70 (s, 1H), 8.50 (s, 1H), 8.29 (s, 1H), 7.69 (d, *J* = 8.9 Hz, 1H), 7.52 (s, 1H), 7.37 (s, 1H), 7.33 (dd, *J* = 7.0, 4.2 Hz, 1H), 7.04 (dd, *J* = 8.9*,* 1.8 Hz, 1H), 5.27 - 4.92 (m, 1H), 4.75-4.62 (m, 1H), 4.44-4.35 (m, 1H), 3.84 (t, *J* = 6.7 Hz, 2H), 2.97 (d, *J* = 9.9 Hz, 1H), 2.77 (s, 6H), 2.74 (t, *J* = 6.7 Hz, 2H), 2.37 - 1.83 (m, 13H), 1.77-1.57 (m, 1H), 1.22 - 1.09 (m, 2H). | | | LCMS (ESI) m/z: [M+1]=747.30. ¹H NMR (400 MHz, DMSO-*d*₆) δ 10.85 (s, 1H), 10.36 (s, 1H), 9.37 (dd, *J* = 7.0, 1.7 Hz, 1H), 8.95 (dd, *J* = 4.2, 1.7 Hz, 1H), 8.72 (s, 1H), 8.70 (s, 1H), 8.51 (d, *J* = 1.0 Hz, 1H), 8.30 (d, *J* = 0.8 Hz, 1H), 7.76 - 7.63 (m, 1H), 7.52 (s, 1H), 7.37 (s, 1H), 7.33 (dd, *J* = 7.0*,* 4.2 Hz, 1H), 7.04 (dd, *J* = 8.9, 1.8 Hz, 1H), 5.08 (d, *J* = 52.1 Hz, 1H), 4.76-4.64 (m, 1H), 4.45-4.33 (m, 1H), 3.84 (t, *J* = 6.7 Hz, 2H), 2.97 (d, *J* = 10.0 Hz, 1H), 2.80-2.69 (m, 8H), 2.39-1.84 (m, 13H), 1.74-1.62 (m, 1H), 1.20-1.08 (m, 2H). | | |

| Example No. | Compound No. | Structure | Example No. | Compound No. | Structure |
|---|---|---|---|---|---|
| Example 32 | TM-32 | 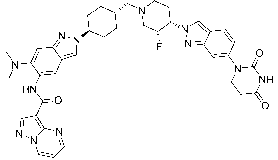 | Example 33 | TM-33 | 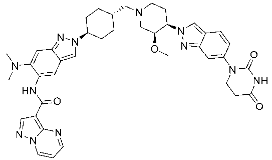 |
| Characterization | | | Characterization | | |
| LCMS (ESI) m/z: [M+1]=747.33. ¹H NMR (400 MHz, DMSO-*d*₆) δ 10.85 (s, 1H), 10.36 (s, 1H), 9.37 (dd, *J* = 7.0, 1.7 Hz, 1H), 8.95 (dd, *J* = 4.1, 1.6 Hz, 1H), 8.72 (s, 1H), 8.70 (s, 1H), 8.44 (s, 1H), 8.29 (s, 1H), 7.70 (d, *J* = 8.9 Hz, 1H), 7.51 (d, *J* = 1.7 Hz, 1H), 7.37 (s, 1H), 7.33 (dd, *J* = 7.0, 4.2 Hz, 1H), 7.04 (dd, *J* = 8.9*,* 1.7 Hz, 1H), 5.13 (d, *J* = 49.7 Hz, 1H), 4.87-4.68 (m, 1H), 4.46-4.34 (m, 1H), 3.84 (t, *J* = 6.7 Hz, 2H), 3.10 (s, 1H), 2.77 (s, 6H), 2.74 (t, *J* = 6.9 Hz, 2H),2.61-2.53 (m, 1H), 2.37-2.21 (m, 4H), 2.19-2.08 (m, 3H), 2.04-1.08 (m, 4H), 1.75-1.61 (m, 1H), 1.21-1.05 (m, 2H). | | | LCMS (ESI) m/z: [M+1]=759.38. ¹H NMR (400 MHz, DMSO-*d*₆) δ 10.85 (s, 1H), 10.36 (s, 1H), 9.37 (dd, *J* = 7.0, 1.7 Hz, 1H), 8.95 (dd, *J* = 4.2, 1.7 Hz, 1H), 8.72 (s, 1H), 8.70 (s, 1H), 8.37 (s, 1H), 8.30 (s, 1H), 7.69 (d, *J* = 9.2 Hz, 2H), 7.49 (s, 1H), 7.36 (s, 1H), 7.33 (dd, *J* = 7.0, 4.2 Hz, 1H), 7.02 (d, *J* = 8.8 Hz, 1H), 4.71-4.57 (m, 1H), 4.44-4.33 (m, 1H), 3.84 (t, *J* = 6.7 Hz, 2H), 3.09-2.99 (m, 4H), 2.77 (s, 6H), 2.73 (t, *J* = 6.7 Hz, 2H), 2.30 - 2.09 (m, 7H), 2.07 - 1.79 (m, 6H), 1.75-1.68 (m, 1H), 1.37 - 1.15 (m, 2H). | | |

| Example No. | Compound No. | Structure | Example No. | Compound No. | Structure |
|---|---|---|---|---|---|
| Example 34 | TM-34 | 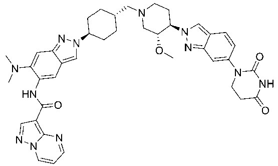 | Example 35 | TM-35 | 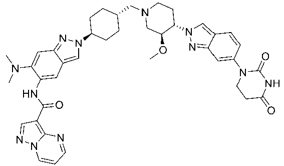 |
| Characterization | | | Characterization | | |
| LCMS (ESI) m/z: [M+1]=759.34. | | | LCMS (ESI) m/z: [M+1]=759.36. | | |
| Example 36 | TM-36 | 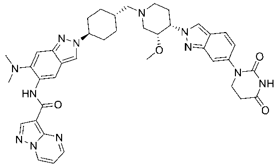 | Example 37 | TM-37 | 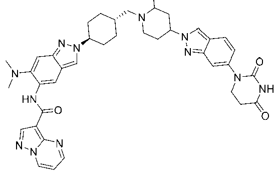 |
| Characterization | | | Characterization | | |
| LCMS (ESI) m/z: [M+1]=759.35. | | | LCMS (ESI) m/z: [M+1]=743.38. | | |

| Example No. | Compound No. | Structure | Example No. | Compound No. | Structure |
|---|---|---|---|---|---|
| Example 38 | TM-38 | 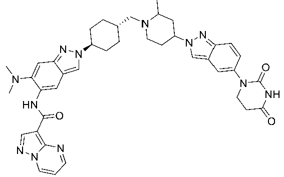 | Example 39 | TM-39 | 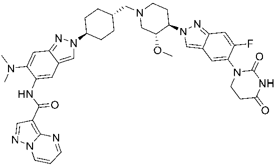 |
| Characterization | | | Characterization | | |
| LCMS (ESI) m/z: [M+1]=743.36. | | | LCMS (ESI) m/z: [M+1]=777.37. | | |

| Example No. | Compound No. | Structure | Example No. | Compound No. | Structure |
|---|---|---|---|---|---|
| Example 40 | TM-40 | 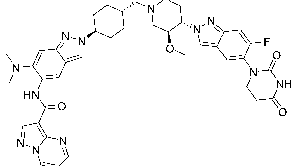 | Example 41 | TM-41 | 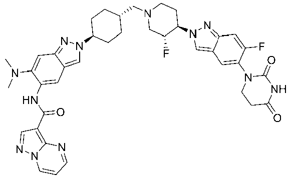 |
| Characterization | | | Characterization 348 | | |
| LCMS (ESI) m/z: [M+1]=777.35. | | | LCMS (ESI) m/z: [M+1]=765.72. ¹H NMR (400 MHz, DMSO-*d*₆) δ 10.85 (s, 1H), 10.47 (s, 1H), 9.37 (d, *J* = 7.0 Hz, 1H), 8.95 (d, *J* = 4.2 Hz, 1H), 8.72 (s, 1H), 8.70 (s, 1H), 8.62 (s, 1H), 8.30 (s, 1H), 7.83 (d, *J* = 7.6 Hz, 1H), 7.51 (d*, J =* 11.1 Hz, 1H), 7.37 (s, 1H), 7.33 (dd,*J* = 7.0, 4.2 Hz, 1H), 5.21-4.95 (m, 1H), 4.81-4.65 (m, 1H), 4.51-4.35 (m, 1H), 3.73 (t, *J* = 6.7 Hz, 2H), 3.02-2.91 (m, 1H), 2.81-2.70 (m, 8H), 2.37-1.84 (m, 13H), 1.78-1.57 (m, 1H), 1.20-1.15 (m, 2H). | | |

| Example No. | Compound No. | Structure | Example No. | Compound No. | Structure |
|---|---|---|---|---|---|
| Example 42 | TM-42 | 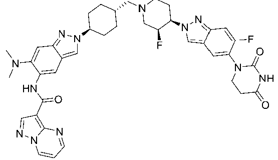 | Example 43 | TM-43 | 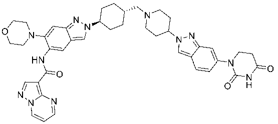 |
| Characterization | | | Characterization | | |
| LCMS (ESI) m/z: [M+1]=765.57. ¹H NMR (400 MHz, DMSO-*d*₆) δ 10.85 (s, 1H), 10.47 (s, 1H), 9.36 (dd, *J* = 7.0, 1.6 Hz, 1H), 8.94 (dd, *J* = 4.1, 1.6 Hz, 1H), 8.72 (s, 1H), 8.70 (s, 1H), 8.53 (s, 1H), 8.29 (s, 1H), 7.81 (d, *J* = 7.7 Hz, 1H), 7.48 (d, *J=* 11.2 Hz, 1H), 7.37 (s, 1H), 7.33 (dd, *J* = 7.0, 4.2 Hz, 1H), 5.11 (d*, J* = 49.9 Hz, 1H), 4.76 (dd, *J* = 30.4, 12.5 Hz, 1H), 4.49 - 4.27 (m, 1H), 3.73 (t, *J* = 6.7 Hz, 2H), 3.24 (t, *J* = 11.3 Hz, 1H), 3.07 (d, *J* = 11.2 Hz, 1H), 2.82-2.71 (m, 8H), 2.62 - 2.21 (m, 5H), 2.20 - 2.07 (m, 3H), 2.04-1.83 (m, 4H), 1.73-1.57 (m, 1H), 1.21 - 1.05 (m, 2H). | | | LCMS (ESI) m/z: [M+1]=771.74. ¹H NMR (400 MHz, DMSO-*d*₆) δ 10.67 (s, 1H), 10.35 (s, 1H), 9.38 (dd, *J* = 7.0, 1.6 Hz, 1H), 8.97 (dd, *J* = 4.2, 1.6 Hz, 1H), 8.75 (s, 1H), 8.72 (s, 1H), 8.44 (s, 1H), 8.32 (s, 1H), 7.67 (d, *J* = 8.9 Hz, 1H), 7.49 (s, 1H), 7.45 (s, 1H), 7.36 (dd, *J* = 7.0, 4.2 Hz, 1H), 7.01 (dd, *J* = 8.9, 1.8 Hz, 1H), 4.54-4.33 (m, 2H), 3.89 (t, *J* = 4.6 Hz, 4H), 3.83 (t, *J* = 6.7 Hz, 2H), 3.06-2.97 (m, 2H), 2.92 (t, *J* = 4.5 Hz, 4H), 2.73 (t, *J* = 6.6 Hz, 2H), 2.28-2.04 (m, 10H), 2.04-1.82 (m, 4H), 1.73-1.57 (m, 1H), 1.21-1.06 (m, 2H). | | |
| Example 44 | TM-44 | 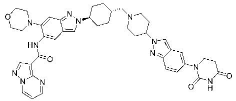 | Example 45 | TM-45 | 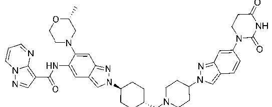 |
| Characterization | | | Characterization | | |
| LCMS (ESI) m/z: [M+1]=771.73. ¹H NMR (400 MHz, DMSO-*d*₆) δ 10.67 (s, 1H), 10.32 (s, 1H), 9.38 (dd, *J* = 7.0, 1.6 Hz, 1H), 8.97 (dd, *J* = 4.2, 1.7 Hz, 1H), 8.75 (s, 1H), 8.72 (s, 1H), 8.45 (d, *J* = 0.9 Hz, 1H), 8.32 (d, *J* = 0.9 Hz, 1H), 7.63 - 7.53 (m, 2H), 7.45 (s, 1H), 7.36 (dd, *J* = 7.0, 4.2 Hz, 1H), 7.19 (dd, *J* = 9.1, 2.1 Hz, 1H), 4.53-4.33 (m, 2H), 3.89 (dd, *J* = 5.8, 4H), 3.79 (t, *J* = 6.7 Hz, 2H), 3.01 (br, 2H), 2.92 (t, *J* = 4.6 Hz, 4H), 2.73 (t, *J* = 6.7 Hz, 2H), 2.32 - 2.04 (m, 10H), 2.03-1.85 (m, 4H), 1.73-1.62 (m, 1H), 1.19-1.06 (m, 2H). | | | LCMS (ESI) m/z: [M+1]=785.91. ¹H NMR (400 MHz, DMSO-*d*₆) δ 10.67 (s, 1H), 10.36 (s, 1H), 9.38 (dd, *J* = 7.0, 1.7 Hz, 1H), 8.94 (dd, *J* = 4.2, 1.6 Hz, 1H), 8.76 (s, 1H), 8.72 (s, 1H), 8.44 (s, 1H), 8.33 (s, 1H), 7.67 (d, *J* = 8.9 Hz, 1H), 7.49 (s, 1H), 7.43 (s, 1H), 7.36 (dd, *J* = 7.0, 4.2 Hz, 1H), 7.02 (dd, *J* = 8.8, 1.8 Hz, 1H), 4.54-4.34 (m, 2H), 4.03 - 3.85 (m, 3H), 3.83 (t, *J* = 6.7 Hz, 2H), 3.06 - 2.77 (m, 5H), 2.73 (t*, J* = 6.7 Hz, 2H), 2.56 (t*, J* = 10.7 Hz, 1H), 2.30 - 2.07 (m, 10H), 2.04-1.85 (m, 4H), 1.73-1.60 (m, 1H), 1.21-1.08 (m, 5H). | | |

| Example No. | Compound No. | Structure | Example No. | Compound No. | Structure |
|---|---|---|---|---|---|
| Example 46 | TM-46 | 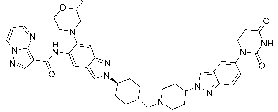 | Example 47 | TM-47 | 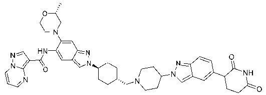 |
| Characterization | | | Characterization | | |
| LCMS (ESI) m/z: [M+1]=785.23. ¹H NMR (400 MHz, DMSO-*d*₆) δ 10.66 (s, 1H), 10.32 (s, 1H), 9.38 (dd, *J* = 7.0, 1.6 Hz, 1H), 8.94 (dd, *J* = 4.2, 1.6 Hz, 1H), 8.76 (s, 1H), 8.72 (s, 1H), 8.45 (s, 1H), 8.32 (s, 1H), 7.65 - 7.55 (m, 2H), 7.43 (s, 1H), 7.36 (dd, *J* = 7.0, 4.2 Hz, 1H), 7.19 (dd, *J* = 9.1, 2.1 Hz, 1H), 4.56-4.35 (m, 2H), 4.04-3.85 (m, 3H), 3.79 (t, *J =* 6.7 Hz, 2H), 3.10 - 2.79 (m, 5H), 2.73 (t*, J* = 6.7 Hz, 2H), 2.56 (t, *J* = 10.9 Hz, 1H), 2.30-2.04 (m, 10H), 2.03-1.85 (m, 4H), 1.72-1.61 (m, 1H), 1.18-1.08 (m, 5H). | | | LCMS (ESI) m/z: [M+1]=784.47. ¹H NMR (400 MHz, DMSO-*d*₆) δ 10.84 (s, 1H), 10.67 (s, 1H), 9.38 (dd, *J* = 7.0, 1.6 Hz, 1H), 8.94 (dd, *J* = 4.2, 1.7 Hz, 1H), 8.76 (s, 1H), 8.72 (s, 1H), 8.39 (s, 1H), 8.33 (s, 1H), 7.55 (d, *J* = 9.0 Hz, 1H), 7.48 (s, 1H), 7.43 (s, 1H), 7.35 (dd, J = 7.0, 4.2 Hz, 1H), 7.08 (dd, *J* = 9.0, 1.7 Hz, 1H), 4.53-4.35 (m, 2H), 4.10 - 3.77 (m, 4H), 3.12 - 2.76 (m, 7H), 2.73-2.62 (m, 2H), 2.60 - 2.53 (m, 1H), 2.29 - 2.06 (m, 10H), 2.02-1.86 (m, 4H), 1.72-1.59 (m, 1H), 1.21 - 1.00 (m, 5H). | | |

| Example No. | Compound No. | Structure | Example No. | Compound No. | Structure |
|---|---|---|---|---|---|
| Example 48 | TM-48 | 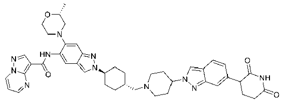 | Example 49 | TM-49 | 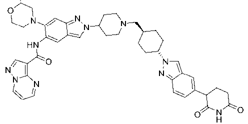 |
| Characterization | | | Characterization | | |
| LCMS (ESI) m/z: [M+1]=784.86. ¹H NMR (400 MHz, DMSO-*d*₆) δ 10.85 (s, 1H), 10.67 (s, 1H), 9.38 (dd, *J* = 7.0, 1.7 Hz, 1H), 8.94 (dd, *J* = 4.2, 1.6 Hz, 1H), 8.76 (s, 1H), 8.72 (s, 1H), 8.40 (d, *J* = 1.0 Hz, 1H), 8.33 (s, 1H), 7.67 - 7.57 (m, 1H), 7.43 (s, 1H), 7.42 (s, 1H), 7.36 (dd, *J* = 7.0, 4.2 Hz, 1H), 6.88 (dd, *J* = 8.7, 1.4 Hz, 1H), 4.52-4.33 (m, 2H), 4.05 - 3.84 (m, 4H), 3.09 - 2.75 (m, 7H), 2.74 - 2.63 (m, 2H),2.60-2.53 (m, 1H), 2.29 - 2.06 (m, 10H), 2.02-1.86 (m, 4H), 1.75-1.60 (m, 1H), 1.21-1.07 (m, 5H). | | | LCMS (ESI) m/z: [M+1] = 784.5. ¹H NMR (400 MHz, DMSO-d6) δ 10.89 (s, 1H), 10.73 (s, 1H), 9.44 (dd, J = 7.0, 1.7 Hz, 1H), 9.00 (dd, J = 4.2, 1.7 Hz, 1H), 8.84 (s, 1H), 8.79 (s, 1H), 8.43 (s, 1H), 8.40 (d, J = 0.9 Hz, 1H), 7.62 (d, J = 8.9 Hz, 1H), 7.55 (d, J = 1.4 Hz, 1H), 7.50 (s, 1H), 7.42 (dd, J = 7.0, 4.2 Hz, 1H), 7.14 (dd, J = 9.0, 1.7 Hz, 1H), 4.57 - 4.41 (m, 2H), 4.11 - 3.92 (m, 4H), 3.04 (d, J = 11.1 Hz, 3H), 2.97 (d, J = 11.3 Hz, 1H), 2.88 (td, J = 11.3, 3.4 Hz, 1H), 2.74 (ddd, J = 16.9, 11.5, 5.2 Hz, 1H), 2.68 - 2.58 (m, 2H), 2.36 - 2.25 (m, 3H), 2.23 - 2.10 (m, 9H), 2.08 - 1.94 (m, 4H), 1.72 (s, 1H), 1.19 (t, J = 7.6 Hz, 5H). | | |
| Example 50 | TM-50 | 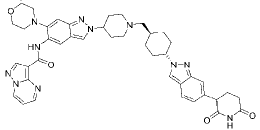 | Example 51 | TM-51 | 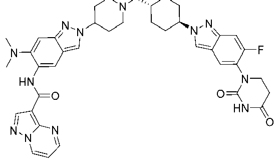 |
| Characterization | | | Characterization | | |
| LCMS (ESI) m/z: [M+1] = 784.4. ¹H NMR (400 MHz, DMSO-d6) δ 10.85 (s, 1H), 10.67 (s, 1H), 9.38 (dd, J = 7.0, 1.6 Hz, 1H), 8.94 (dd, J = 4.2, 1.7 Hz, 1H), 8.78 (s, 1H), 8.73 (s, 1H), 8.37 (d, J = 0.8 Hz, 1H), 8.35 (d, J = 0.9 Hz, 1H), 7.63 (dd, J = 8.6, 0.8 Hz, 1H), 7.44 (s, 1H), 7.42 (s, 1H), 7.36 (dd, J = 7.0, 4.2 Hz, 1H), 6.88 (dd, J = 8.7, 1.5 Hz, 1H), 4.51 - 4.36 (m, 2H), 4.06 - 3.83 (m, 4H), 2.98 (d, J = 11.1 Hz, 3H), 2.91 (d, J = 10.5 Hz, 1H), 2.82 (td, J = 11.3, 3.4 Hz, 1H), 2.69 (ddd, J = 17.0, 11.7, 5.3 Hz, 1H), 2.62 - 2.52 (m, 2H), 2.34 - 2.20 (m, 3H), 2.12 (q, J = 10.6, 7.6 Hz, 9H), 2.02 - 1.87 (m, 4H), 1.66 (s, 1H), 1.12 (d, J = 6.2 Hz, 5H). | | | LCMS (ESI) m/z: [M+1]=747.36. | | |

| Example No. | Compound No. | Structure | Example No. | Compound No. | Structure |
|---|---|---|---|---|---|
| Example 52 | TM-52 | 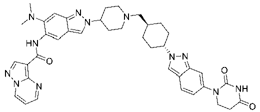 | Example 53 | TM-53 | 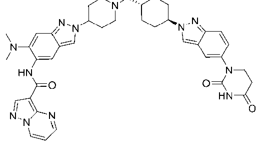 |
| Characterization | | | Characterization | | |
| LCMS (ESI) m/z: [M+1]=729.59. ¹H NMR (400 MHz, DMSO-*d*₆) δ 10.85 (s, 1H), 10.35 (s, 1H), 9.36 (dd, *J* = 7.0, 1.6 Hz, 1H), 8.95 (dd, *J* = 4.2, 1.6 Hz, 1H), 8.72 (s, 1H), 8.70 (s, 1H), 8.40 (d, *J* = 1.0 Hz, 1H), 8.33 (s, 1H), 7.67 (d, *J* = 8.9 Hz, 1H), 7.49 (d, *J =* 1.7 Hz, 1H), 7.37 (s, 1H), 7.33 (dd, *J* = 7.0, 4.2 Hz, 1H), 7.01 (dd, *J* = 8.9, 1.8 Hz, 1H), 4.54-4.33 (m, 2H), 3.83 (t, *J* = 6.7 Hz, 2H), 3.09-2.94 (m, 2H), 2.77 (s, 6H), 2.73 (t, *J* = 6.7 Hz, 2H), 2.28 - 2.05 (m, 10H),2.05-1.87 (m, 4H), 1.74-1.59 (m, 1H), 1.21-1.07 (m, 2H). | | | LCMS (ESI) m/z: [M+1]=729.75. ¹H NMR (400 MHz, DMSO-*d*₆) δ 10.85 (s, 1H), 10.32 (s, 1H), 9.36 (dd, *J* = 7.0, 1.6 Hz, 1H), 8.95 (dd, *J* = 4.2, 1.7 Hz, 1H), 8.72 (s, 1H), 8.70 (s, 1H), 8.41 (s, 1H), 8.33 (d, *J* = 0.9 Hz, 1H), 7.70 - 7.52 (m, 2H), 7.37 (s, 1H), 7.33 (dd, *J* = 7.0, 4.2 Hz, 1H), 7.18 (dd, *J* = 8.9, 2.2 Hz, 1H), 4.52-4.34 (m, 2H), 3.79 (t, *J* = 6.7 Hz, 2H), 3.04-2.93 (m, 2H), 2.77 (s, 6H), 2.73 (t, *J* = 6.7 Hz, 2H), 2.27 - 2.05 (m, 10H), 2.05 - 1.82 (m, 4H), 1.74-1.60 (m, 1H), 1.22-1.06 (m, 2H). | | |

| Example No. | Compound No. | Structure | Example No. | Compound No. | Structure |
|---|---|---|---|---|---|
| Example 54 | TM-54 | 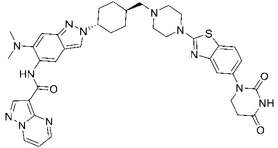 | Example 55 | TM-55 | 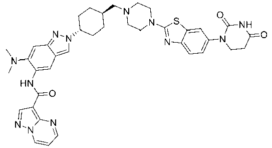 |
| Characterization | | | Characterization | | |
| LCMS (ESI) m/z: [M+1]=747.18. ¹H NMR (400 MHz, DMSO-*d*₆) δ 10.85 (s, 1H), 10.35 (s, 1H), 9.36 (dd, *J* = 7.0, 1.7 Hz, 1H), 8.95 (dd, *J* = 4.2, 1.7 Hz, 1H), 8.72 (s, 1H), 8.70 (s, 1H), 8.29 (s, 1H), 7.75 (d, *J* = 8.4 Hz, 1H), 7.41 (d*, J* = 2.1 Hz, 1H), 7.37 (s, 1H), 7.33 (dd, *J* = 7.0, 4.2 Hz, 1H), 7.05 (dd, *J* = 8.4, 2.1 Hz, 1H), 4.44-4.30 (m, 1H), 3.80 (t, *J* = 6.7 Hz, 2H), 3.57 (d, *J =* 5.6 Hz, 4H), 2.77 (s, 6H), 2.72 (t, *J* = 6.7 Hz, 2H), 2.23 (d, *J* = 7.1 Hz, 2H), 2.02-1.84 (m, 4H), 1.73-1.60 (m, 1H), 1.21-1.06 (m, 2H). | | | LCMS (ESI) m/z: [M+1]=747.33. ¹H NMR (400 MHz, DMSO-*d*₆) δ 10.85 (s, 1H), 10.34 (s, 1H), 9.35 (dd, *J* = 7.0, 1.7 Hz, 1H), 8.94 (dd, *J* = 4.2, 1.6 Hz, 1H), 8.71 (s, 1H), 8.70 (s, 1H), 8.29 (s, 1H), 7.74 (d, *J* = 2.3 Hz, 1H), 7.44 (d, *J* = 8.6 Hz, 1H), 7.37 (s, 1H), 7.33 (dd, *J* = 7.0, 4.2 Hz, 1H), 7.23 (dd, *J* = 8.5*,* 2.2 Hz, 1H), 4.46 - 4.27 (m, 1H), 3.77 (t, *J* = 6.7 Hz, 2H), 3.57 (t*, J* = 5.7 Hz, 4H), 2.76 (s, 6H), 2.71 (t, *J* = 6.7 Hz, 2H), 2.23 (d, *J* = 7.0 Hz, 2H), 2.14 (d, *J* = 12.1 Hz, 2H), 2.03-1.84 (m, 4H), 1.73-1.58 (m, 1H), 1.21-1.06 (m, 2H). | | |
| Example 56 | TM-56 | 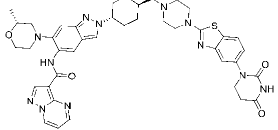 | Example 57 | TM-57 | 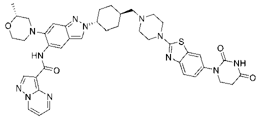 |
| Characterization | | | Characterization | | |
| LCMS (ESI) m/z: [M+1]= 803.40. ¹H NMR (400 MHz, DMSO-*d*₆) δ 10.66 (s, 1H), 10.35 (s, 1H), 9.37 (dd, *J* = 7.0, 1.6 Hz, 1H), 8.93 (dd, *J* = 4.3, 1.7 Hz, 1H), 8.76 (s, 1H), 8.72 (s, 1H), 8.32 (s, 1H), 7.75 (d, *J* = 8.4 Hz, 1H), 7.43 (s, 1H), 7.41 (d, *J* = 2.1 Hz, 1H), 7.35 (dd, *J* = 7.0, 4.2 Hz, 1H), 7.05 (dd, *J* = 8.4, 2.1 Hz, 1H), 4.45-4.33 (m, 1H), 4.09 - 3.86 (m, 3H), 3.80 (t, *J* = 6.7 Hz, 2H), 3.66-3.54 (m, 4H), 2.98 (d, *J* = 11.1 Hz, 1H), 2.93 - 2.76 (m, 2H), 2.72 (t, *J* = 6.6 Hz, 2H), 2.56 (t, *J* = 10.7 Hz, 1H), 2.23 (d, *J* = 7.0 Hz, 2H), 2.14 (d, *J* = 11.6 Hz, 2H), 2.03-1.84 (m, 4H), 1.74-1.60 (m, 1H), 1.20-1.07 (m, 5H). | | | LCMS (ESI) m/z: [M+1]=803.76. ¹H NMR (400 MHz, DMSO-*d*₆) δ 10.65 (s, 1H), 10.32 (s, 1H), 9.37 (dd, *J* = 7.0, 1.6 Hz, 1H), 8.93 (dd, *J* = 4.2, 1.7 Hz, 1H), 8.75 (s, 1H), 8.71 (s, 1H), 8.29 (s, 1H), 7.72 (d, *J* = 2.1 Hz, 1H), 7.48 - 7.41 (m, 2H), 7.35 (dd, *J* = 7.0, 4.2 Hz, 1H), 7.21 (dd, *J* = 8.6, 2.2 Hz, 1H), 4.42 - 4.29 (m, 1H), 4.08 - 3.85 (m, 3H), 3.80-3.70 (m, 4H), 3.05 - 2.75 (m, 6H), 2.68 (t, *J* = 6.7 Hz, 2H), 2.58 (t, *J* = 10.7 Hz, 3H), 2.26 (d, *J* = 7.1 Hz, 2H), 2.17 - 2.03 (m, 2H), 1.99 - 1.81 (m, 4H), 1.64-1.49 (m, 1H), 1.17-1.01 (m, 5H). | | |

| Example No. | Compound No. | Structure | Example No. | Compound No. | Structure |
|---|---|---|---|---|---|
| Example 58 | TM-58 | 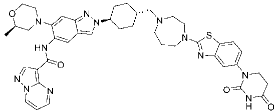 | Example 59 | TM-59 | 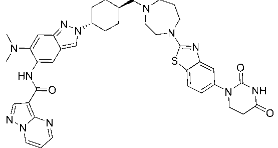 |
| Characterization | | | Characterization | | |
| LCMS (ESI) m/z: [M+1]=817.22. ¹H NMR (400 MHz, DMSO-*d*₆) δ 10.66 (s, 1H), 10.33 (s, 1H), 9.37 (dd, *J* = 7.0, 1.6 Hz, 1H), 8.93 (dd, *J* = 4.2, 1.7 Hz, 1H), 8.76 (s, 1H), 8.72 (s, 1H), 8.30 (s, 1H), 7.71 (d, *J* = 8.3 Hz, 1H), 7.47 - 7.34 (m, 2H), 7.35 (dd, *J* = 7.0, 4.2 Hz, 1H), 6.99 (dd, *J* = 8.4, 2.1 Hz, 1H), 4.41-4.30 (m, 1H), 4.10 - 3.83 (m, 3H), 3.84 - 3.54 (m, 6H), 3.15 - 2.77 (m, 5H), 2.77 - 2.53 (m, 5H), 2.32 (d, *J* = 7.0 Hz, 2H), 2.24 - 2.05 (m, 2H), 2.0-1.77 (m, 6H), 1.64-1.51 (m, 1H), 1.09-1.01 (m, 5H). | | | LCMS (ESI) m/z: [M+1]=761.69. ¹H NMR (400 MHz, DMSO-*d*₆) δ 10.85 (s, 1H), 10.33 (s, 1H), 9.36 (dd, *J* = 7.0, 1.7 Hz, 1H), 8.94 (dd, *J* = 4.1, 1.6 Hz, 1H), 8.71 (s, 1H), 8.70 (s, 1H), 8.27 (d, *J* = 0.9 Hz, 1H), 7.72 (d, *J* = 8.5 Hz, 1H), 7.44 - 7.32 (m, 2H), 7.33 (dd, *J* = 7.0, 4.2 Hz, 1H), 6.99 (dd, *J* = 8.4*,* 2.1 Hz, 1H), 4.39-4.29 (m, 1H), 3.89 - 3.59 (m, 6H), 2.88-2.79 (br, 2H), 2.76 (s, 6H), 2.67-2.60 (m, 2H), 2.63 (d, *J* = 6.5 Hz, 2H), 2.32 (d, *J* = 6.8 Hz, 2H), 2.22 - 2.04 (m, 2H), 1.99-1.79 (m, 6H), 1.66-1.49 (m, 1H), 1.16-1.01 (m, 2H). | | |

| Example No. | Compound No. | Structure | Example No. | Compound No. | Structure |
|---|---|---|---|---|---|
| Example 60 | TM-60 | 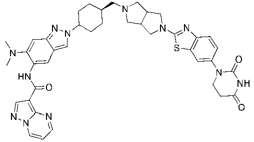 | Example 61 | TM-61 | 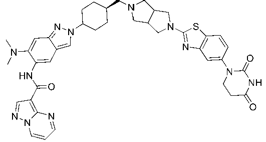 |
| Characterization | | | Characterization | | |
| LCMS (ESI) m/z: [M+1]=773.49. ¹H NMR (400 MHz, DMSO-*d*₆) δ 10.84 (s, 1H), 10.32 (s, 1H), 9.36 (dd, *J* = 7.0, 1.7 Hz, 1H), 8.94 (dd, *J* = 4.3, 1.7 Hz, 1H), 8.70 (d, *J* = 1.5 Hz, 2H), 8.26 (s, 1H), 7.72 (d, *J* = 2.2 Hz, 1H), 7.42 (s, 1H), 7.35 (s, 1H), 7.33 (dd, *J* = 7.0, 4.2 Hz, 1H), 7.21 (dd, *J* = 8.6, 2.2 Hz, 1H), 4.39-4.28 (m, 1H), 3.88 - 3.66 (m, 4H), 2.97 (s, 2H), 2.76 (s, 6H), 2.68 (t, *J* = 6.6 Hz, 2H), 2.60 (d, *J* = 9.1 Hz, 2H), 2.26 (d, *J* = 7.1 Hz, 2H), 2.11 - 2.01 (m, 2H), 1.88 (dt, *J* = 25.8, 11.7 Hz, 4H), 1.61-1.47 (m, 1H), 1.14-1.02 (m, 2H). | | | LCMS (ESI) m/z: [M+1]=773.71. ¹H NMR (400 MHz, DMSO-*d*₆) δ 10.84 (s, 1H), 10.33 (s, 1H), 9.36 (dd, *J* = 7.0, 1.7 Hz, 1H), 8.94 (dd, *J* = 4.2, 1.7 Hz, 1H), 8.70 (d, *J* = 1.3 Hz, 2H), 8.26 (d, *J=* 0.9 Hz, 1H), 7.73 (d, *J* = 8.4 Hz, 1H), 7.40 (d, *J* = 2.1 Hz, 1H), 7.35 (s, 1H), 7.33 (dd, *J* = 7.0, 4.2 Hz, 1H), 7.01 (dd, *J* = 8.4*,* 2.1 Hz, 1H), 4.40-4.26 (m, 1H), 3.76 (t, *J* = 6.6 Hz, 2H), 2.97 (s, 2H), 2.76 (s, 6H), 2.68 (t, *J* = 6.5 Hz, 2H), 2.60 (d, *J* = 9.0 Hz, 2H), 2.26 (d, *J* = 7.1 Hz, 2H), 2.12-2.03 (m, 2H), 1.97-1.78 (m, 4H), 1.63-1.47 (m, 1H), 1.16 - 1.00 (m, 2H). | | |
| Example 62 | TM-62 | 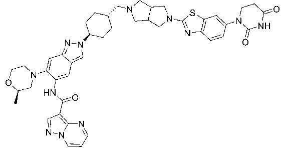 | Example 63 | TM-63 | 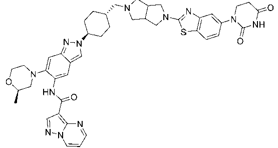 |
| Characterization | | | Characterization | | |
| LCMS (ESI) m/z: [M+1]=829.17. ¹H NMR (400 MHz, DMSO-*d*₆) δ 10.66 (s, 1H), 10.34 (s, 1H), 9.38 (dd, *J* = 7.0, 1.7 Hz, 1H), 8.94 (dd, *J* = 4.3, 1.7 Hz, 1H), 8.76 (s, 1H), 8.72 (s, 1H), 8.32 (s, 1H), 7.75 (d, *J* = 2.2 Hz, 1H), 7.47-7.42 (m, 2H), 7.36 (dd, *J* = 7.0*,* 4.2 Hz, 1H), 7.23 (dd, *J* = 8.6, 2.2 Hz, 1H), 4.47-4.33 (m, 1H), 4.08 - 3.85 (m, 3H), 3.77 (t, *J* = 6.7 Hz, 2H), 3.63-3.53 (m, 4H), 2.98 (d, *J* = 11.2 Hz, 1H), 2.91 (d, *J* = 11.2 Hz, 1H), 2.86-2.78 (m, 1H), 2.71 (t, *J* = 6.6 Hz, 2H), 2.56 (t, *J* = 10.8 Hz, 2H), 2.23 (d, *J* = 7.1 Hz, 2H), 2.14 (d, *J =* 11.8 Hz, 2H), 2.03-1.86 (m, 4H), 1.73-1.61 (m, 1H), 1.21-1.08 (m, 5H). | | | LCMS (ESI) m/z: [M+1]=829.89. ¹H NMR (400 MHz, DMSO-*d*₆) δ 10.65 (s, 1H), 10.33 (s, 1H), 9.37 (dd, *J* = 6.9, 1.6 Hz, 1H), 8.93 (dd, *J* = 4.2, 1.7 Hz, 1H), 8.74 (s, 1H), 8.71 (s, 1H), 8.29 (s, 1H), 7.73 (d, *J* = 8.4 Hz, 1H), 7.44 - 7.38 (m, 2H), 7.35 (dd, *J* = 7.0, 4.2 Hz, 1H), 7.01 (dd, *J =* 8.4, 2.1 Hz, 1H), 4.41-4.30 (m, 1H), 4.05-3.84 (m, 3H), 3.76 (t, *J =* 6.7 Hz, 4H), 3.03 - 2.78 (m, 6H), 2.68 (t, *J* = 6.6 Hz, 2H), 2.58 (t, *J* = 10.6 Hz, 3H), 2.26 (d, *J* = 7.1 Hz, 2H), 2.07 (d, *J* = 11.5 Hz, 2H), 2.00 - 1.80 (m, 4H), 1.64-1.44 (m, 1H), 1.17 - 0.98 (m, 5H). | | |
| | | | | | |

| Example No. | Compound No. | Structure | Example No. | Compound No. | Structure |
|---|---|---|---|---|---|
| Example 64 | TM-64 | 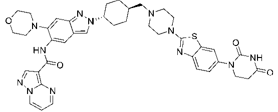 | Example 65 | TM-65 | 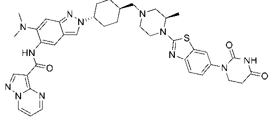 |
| Characterization | | | Characterization | | |
| LCMS (ESI) m/z: [M+1]=789.33. | | | LCMS (ESI) m/z: [M+1]=761.12. | | |

| Example No. | Compound No. | Structure | Example No. | Compound No. | Structure |
|---|---|---|---|---|---|
| Example 66 | TM-66 | 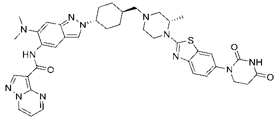 | Example 67 | TM-67 | 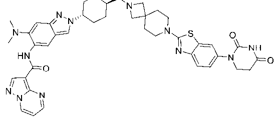 |
| Characterization | | | Characterization | | |
| LCMS (ESI) m/z: [M+1]=761.30. ¹H NMR (400 MHz, Chloroform-d) δ 10.83 (s, 1H), 8.86 (s, 1H), 8.82 (dd, *J* = 7.1, 1.8 Hz, 1H), 8.80 (s, 1H), 8.72 (dd, *J* = 4.1, 1.8 Hz, 1H), 7.88 (s, 1H), 7.61 - 7.50 (m, 2H), 7.45 (s, 1H), 7.17 (dd, *J* = 8.5, 2.3 Hz, 1H), 7.03 (dd, *J* = 7.0, 4.1 Hz, 1H), 4.42-4.30 (m, 1H), 4.30-4.16 (m, 1H), 3.87 (t, *J* = 6.6 Hz, 2H), 3.49 (br, 7H), 2.95-2.69 (m, 8H), 2.39 - 1.93 (m, 8H), 1.74-1.62 (m, 1H), 1.42 (d, *J* = 6.6 Hz, 3H), 1.25-1.09 (m, 2H), 1.03-0.92 (m, 2H). | | | LCMS (ESI) m/z: [M+1]=787.23. ¹H NMR (400 MHz, DMSO-*d*₆) δ 10.85 (s, 1H), 10.34 (s, 1H), 9.36 (dd, *J* = 7.1, 1.7 Hz, 1H), 8.94 (dd, *J* = 4.2, 1.7 Hz, 1H), 8.71 (s, 1H), 8.70 (s, 1H), 8.28 (s, 1H), 7.72 (d, *J* = 2.2 Hz, 1H), 7.41 (d, *J* = 8.6 Hz, 1H), 7.36 (s, 1H), 7.33 (dd, *J* = 7.0, 4.2 Hz, 1H), 7.21 (dd, *J* = 8.6, 2.2 Hz, 1H), 4.39-4.28 (m, 1H), 3.76 (t, *J =* 6.6 Hz, 2H), 3.58-3.51 (m, 4H), 3.00 (s, 4H), 2.76 (s, 6H), 2.71 (t, *J* = 6.6 Hz, 2H), 2.15 - 2.05 (m, 3H), 1.97-1.72 (m, 8H), 1.65-1.59 (m, 1H), 1.20-1.08 (m, 2H). | | |

| Example No. | Compound No. | Structure | Example No. | Compound No. | Structure |
|---|---|---|---|---|---|
| Example 68 | TM-68 | 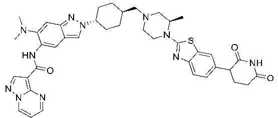 | Example 69 | TM-69 | 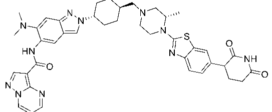 |
| Characterization | | | Characterization | | |
| LCMS (ESI) m/z: [M+1]=760.24. | | | LCMS (ESI) m/z: [M+1]=760.20. | | |
| Example 70 | TM-70 | 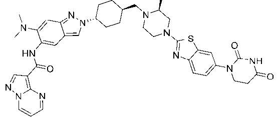 | Example 71 | TM-71 | 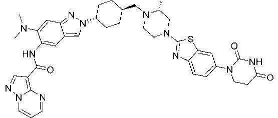 |
| Characterization | | | Characterization | | |
| LCMS (ESI) m/z: [M+1]=761.22. | | | LCMS (ESI) m/z: [M+1]=761.13. | | |

| Example No. | Compound No. | Structure | Example No. | Compound No. | Structure |
|---|---|---|---|---|---|
| Example 72 | TM-72 | 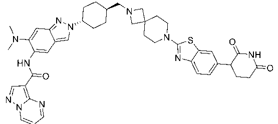 | Example 73 | TM-73 | 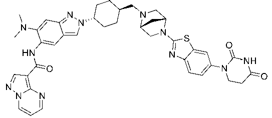 |
| Characterization | | | Characterization | | |
| LCMS (ESI) m/z: [M+1]=786.14. | | | LCMS (ESI) m/z: [M+1]=759.22. | | |

| Example No. | Compound No. | Structure | Example No. | Compound No. | Structure |
|---|---|---|---|---|---|
| Example 74 | TM-74 | 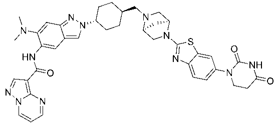 | Example 75 | TM-75 | 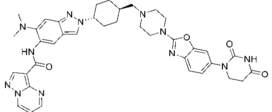 |
| Characterization | | | Characterization | | |
| LCMS (ESI) m/z: [M+1]=759.12. ¹H NMR (400 MHz, DMSO-*d*₆) δ 10.84 (s, 1H), 10.34 (s, 1H), 9.36 (dd, *J* = 7.0, 1.7 Hz, 1H), 8.94 (dd, *J* = 4.2, 1.7 Hz, 1H), 8.71 (s, 1H), 8.70 (s, 1H), 8.27 (d, *J* = 0.8 Hz, 1H), 7.73 (d, *J* = 2.3 Hz, 1H), 7.44 (d, *J* = 8.6 Hz, 1H), 7.35 (s, 1H), 7.33 (dd, *J* = 7.0, 4.2 Hz, 1H), 7.22 (dd, *J* = 8.6, 2.2 Hz, 1H), 4.51-4.31 (m, 2H), 3.76 (t, *J* = 6.7 Hz, 2H), 3.67-3.52 (m, 2H), 3.46 (d, *J* = 8.6 Hz, 1H), 3.01 (d, *J* = 9.0 Hz, 1H), 2.76 (s, 6H), 2.71 (t, *J* = 6.7 Hz, 2H), 2.59 (d, *J* = 9.2 Hz, 1H), 2.41 (d, *J* = 6.8 Hz, 2H), 2.16-2.04 (m, 1H), 2.02-1.79 (m, 6H), 1.49-1.39 (m, 1H), 1.18 - 0.97 (m, 2H). | | | LCMS (ESI) m/z: [M+1]=731.12. ¹H NMR (400 MHz, DMSO-*d*₆) δ 10.85 (s, 1H), 10.34 (s, 1H), 9.36 (d, *J* = 6.9 Hz, 1H), 8.95 (d, *J* = 4.2 Hz, 1H), 8.71 (s, 1H), 8.70 (s, 1H), 8.29 (s, 1H), 7.42 (s, 1H), 7.37 (s, 1H), 7.35 - 7.29 (m, 1H), 7.27 (d, *J* = *8.3* Hz, 1H), 7.11 (d, *J* = 8.9 Hz, 1H), 4.45-4.31 (m, 1H), 3.76 (t, *J* = 6.7 Hz, 2H), 3.68-3.57 (m, 4H), 2.80-2.68 (m, 8H), 2.23 (d, *J* = 6.7 Hz, 2H), 2.14 (d, *J* = 11.7 Hz, 2H), 2.02-1.68 (m, 4H), 1.74-1.61 (m, 1H), 1.22-1.60 (m, 2H). | | |

| Example No. | Compound No. | Structure | Example No. | Compound No. | Structure |
|---|---|---|---|---|---|
| Example 76 | TM-76 | 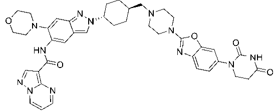 | Example 77 | TM-77 | 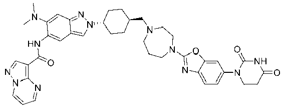 |
| Characterization | | | Characterization | | |
| LCMS (ESI) m/z: [M+1]=773.12. | | | LCMS (ESI) m/z: [M+1]=745.18. | | |

| Example No. | Compound No. | Structure | Example No. | Compound No. | Structure |
|---|---|---|---|---|---|
| Example 78 | TM-78 | 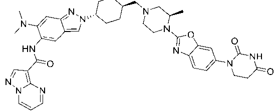 | Example 79 | TM-79 | 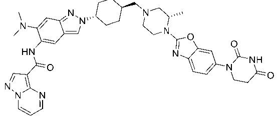 |
| Characterization | | | Characterization | | |
| LCMS (ESI) m/z: [M+1]=745.26. | | | LCMS (ESI) m/z: [M+1]=745.18. | | |

| Example No. | Compound No. | Structure | Example No. | Compound No. | Structure |
|---|---|---|---|---|---|
| Example 80 | TM-80 | 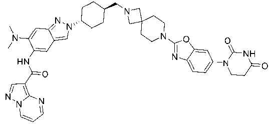 | Example 81 | TM-81 | 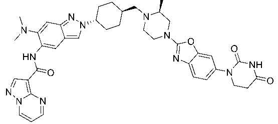 |
| Characterization | | | Characterization | | |
| LCMS (ESI) m/z: [M+1]=771.28. | | | LCMS (ESI) m/z: [M+1]=745.14. | | |

| Example No. | Compound No. | Structure | Example No. | Compound No. | Structure |
|---|---|---|---|---|---|
| Example 82 | TM-82 | 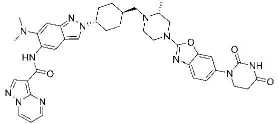 | Example 83 | TM-83 | 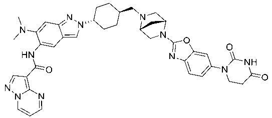 |
| Characterization | | | Characterization | | |
| LCMS (ESI) m/z: [M+1]=745.16. | | | LCMS (ESI) m/z: [M+1]=743.16. | | |

| Example No. | Compound No. | Structure | Example No. | Compound No. | Structure |
|---|---|---|---|---|---|
| Example 84 | TM-84 | 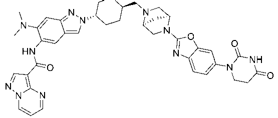 | Example 85 | TM-85 | 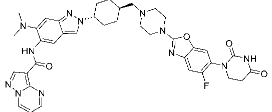 |
| Characterization | | | Characterization | | |
| LCMS (ESI) m/z: [M+1]=743.18. | | | LCMS (ESI) m/z: [M+1]=749.23. ¹H NMR (400 MHz, DMSO-*d*₆) δ 10.85 (s, 1H), 10.45 (s, 1H), 9.36 (dd, *J* = 7.0, 1.7 Hz, 1H), 8.95 (dd, *J* = 4.3, 1.7 Hz, 1H), 8.71 (s, 1H), 8.70 (s, 1H), 8.43 (s, 2H), 8.29 (s, 1H), 7.53 (d, *J* = 6.6 Hz, 1H), 7.37 (s, 1H), 7.33 (dd, *J* = 7.0, 4.2 Hz, 1H), 7.23 (d, *J* = 10.2 Hz, 1H), 4.43-4.33 (m, 1H), 3.79 - 3.59 (m, 6H), 2.77 (s, 6H), 2.72 (t, *J* = 6.7 Hz, 2H), 2.22 (d, *J* = 7.0 Hz, 2H), 2.14 (d, *J* = 12.0 Hz, 2H), 2.03-1.86 (m, 5H), 1.74-1.61 (m, 1H), 1.20-1.06 (m, 2H). | | |

| Example No. | Compound No. | Structure | Example No. | Compound No. | Structure |
|---|---|---|---|---|---|
| Example 86 | TM-86 | 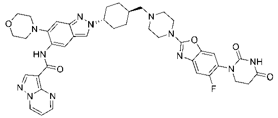 | Example 87 | TM-87 | 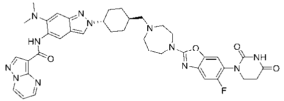 |
| Characterization | | | Characterization | | |
| LCMS (ESI) m/z: [M+1]=791.35. | | | LCMS (ESI) m/z: [M+1]=763.05. | | |

| Example No. | Compound No. | Structure | Example No. | Compound No. | Structure |
|---|---|---|---|---|---|
| Example 88 | TM-88 | 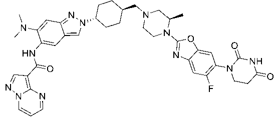 | Example 89 | TM-89 | 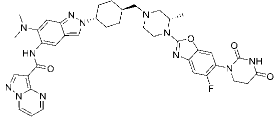 |
| Characterization | | | Characterization | | |
| LCMS (ESI) m/z: [M+1]=763.10. | | | LCMS (ESI) m/z: [M+1]=763.14. | | |

| Example No. | Compound No. | Structure | Example No. | Compound No. | Structure |
|---|---|---|---|---|---|
| Example 90 | TM-90 | 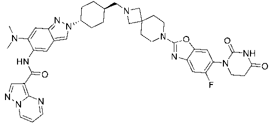 | Example 91 | TM-91 | 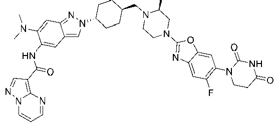 |
| Characterization | | | Characterization | | |
| LCMS (ESI) m/z: [M+1]=789.18. ¹H NMR (400 MHz, DMSO-*d*₆) δ 10.85 (s, 1H), 10.45 (s, 1H), 9.36 (dd, *J* = 7.0, 1.7 Hz, 1H), 8.94 (dd, *J* = 4.2, 1.7 Hz, 1H), 8.71 (s, 1H), 8.70 (s, 1H), 8.28 (s, 1H), 7.51 (d, *J* = 6.5 Hz, 1H), 7.36 (s, 1H), 7.33 (dd, *J* = 7.1, 4.2 Hz, 1H), 7.20 (d, *J* = 10.3 Hz, 1H), 4.39-4.20 (m, 1H), 3.67 (t, *J* = 6.7 Hz, 2H), 3.58 (s, 4H), 3.03 (s, 4H), 2.76 (s, 6H), 2.72 (t, *J* = | | | LCMS (ESI) m/z: [M+1]=763.04. | | |
| 6.7 Hz, 2H),2.40-2.30 (m, 2H), 2.16-2.05 (m, 2H), 1.95-1.74 (m, 8H), 1.47-1.34 (m, 1H), 1.20-1.06 (m, 2H). | | | | | |

| Example No. | Compound No. | Structure | Example No. | Compound No. | Structure |
|---|---|---|---|---|---|
| Example 92 | TM-92 | 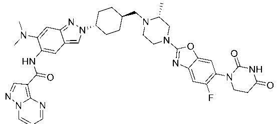 | Example 93 | TM-93 | 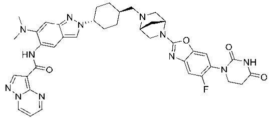 |
| Characterization | | | Characterization | | |
| LCMS (ESI) m/z: [M+1]=763.08. | | | LCMS (ESI) m/z: [M+1]=761.22. | | |

| Example No. | Compound No. | Structure | Example No. | Compound No. | Structure |
|---|---|---|---|---|---|
| Example 94 | TM-94 | 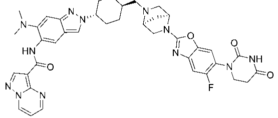 | Example 95 | TM-95 | 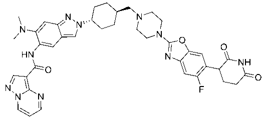 |
| Characterization | | | Characterization | | |
| LCMS (ESI) m/z: [M+1]=761.22. | | | LCMS (ESI) m/z: [M+1]=748.12. | | |

| Example No. | Compound No. | Structure | Example No. | Compound No. | Structure |
|---|---|---|---|---|---|
| Example 96 | TM-96 | 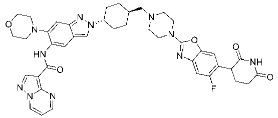 | Example 97 | TM-97 | 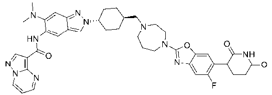 |
| Characterization | | | Characterization | | |
| LCMS (ESI) m/z: [M+1]=790.16. | | | LCMS (ESI) m/z: [M+1]=762.14. | | |

| Example No. | Compound No. | Structure | Example No. | Compound No. | Structure |
|---|---|---|---|---|---|
| Example 98 | TM-98 | 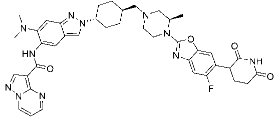 | Example 99 | TM-99 | 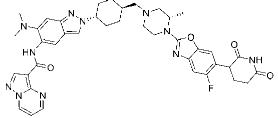 |
| Characterization | | | Characterization | | |
| LCMS (ESI) m/z: [M+1]=762.18. | | | LCMS (ESI) m/z: [M+1]=762.14. | | |

| Example No. | Compound No. | Structure | Example No. | Compound No. | Structure |
|---|---|---|---|---|---|
| Example 100 | TM-100 | 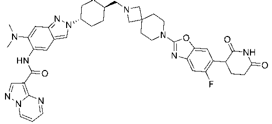 | Example 101 | TM-101 | 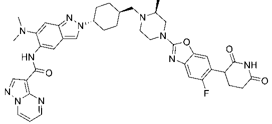 |
| Characterization | | | Characterization | | |
| LCMS (ESI) m/z: [M+1]=788.17. | | | LCMS (ESI) m/z: [M+1]=762.12. | | |

| Example No. | Compound No. | Structure | Example No. | Compound No. | Structure |
|---|---|---|---|---|---|
| Example 102 | TM-102 | 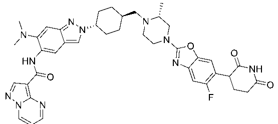 | Example 103 | TM-103 | 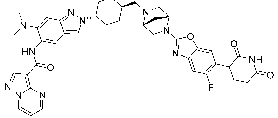 |
| Characterization | | | Characterization | | |
| LCMS (ESI) m/z: [M+1]=762.08. | | | LCMS (ESI) m/z: [M+11=760.18. | | |
| Example 104 | TM-104 | 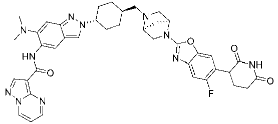 | Example 105 | TM-105 | 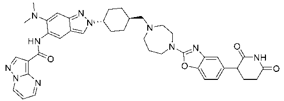 |
| Characterization | | | Characterization | | |
| LCMS (ESI) m/z: [M+1]=760.13. | | | LCMS (ESI) m/z: [M+1]=744.28. | | |

| Example No. | Compound No. | Structure | Example No. | Compound No. | Structure |
|---|---|---|---|---|---|
| Example 106 | TM-106 | 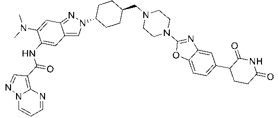 | Example 107 | TM-107 | 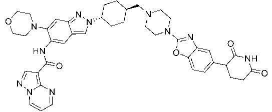 |
| Characterization | | | Characterization | | |
| LCMS (ESI) m/z: [M+1]=730.24. ¹H NMR (400 MHz, DMSO-*d*₆) δ 10.85 (s, 1H), 10.81 (s, 1H), 9.36 (dd,*J* = 7.0, 1.7 Hz, 1H), 8.95 (dd, *J* = 4.1, 1.6 Hz, 1H), 8.72 (s, 1H), 8.70 (s, 1H), 8.29 (s, 1H), 7.40 - 7.29 (m, 3H), 7.14 (d, *J* = 1.7 Hz, 1H), 6.87 (dd, *J* = 8.3, 1.7 Hz, 1H), 4.46-4.32 (m, 1H), 3.88 (dd, *J =* 11.5, 4.9 Hz, 1H), 3.68-3.54 (m, 4H), 2.77 (s, 6H), 2.72-2.60 (m, 1H), 2.29-1.84 (m, 10H), 1.74-1.60 (m, 2H), (dd, *J =* 32.6, 9.2 Hz, 2H), 2.10 - 1.83 (m, 10H), 1.67-1.54 (m, 1H), 1.21-1.07 (m, 2H). | | | LCMS (ESI) m/z: [M+1]=772.16. | | |

| Example No. | Compound No. | Structure | Example No. | Compound No. | Structure |
|---|---|---|---|---|---|
| Example 108 | TM-108 | 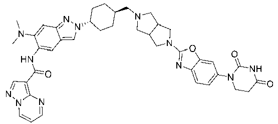 | Example 109 | TM-109 | 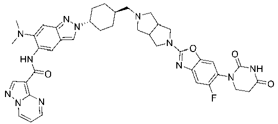 |
| Characterization | | | Characterization | | |
| LCMS (ESI) m/z: [M+1]=757.18. | | | LCMS (ESI) m/z: [M+1]=775.15. | | |

| Example No. | Compound No. | Structure | Example No. | Compound No. | Structure |
|---|---|---|---|---|---|
| Example 110 | TM-110 | 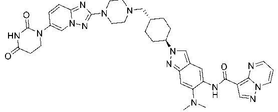 | Example 111 | TM-111 | 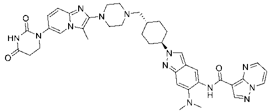 |
| Characterization | | | Characterization | | |
| LCMS (ESI) m/z: [M+1]=731.14. ¹H NMR (400 MHz, DMSO-*d*₆) δ 10.85 (s, 1H), 10.50 (s, 1H), 9.37 (dd, *J =* 7.0, 1.6 Hz, 1H), 8.95 (dd, *J* = 4.2, 1.7 Hz, 1H), 8.78 (d, *J* = 2.0 Hz, 1H), 8.72 (s, 1H), 8.70 (s, 1H), 8.29 (s, 1H), 7.54 (dd, *J =* 9.4, 2.0 Hz, 1H), 7.47 (d, *J =* 9.4 Hz, 1H), 7.37 (s, 1H), 7.33 (dd, *J =* 7.0, 4.2 Hz, 1H), 4.43-4.32 (m, 1H), 3.80 (t, *J =* 6.7 Hz, 2H), 3.51 (s, 4H), 2.80-2.72 (m, 8H), 2.25-2.10 (m, 4H), 2.04-1.84 (m, 5H), 1.73-1.60 (m, 1H), 1.19-1.07 (m, 2H). | | | LCMS (ESI) m/z: [M+1]=744.28. | | |

| Example No. | Compound No. | Structure | Example No. | Compound No. | Structure |
|---|---|---|---|---|---|
| Example 112 | TM-112 | 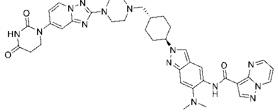 | Example 113 | TM-113 | 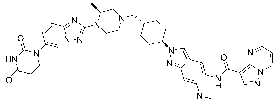 |
| Characterization | | | Characterization | | |
| LCMS (ESI) m/z: [M+1]=731.17. | | | LCMS (ESI) m/z: [M+1]=745.08. | | |
| Example 114 | TM-114 | 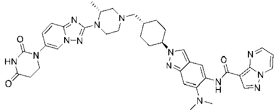 | Example 115 | TM-115 | 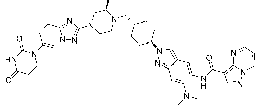 |
| Characterization | | | Characterization | | |
| LCMS (ESI) m/z: [M+1]=745.06. | | | LCMS (ESI) m/z: [M+1]=745.18. | | |

| Example No. | Compound No. | Structure | Example No. | Compound No. | Structure |
|---|---|---|---|---|---|
| Example 116 | TM-116 | 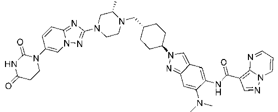 | Example 117 | TM-117 | 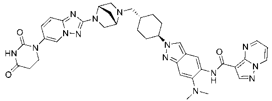 |
| Characterization | | | Characterization | | |
| LCMS (ESI) m/z: [M+1]=745.24. | | | LCMS (ESI) m/z: [M+1]=743.24. | | |

| Example No. | Compound No. | Structure | Example No. | Compound No. | Structure |
|---|---|---|---|---|---|
| Example 118 | TM-118 | 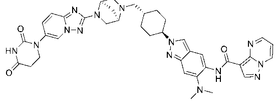 | Example 119 | TM-119 | 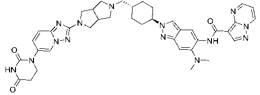 |
| Characterization | | | Characterization | | |
| LCMS (ESI) m/z: [M+1]=743.21. | | | LCMS (ESI) m/z: [M+1]=757.23. | | |

| Example No. | Compound No. | Structure | Example No. | Compound No. | Structure |
|---|---|---|---|---|---|
| Example 120 | TM-120 | 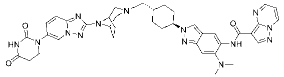 | Example 121 | TM-121 | 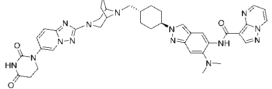 |
| Characterization | | | Characterization | | |
| LCMS (ESI) m/z: [M+1]=757.15. | | | LCMS (ESI) m/z: [M+1]=757.12. | | |

| Example No. | Compound No. | Structure | Example No. | Compound No. | Structure |
|---|---|---|---|---|---|
| Example 122 | TM-122 | 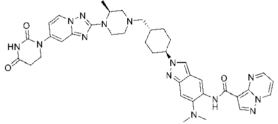 | Example 123 | TM-123 | 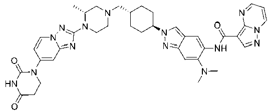 |
| Characterization | | | Characterization | | |
| LCMS (ESI) m/z: [M+1]=745.18. | | | LCMS (ESI) m/z: [M+1]=745.16. | | |
| Example 124 | TM-124 | 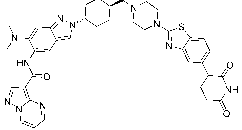 | Example 125 | TM-125 | 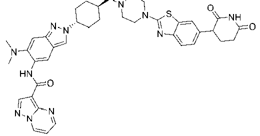 |
| Characterization | | | Characterization | | |
| LCMS (ESI) m/z: [M+1]=746.12. | | | LCMS (ESI) m/z: [M+1]=746.15. | | |

| Example No. | Compound No. | Structure | Example No. | Compound No. | Structure |
|---|---|---|---|---|---|
| Example 126 | TM-126 | 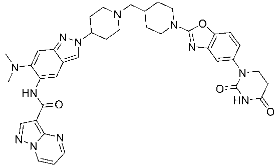 | Example 127 | TM-127 | 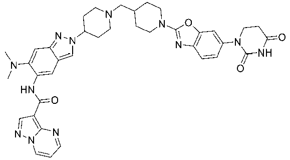 |
| Characterization | | | Characterization | | |
| LCMS (ESI) m/z: [M+1]=731.34. | | | LCMS (ESI) m/z: [M+1]=731.28. | | |
| Example 128 | TM-128 | 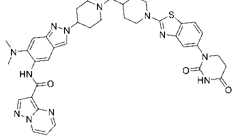 | Example 129 | TM-129 | 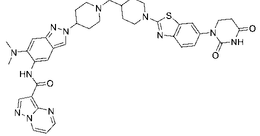 |
| Characterization | | | Characterization | | |
| LCMS (ESI) m/z: [M+1]=747.62. ¹H NMR (400 MHz, DMSO-*d*₆) δ 10.86 (s, 1H), 10.34 (s, 1H), 9.36 (dd, *J =* 7.0, 1.6 Hz, 1H), 8.95 (dd, *J* = 4.2, 1.7 Hz, 1H), 8.72 (s, 1H), 8.70 (s, 1H), 8.33 (d, *J=* 0.9 Hz, 1H), 7.73 (d, *J =* 8.4 Hz, 1H), 7.39 (d, *J* = 2.1 Hz, 1H), 7.37 (s, 1H), 7.33 (dd, *J =* 7.0, 4.2 Hz, 1H), 7.02 (dd, *J =* 8.4, 2.1 Hz, 1H), 4.47-4.33 (m, 1H), 4.04 (d, *J =* 12.7 Hz, 2H), 3.80 (t, *J =* 6.7 Hz, 2H), 3.25 - 3.14 (m, 2H), 3.05-2.93 (m, 2H), 2.77 (s, 6H), 2.72 (t, *J* = 6.7 Hz, 2H), 2.24 (d, *J =* 6.7 Hz, 2H), 2.16-2.04 (m, 6H), 1.94-1.86 (m, 3H), 1.29-1.14 (m, 3H). | | | LCMS (ESI) m/z: [M+1]=747.23. | | |

| Example No. | Compound No. | Structure | Example No. | Compound No. | Structure |
|---|---|---|---|---|---|
| Example 130 | TM-130 | 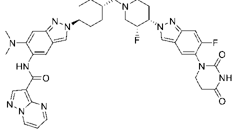 | Example 131 | TM-131 | 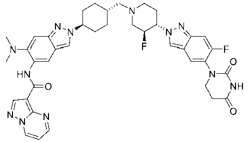 |
| Characterization | | | Characterization | | |
| LCMS (ESI) m/z: [M+1]=765.71. ¹H NMR (400 MHz, DMSO-*d*₆) δ 10.85 (s, 1H), 10.46 (s, 1H), 9.36 (dd, *J =* 7.1, 1.7 Hz, 1H), 8.95 (dd, *J* = 4.2, 1.7 Hz, 1H), 8.72 (s, 1H), 8.70 (s, 1H), 8.54 (s, 1H), 8.29 (s, 1H), 7.81 (d, *J* = 7.7 Hz, 1H), 7.48 (d, *J =* 11.2 Hz, 2H), 7.37 (s, 1H), 7.33 (dd, *J =* 7.0, 4.2 Hz, 1H), 5.12 (d, *J =* 49.8 Hz, 1H), 4.77 (dd, *J =* 30.7, 12.6 Hz, 1H), 4.37 (d, *J =* 12.7 Hz, 1H), 3.73 (t*, J =* 6.7 Hz, 2H), 3.24-3.16 (m, 1H), 3.11-3.04 (m, 1H), 2.81-2.71 (m, 8H), 2.61-1.82 (m, 12H), 1.76-1.56 (m, 1H), 1.20-1.06 (m, 2H). | | | LCMS (ESI) m/z: [M+1] = 765.68. ¹H NMR (400 MHz, DMSO-*d*₆) δ 10.79 (s, 1H), 10.40 (s, 1H), 9.30 (dd, *J =* 7.0, 1.7 Hz, 1H), 8.96 - 8.75 (m, 1H), 8.66 (s, 1H), 8.64 (s, 1H), 8.54 (s, 1H), 8.23 (s, 1H), 7.76 (d, *J =* 7.7 Hz, 1H), 7.43 (d, *J =* 11.1 Hz, 1H), 7.30 (s, 1H), 7.27 (dd, *J* = 7.0, 4.2 Hz, 11),5.13-4.90 (m, 1H), 4.72-4.57 (m, 1H), 4.40-4.25 (m, 1H), 3.67 (t, *J =* 6.7 Hz, 2H), 2.97-2.85 (m, 1H), 2.74-2.64 (m, 8H), 2.30-1.80 (m, 13H), 1.68-1.52 (m, 1H), 1.15-1.03 (m, 2H). | | |

| Example No. | Compound No. | Structure | Example No. | Compound No. | Structure |
|---|---|---|---|---|---|
| Example 132 | TM-132 | 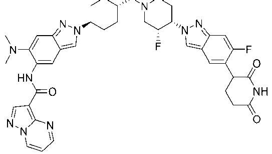 | Example 133 | TM-133 | 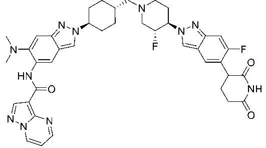 |
| Characterization | | | Characterization | | |
| LCMS (ESI) m/z: [M+1] = 764.23. 1H NMR (400 MHz, DMSO-d6) δ 10.87 (s, 1H), 10.85 (s, 1H), 9.36 (dd, J = 7.0, 1.6 Hz, 1H), 8.95 (dd, J = 4.2, 1.7 Hz, 1H), 8.72 (s, 1H), 8.70 (s, 1H), 8.46 (s, 1H), 8.29 (d, J = 0.9 Hz, 1H), 7.66 (d, J = 7.4 Hz, 1H), 7.39-7.31 (m, 3H), 5.11 (d, J = 49.9 Hz, 1H), 4.74 (dd, J = 30.3, 12.5 Hz, 1H), 4.42 - 4.33 (m, 1H), 4.09-4.04 (m, 1H), 3.29 - 3.21 (m, 1H), 3.12-3.03 (m, 1H), 2.77 (s, 6H), 2.60-1.83 (m, 16H), 1.74-1.65 (m, 1H), 1.23-1.06 (m, 2H). | | | LCMS (ESI) m/z: [M+1] = 764.76. ¹H NMR (400 MHz, DMSO-*d*₆) δ 10.87 (s, 1H), 10.85 (s, 1H), 9.36 (dd, *J =* 7.1, 1.7 Hz, 1H), 8.95 (dd, *J* = 4.2, 1.7 Hz, 1H), 8.72 (s, 1H), 8.70 (s, 1H), 8.53 (s, 1H), 8.29 (s, 1H), 7.67 (d, *J=* 7.5 Hz, 1H), 7.50 - 7.18 (m, 3H), 5.07 (d, *J =* 48.8 Hz, 1H), 4.73-4.62 (m, 1H), 4.45-4.33 (m, 1H), 4.06 (dd, *J =* 12.4, 4.9 Hz, 1H), 3.14 - 3.06 (m, 1H), 3.02-2.90 (m, 1H), 2.77 (s, 6H), 2.60-2.53 (m, 2H), 2.39 - 1.08 (m, 17H). | | |
| Example 134 | TM-134 | 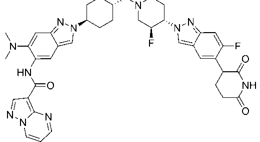 | Example 135 | TM-135 | 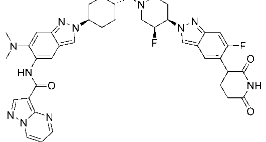 |
| Characterization | | | Characterization | | |
| LCMS (ESI) m/z: [M+1] = 764.76. ¹H NMR (400 MHz, DMSO-*d*₆) δ 10.87 (s, 1H), 10.85 (s, 1H), 9.36 (dd, *J =* 7.0, 1.7 Hz, 1H), 8.95 (dd, *J* = 4.2, 1.7 Hz, 1H), 8.72 (s, 1H), 8.70 (s, 1H), 8.53 (s, 1H), 8.29 (s, 1H), 7.67 (d, *J =* 7.4 Hz, 1H), 7.39 - 7.26 (m, 3H), 5.06 (d, *J =* 53.1 Hz, 1H), 4.74-4.61 (m, 1H), 4.44-4.32 (m, 1H), 4.06 (dd, *J =* 12.4, 4.9 Hz, 1H), 3.14-3.06 (m, 1H), 3.00-2.93 (m, 1H), 2.77 (s, 6H), 2.61-2.52 (m, 2H), 2.38-1.08 (m, 17H). | | | LCMS (ESI) m/z: [M+1] = 764.65. ¹H NMR (400 MHz, DMSO-*d*₆) δ 10.87 (s, 1H), 10.85 (s, 1H), 9.36 (dd, *J =* 7.0, 1.6 Hz, 1H), 8.95 (dd, *J* = 4.3, 1.6 Hz, 1H), 8.71 (s, 1H), 8.70 (s, 1H), 8.46 (s, 1H), 8.29 (s, 1H), 7.66 (d, *J =* 7.4 Hz, 1H), 7.41 - 7.28 (m, 3H), 5.11 (d, *J =* 49.8 Hz, 1H), 4.82-4.67 (m, 1H), 4.45-4.33 (m, 1H), 4.06 (dd, *J =* 12.4, 4.9 Hz, 1H), 3.15-3.03 (m, 2H), 2.77 (s, 6H), 2.60-2.53 (m, 2H), 2.35 - 1.08 (m, 17H). | | |

| Example No. | Compound No. | Structure | Example No. | Compound No. | Structure |
|---|---|---|---|---|---|
| Example 136 | TM-136 | 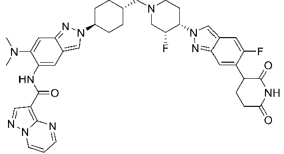 | Example 137 | TM-137 | 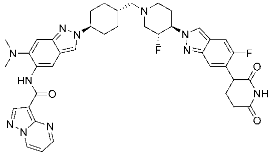 |
| Characterization | | | Characterization | | |
| LCMS (ESI) m/z: [M+1] = 764.59. ¹H NMR (400 MHz, DMSO-*d*₆) δ 10.88 (s, 1H), 10.85 (s, 1H), 9.36 (dd, *J =* 7.0, 1.6 Hz, 1H), 8.95 (dd, *J* = 4.1, 1.6 Hz, 1H), 8.71 (s, 1H), 8.70 (s, 1H), 8.40 (s, 1H), 8.29 (d, *J =* 1.1 Hz, 1H), 7.59 (d, *J =* 6.5 Hz, 1H), 7.44 (d, *J* = 11.1 Hz, 1H), 7.37 (s, 1H), 7.33 (dd, *J =* 7.0, 4.2 Hz, 1H), 5.10 (d, *J* = 49.7 Hz, 1H), 4.76 (dd, *J =* 30.2, 12.5 Hz, 1H), 4.45-4.29 (m, 1H), 4.10 (dd, *J =* 12.7, 5.0 Hz, 1H), 3.13-3.01 (m, 2H), 2.76 (s, 6H), 2.63-2.51 (m, 2H), 2.41-1.02 (m, 17H). | | | LCMS (ESI) m/z: [M+1] = 764.95. ¹H NMR (400 MHz, DMSO-*d*₆) δ 10.88 (s, 1H), 10.85 (s, 1H), 9.36 (dd, *J =* 7.0, 1.7 Hz, 1H), 8.95 (dd, *J* = 4.2, 1.7 Hz, 1H), 8.72 (s, 1H), 8.70 (s, 1H), 8.47 (s, 1H), 8.29 (s, 1H), 7.60 (d, *J =* 6.4 Hz, 1H), 7.45 (d, *J =* 11.1 Hz, 1H), 7.37 (s, 1H), 7.33 (dd, *J =* 7.0, 4.2 Hz, 1H), 5.07 (d, *J* = 49.4 Hz, 1H), 4.75-4.62 (m, 1H), 4.45-4.31 (m, 1H), 4.10 (dd, *J =* 12.7, 4.9 Hz, 1H),3.01-2.91 (m, 1H), 2.81-2.71 (m, 7H),2.62-2.52 (m, 2H), 2.37-1.06 (m, 17H). | | |

| Example No. | Compound No. | Structure | Example No. | Compound No. | Structure |
|---|---|---|---|---|---|
| Example 138 | TM-138 | 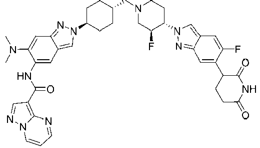 | Example 139 | TM-139 | 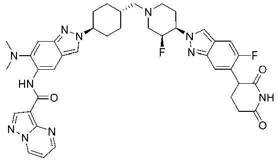 |
| Characterization | | | Characterization | | |
| LCMS (ESI) m/z: [M+1]=764.71. | | | LCMS (ESI) m/z: [M+1]=764.69. | | |

| Example No. | Compound No. | Structure | Example No. | Compound No. | Structure |
|---|---|---|---|---|---|
| Example 140 | TM-140 | 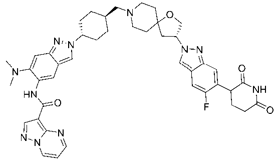 | Example 141 | TM-141 | 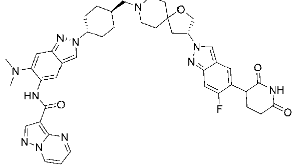 |
| Characterization | | | Characterization | | |
| LCMS (ESI) m/z: [M+1]=803.18. | | | LCMS (ESI) m/z: [M+1]=802.68. | | |
| Example 142 | TM-142 | 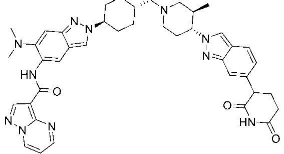 | Example 143 | TM-143 | 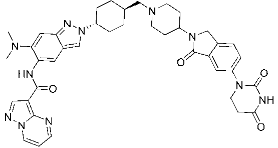 |
| Characterization | | | Characterization | | |
| LCMS (ESI) m/z: [M+Na] = 764.78. ¹H NMR (400 MHz, DMSO-*d*₆) δ 10.85 (d, *J =* 2.6 Hz, 2H), 9.37 (dd, *J* = 7.0, 1.7 Hz, 1H), 8.95 (dd, *J =* 4.2, 1.7 Hz, 1H), 8.73 (s, 1H), 8.70 (s, 0H), 8.41 (s, 1H), 8.30 (s, 0H), 7.66 (d, *J* = 8.6 Hz, 1H), 7.43 (s, 1H), 7.36 (s, 0H), 7.34 (dd, *J =* 7.0, 4.2 Hz, 1H), 6.91 (d, *J =* 8.7 Hz, 0H), 4.46-4.32 (m, 1H), 3.93 (dd, *J =* 11.6, 4.9 Hz, 1H), 2.77 (s, 6H), 2.70-2.64 (m, 1H), 2.58-2.52 (m, 1H),2.37-1.88 (m, 12H), 1.32-1.11 (m, 2H), 0.63 (s, 3H). | | | LCMS (ESI) m/z: [M+1]=744.43. ¹H NMR (400 MHz, DMSO-*d*₆) δ 10.85 (s, 1H), 10.41 (s, 1H), 9.37 (dd,*J* = 7.0, 1.7 Hz, 1H), 8.95 (dd, *J* = 4.2, 1.7 Hz, 1H), 8.72 (s, 1H), 8.70 (s, 1H), 8.29 (s, 1H), 7.66 - 7.50 (m, 3H), 7.37 (s, 1H), 7.33 (dd, *J =* 7.0, 4.2 Hz, 1H), 4.47 (s, 2H), 4.43-4.32 (m, 1H), 4.07-3.97 (m, 1H), 3.84 (t, *J =* 6.6 Hz, 2H), 2.97 (d, *J =* 11.0 Hz, 2H), 2.81-2.70 (m, 8H), 2.23-1.59 (m, 15H), 1.18 - 1.06 (m, 2H). | | |

| Example No. | Compound No. | Structure | Example No. | Compound No. | Structure |
|---|---|---|---|---|---|
| Example 144 | TM-144 | 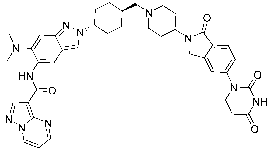 | Example 145 | TM-145 | 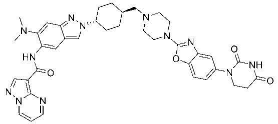 |
| Characterization | | | Characterization | | |
| LCMS (ESI) m/z: [M+1]=744.77. ¹H NMR (400 MHz, DMSO-*d*₆) δ 10.85 (s, 1H), 10.46 (s, 1H), 9.37 (dd, *J =* 7.0, 1.7 Hz, 1H), 8.95 (dd, *J* = 4.2, 1.7 Hz, 1H), 8.72 (s, 1H), 8.70 (s, 1H), 8.29 (s, 1H), 7.68 (d, *J =* 8.2 Hz, 1H), 7.55 (s, 1H), 7.43 (dd, *J =* 8.2, 1.8 Hz, 1H), 7.37 (s, 1H), 7.33 (dd, *J =* 7.0, 4.2 Hz, 1H), 4.47 (s, 2H), 4.43-4.36 (m, 1H), 4.07-3.95 (m, 1H), 3.85 (t, *J =* 6.6 Hz, 2H), 2.97 (d, *J =* 10.8 Hz, 2H), 2.84-2.70 (m, 8H), 2.25-1.57 (m, 15H), 1.18-1.06 (m, 2H). | | | LCMS (ESI) m/z: [M+1] = 731.55. ¹H NMR (400 MHz, DMSO-*d*₆) δ 10.85 (s, 1H), 10.32 (s, 1H), 9.36 (dd, *J =* 7.0, 1.6 Hz, 1H), 8.95 (dd, *J* = 4.2, 1.6 Hz, 1H), 8.72 (s, 1H), 8.29 (s, 1H), 8.70 (s, 1H), 7.40 (d, *J =* 8.5 Hz, 1H), 7.37 (s, 1H), 7.33 (dd, *J =* 7.0, 4.2 Hz, 1H), 7.24 (d, *J =* 2.1 Hz, 1H), 6.97 (dd, *J* = 8.5, 2.1 Hz, 1H), 4.45 - 4.32 (m, 1H), 3.76 (t, *J =* 6.7 Hz, 2H), 3.69-3.57 (m, 4H), 2.77 (s, 6H), 2.71 (t, *J* = 6.7 Hz, 2H), 2.22 (d, *J* = 7.1 Hz, 2H), 2.14 (d, *J =* 11.8 Hz, 2H), 2.05-1.85 (m, 4H), 1.75-1.59 (m, 4H), 1.74-1.60 (m, 1H), 1.14 (q, *J* = 12.5 Hz, 2H). | | |

| Example No. | Compound No. | Structure | Example No. | Compound No. | Structure |
|---|---|---|---|---|---|
| Example 146 | TM-146 | 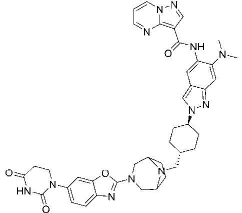 | Example 147 | TM-147 | 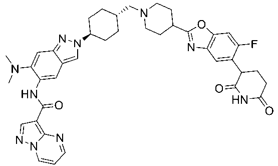 |
| Characterization | | | Characterization | | |
| LCMS (ESI) m/z: [M+1] = 757.86. ¹H NMR (400 MHz, DMSO-*d*₆) δ 10.85 (s, 1H), 10.34 (s, 1H), 9.36 (dd, *J =* 7.0, 1.7 Hz, 1H), 8.95 (dd, *J* = 4.2, 1.7 Hz, 1H), 8.71 (s, 1H), 8.70 (s, 1H), 8.28 (s, 1H), 7.44 (d, *J* = 2.0 Hz, 1H), 7.36 (s, 1H), 7.34 - 7.25 (m, 2H), 7.12 (dd, *J* = 8.3, 2.0 Hz, 1H), 4.45-4.30 (m, 3H), 3.77 (t, *J =* 6.6 Hz, 2H), 2.88 - 2.63 (m, 10H), 2.40 - 2.27 (m, 2H), 2.20-2.09 (m, 3H), 2.01-1.82 (m, 8H), 1.69-1.54 (m, 1H), 1.19-1.03 (m, 2H). | | | LCMS (ESI) m/z: [M+1]=747.59. | | |
| Example 148 | TM-148 | 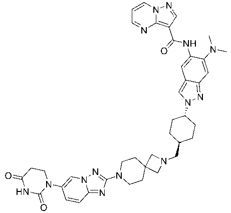 | Example 149 | TM-149 | 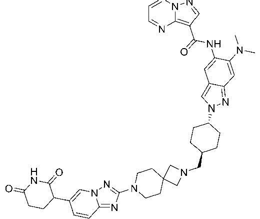 |
| Characterization | | | Characterization | | |
| LCMS (ESI) m/z: [M+1] = 772.18. ¹H NMR (400 MHz, DMSO-*d*₆) δ 10.85 (s, 1H), 10.49 (s, 1H), 9.36 (dd, *J =* 7.0, 1.7 Hz, 1H), 8.95 (dd, *J* = 4.2, 1.7 Hz, 1H), 8.77 - 8.73 (m, 1H), 8.71 (s, 1H), 8.70 (s, 1H), 8.28 (s, 1H), 7.51 (dd, *J* = 9.3, 2.0 Hz, 1H), 7.44 (d, *J =* 9.1 Hz, 1H), 7.36 (s, 1H), 7.33 (dd, *J* = 7.0, 4.2 Hz, 1H), 4.40-4.29 (m, 1H), 3.79 (t, *J =* 6.7 Hz, 2H), 3.46 (s, 4H), 2.97 (s, 4H), 2.80-2.69 (m, 8H), 2.34-2.29 (m, 2H), 2.20 - 2.07 (m, 2H), 1.96-1.80 (m, 5H), 1.76-1.60 (m, 4H), 1.44-1.33 (m, 1H), 1.19-1.05 (m, 2H). | | | LCMS (ESI) m/z: [M+1] = 770.85. ¹H NMR (400 MHz, DMSO-*d*₆) δ 10.90 (s, 1H), 10.85 (s, 1H), 9.36 (d, *J =* 7.0 Hz, 1H), 9.04 - 8.85 (m, 1H), 8.72-8.68 (m, 2H), 8.55 (s, 1H), 8.30 (d, *J* = 21.9 Hz, 1H), 7.43-7.35 (m, 2H), 7.33 (dd, *J* = 7.0, 4.2 Hz, 1H), 4.41-4.28 (m, 1H), 3.94 (dd, *J =* 12.8, 4.8 Hz, 1H), 3.45 (s, 4H), 2.97 (s, 4H), 2.76 (d, *J =* 1.6 Hz, 6H),2.73-2.64 (m, 1H), 2.61-2.54 (m, 1H), 2.47-2.40 (m, 1H), 236-2.29 (m, 3H), 2.18-2.00 (m, 4H), 1.95-1.80 (m, 4H), 1.76-1.58 (m, 6H), 1.45-1.32 (m, 1H), 1.19-1.03 (m, 2H). | | |

| Example No. | Compound No. | Structure | Example No. | Compound No. | Structure |
|---|---|---|---|---|---|
| Example 150 | TM-150 | 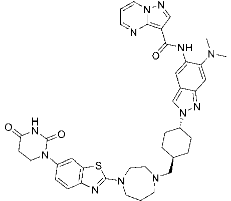 | Example 151 | TM-151 | 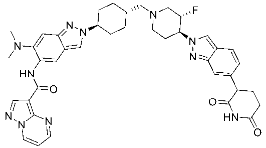 |
| Characterization | | | Characterization | | |
| LCMS (ESI) m/z: [M+1] = 761.33. ¹H NMR (400 MHz, DMSO-*d*₆) δ 10.85 (s, 1H), 10.32 (s, 1H), 9.36 (dt, *J =* 7.1, 1.4 Hz, 1H), 8.95 (dt, *J* = 4.2, 1.2 Hz, 1H), 8.71 (s, 1H), 8.70 (s, 1H), 8.27 (s, 1H), 7.71 (dd, *J* = 2.3, 1.0 Hz, 1H), 7.41 (d, *J* = 8.6 Hz, 1H), 7.36 (s, 1H), 7.33 (ddd, *J* = 7.1, 4.2, 1.1 Hz, 1H), 7.20 (dd, *J* = 8.5, 2.2 Hz, 1H), 4.41-4.29 (m, 1H), 3.85 - 3.54 (m, 6H), 2.87-2.79 (m, 2H), 2.76 (s, 6H), 2.72 - 2.60 (m, 4H), 2.32 (d, *J= 6.7* Hz, 2H), 2.21 - 2.05 (m, 2H), 1.97-1.80 (m, 6H), 1.64-1.50 (m, 1H), 1.15-1.02 (m, 2H). | | | LCMS (ESI) m/z: [M+23] = 768.84. ¹H NMR (400 MHz, DMSO-*d*₆) δ 10.85 (s, 2H), 9.37 (dd, *J* = 7.0, 1.7 Hz, 1H), 8.95 (dd, *J* = 4.2, 1.7 Hz, 1H), 8.73 (s, 1H), 8.70 (s, 1H), 8.48 (s, 1H), 8.30 (s, 1H), 7.67 (d, *J =* 8.7 Hz, 1H), 7.45 (s, 1H), 7.37 (s, 1H), 7.34 (dd, *J =* 7.0, 4.2 Hz, 1H), 6.93 (d, *J =* 8.7 Hz, 1H), 5.38-4.78 (m, 2H), 4.41 (s, 1H), 3.95 (dd, *J =* 11.5, 4.9 Hz, 1H), 2.77 (s, 6H), 2.71-2.64 (m, 1H), 2.69 (dd, *J =* 11.1, 6.1 Hz, 1H), 2.56-1.88 (m, 16H), 1.30-1.09 (m, 5H). | | |

| Example No. | Compound No. | Structure | Example No. | Compound No. | Structure |
|---|---|---|---|---|---|
| Example 152 | TM-152 | 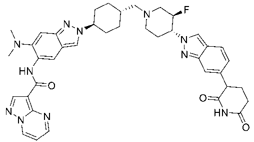 | Example 153 | TM-153 | 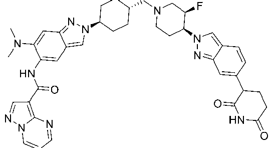 |
| Characterization | | | Characterization | | |
| LCMS (ESI) m/z: [M+1] = 746.96. ¹H NMR (400 MHz, DMSO-*d*₆) δ 10.85 (d, *J =* 2.0 Hz, 2H), 9.37 (dd, *J =* 7.0, 1.6 Hz, 1H), 8.95 (dd, *J* = 4.2, 1.7 Hz, 1H), 8.72 (s, 1H), 8.70 (s, 1H), 8.47 (s, 1H), 8.30 (s, 1H), 7.66 (d, *J =* 8.7 Hz, 1H), 7.45 (s, 1H), 7.37 (s, 1H), 7.33 (dd, *J =* 7.0, 4.2 Hz, 1H), 6.92 (dd, *J =* 8.7, 1.4 Hz, 1H), 5.33-5.00 (m, 1H), 4.83-4.63 (m, 1H), 4.46-4.33 (m, 1H), 3.94 (dd, *J =* 11.6, 4.9 Hz, 1H), 2.77 (s, 6H), 2.72 - 2.65 (m, 1H), 2.57-1.85 (m, 16 H), 1.82-1.59 (m, 2H), 1.28-1.10 (m, 2H). | | | LCMS (ESI) m/z: [M+1] = 746.74. ¹H NMR (400 MHz, DMSO-*d*₆) δ 10.85 (s, 2H), 9.36 (dd, *J =* 7.1, 1.7 Hz, 1H), 8.95 (dd, *J =* 4.2, 1.7 Hz, 1H), 8.72 (s, 1H), 8.70 (s, 1H), 8.44 (s, 1H), 8.40 (s, 1H), 8.29 (s, 1H), 7.66 (d, *J =* 8.6 Hz, 1H), 7.43 (s, 1H), 7.37 (s, 1H), 7.33 (dd, *J =* 7.1, 4.2 Hz, 1H), 6.90 (d, *J* = 8.4 Hz, 1H), 5.11 (d, *J* = 49.4 Hz, 1H), 4.84 - 4.68 (m, 1H), 4.45-4.32 (m, 1H), 3.94 (dd, *J =* 11.5, 4.9 Hz, 1H), 3.15-3.05 (m, 1H), 2.77 (s, 6H), 2.71 - 2.63 (m, 1H), 2.38 - 1.59 (m, 16H), 1.72-1.58 (m, 2H), 1.21-1.07 (m, 2H). | | |
| Example 154 | TM-154 | 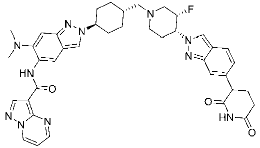 | Example 155 | TM-155 | 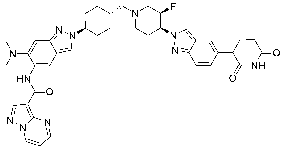 |
| Characterization | | | Characterization 349 | | |
| LCMS (ESI) m/z: [M+23] = 768.85. ¹H NMR (400 MHz, DMSO-*d*₆) δ 10.85 (s, 2H), 9.36 (dd, *J* = 7.0, 1.7 Hz, 1H), 8.95 (dd, *J* = 4.2, 1.6 Hz, 1H), 8.72 (s, 1H), 8.70 (s, 1H), 8.46 (s, 1H), 8.40 (s, 1H), 8.29 (s, 1H), 7.66 (d, *J* = 8.7 Hz, 1H), 7.43 (s, 1H), 7.37 (s, 1H), 7.33 (dd, *J* = 7.0, 4.2 Hz, 1H), 6.90 (d, *J =* 8.6 Hz, 1H), 5.11 (d, *J* = 49.8 Hz, 1H), 4.86 - 4.55 (m, 1H), 4.43-4.34 (m, 1H), 3.94 (dd, *J* = 11.5, 4.8 Hz, 1H), 3.13-3.04 (m, 1H), 2.77 (s, 6H), 2.72 - 2.63 (m, 1H), 2.40 - 1.88 (m, 16H), 1.74-1.57 (m, 1H), 1.21-1.06 (m, 1H). | | | LCMS (ESI) m/z: [M+1] = 746.61. ¹H NMR (400 MHz, DMSO-*d*₆) δ 10.85 (s, 1H), 10.83 (s, 1H), 9.36 (dd, *J =* 7.0, 1.6 Hz, 1H), 8.94 (dd, *J* = 4.2, 1.7 Hz, 1H), 8.72 (s, 1H), 8.70 (s, 1H), 8.39 (s, 1H), 8.29 (s, 1H), 7.57 (d, *J =* 8.9 Hz, 1H), 7.51 (s, 1H), 7.37 (s, 1H), 7.33 (dd, *J* = 7.0, 4.2 Hz, 1H), 7.11 (dd, *J =* 9.0, 1.7 Hz, 1H), 5.25 - 4.61 (m, 2H), 4.43-4.31 (m, 1H), 3.24-2.95 (m, 3H), 2.77 (s, 6H), 2.73-2.61 (m, 1H), 2.40 - 1.83 (m, 14H), 1.72-1.58 (m, 1H), 1.19-1.04 (m, 2H). | | |

| Example No. | Compound No. | Structure | Example No. | Compound No. | Structure |
|---|---|---|---|---|---|
| Example 156 | TM-156 | 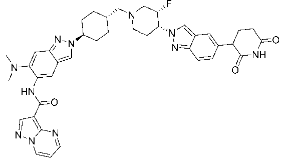 | Example 157 | TM-157 | 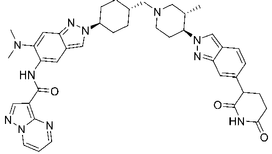 |
| Characterization 350 | | | Characterization 327 | | |
| LCMS (ESI) m/z: [M+1] = 746.68. ¹H NMR (400 MHz, DMSO-*d*₆) δ 10.85 (s, 1H), 10.83 (s, 1H), 9.37 (dd, *J =* 7.0, 1.6 Hz, 1H), 8.95 (dd, *J* = 4.1, 1.5 Hz, 1H), 8.72 (s, 1H), 8.70 (s, 1H), 8.39 (s, 1H), 8.29 (s, 1H), 7.57 (d, *J =* 8.9 Hz, 1H), 7.51 (s, 1H), 7.37 (s, 1H), 7.33 (dd, *J* = 7.0, 4.2 Hz, 1H), 7.11 (d, *J =* 9.3 Hz, 1H), 5.24 - 4.69 (m, 2H), 4.44-4.32 (m, 1H), 3.91 (dd, *J =* 11.2, 4.8 Hz, 1H), 3.27 - 2.94 (m, 3H), 2.77 (s, 6H), 2.73-2.63 (m, 1H), 2.40-1.77 (m, 14H), 1.73-1.58 (m, 1H), 1.21-1.04 (m, 2H). | | | LCMS (ESI) m/z: [M+1]=742.19. | | |

| Example No. | Compound No. | Structure | Example No. | Compound No. | Structure |
|---|---|---|---|---|---|
| Example 158 | TM-158 | 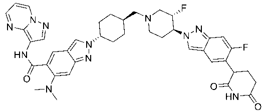 | Example 159 | TM-159 | 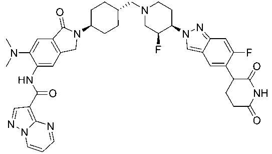 |
| Characterization | | | Characterization | | |
| LCMS (ESI) m/z: [M+1]=764.38. | | | LCMS (ESI) m/z: [M+1]=779.36. | | |

| Example No. | Compound No. | Structure | Example No. | Compound No. | Structure |
|---|---|---|---|---|---|
| Example 160 | TM-160 | 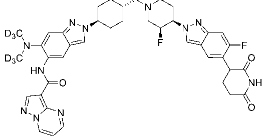 | Example 161 | TM-161 | 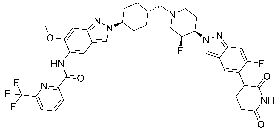 |
| Characterization | | | Characterization | | |
| LCMS (ESI) m/z: [M+1]=770.35 | | | LCMS (ESI) m/z: [M+1]=779.31 | | |
| Example 162 | TM-162 | 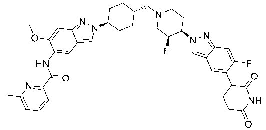 | Example 163 | TM-163 | 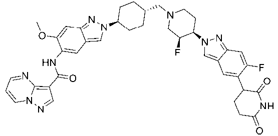 |
| Characterization | | | Characterization | | |
| LCMS (ESI) m/z: [M+1]=725.33 | | | LCMS (ESI) m/z: [M+1]=751.34 | | |

| Example No. | Compound No. | Structure | Example No. | Compound No. | Structure |
|---|---|---|---|---|---|
| Example 164 | TM-164 | 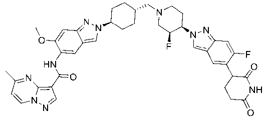 | Example 165 | TM-165 | 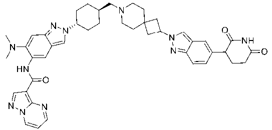 |
| Characterization | | | Characterization | | |
| LCMS (ESI) m/z: [M+1]=765.35 | | | LCMS (ESI) m/z: [M+1]=768.36 | | |

| Example No. | Compound No. | Structure | Example No. | Compound No. | Structure |
|---|---|---|---|---|---|
| Example 166 | TM-166 | 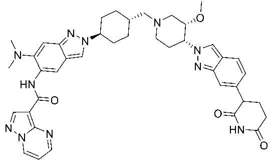 | Example 167 | TM-167 | 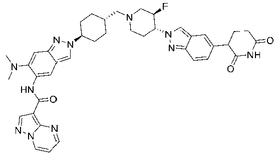 |
| Characterization | | | Characterization | | |
| LCMS (ESI) m/z: [M+Na]=780.95 | | | LCMS (ESI) m/z: [M+1]=746.68. | | |

| Example No. | Compound No. | Structure | Example No. | Compound No. | Structure |
|---|---|---|---|---|---|
| Example 168 | TM-168 | 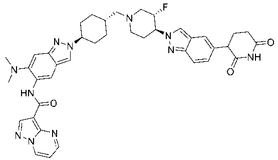 | Example 169 | TM-169 | 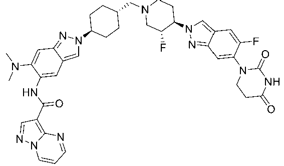 |
| Characterization 351 | | | Characterization | | |
| LCMS (ESI) m/z: [M+1] = 746.46. ¹H NMR (400 MHz, DMSO-*d*₆) δ 10.85 (s, 1H), 10.84 (s, 1H), 9.37 (dd, *J =* 7.0, 1.7 Hz, 1H), 8.95 (dd, *J* = 4.1, 1.7 Hz, 1H), 8.73 (s, 1H), 8.70 (s, 1H), 8.47 (s, 1H), 8.30 (s, 1H), 7.61 (d, *J =* 9.0 Hz, 1H), 7.55 (s, 1H), 7.37 (s, 1H), 7.33 (dd, *J =* 7.0, 4.2 Hz, 1H), 7.15 (d, *J =* 9.0 Hz, 1H), 5.67-5.39 (m, 1H), 5.12-4.88 (m, 1H), 4.49-4.36 (m, 1H), 3.92 (dd, *J =* 11.4, 4.9 Hz, 1H), 3.28-3.04 (m, 2H), 2.77 (s, 6H), 2.71 - 2.60 (m, 1H), 2.38-1.84 (m, 15H), 1.36-1.14 (m, 2H). | | | LCMS (ESI) m/z: [M+1]=765.58. | | |

| Example No. | Compound No. | Structure | Example No. | Compound No. | Structure |
|---|---|---|---|---|---|
| Example 170 | TM-170 | 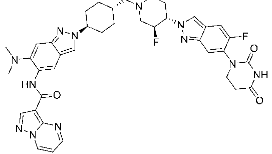 | Example 171 | TM-171 | 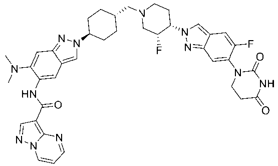 |
| Characterization | | | Characterization | | |
| LCMS (ESI) m/z: [M+1]=765.45. | | | LCMS (ESI) m/z: [M+1]=765.33. | | |
| Example 172 | TM-172 | 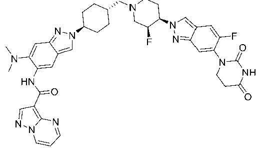 | Example 173 | TM-173 | 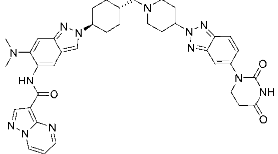 |
| Characterization | | | Characterization | | |
| LCMS (ESI) m/z: [M+1]=765.56. | | | LCMS (ESI) m/z: [M+1] = 730.82. ¹H NMR (400 MHz, DMSO-*d*₆) δ 10.85 (s, 1H), 10.49 (s, 1H), 9.36 (dd, *J =* 7.0, 1.7 Hz, 1H), 8.94 (dd, *J* = 4.2, 1.6 Hz, 1H), 8.72 (s, 1H), 8.70 (s, 1H), 8.29 (s, 1H), 8.02 (d, *J =* 8.9 Hz, 1H), 7.92 (d, *J =* 1.8 Hz, 1H), 7.41 (dd, *J =* 8.9, 1.8 Hz, 1H), 7.37 (s, 1H), 7.32 (dd, *J =* 7.0, 4.2 Hz, 1H), 5.03 - 4.67 (m, 1H), 4.38 (t, *J* = 11.7 Hz, 1H), 3.91 (t, *J =* 6.6 Hz, 2H), 3.04 (d, *J =* 9.5 Hz, 2H), 2.80-2.71 (m, 8H), 2.34 - 2.06 (m, 10H), 2.04 - 1.83 (m, 4H), 1.66 (s, 1H), 1.20-1.07 (m, 2H). | | |

| Example No. | Compound No. | Structure | Example No. | Compound No. | Structure |
|---|---|---|---|---|---|
| Example 174 | TM-174 | 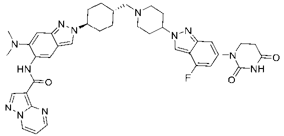 | Example 175 | TM-175 | 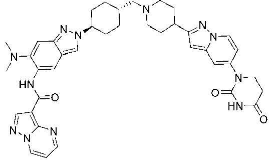 |
| Characterization 362 | | | Characterization | | |
| LCMS (ESI) m/z: [M+1] = 746.44. ¹H NMR (400 MHz, DMSO-*d*₆) δ 10.85 (s, 1H), 10.41 (s, 1H), 9.37 (dd, *J =* 7.0, 1.7 Hz, 1H), 8.95 (dd, *J* = 4.2, 1.7 Hz, 1H), 8.72 (s, 1H),8.70 (s, 1H), 8.64 (s, 1H), 8.29 (s, 1H), 7.41 - 7.30 (m, 3H), 6.88 (dd, *J =* 11.8, 1.5 Hz, 1H), 4.59 - 4.33 (m, 2H), 3.84 (t, *J =* 6.6 Hz, 2H), 3.08-2.95 (m, 2H), 2.77 (s, 6H), 2.73(t, *J =* 6.6 Hz, 2H), 2.26-2.07 (m, 10H), 1.99 - 1.85 (m, 4H), 1.72-1.60 (m, 1H), 1.20-1.08 (m, 2H). | | | LCMS (ESI) m/z: [M+1]=729.42 | | |

| Example No. | Compound No. | Structure | Example No. | Compound No. | Structure |
|---|---|---|---|---|---|
| Example 176 | TM-176 | 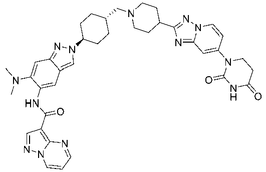 | Example 177 | TM-177 | 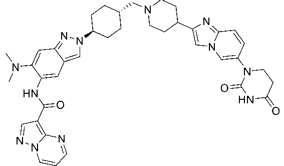 |
| Characterization | | | Characterization | | |
| LCMS (ESI) m/z: [M+1]=730.65. | | | LCMS (ESI) m/z: [M+1] = 729.34. ¹H NMR (400 MHz, DMSO-*d*₆) δ 10.85 (s, 1H), 10.46 (s, 1H), 9.36 (dd, *J =* 7.1, 1.6 Hz, 1H), 8.95 (dd, *J* = 4.3, 1.6 Hz, 1H), 8.71 (s, 1H), 8.70 (s, 1H), 8.54 (d, *J* = 2.0 Hz, 1H), 8.29 (s, 1H), 7.71 (s, 1H), 7.47 (d, *J* = 9.5 Hz, 1H), 7.37 (s, 1H), 7.33 (dd, *J =* 7.0, 4.1 Hz, 1H), 7.20 (dd, *J* = 9.6, 2.0 Hz, 1H), 4.42-4.34 (m, 1H), 3.77 (t, *J =* 6.6 Hz, 2H), 2.98-2.88 (m, 2H), 2.79-2.71 (m, 8H), 2.70-2.62 (m, 1H), 2.27 - 1.85 (m, 12H), 1.77-1.57 (m, 4H), 1.20-1.04 (m, 2H). | | |
| Example 178 | TM-178 | 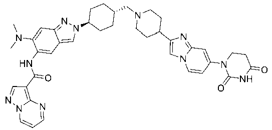 | Example 179 | TM-179 | 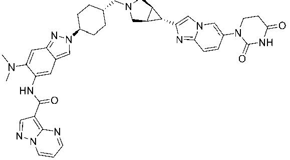 |
| Characterization | | | Characterization | | |
| LCMS (ESI) m/z: [M+1] = 729.38. ¹H NMR (400 MHz, DMSO-*d*₆) δ 10.85 (s, 1H), 10.48 (s, 1H), 9.36 (dd, *J =* 6.9, 1.7 Hz, 1H), 8.95 (dd, *J =* 4.2, 1.6 Hz, 1H), 8.72 (s, 1H), 8.70 (s, 1H), 8.40 (d, *J =* 7.4 Hz, 1H), 8.29 (s, 1H), 7.66 (s, 1H), 7.42 - 7.29 (m, 3H), 6.91 (dd, *J =* 7.3, 2.1 Hz, 1H), 4.43-4.33 (m, 1H), 3.85 (t, *J =* 6.6 Hz, 3H), 3.03-2.88 (m, 2H), 2.80-2.71 (m, 8H), 2.70-2.62 (m, 1H), 2.28 - 1.85 (m, 12H), 1.76-1.59 (m, 4H), 1.19-1.03 (m, 2H). | | | LCMS (ESI) m/z: [M+1]=727.34 | | |

| Example No. | Compound No. | Structure | Example No. | Compound No. | Structure |
|---|---|---|---|---|---|
| Example 180 | TM-180 | 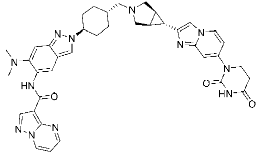 | Example 181 | TM-181 | 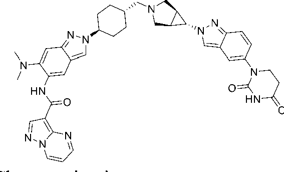 |
| Characterization | | | Characterization | | |
| LCMS (ESI) m/z: [M+1]=727.28 | | | LCMS (ESI) m/z: [M+1] = 727.42. ¹H NMR (400 MHz, DMSO-*d*₆) δ 10.85 (s, 1H), 10.32 (s, 1H), 9.36 (dd, *J =* 7.0, 1.6 Hz, 1H), 8.94 (dd, *J* = 4.2, 1.6 Hz, 1H), 8.72 (s, 1H), 8.70 (s, 1H), 8.50 (s, 1H), 8.29 (s, 1H), 7.54 (dd, *J* = 5.6, 3.5 Hz, 2H), 7.37 (s, 1H), 7.33 (dd, *J* = 7.0, 4.2 Hz, 1H), 7.18 (dd, *J* = 9.2, 1.9 Hz, 1H), 4.47 - 4.31 (m, 1H), 4.25 (s, 1H), 3.78 (t, *J =* 6.7 Hz, 2H), 3.23 (d, *J =* 8.9 Hz, 2H), 2.77 (s, 6H), 2.72 (t, *J =* 6.7 Hz, 2H), 2.47 - 2.39 (m, 2H), 2.37 - 2.26 (m, 4H), 2.13 (d, *J =* 12.0 Hz, 2H), 1.95 (dd, *J =* 25.8, 12.9 Hz, 4H), 1.61-1.49 (m, 1H), 1.18-1.03 (m, 2H). | | |

| Example No. | Compound No. | Structure | Example No. | Compound No. | Structure |
|---|---|---|---|---|---|
| Example 182 | TM-182 | 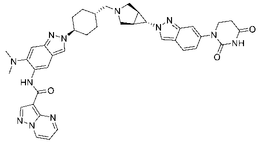 | Example 183 | TM-183 | 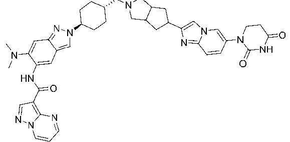 |
| Characterization | | | Characterization | | |
| LCMS (ESI) m/z: [M+1] = 727.52. ¹H NMR (400 MHz, DMSO-*d*₆) δ 10.85 (s, 1H), 10.35 (s, 1H), 9.37 (dd, *J =* 7.0, 1.7 Hz, 1H), 8.95 (dd, *J* = 4.2, 1.6 Hz, 1H), 8.72 (s, 1H), 8.70 (s, 1H), 8.49 (s, 1H), 8.30 (s, 1H), 7.63 (d, *J =* 8.9 Hz, 1H), 7.45 (s, 1H), 7.37 (s, 1H), 7.34 - 7.30 (m, 1H), 7.01 (dd, *J =* 8.9, 1.8 Hz, 1H), 4.43-4.35 (m, 1H), 4.24 (s, 1H), 3.82 (t, *J* = 6.7 Hz, 2H), 3.24 (d, *J* = 8.9 Hz, 2H), 2.46-1.53 (m, 13H), 1.19-1.08 (m, 2H). | | | LCMS (ESI) m/z: [M+1]=755.32 | | |

| Example No. | Compou nd No. | Structure | Example No. | Compou nd No. | Structure |
|---|---|---|---|---|---|
| Example 184 | TM-184 | 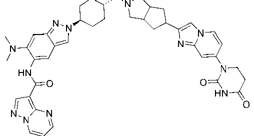 | Example 185 | TM-185 | 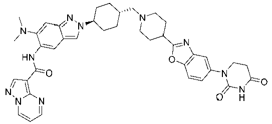 |
| Characterization | | | Characterization | | |
| LCMS (ESI) m/z: [M+1]=755.42 | | | LCMS (ESI) m/z: [M+1] = 730.73. ¹H NMR (400 MHz, DMSO-*d*₆) δ 10.85 (s, 1H), 10.42 (s, 1H), 9.36 (dd, *J =* 7.0, 1.6 Hz, 1H), 8.94 (dd, *J* = 4.2, 1.6 Hz, 1H), 8.72 (s, 1H), 8.70 (s, 1H), 8.28 (s, 1H), 7.68 (dd, *J* = 5.2, 3.2 Hz, 2H), 7.38 (s, 1H), 7.34 - 7.29 (m, 2H), 4.41 - 4.31 (m, 2H), 3.82 (t, *J =* 6.6 Hz, 2H), 3.05-2.95 (m, 1H), 2.93-2.82 (m, 2H), 2.80-2.70 (m, 8H), 2.76 (s, 6H), 2.73 (d, *J* = 6.6 Hz, 1H), 2.22 - 2.04 (m, 8H), 1.99-1.77 (m, 6H), 1.69-1.56 (m, 1H), 1.16 - 1.05 (m, 2H). | | |

| Example No. | Compound No. | Structure | Example No. | Compound No. | Structure |
|---|---|---|---|---|---|
| Example 186 | TM-186 | 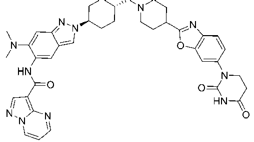 | Example 187 | TM-187 | 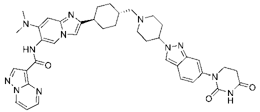 |
| Characterization | | | Characterization | | |
| LCMS (ESI) m/z: [M+1] = 730.45. ¹H NMR (400 MHz, DMSO-*d*₆) δ 10.85 (s, 1H), 10.38 (s, 1H), 9.36 (dd, *J =* 7.0, 1.6 Hz, 1H), 8.94 (dd, *J* = 4.2, 1.7 Hz, 1H), 8.72 (s, 1H), 8.70 (s, 1H), 8.28 (s, 1H), 7.72 - 7.63 (m, 2H), 7.43 - 7.28 (m, 3H), 4.44 - 4.28 (m, 1H), 3.81 (t, *J =* 6.7 Hz, 2H), 3.07-2.96 (m, 1H), 2.96 - 2.82 (m, 2H), 2.81-2.71 (m, 8H), 2.22-2.03 (m, 8H), 1.99 - 1.79 (m, 6H), 1.70-1.57 (m, 1H), 1.17 - 1.03 (m, 2H). | | | LCMS (ESI) m/z: [M+1]=729.38 | | |

| Example No. | Compound No. | Structure | Example No. | Compound No. | Structure |
|---|---|---|---|---|---|
| Example 188 | TM-188 | 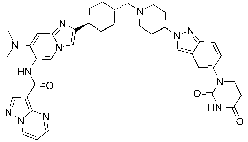 | Example 189 | TM-189 | 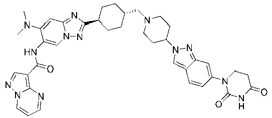 |
| Characterization | | | Characterization | | |
| LCMS (ESI) m/z: [M+1]=729.46 | | | LCMS (ESI) m/z: [M+1]=730.44 | | |

| Example No. | Compound No. | Structure | Example No. | Compound No. | Structure |
|---|---|---|---|---|---|
| Example 190 | TM-190 | 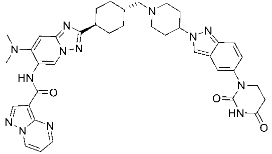 | Example 191 | TM-191 | 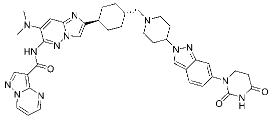 |
| Characterization | | | Characterization | | |
| LCMS (ESI) m/z: [M+1]=730.53 | | | LCMS (ESI) m/z: [M+1]=730.62. | | |

| Example No. | Compound No. | Structure | Example No. | Compound No. | Structure |
|---|---|---|---|---|---|
| Example 192 | TM-192 | 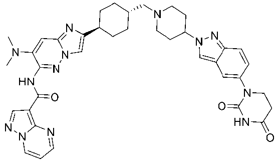 | Example 193 | TM-193 | 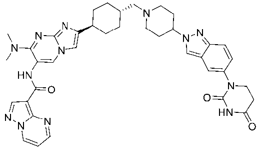 |
| Characterization | | | Characterization | | |
| LCMS (ESI) m/z: [M+1]=730.52 | | | LCMS (ESI) m/z: [M+1]=730.46 | | |
| Example 194 | TM-194 | 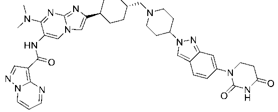 | Example 195 | TM-195 | 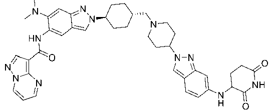 |
| Characterization | | | Characterization | | |
| LCMS (ESI) m/z: [M+1]=730.48 | | | LCMS (ESI) m/z: [M+1] = 743.73. ¹H NMR (400 MHz, DMSO-*d*₆) δ 10.91 (s, 1H), 10.88 (s, 1H), 9.42 (dd, *J =* 7.0, 1.7 Hz, 1H), 9.01 (dd, *J* = 4.2, 1.6 Hz, 1H), 8.81 - 8.75 (m, 2H), 8.39 (s, 1H), 8.18 (s, 1H), 7.47-7.37 (m, H), 7.39 (dd, *J =* 7.0, 4.2 Hz, 1H), 6.70 (dd, *J* = 9.0, 1.9 Hz, 1H), 6.59 (s, 1H), 5.86 (d, *J* = 7.1 Hz, 1H), 4.55 - 4.33 (m, 3H), 3.04 (s, 2H), 2.92 - 2.79 (m, 7H), 2.73 - 2.63 (m, 1H), 2.40 - 2.12 (m, 10H), 2.07 - 1.87 (m, 6H), 1.80-1.63 (m, 1H), 1.25-1.13 (m, 2H). | | |

| Example No. | Compound No. | Structure | Example No. | Compound No. | Structure |
|---|---|---|---|---|---|
| Example 196 | TM-196 | 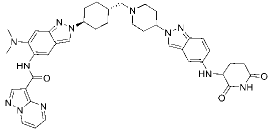 | Example 197 | TM-197 | 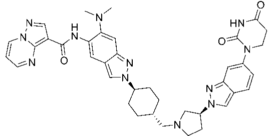 |
| Characterization | | | Characterization | | |
| LCMS (ESI) m/z: [M+1] = 743.87. ¹H NMR (400 MHz, DMSO-*d*₆) δ 10.85 (s, 1H), 10.80 (s, 1H), 9.36 (dd, *J =* 7.0, 1.6 Hz, 1H), 8.94 (dd, *J* = 4.2, 1.7 Hz, 1H), 8.72 (s, 1H), 8.70 (s, 1H), 8.29 (s, 1H), 8.01 (s, 1H), 7.40-7.35 (m, 2H), 7.33 (dd, *J* = 7.0, 4.2 Hz, 1H), 6.87 (dd, *J =* 9.2, 2.1 Hz, 1H), 6.58 (d, *J* = 2.0 Hz, 1H), 5.60 (d, *J* = 7.0 Hz, 1H), 4.43 - 4.25 (m, 3H), 3.02 (br, 2H), 2.82-2.71 (m, 7H), 2.68-2.57 (m, 1H), 2.23 - 2.05 (m, 10H), 2.00 - 1.81 (m, 6H), 1.72-1.58 (m, 1H), 1.13 (q, *J* = 12.3 Hz, 2H). | | | LCMS (ESI) m/z: [M+1] = 715.36. ¹H NMR (400 MHz, DMSO-*d*₆) δ 10.85 (s, 1H), 10.36 (s, 1H), 9.36 (dd, *J =* 7.0, 1.7 Hz, 1H), 8.94 (dd, *J* = 4.2, 1.7 Hz, 1H), 8.71(s, 1H), 8.70 (s, 1H), 8.44 (s, 1H), 8.28 (s, 1H), 7.68 (d, *J =* 8.9 Hz, 1H), 7.50 (s, 1H), 7.37 (s, 1H), 7.33 (dd, *J =* 7.0, 4.2 Hz, 1H), 7.02 (dd, *J =* 8.9, 1.9 Hz, 1H), 5.26 - 5.18 (m, 1H), 4.42 - 4.33 (m, 1H), 3.83 (t, *J =* 6.7 Hz, 2H), 3.02 (dd, *J =* 9.8, 7.3 Hz, 1H), 2.95-2.87 (m, 2H), 2.76 (s, 6H), 2.73 (t, *J =* 6.6 Hz, 2H), 2.65-2.57 (m, 1H), 2.38 (d, *J* = 7.2 Hz, 2H), 2.25 - 1.86 (m, 8H), 1.72-1.55 (m, 1H), 1.15 (q, *J* = 12.1 Hz, 2H). | | |

| Example No. | Compound No. | Structure | Example No. | Compound No. | Structure |
|---|---|---|---|---|---|
| Example 198 | TM-198 | 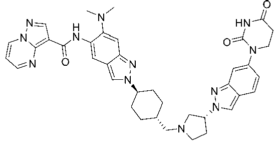 | Example 199 | TM-199 | 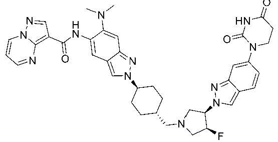 |
| Characterization | | | Characterization | | |
| LCMS (ESI) m/z: [M+1] = 715.51. ¹H NMR (400 MHz, DMSO-*d*₆) δ 10.85 (s, 1H), 10.36 (s, 1H), 9.36 (dd, *J =* 7.0, 1.7 Hz, 1H), 8.95 (dd, *J* = 4.2, 1.6 Hz, 1H), 8.71 (s, 1H), 8.70 (s, 1H), 8.45 (s, 1H), 8.28 (s, 1H), 7.68 (d, *J =* 8.9 Hz, 1H), 7.50 (d, *J* = 1.6 Hz, 1H), 7.36 (s, 1H), 7.33 (dd, *J =* 7.0, 4.2 Hz, 1H), 7.02 (dd, *J =* 8.9, 1.8 Hz, 1H), 5.26-5.16 (m, 1H), 4.44-4.33 (m, 1H), 3.83 (t, *J =* 6.7 Hz, 2H), 3.08-3.03 (m, 1H), 2.97-2.86 (m, 2H), 2.76 (s, 6H), 2.73 (t, *J =* 6.8 Hz, 2H), 2.68 - 2.62 (m, 1H), 2.39 (d, *J =* 7.0 Hz, 2H), 2.23 - 1.87 (m, 8H), 1.61 (s, 1H), 1.22 - 1.11 (m, 2H). | | | LCMS (ESI) m/z: [M+1] = 733.32. ¹H NMR (400 MHz, DMSO-*d*₆) δ 10.85 (s, 1H), 10.34 (s, 1H), 9.36 (dd, *J =* 7.0, 1.6 Hz, 1H), 8.95 (dd, *J* = 4.2, 1.6 Hz, 1H), 8.72 (s, H), 8.70 (s, 1H), 8.45 (s, 1H), 8.30 (s, 1H), 7.65 - 7.57 (m, 2H), 7.34 (s, 1H), 7.33 (dd, *J =* 7.0, 4.2 Hz, 1H), 7.22 (dd, *J* = 9.1, 2.1 Hz, 1H), 5.54 - 5.28 (m, 2H), 4.45 - 4.36 (m, 1H), 3.80 (t, *J =* 6.7 Hz, 2H), 3.44-3.36 (m, 1H), 3.24 - 3.14 (m, 2H), 3.12 - 2.91 (m, 2H), 2.81-2.68 (m, 8H), 2.16 (d, *J =* 12.0 Hz, 2H), 2.09 - 1.86 (m, 4H), 1.77-1.56 (m, 1H), 1.26-1.12 (m, 2H). | | |

| Example No. | Compound No. | Structure | Example No. | Compound No. | Structure |
|---|---|---|---|---|---|
| Example 200 | TM-200 | 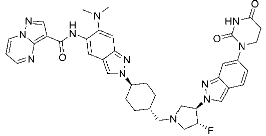 | Example 201 | TM-201 | 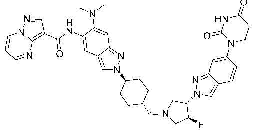 |
| Characterization | | | Characterization | | |
| LCMS (ESI) m/z: [M+1] = 733.33. ¹H NMR (400 MHz, DMSO-*d*₆) δ 10.85 (s, 1H), 10.37 (s, 1H), 9.36 (dd, *J =* 7.0, 1.7 Hz, 1H), 8.95 (dd, *J* = 4.2, 1.7 Hz, 1H), 8.72 (s, 1H), 8.70 (s, 1H), 8.54 (s, 1H), 8.29 (s, 1H), 7.71 (d, *J =* 8.9 Hz, 1H), 7.53 (s, 1H), 7.37 (s, 1H), 7.33 (dd, *J =* 7.0, 4.1 Hz, 1H) , 7.06 (dd, *J =* 8.9, 1.8 Hz, 1H), 5.59-5.31 (m, 2H), 5.47 - 5.31 (m, 2H), 4.44 - 4.34 (m, 1H), 3.84 (t, *J =* 6.7 Hz, 2H), 3.49 (t, *J =* 8.5 Hz, 1H), 3.15 (dd,*J* = 23.3, 11.4 Hz, 1H), 2.98-2.84 (m, 1H), 2.80-2.70 (m, 8H), 2.43-2.38 (m, 2H), 2.14 (d, *J =* 12.0 Hz, 2H), 2.04 - 1.87 (m, 4H), 1.73-1.55 (m, 1H), 1.25-1.10 (m, 2H). | | | LCMS (ESI) m/z: [M+1] = 733.53. ¹H NMR (400 MHz, DMSO-*d*₆) δ 10.84 (s, 1H), 10.37 (s, 1H), 9.34 (dd, *J =* 7.0, 1.6 Hz, 1H), 8.93 (dd, *J* = 4.2, 1.6 Hz, 1H), 8.70 (s, 1H), 8.69 (s, 1H), 8.52 (s, 1H), 8.28 (s, 1H), 7.76 - 7.63 (m, 1H), 7.52 (s, 1H), 7.35 (s, 1H), 7.32 (dd, ), 7.32 (dd, *J =* 7.0, 4.2 Hz, 1H), 7.05 (dt, *J* = 8.9, 1.7 Hz, 1H), 5.59 - 5.29 (m, 2H), 4.43 - 4.32 (m, 1H), 3.83 (t*, J =* 6.7 Hz, 2H), 3.48 (t, *J* = 8.6 Hz, 1H), 3.13 (dd, *J* = 23.4, 11.5 Hz, 1H), 2.99 - 2.82 (m, 1H), 2.80 - 2.67 (m, 9H), 2.42-2.36 (m, 1H), 2.23-2.08 (m, 2H), 2.02 - 1.85 (m, 4H), 1.74-1.52 (m, 2H), 1.23-1.08 (m, 2H). | | |

| Example No. | Compound No. | Structure | Example No. | Compound No. | Structure |
|---|---|---|---|---|---|
| Example 202 | TM-202 | 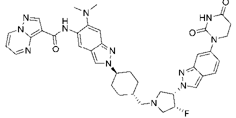 | Example 203 | TM-203 | 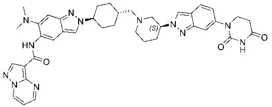 |
| Characterization | | | Characterization | | |
| LCMS (ESI) m/z: [M+1] = 733.45. ¹H NMR (400 MHz, DMSO-*d*₆) δ 10.85 (s, 1H), 10.37 (s, 1H), 9.36 (dd, *J =* 7.0, 1.7 Hz, 1H), 8.95 (dd, *J* = 4.2, 1.7 Hz, 1H), 8.72 (s, 1H), 8.70 (s, 1H), 8.45 (s, 1H), 8.30 (s, 1H), 7.71 (dd, *J* = 8.9, 0.7 Hz, 1H), 7.50 (s, 1H), 7.37 (s, 1H), 7.33 (dd, *J* = 7.0, 4.1 Hz, 1H), 7.04 (dd, *J* = 8.9, 1.8 Hz, 1H), 5.52-5.27 (m, 2H), 5.33 (tt, *J =* 12.9, 6.9 Hz, 2H), 4.46 - 4.33 (m, 1H), 3.84 (t, *J =* 6.7 Hz, 2H), 3.43-3.36 (m, 1H), 3.26 - 2.88 (m, 3H), 2.77 (s, 6H), 2.74 (t, *J =* 6.7 Hz, 2H), 2.22 - 1.87 (m, 6H), 1.73-1.56 (m, 1H), 1.18 (q, *J =* 12.3 Hz, 2H). | | | LCMS (ESI) m/z: [M+1] = 729.66. ¹H NMR (400 MHz, DMSO-*d*₆) δ 10.85 (s, 1H), 10.36 (s, 1H), 9.37 (dd, *J =* 7.0, 1.7 Hz, 1H), 8.95 (dd, *J* = 4.2, 1.7 Hz, 1H), 8.71 (s, 1H), 8.70 (s, 1H), 8.51 (s, 1H), 8.28 (s, 1H), 7.68 (d, *J =* 8.9 Hz, 1H), 7.50 (s, 1H), 7.36 (s, 1H), 7.33 (dd, *J =* 7.0, 4.2 Hz, 1H), 7.03 (dd, *J =* 8.9, 1.8 Hz, 1H), 4.69 - 4.59 (m, 1H), 4.42-433 (m, 1H), 3.83 (t, *J* = 6.7 Hz, 2H), 3.14 (d, *J* = 10.8 Hz, 1H), 2.83 (d, *J* = 10.5 Hz, 1H), 2.76 (s, 6H), 2.74 (t, *J =* 6.7 Hz, 2H), 2.27 - 1.68 (m, 15H), 1.18 - 1.09 (m, 2H). | | |

| Example No. | Compound No. | Structure | Example No. | Compound No. | Structure |
|---|---|---|---|---|---|
| Example 204 | TM-204 | 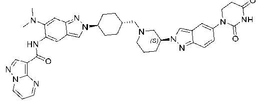 | Example 205 | TM-205 | 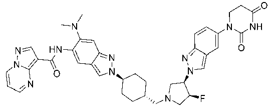 |
| Characterization | | | Characterization | | |
| LCMS (ESI) m/z: [M+1] = 729.66. ¹H NMR (400 MHz, DMSO-*d*₆) δ 10.85 (s, 1H), 10.33 (s, 1H), 9.37 (dd, *J =* 7.0, 1.7 Hz, 1H), 8.95 (dd, *J* = 4.2, 1.7 Hz, 1H), 8.71 (s, 1H),8.70 (s, 1H), 8.52 (s, 1H), 8.28 (s, 1H), 7.63-7.57 (m, 2H), 7.36 (s, 1H), 7.33 (dd, *J =* 7.0, 4.2 Hz, 1H), 7.20 (dd, *J =* 9.1, 2.0 Hz, 1H), 4.70-4.58 (m, 1H), 4.42 - 4.31 (m, 1H), 3.79 (t, *J =* 6.7 Hz, 2H), 3.14 (d, *J* = 9.9 Hz, 1H), 2.82 (d, *J =* 4.5 Hz, 1H), 2.76 (s, 6H), 2.73 (t, *J* = 6.7 Hz, 2H), 2.27 - 1.63 (m, 15H), 1.20-1.07 (m, 2H). | | | LCMS (ESI) m/z: [M+1] = 733.44. ¹H NMR (400 MHz, DMSO-*d*₆) δ 10.85 (s, 1H), 10.33 (s, 1H), 9.36 (dd, *J =* 7.0, 1.7 Hz, 1H), 8.95 (dd, *J* = 4.2, 1.7 Hz, 1H), 8.75 - 8.67 (m, 2H), 8.45 (s, 1H), 8.30 (s, 1H), 7.64 - 7.55 (m, 2H), 7.41 - 7.31 (m, 2H), 7.22 (dt, *J* = 9.0*,* 1.9 Hz, 1H), 5.52-5.28 (m, 1H), 4.54 - 4.32 (m, 1H),4.45-4.35 (m, 1H), 3.80 (td, *J =* 6.7, 2.0 Hz, 2H), 3.27 - 2.90 (m, 4H), 2.76 (s, 6H), 2.73 (t, *J =* 6.92, 2H), 2.65-2.58 (m, 1H), 2.29 - 1.84 (m, 7H), 1.67 (d, *J* = 23.8 Hz, 2H), 1.28 - 1.11 (m, 2H). | | |

| Example No. | Compound No. | Structure | Example No. | Compound No. | Structure |
|---|---|---|---|---|---|
| Example 206 | TM-206 | 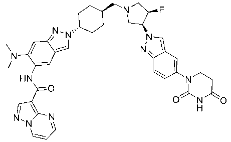 | Example 207 | TM-207 | 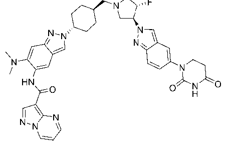 |
| Characterization | | | Characterization | | |
| LCMS (ESI) m/z: [M+1] = 733.62. ¹H NMR (400 MHz, DMSO-*d*₆) δ 10.85 (s, 1H), 10.33 (s, 1H), 9.35 (dt, *J =* 7.1, 1.3 Hz, 1H), 8.94 (dd, *J* = 4.2, 1.9 Hz, 1H), 8.72 (s, 1H), 8.70 (s, 1H), 8.45 (s, 1H), 8.29 (s, 1H), 7.65 - 7.57 (m, 2H), 7.36 (s, 1H), F7.32 (dd, *J =* 7.0, 4.2 Hz, 1H), | | | LCMS (ESI) m/z: [M+1] = 733.47. ¹H NMR (400 MHz, DMSO-*d*₆) δ 10.85 (s, 1H), 10.34 (s, 1H), 9.37 (dd, *J =* 7.0, 1.6 Hz, 1H), 8.95 (dd, *J* = 4.2, 1.7 Hz, 1H), 8.72 (s, 1H), 8.70 (s, 1H), 8.55 (s, 1H), 8.29 (s, 1H), 7.71 (d, *J =* 8.9 Hz, 1H), 7.53 (s, 1H), 7.37 (s, 1H), 7.33 (dd, *J =* 7.0, | | |
| 7.21 (dt, *J =* 9.1, 2.0 Hz, 1H), 5.53 - 5.28 (m, 2H), 4.44 - 4.34 (m, 1H), 3.80 (t, *J =* 6.7 Hz, 2H), 3.27 - 2.90 (m, 4H), 2.76 (s, 6H), 2.73 (t, *J =* 6.92, 2H),2.64-2.58 (m, 1H), 2.27-1.58 (m, 9H), 1.26-1.10 (m, 2H). | | | 4.1 Hz, 1H) , 7.06 (dd, *J =* 8.9, 1.8 Hz, 1H), 5.59-5.31 (m, 2H), 5.47 - 5.31 (m, 2H), 4.44 - 4.34 (m, 1H), 3.84 (t, *J =* 6.7 Hz, 2H), 3.49 (t, *J* = 8.5 Hz, 1H), 3.15 (dd, *J =* 23.3, 11.4 Hz, 1H), 2.98-2.84 (m, 1H), 2.80-2.70 (m, 8H), 2.43-2.38 (m, 2H), 2.14 (d, *J =* 12.0 Hz, 2H), 2.04 - 1.87 (m, 4H), 1.73-1.55 (m, 1H), 1.25-1.10 (m, 2H). | | |

| Example No. | Compound No. | Structure | Example No. | Compound No. | Structure |
|---|---|---|---|---|---|
| Example 208 | TM-208 | 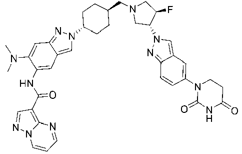 | Example 209 | TM-209 | 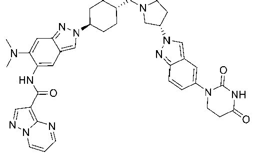 |
| Characterization | | | Characterization | | |
| LCMS (ESI) m/z: [M+1] = 733.87. ¹H NMR (400 MHz, DMSO-*d*₆) δ 10.85 (s, 1H), 10.34 (s, 1H), 9.36 (d, *J =* 7.1 Hz, 1H), 8.95 (d, *J* = 3.5 Hz, 1H), 8.71 (s, 1H), 8.70 (s, 1H), 8.55 (s, 1H), 8.29 (s, 1H), 7.66-7.60 (m, 2H), 7.37 (s, 1H), 7.33 (dd, *J =* 7.0, 4.2 Hz, 1H), 7.24 (d, *J =* 10.5 Hz, 1H), 5.64 - 5.24 (m, 2H), 4.46-4.32 (m, 1H), 3.80 (t, *J =* 6.6 Hz, 2H), 3.49 (t, *J* = 8*.*6 Hz, 1H), 3.14 (dd, *J =* 23.6, 11.1 Hz, 1H), 3.00-2.83 (m, 1H), 2.79-2.71 (m, 8H), 2.43-2.36 (m, 2H), 2.18-2.09 (m, 2H), 2.05-1.87(m, 4H), 1.71-1.57 (m, 1H), 1.14-1.12 (m, 2H). | | | LCMS (ESI) m/z: [M+1] = 715.55. ¹H NMR (400 MHz, DMSO-*d*₆) δ 10.85 (s, 1H), 10.32 (s, 1H), 9.36 (dd, *J =* 7.0, 1.7 Hz, 1H), 8.94 (dd, *J =* 4.2, 1.6 Hz, 1H), 8.79 - 8.65 (m, 2H), 8.45 (s, 1H), 8.28 (s, 1H), 7.64 - 7.54 (m, 2H), 7.40 - 7.30 (m, 2H), 7.20 (dt, *J* = 9.2*,* 1.7 Hz, 1H), 5.29-5.22 (m, 1H), 4.43 - 4.31 (m, 1H), 3.78 (td, *J =* 6.7, 1.8 Hz, 2H), 3.11-2.86 (m, 3H), 2.76 (s, 6H), 2.72 (t, *J =* 6.7 Hz, 2H), 2.69-2.60 (m, 1H), 2.44 -2.35 (m, 2H), 2.40 (d, *J =* 6.8 Hz, 1H), 2.28 - 2.09 (m, 3H), 2.05 - 1.84 (m, 4H), 1.73-1.55 (m, 2H), 1.29 - 1.08 (m, 2H). | | |

| Example No. | Compound No. | Structure | Example No. | Compound No. | Structure |
|---|---|---|---|---|---|
| Example 210 | TM-210 | 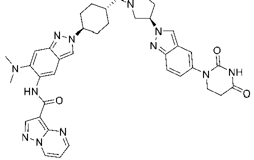 | Example 211 | TM-211 | 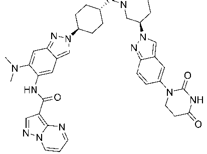 |
| Characterization | | | Characterization | | |
| LCMS (ESI) m/z: [M+1] = 715.67. ¹H NMR (400 MHz, DMSO-*d*₆) δ 10.85 (s, 1H), 10.33 (s, 1H), 9.36 (dd, *J =* 7.0, 1.6 Hz, 1H), 8.95 (dd, *J =* 4.2, 1.6 Hz, 1H), 8.76 - 8.65 (m, 2H), 8.45 (d, *J =* 4.0 Hz, 1H), 8.28 (s, 1H), 7.69 - 7.55 (m, 2H), 7.46 - 7.29 (m, 2H), 7.20 (dt, *J* = 9.1*,* 1.8 Hz, 1H), 5.27-5.18 (m, 1H), 4.53 - 4.23 (m, 1H), 3.79 (td, *J =* 6.7, 1.8 Hz, 2H), 3.10 - 2.86 (m, 3H), 2.77 (s, 6H), 2.72 (t, *J =* 6.7 Hz, 2H), 2.67 - 2.58 (m, 1H), 2.39 (d, *J =* 7.2 Hz, 2H), 2.33 - 2.10 (m, 2H), 2.08 - 1.84 (m, 4H), 1.74-1.55 (m, 2H), 1.31 - 1.07 (m, 2H). | | | LCMS (ESI) m/z: [M+1] = 729.64. ¹H NMR (400 MHz, DMSO-*d*₆) δ 10.85 (s, 1H), 10.33 (s, 1H), 9.36 (dd, *J =* 7.0, 1.7 Hz, 1H), 8.94 (dd, *J* = 4.2, 1.7 Hz, 1H), 8.71 (s, 1H), 8.70 (s, 1H), 8.51 (s, 1H), 8.27 (s, 1H), 7.65 - 7.49 (m, 2H), 7.42 - 7.26 (m, 2H), 7.20 (dd, *J =* 9.0, 2.1 Hz, 1H), 4.69-4.59 (m, 1H), 4.42-4.30 (m, 1H), 3.79 (t, *J =* 6.7 Hz, 2H), 3.13 (d, *J =* 10.8 Hz, 1H), 2.85-2.69 (m, 9H), 2.24 (t, *J =* 7.0 Hz, 2H), 2.17-2.05 (m, 4H), 2.01 - 1.74 (m, 6H), 1.72-1.58 (m, 2H), 1.19-1.04 (m, 2H). | | |

| Example No. | Compound No. | Structure | Example No. | Compound No. | Structure |
|---|---|---|---|---|---|
| Example 212 | TM-212 | 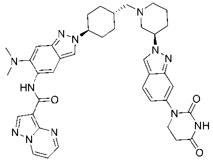 | Example 213 | TM-213 | 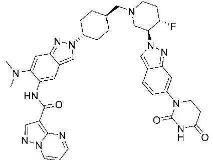 |
| Characterization | | | Characterization | | |
| LCMS (ESI) m/z: [M+1] = 729.80. ¹H NMR (400 MHz, DMSO-*d*₆) δ 10.85 (s, 1H), 10.36 (s, 1H), 9.35 (dt, *J =* 7.0, 1.5 Hz, 1H), 8.93 (dd, *J* = 4.1, 1.7 Hz, 1H), 8.71 (s, 1H), 8.70 (s, 1H), 8.49 (s, 1H), 8.26 (s, 1H), 7.67 (d, *J =* 8.9 Hz, 1H), 7.50 (s, 1H), 7.48 - 7.28 (m, 2H), 7.02 (dd, *J* = 8.9, 1.8 Hz, 1H), 4.63 (tt, *J =* 9.8, 4.0 Hz, 1H), 4.44 - 4.30 (m, 1H), 3.83 (t, *J* = 6.7 Hz, 2H), 3.13 (d, *J=* 10.0 Hz, 1H), 2.87-2.70 (m, 9H), 2.50-2.42 (m, 1H), 2.30- | | | LCMS (ESI) m/z: [M+1] = 747.37. ¹H NMR (400 MHz, DMSO-*d*₆) δ 10.85 (s, 1H), 10.38 (s, 1H), 9.35 (dd, *J =* 7.0, 1.7 Hz, 1H), 8.93 (dd, *J* = 4.2, 1.7 Hz, 1H), 8.71 (d, *J* = 5.1 Hz, 2H), 8.55 (d, *J =* 1.0 Hz, 1H), 8.26 (s, 1H), 7.70 (d, *J =* 8.9 Hz, 1H), 7.55 - 7.49 (m, 1H), 7.39 - 7.30 (m, 2H), 7.05 (dd, *J =* 8.9, 1.8 Hz, 1H), 5.26 - 5.03 (m, 1H), 4.82 (qd, *J =* 10.1, 4.4 Hz, 1H), 4.43 - 4.28 (m, 1H), 3.84 (t, *J =* 6.6 Hz, 2H), 3.20 - 3.10 (m, 1H), 2.97 | | |
| 2.01 (m, 6H), 2.01-1.73 (m, 5H), 1.73-1.55 (m, 2H), 1.18-1.02 (m, 2H). | | | (d, *J* = 11.5 Hz, 1H), 2.76 (s, 8H), 2.63 (t, *J* = 11.0 Hz, 1H), 2.37 - 2.03 (m, 6H), 1.92 (dd, *J =* 32.2, 12.8 Hz, 5H), 1.61 (s, 1H), 1.10 *J =* 12.4 Hz, 2H). | | |

| Example No. | Compound No. | Structure | Example No. | Compound No. | Structure |
|---|---|---|---|---|---|
| Example 214 | TM-214 | 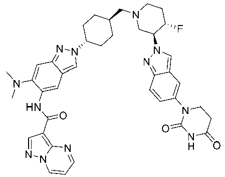 | Example 215 | TM-215 | 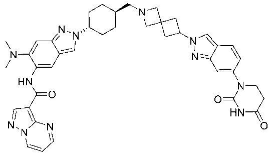 |
| Characterization | | | Characterization | | |
| LCMS (ESI) m/z: [M+1] = 747.68. ¹H NMR (400 MHz, DMSO-*d*₆) δ 10.85 (s, 1H), 10.34 (s, 1H), 9.36 (dd, *J =* 7.0, 1.6 Hz, 1H), 8.94 (dd, *J* = 4.2, 1.7 Hz, 1H), 8.70 (d, *J =* 3.9 Hz, 2H), 8.57 (s, 1H), 8.27 (s, 1H), 7.62 (dd, *J =* 5.7, 3.5 Hz, 2H), 7.40 - 7.30 (m, 2H), 7.23 (dd, *J* = 9.3*,* 1.9 Hz, 1H), 5.15 (ddd, *J =* 50.9, 15.8, 9.9 Hz, 1H), 4.81 (td, *J =* 10.0, 4.4 Hz, 1H), 4.35 (d, *J =* 11.5 Hz, 1H), 3.80 (t, *J =* 6.7 Hz, 2H), 3.15 (d, *J =* 10.8 Hz, 1H), 2.98 (d, *J =* 10.7 Hz, 1H), 2.75 (d, *J* = 9.7 Hz, 8H), 2.69 - 2.58 (m, 1H), 2.36 - 2.07 (m, 6H), 2.03 - 1.77 (m, 5H), 1.62 (s, 1H), 1.19 - 1.01 (m, 2H). | | | LCMS (ESI) m/z: [M+1] = 741.84. ¹H NMR (400 MHz, DMSO-*d*₆) δ 10.85 (s, 1H), 10.36 (s, 1H), 9.36 (dd, *J =* 7.0, 1.7 Hz, 1H), 8.95 (dd, *J* = 4.2, 1.7 Hz, 1H), 8.71 (d, *J* = 3.8 Hz, 2H), 8.42 - 8.26 (m, 2H), 7.65 (d, *J =* 8.9 Hz, 1H), 7.51 (s, 1H), 7.40 - 7.30 (m, 2H), 7.02 (dd, *J =* 8.9, 1.8 Hz, 1H), 5.06 (q, *J =* 8.0 Hz, 1H), 4.40 - 4.29 (m, 1H), 3.83 (t, *J =* 6.6 Hz, 2H), 2.80 - 2.66 (m, 12H), 2.29 (d, *J =* 6.6 Hz, 2H), 2.16 - 2.06 (m, 3H), 1.89 (t, *J =* 11.4 Hz, 4H), 1.62 (s, 2H), 1.37 (s, 1H), 1.26 - 1.02 (m, 3H). | | |

| Example No. | Compound No. | Structure | Example No. | Compound No. | Structure |
|---|---|---|---|---|---|
| Example 216 | TM-216 | 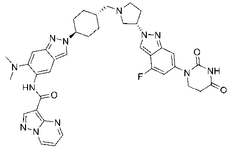 | Example 217 | TM-217 | 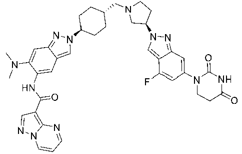 |
| Characterization | | | Characterization | | |
| LCMS (ESI) m/z: [M+1] = 733.33. ¹H NMR (400 MHz, DMSO-*d*₆) δ 10.85 (s, 1H), 10.42 (s, 1H), 9.36 (dd, *J =* 7.0, 1.7 Hz, 1H), 8.94 (dd, *J* = 4.2, 1.6 Hz, 1H), 8.78 - 8.67 (m, 2H), 8.61 (d, *J* = 5.2 Hz, 1H), 8.28 (s, 1H), 7.46 - 7.22 (m, 3H), 6.88 (dt, *J =* 11.8, 1.7 Hz, 1H), 5.29-5.19 (m, 1H), 4.46 - 4.32 (m, 1H), 3.84 (t, *J =* 6.6 Hz, 2H), 3.09-2.87 (m, 3H), 2.73 (t, *J =* 6.7 Hz, 2H), 2.65-2.59 (m, 1H), 2.38 (d, *J* = 7.1 Hz, 2H), 2.30 - 2.08 (m, 3H), 2.06-1.08 (m, 4H), 1.72-1.54 (m, 2H), 1.21-1.08 (m, 2H). | | | LCMS (ESI) m/z: [M+1] = 733.54. ¹H NMR (400 MHz, DMSO-*d*₆) δ 10.85 (s, 1H), 10.42 (s, 1H), 9.36 (dd, *J =* 7.0, 1.7 Hz, 1H), 8.94 (dd, *J* = 4.2, 1.6 Hz, 1H), 8.80 - 8.68 (m, 2H), 8.61 (d, *J* = 5.1 Hz, 1H), 8.28 (s, 1H), 7.46 - 7.21 (m, 3H), 6.88 (dt, *J =* 11.8, 1.8 Hz, 1H), 5.29-5.18 (m, 1H), 4.43-4.33 (m, 1H), 3.84 (t, *J =* 6.6 Hz, 2H), 3.08-2.87 (m, 3H), 2.76 (s, 6H), 2.73 (t, *J =* 6.7 Hz, 2H), 2.62 (d, *J* = 6.2 Hz, 0H), 2.38 (d, *J =* 7.2 Hz, 2H), 2.30 - 2.08 (m, 3H), 2.05-1.83 (m, 4H), 1.71-1.56 (m, 2H), 1.22-1.07 (m, 2H). | | |

| Example No. | Compound No. | Structure | Example No. | Compound No. | Structure |
|---|---|---|---|---|---|
| Example 218 | TM-218 | 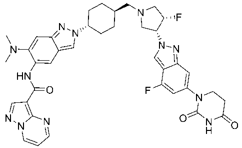 | Example 219 | TM-219 | 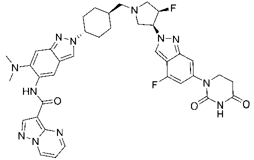 |
| Characterization | | | Characterization | | |
| LCMS (ESI) m/z: [M+1] = 751.32. ¹H NMR (400 MHz, DMSO-*d*₆) δ 10.85 (s, 1H), 10.43(, 1H), 9.37 (dd, *J =* 7.0, 1.7 Hz, 1H), 8.95 (dd, *J* = 4.2, 1.7 Hz, 1H), 8.72 (s, 1H), 8.70 (s, 1H), 8.63 (s, 1H), 8.30 (s, 1H), 7.41 - 7.36 (m, 2H), 7.33 (dd, *J =* 7.0, 4.2 Hz, 1H), 6.91 (dd, *J =* 11.8, 1.5 Hz, 1H), 5.54-5.28 (m, 1H), 4.47 - 4.36 (m, 1H), 3.86 (t, *J =* 6.7 Hz, 2H), 3.44 (t, *J =* 8.5 Hz, 1H), 3.28 - 3.09 (m, 2H), 3.05-2.90 (m, 2H), 2.77 (s, 6H), | | | LCMS (ESI) m/z: [M+1] = 751.47. ¹H NMR (400 MHz, DMSO-*d*₆) δ 10.85 (s, 1H), 10.43 (s, 1H), 9.37 (dd, *J =* 7.0, 1.7 Hz, 1H), 8.95 (dd, *J* = 4.2, 1.7 Hz, 1H), 8.72 (s, 1H), 8.70 (s, 1H), 8.63 (s, 1H), 8.30 (s, 1H), 7.41 - 7.37 (m, 2H), 7.33 (dd, *J =* 7.0, 4.2 Hz, 1H), 6.91 (dd, *J =* 11.8, 1.5 Hz, 1H), 5.56-5.28 (m, 2H), 4.46-4.34 (m, 1H), 3.86 (t, *J =* 6.7 Hz, 2H), 3.44 (t, *J =* 8.4 Hz, 1H), 3.27 - 3.18 (m, 2H), 3.05-2.89 (m, 2H), 2.77 (s, 6H), 2.74 (t, *J* | | |
| 2.74 (t, *J =* 6.8 Hz, 2H), 2.20-1.86 (m, 6H), 1.75-1.54 (m, 2H), 1.46-1.30 (m, 1H), 1.27-1.12 (m, 2H). | | | = 6.7 Hz, 2H), 2.24-1.86 (m, 6H), 1.76-1.52 (m, 1H), 1.47-1.28 (m, 1H), 1.26-1.12 (m, 2H). | | |

| Example No. | Compound No. | Structure | Example No. | Compound No. | Structure |
|---|---|---|---|---|---|
| Example 220 | TM-220 | 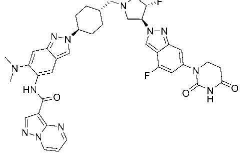 | Example 221 | TM-221 | 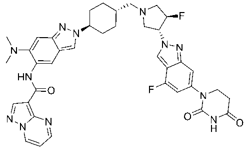 |
| Characterization | | | Characterization | | |
| LCMS (ESI) m/z: [M+1] = 751.65. ¹H NMR (400 MHz, DMSO-*d*₆) δ 10.85 (s, 1H), 10.44 (s, 1H), 9.35 (dd, *J =* 7.0, 1.7 Hz, 1H), 8.94 (dd, *J* = 4.3, 1.6 Hz, 1H), 8.79 - 8.60 (m, 3H), 8.28 (s, 1H), 7.52 - 7.25 (m, 3H), 6.93 (dd, *J =* 11.8, 1.5 Hz, 1H), 5.60-5.31 (m, 1H), 4.44-4.33 (m, 1H), 3.85 (t, *J =* 6.6 Hz, 2H), 3.50 (t, *J =* 8.6 Hz, 1H), 3.14 (dd, *J =* 23.5, 11.4 Hz, 2H), 2.91 (ddd, *J =* 30.8, 11.5, 5.6 Hz, 1H), 2.76 (s, 7H), 2.73 (t, *J =* 6.8 Hz, 3H), 2.43-2.35 (m, 2H), 2.24-1.84 (m, 6H), 1.73-1.54 (m, 1H), 1.26-1.08 (m, 2H). | | | LCMS (ESI) m/z: [M+1] = 751.35. ¹H NMR (400 MHz, DMSO-*d*₆) δ 10.85 (s, 1H), 10.44 (s, 1H), 9.35 (dd, *J =* 7.0, 1.6 Hz, 1H), 9.02 - 8.89 (m, 1H), 8.79 - 8.65 (m, 3H), 8.28 (s, 1H), 7.43 - 7.28 (m, 3H), 6.93 (d, *J =* 11.8 Hz, 1H), 5.63-5.29 (m, 1H), 4.45-4.31 (m, 1H), 5.51 - 5.38 (m, 1H), 4.46 - 4.30 (m, 1H), 3.85 (t, *J =* 6.7 Hz, 2H), 3.50 (t*, J =* 8.6 Hz, 1H), 3.14 (dd, *J* = 23.3, 11.4 Hz, 1H), 2.98-2.83 (m, 1H), 2.76 (s, 7H), 2.72 (d, *J =* 6.7 Hz, 2H), 2.44-2.34 (m, 2H), 2.23-1.83 (m, 6H), 1.71-1.55 (m, 1H), 1.29-1.08 (m, 2H). | | |

| Example No. | Compound No. | Structure | Example No. | Compound No. | Structure |
|---|---|---|---|---|---|
| Example 222 | TM-222 | 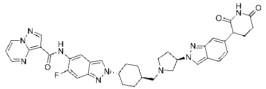 | Example 223 | TM-223 | 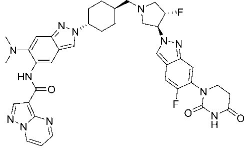 |
| Characterization | | | Characterization | | |
| LCMS (ESI) m/z: [M+1] = 690.28. ¹H NMR (400 MHz, DMSO-*d*₆) δ 10.35 (s, 1H), 10.18 (d, *J =* 3.5 Hz, 1H), 9.39 (dd, *J =* 7.0, 1.6 Hz, 1H), 8.92 (dd, *J =* 4.3, 1.6 Hz, 1H), 8.73 (s, 1H), 8.65 (d, *J =* 7.9 Hz, 1H), 8.46 - 8.41 (m, 1H), 7.67 (dd, *J =* 8.9, 4.1 Hz, 1H), 7.59 - 7.47 (m, 2H), 7.35 (dd, *J =* 7.0, 4.3 Hz, 1H), 7.02 (dd, *J =* 8.9, 1.9 Hz, 1H), 3.82 (t, *J =* 6.6 Hz, 2H), 3.08 - 2.83 (m, 5H), 2.73 (t, *J =* 6.6 Hz, 2H), 2.69 - 2.57 (m, 2H), 2.42 - 2.31 (m, 2H), 2.27 - 1.82 (m, 8H), 1.66 (s, 2H), 1.18 (dd, *J =* 24.2, 11.4 Hz, 2H). | | | LCMS (ESI) m/z: [M+1]=751.23. | | |

| Example No. | Compound No. | Structure | Example No. | Compound No. | Structure |
|---|---|---|---|---|---|
| Example 224 | TM-224 | 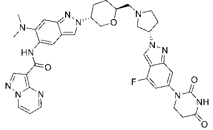 | Example 225 | TM-225 | 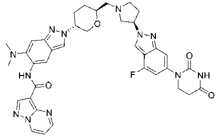 |
| Characterization | | | Characterization | | |
| LCMS (ESI) m/z: [M+1]=735.18. | | | LCMS (ESI) m/z: [M+1]=735.38. | | |

| Example No. | Compound No. | Structure | Example No. | Compound No. | Structure |
|---|---|---|---|---|---|
| Example 226 | TM-226 | 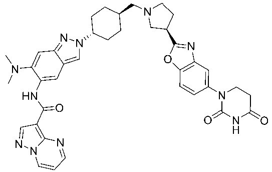 | Example 227 | TM-227 | 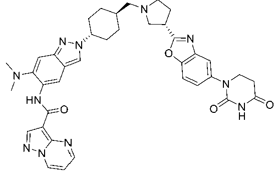 |
| Characterization | | | Characterization | | |
| LCMS (ESI) m/z: [M+1] = 716.54. ¹H NMR (400 MHz, DMSO-*d*₆) δ 10.85 (s, 1H), 10.38 (s, 1H), 9.35 (dd, *J =* | | | LCMS (ESI) m/z: [M+1] = 716.30. ¹H NMR (400 MHz, DMSO-*d*₆) δ 10.84 (s, 1H), 10.37 (s, 1H), 9.34 (dd, *J =* | | |
| 7.0, 1.7 Hz, 1H), 8.94 (dd, *J =* 4.1, 1.6 Hz, 1H), 8.71 (s, 1H), 8.70 (s, 1H), 8.28 (s, 1H), 7.71 - 7.64 (m, 2H), 7.41 - 7.28 (m, 3H), 4.43 - 4.32 (m, 1H), 3.81 (t, *J =* 6.6 Hz, 2H), 3.75-3.67 (m, 1H), 3.07 - 2.96 (m, 1H), 2.87-2.80 (m, 1H), 2.78-2.71 (m, 8H), 2.71-2.58 (m, 2H), 2.37-2.07 (m, 6H), 2.02-1.83 (m, 4H), 1.71-1.54 (m, 2H), 1.18-1.06 (m, 2H). | | | 7.0, 1.7 Hz, 1H), 8.93 (dd, *J =* 4.2, 1.7 Hz, 1H), 8.70 (s, 1H), 8.69 (s, 1H), 8.26 (s, 1H), 7.70 - 7.62 (m, 2H), 7.38 - 7.28 (m, 3H), 4.47 - 4.30 (m, 1H), 3.79 (t, *J =* 6.7 Hz, 2H), 3.74-3.65 (m, 1H), 3.06-2.96 (m, 1H), 2.81 - 2.57 (m, 11H), 2.37 - 2.03 (m, 6H), 1.99 - 1.78 (m, 4H), 1.69-1.51 (m, 2H), 1.18-1.04 (m, 2H). | | |

| Example No. | Compound No. | Structure | Example No. | Compound No. | Structure |
|---|---|---|---|---|---|
| Example 228 | TM-228 | 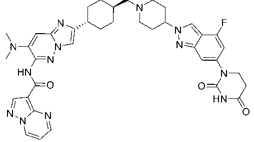 | Example 229 | TM-229 | 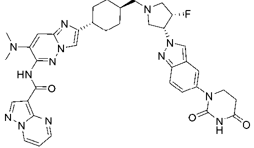 |
| Characterization | | | Characterization | | |
| LCMS (ESI) m/z: [M+1]=748.71. | | | LCMS (ESI) m/z: [M+1]=734.44. | | |

| Example No. | Compound No. | Structure | Example No. | Compound No. | Structure |
|---|---|---|---|---|---|
| Example 230 | TM-230 | 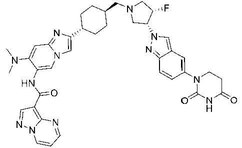 | Example 231 | TM-231 | 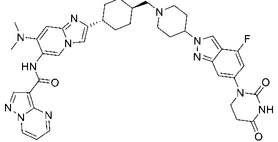 |
| Characterization | | | Characterization | | |
| LCMS (ESI) m/z: [M+1] = 733.44. ¹H NMR (400 MHz, DMSO-*d*₆) δ 10.49 (s, 1H), 10.34 (s, 1H), 9.44 (s, 1H), 9.40 (dd, *J* = 7.1*,* 1.7 Hz, 1H), 8.98 - 8.95 (m, 1H), 8.73 (s, 1H), 8.45 (d, *J =* 8.3 Hz, 1H), 8.10 (d, J = 27.8 Hz, 1H), 7.66-7.57 (m, 2H), 7.36 (dd, *J* = 7.0, 4.2 Hz, 1H), 7.24 - 7.16 (m, 2H), 5.50-5.24 (m, 1H), 3.80 (t, *J =* 6.8 Hz, 2H), 3.23-2.87 (m, 4H), 2.79 (s, 6H), 2.73 (t, *J =* 6.6 Hz, 2H), 2.13 - 1.04 (m, 12H). | | | LCMS (ESI) m/z: [M+1] = 747.58. ¹H NMR (400 MHz, DMSO-*d*₆) δ 10.48 (s, 1H), 10.42 (s, 1H), 9.45 (s, 1H), (d, *J =* 26.9 Hz, 2H), 9.40 (dd, *J =* 7.0, 1.6 Hz, 1H), 8.97 (dd, *J* = 4.2, 1.6 Hz, 1H), 8.73 (s, 1H), 8.64 (s, 1H), 7.98 (dd, *J* = 7.9, 1.2 Hz, 1H), 7.70 (dd, *J* = 7.7, 1.7 Hz, 1H), 7.48 (td, *J =* 7.5, 1.2 Hz, 1H), 7.40 - 7.35 (m, 2H), 7.23 (td, *J =* 7.6, 1.8 Hz, 1H), 6.88 (dd, *J =* 11.8, 1.4 Hz, 1H), 4.60 (d, *J* = 46.0 Hz, 3H), 4.03 (q, *J =* 7.1 Hz, 1H), 3.84 (t, *J =* 6.6 Hz, 2H), 2.93 (d, *J =* 7.4 Hz, 2H), 2.72 (d, *J =* 6.7 Hz, 3H), 2.11 (d, *J =* 34.6 Hz, 8H), 1.92 (d, *J =* 11.0 Hz, 3H), 1.59 - 1.38 (m, 5H). | | |

| Example No. | Compound No. | Structure | Example No. | Compound No. | Structure |
|---|---|---|---|---|---|
| Example 232 | TM-232 | 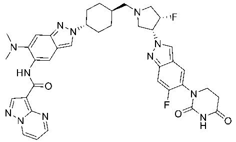 | Example 233 | TM-233 | 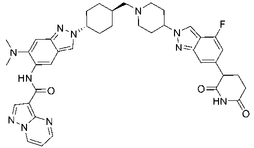 |
| Characterization | | | Characterization | | |
| LCMS (ESI) m/z: [M+1] = 751.36. ¹H NMR (400 MHz, DMSO-*d*₆) δ 10.85 (s, 1H), 10.47 (s, 1H), 9.37 (dd, *J =* 7.0, 1.6 Hz, 1H), 8.95 (dd, *J* = 4.2, 1.7 Hz, 1H), 8.72 (s, 1H), 8.70 (s, 1H), 8.55 (s, 1H), 8.30 (s, 1H), 7.84 (d, *J =* 4.4 Hz, 1H), 7.48 (d, *J =* 11.4 Hz, 1H), 7.37 (s, 1H), 7.33 (dd, *J =* 7.0, 4.1 Hz, 1H), 5.51 - 5.29 (m, 2H), 4.47-4.35 (m, 1H), 3.74 (t, *J =* 6.6 Hz, 2H), 3.45-3.36 (m, 2H), 3.24-3.05 (m, 3H), 3.04-2.91 (m, 1H), 2.77 (s, 6H), 2.73 (d, *J* = 6.7 Hz, 2H), 2.23-1.11 (m, 9H). | | | LCMS (ESI) m/z: [M+1] = 746.61. ¹H NMR (400 MHz, DMSO-*d*₆) δ 10.85 (s, 1H), 9.36 (dd, *J =* 7.0, 1.6 Hz, 1H), 8.94 (dd, *J* = 4.1, 1.7 Hz, 1H), 8.72 (s, 1H), 8.70 (s, 1H), 8.59 (s, 1H), 8.29 (s, 1H), 7.43 - 7.20 (m, 3H), 6.71 (d, *J =* 11.6 Hz, 1H), 4.67 - 4.21 (m, 1H), 3.96 (dd, *J =* 11.9, 4.8 Hz, 1H), 2.99 (d, *J =* 6.6 Hz, 1H), 2.77 (s, 6H), 2.74-2.62 (m, 1H), 2.58-2.53 (m, 1H), 2.39-1.83 (m, 14H), 1.71-1.57 (m, 1H), 1.20-1.04 (m, 2H). | | |
| Example 234 | TM-234 | 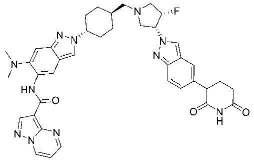 | Example 235 | TM-235 | 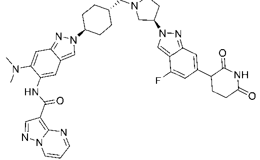 |
| Characterization | | | Characterization | | |
| LCMS (ESI) m/z: [M+1] = 732.64. ¹H NMR (400 MHz, DMSO-*d*₆) δ 10.85 (s, 1H), 10.83 (s, 1H), (d, *J* = 8.3 Hz, 2H), 9.36 (dd, *J* = 7.0*,* 1.7 Hz, 1H), 8.94 (dd, *J* = 4.2, 1.6 Hz, 1H), 8.77 - 8.66 (m, 2H), 8.44 - 8.26 (m, 2H), 7.61 - 7.49 (m, 2H), 7.42 - 7.28 (m, 2H), 7.10 (dd, *J* = 8.9, 1.8 Hz, 1H), 5.50-5.26 (m, 2H), 4.53 - 4.34 (m, 1H), 3.90 (dd, *J =* 11.3, 4.9 Hz, 1H), 3.45-2.89 (m, 4H), 2.76 (s, 6H), 2.73-2.58 (m, 2H), 2.28-1.57 (m, 11H), 1.19-1.12 (m, 2H). | | | LCMS (ESI) m/z: [M+1]=732.90. | | |

| Example No. | Compound No. | Structure | Example No. | Compound No. | Structure |
|---|---|---|---|---|---|
| Example 236 | TM-236 | 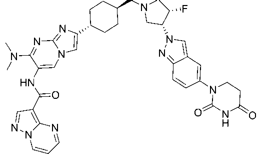 | Example 237 | TM-237 | 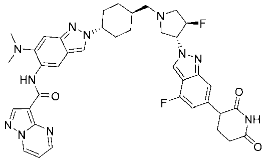 |
| Characterization | | | Characterization | | |
| LCMS (ESI) m/z: [M+1]=734.33. | | | LCMS (ESI) m/z: [M+1] = 750.75. ¹H NMR (400 MHz, DMSO-*d*₆) δ 10.85 (s, 1H), 10.66 (s, 1H), 9.37 (dd, *J =* 7.0, 1.7 Hz, 1H), 8.95 (dd, *J* = 4.2, 1.7 Hz, 1H), 8.73-8.69 (m, 2H),8.29 (s, 1H), 7.51 (s, 1H), 7.44 - 7.23 (m, 3H), 5.37-5.17 (m, 1H), 4.97-4.81 (m, 1H), 4.45-4.31 (m, 1H), 3.12-2.99 (m, 2H), 2.77 (s, 6H), 2.65-1.08 (m, 15H). | | |

| Example No. | Compound No. | Structure | Example No. | Compound No. | Structure |
|---|---|---|---|---|---|
| Example 238 | TM-238 | 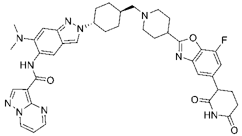 | Example 239 | TM-239 | 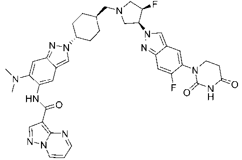 |
| Characterization | | | Characterization | | |
| LCMS (ESI) m/z: [M+1] = 747.41. ¹H NMR (400 MHz, DMSO-*d*₆) δ 10.87 (s, 1H), 10.85 (s, 1H), 9.37 (dd, *J =* 6.9, 1.7 Hz, 1H), 8.95 (dd, *J =* 4.2, 1.7 Hz, 1H), 8.72 (s, 1H), 8.70 (s, 1H), 8.29 (s, 1H), 7.46 (s, 1H), 7.37 (s, 1H), 7.33 (dd, *J* = 7.0, 4.2 Hz, 1H), 7.23 (dd, *J =* 11.5, 1.3 Hz, 1H), 4.43-4.31 (m, 1H), 4.01 (dd, *J =* 12.4, 4.8 Hz, 1H), 3.11-3.01 (m, 1H), 2.94-2.84 (m, 2H), 2.77 (s, 6H), 2.72 - 2.56 (m, 2H), 2.41-2.29 (m, 1H), 2.24-2.20 (m, 9H), 2.00-1.80 (m, 6H), 1.71-1.57 (m, 1H), 1.19-1.04 (m, 2H). | | | LCMS (ESI) m/z: [M+1] = 751.61. ¹H NMR (400 MHz, DMSO-*d*₆) δ 10.86 (s, 1H), 10.47 (s, 1H), 9.37 (dd, *J =* 7.0, 1.7 Hz, 1H), 8.95 (dd, *J* = 4.2, 1.6 Hz, 1H), 8.72 (s, 1H), 8.70 (s, 1H), 8.55 (s, 1H), 8.30 (s, 1H), 7.84 (d, *J =* 7.9 Hz, 1H), 7.48 (d, *J =* 11.2 Hz, 1H), 7.38 (s, 1H), 7.33 (dd, *J =* 7.1, 4.1 Hz, 1H), 5.50 - 5.26 (m, 2H), 4.45 - 4.35 (m, 1H), 3.74 (t, *J =* 6.6 Hz, 2H), 3.25-2.91 (m, 3H), 2.77 (s, 6H), 2.74 (t, *J =* 6.6 Hz, 2H), 2.23-1.11 (m, 9H). | | |

| Example No. | Compound No. | Structure | Example No. | Compound No. | Structure |
|---|---|---|---|---|---|
| Example 240 | TM-240 | 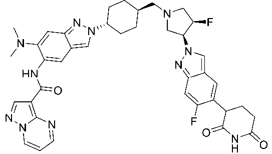 | Example 241 | TM-241 | 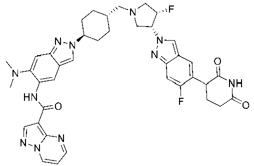 |
| Characterization | | | Characterization | | |
| LCMS (ESI) m/z: [M+1] = 750.93. ¹H NMR (400 MHz, DMSO-*d*₆) δ 10.86 (s, 1H), 10.85 (s, 1H), 9.37 (dd, *J =* 7.0, 1.7 Hz, 1H), 8.96 - 8.94 (m, 1H), 8.72 (s, 1H), 8.70 (s, 1H), 8.48 (s, 1H), 8.30 (s, 1H), 7.69 (d, *J =* 7.5 Hz, 1H), 7.37 (s, 1H), 7.36 - 7.31 (m, 2H), 5.51-5.23 (m, 2H), 4.47-4.33 (m, 1H), 4.06 (dd, *J =* 12.5, 5.1 Hz, 1H), 3.25-2.90 (m, 4H), 2.77 (s, 6H), 2.62-1.12 (m, 15H). | | | LCMS (ESI) m/z: [M+1] = 750.79. ¹H NMR (400 MHz, DMSO-*d*₆) δ 10.87 (s, 1H), 10.85 (s, 1H), 9.36 (dd, *J =* 7.0, 1.7 Hz, 1H), 8.94 (dd, *J* = 4.2, 1.6 Hz, 1H), 8.72 (s, 1H), 8.70 (s, 1H), 8.47 (s, 1H), 8.29 (s, 1H), 7.49 - 7.18 (m, 3H), 5.58 - 5.15 (m, 1H), 4.59 - 4.27 (m, 1H), 4.06 (dd, *J =* 12.4, 4.9 Hz, 1H), 3.29 - 2.88 (m, 4H), 2.77 (s, 6H), 2.62-1.12 (m, 15H). | | |

| Example No. | Compound No. | Structure | Example No. | Compound No. | Structure |
|---|---|---|---|---|---|
| Example 242 | TM-242 | 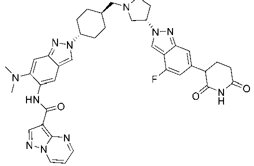 | Example 243 | TM-243 | 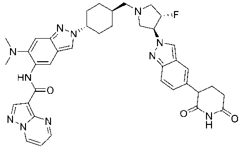 |
| Characterization | | | Characterization | | |
| LCMS (ESI) m/z: [M+1] = 732.44. ¹H NMR (400 MHz, DMSO-*d*₆) δ 10.85 (d, *J =* 3.6 Hz, 2H), 9.36 (dd, *J =* 7.0, 1.6 Hz, 1H), 9.05 - 8.88 (m, 1H), 8.71 (s, 1H), 8.70 (s, 1H), 8.58 (s, 1H), 8.28 (s, 1H), 7.43-7.28 (m, 2H), 6.72 (d, *J =* 11.7 Hz, 1H), 5.27-5.19 (m, 1H), 5.23 (d, *J =* 9.1 Hz, 0H), 4.53 - 4.27 (m, 1H), 3.96 (dd, *J* = 11.9, 4.7 Hz, 1H), 3.07-2.86 (m, 4H), 2.76 (s, 6H), 2.72-1.07 (m, 15H). | | | LCMS (ESI) m/z: [M+1] = 732.10. ¹H NMR (400 MHz, DMSO-*d*₆) δ 10.85 (s, 1H), 10.83 (s, 1H), 9.36 (dd, *J =* 7.0, 1.7 Hz, 1H), 8.95 (dd, *J* = 4.2, 1.6 Hz, 1H), 8.72 (s, 1H), 8.70 (s, 1H), 8.49 (s, 1H), 8.29 (s, 1H), 7.59 (d, *J =* 8.9 Hz, 1H), 7.54 (s, 1H), 7.37 (s, 1H), 7.33 (dd, *J =* 7.0, 4.2 Hz, 1H), 7.13 (dd, *J =* 8.9, 1.6 Hz, 1H), 5.59-5.28 (m, 1H), 4.44-4.31 (m, 1H), 3.91 (dd, *J =* 11.4, 4.9 Hz, 1H), 3.48 (t, *J =* 8.2 Hz, 1H), 3.2-2.84 (m, 3H), 2.76 (s, 6H), 2.72-1.06 (m, 15H). | | |

| Example No. | Compound No. | Structure | Example No. | Compound No. | Structure |
|---|---|---|---|---|---|
| Example 244 | TM-244 | 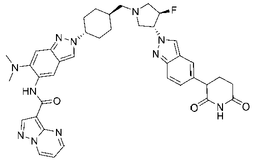 | Example 245 | TM-245 | 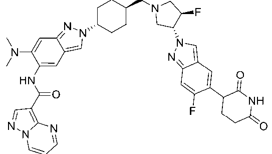 |
| Characterization | | | Characterization | | |
| LCMS (ESI) m/z: [M+1] = 732.36. ¹H NMR (400 MHz, DMSO-*d*₆) δ 10.97 - 10.50 (m, 2H), 9.36 (dd, *J =* 7.0, 1.6 Hz, 1H), 8.94 (dd, *J* = 4.2*,* 1.7 Hz, 1H), 8.71 (s, 1H), 8.70 (s, 1H), 8.49 (s, 0.5 H), 8.29 (d, *J =* 2.4 Hz, 1H), 7.64 - 7.09 (m, 5H), 5.66 - 5.16 (m, 2H), 4.94-4.79 (m, 1H), 4.48 - 4.35 (m, 1H), 3.91 (dd, *J =* 11.4, 4.9 Hz, 1H), 3.51-2.83 (m, 4H), 2.76 (s, 6H), 2.73-1.08 (m, 15H). | | | LCMS (ESI) m/z: [M+1] = 750.88. ¹H NMR (400 MHz, DMSO-*d*₆) δ 10.87 (s, 1H), 10.85 (s, 1H), 9.36 (dd, *J =* 7.0, 1.6 Hz, 1H), 8.95 (dd, *J* = 4.2*,* 1.5 Hz, 1H), 8.71 (s, 1H), 8.70 (s, 1H), 8.55 (s, 1H), 8.29 (s, 1H), 7.69 (d, *J =* 7.4 Hz, 1H), 7.46 - 7.27 (m, 3H), 5.48 - 5.23 (m, 2H), 4.57 - 4.34 (m, 1H), 4.06 (dd, *J =* 12.5, 4.9 Hz, 1H), 3.47-2.83 (m, 4H), 2.76 (s, 6H), 2.73-1.09 (m, 15H). | | |

| Example No. | Compound No. | Structure | Example No. | Compound No. | Structure |
|---|---|---|---|---|---|
| Example 246 | TM-246 | 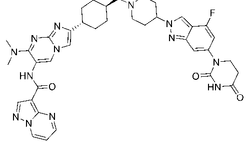 | Example 247 | TM-247 | 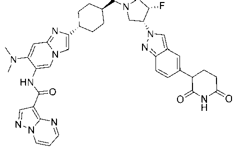 |
| Characterization | | | Characterization | | |
| LCMS (ESI) m/z: [M+1] = 748.66. ¹H NMR (400 MHz, DMSO-*d*₆) δ 10.41 (s, 1H), 9.79 (s, 1H), 9.40 (dd, *J =* 7.0, 1.6 Hz, 1H), 8.97 - 8.90 (m, 1H), 8.74 (s, 1H), 8.64 (s, 1H), 7.98 (d, *J =* 7.9 Hz, 1H), 7.70 (d, *J =* 7.7 Hz, 1H), 7.37 (d, *J =* 8.1 Hz, 2H), 6.88 (d, *J =* 11.8 Hz, 1H), 3.83 (t, *J =* 6.7 Hz, 2H), 3.02 (d, *J =* 12.5 Hz, 6H), 2.73 (t, *J =* 6.6 Hz, 3H), 2.30 - 2.00 (m, 9H), 1.90 (s, 3H), 1.47 - 1.10 (m, 8H). | | | LCMS (ESI) m/z: [M+1] = 732.46. ¹H NMR (400 MHz, DMSO-*d*₆) δ 10.83 (s, 1H), 10.48 (s, 1H), 9.47 - 9.36 (m, 2H), 8.96 (dd, *J* = 4.2*,* 1.6 Hz, 1H), 8.73 (d, *J* = 3.1 Hz, 1H), 8.40 (s, 1H), 7.65 (s, 1H), 7.58 - 7.51 (m, 2H), 7.36 (dd, *J =* 7.0, 4.3 Hz, 1H), 7.18 (d, *J =* 11.2 Hz, 1H), 7.10 (dd, *J =* 9.0, 1.8 Hz, 1H), 5.51 - 5.22 (m, 3H), 3.90 (dd, *J =* 11.3, 4.9 Hz, 1H), 2.75 - 2.56 (m, 4H), 2.25 (q, *J* = 11.4 Hz, 4H), 2.14 - 1.92 (m, 6H), 1.83 - 1.34 (m, 8H), 1.17 - 1.00 (m, 3H). | | |
| Example 248 | TM-248 | 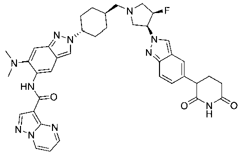 | Example 249 | TM-249 | 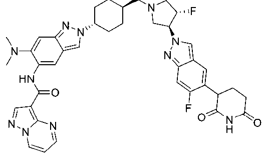 |
| Characterization | | | Characterization | | |
| LCMS (ESI) m/z: [M+1] = 732.46. ¹H NMR (400 MHz, DMSO-*d*₆) δ 10.87 (s, 1H), 10.85 (s, 1H), 9.37 (dd, J= 7.0, 1.7 Hz, 1H), 8.96 - 8.94 (m, 1H), 8.72 (s, 1H), 8.70 (s, 1H), 8.48 (s, 1H), 8.30 (s, 1H), 7.69 (d, *J=* 7.5 Hz, 1H), 7.37 (s, 1H), 7.36 - 7.31 (m, 3H), 5.50-5.24 (m, 2H), 4.46-4.35 (m, 1H), 4.06 (dd, *J =* 12.5, 5.1 Hz, 1H), 3.25-2.91 (m, 4H), 2.77 (s, 6H), 2.71-1.10 (m, 15H). | | | LCMS (ESI) m/z: [M+1] = 750.71. ¹H NMR (400 MHz, DMSO-*d*₆) δ 10.87 (s, 1H), 10.85 (s, 1H), 9.36 (dd, J= 7.0, 1.7 Hz, 1H), 8.95 (dd, *J* = 4.2, 1.7 Hz, 1H), 8.72 (s, 1H), 8.70 (s, 1H), 8.56 (s, 1H), 8.29 (s, 1H), 7.70 (d, *J =* 7.5 Hz, 1H), 7.43-7.30 (m, 3H), 5.58-5.28 (m, 1H), 4.46-4.33 (m, 1H), 4.07 (dd, *J =* 12.5, 4.9 Hz, 1H), 3.52-2.83 (m, 4H), 2.77 (s, 6H), 2.62-1.10 (m, 15H). | | |

| Example No. | Compound No. | Structure | Example No. | Compound No. | Structure |
|---|---|---|---|---|---|
| Example 250 | TM-250 | 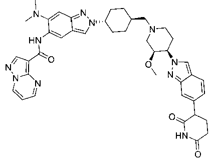 | Example 251 | TM-251 | 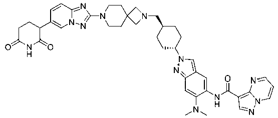 |
| Characterization | | | Characterization | | |
| LCMS (ESI) m/z: [M+1] = 716.41. ¹H NMR (400 MHz, DMSO-*d*₆) δ 10.53 (s, 1H), 10.35 (s, 1H), 9.38 (dd, *J =* 6.9, 1.7 Hz, 1H), 8.95 (dd, *J =* 4.2, 1.6 Hz, 1H), 8.69 (d, *J* = 8.2 Hz, 2H), 8.44 (s, 1H), 8.28 (s, 1H), 7.66 (d, *J =* 8.9 Hz, 1H), 7.49 (s, 1H), 7.35 (dd, *J* = 7.0, 4.3 Hz, 1H), 7.10 (s, 1H), 7.01 (d, *J =* 9.1 Hz, 1H), 4.55-4.31 (m, 2H), 4.02 (s, 3H), 3.83 (t, *J =* 6.6 Hz, 2H), 3.00 (s, 1H), 2.73 (t, *J =* 6.6 Hz, 2H), 2.29-2.01 (m, 9H), 2.03-1.81 (m, 4H), 1.73-1.57 (m, 1H), 1.20-1.27 (m, 2H). | | | LCMS (ESI) m/z: [M+1] = 770.85. ¹H NMR (400 MHz, DMSO-*d*₆) δ 10.90 (s, 1H), 10.85 (s, 1H), 9.36 (d, *J =* 7.0 Hz, 1H), 9.02 - 8.92 (m, 1H), 8.71 (dd, *J* = 3.6, 1.9 Hz, 2H), 8.55 (s, 1H), 8.30 (d, *J =* 21.9 Hz, 1H), 7.48 - 7.21 (m, 3H), 4.48-4.27 (m, 1H), 3.94 (dd, *J* = 12.8, 4.8 Hz, 1H), 3.45 (s, 4H), 2.97 (s, 4H), 2.76 (s, 6H), 2.74 - 2.64 (m, 2H), 2.39-2.27 (m, 2H), 2.19-2.00 (m, 3H), 1.98-1.80 (m, 4H), 1.78-1.68 (m, 4H), 1.66-1.56 (m, 2H), 1.42-1.32 (m, 1H), 1.20-1.04 (m, 2H). | | |

**The following test examples are used to illustrate the beneficial effects of the compounds**

### of the present invention.

The positive compound used in the present invention is derived from patent No. WO2020113233 of Kymera Therapeutics, Inc. Its structure is as follows:

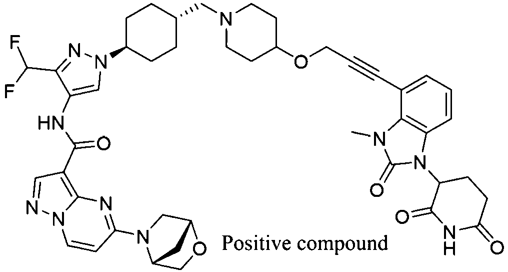

**Test Example 1:** Inhibitory effect of the compounds of the present invention on R848-induced IL-6 secretion in human peripheral blood mononuclear cells (PBMCs)
(1) Experimental method:
   This experiment was carried out in RPMI 1640 medium containing 10% FBS and 1% penicillin/streptomycin, with each of the compounds at an initial test concentration of 1 µM. The compound was dissolved in DMSO to prepare a stock solution, which was serially diluted into a series of working solutions with a 4-fold concentration gradient using the culture medium and then added to a 96-well plate at 10 µL per well. PBMCs (Batch No.: P122101102C; Milecell Biotechnology Inc.) were counted and diluted to a cell concentration of 1.25×10⁶ cells/mL, and then added to the aforementioned compound-containing 96-well plate at 160 µL per well. The system was mixed well and incubated in a 5% CO₂ incubator at 37°C for 20 h. Then 10 µL of R848 (final concentration: 2.5 µg/mL) was added, and the culture was continued for 24 h in a 5% CO₂ incubator at 37°C. After incubation, the 96-well plate was centrifuged at 2000 rpm for 4 min. The supernatant was collected and diluted 120-fold, then detected using an IL-6 ELISA kit. The OD₄₅₀ values were read, and converted to IL-6 concentrations based on a standard curve. The dose-response curves were fitted using GraphPad 8.0 to calculate the IC₅₀ values, as shown in Table 23.

**Table 23 Inhibitory effect of the compounds on R848-induced secretion of IL-6 in PBMC cells**

| Compound No. | IC₅₀ (nM) | Compound No. | IC₅₀ (nM) |
|---|---|---|---|
| Positive compound | 12.11 (mean result of two tests) | TM-222 | 13.98 |
| TM-003 | 3.53 | TM-223 | 10.81 |
| TM-004 | 10.49 | TM-224 | 15.5 |
| TM-132 | 7.83 | TM-225 | 12.26 |
| TM-135 | 4.53 | TM-226 | 4.281 |
| TM-167 | 4.3 | TM-227 | 13.79 |
| TM-174 | 3.19 | TM-228 | 14.69 |
| TM-185 | 8.82 | TM-229 | 19.46 |
| TM-196 | 2.94 | TM-230 | 6.97 |
| TM-197 | 4.932 | TM-231 | 11.59 |
| TM-198 | 20.75 | TM-232 | 18.87 |
| TM-199 | 25.59 | TM-233 | 3.34 |
| TM-202 | 3.606 | TM-234 | 14.42 |
| TM-205 | 3.78 | TM-235 | 6.078 |
| TM-206 | 5.49 | TM-236 | 22.36 |
| TM-207 | 10.33 | TM-237 | 25.02 |
| TM-208 | 10.76 | TM-238 | 4.18 |
| TM-211 | 1.73 | TM-239 | 10.44 |
| TM-216 | 13.42 | TM-240 | 2.68 |
| TM-217 | 8.59 | TM-241 | 9.08 |
| TM-218 | 15.57 | TM-243 | 7.67 |
| TM-219 | 14.83 | TM-244 | 14.43 |
| TM-220 | 8.10 | TM-245 | 4.02 |
| TM-221 | 7.101 | TM-249 | 4.37 |

**Test Example 2:** Inhibitory effect of the compounds of the present invention on LPS/IL-1β-induced IL-6 secretion in human peripheral blood mononuclear cells (PBMCs)

This experiment was carried out in RPMI 1640 medium containing 10% FBS and 1% penicillin/streptomycin, with each of the compounds at an initial test concentration of 1 µM.

The compound was dissolved in DMSO to prepare a stock solution, which was serially diluted into some working solutions with a 5-fold concentration gradient using the culture medium and then added to a 96-well plate at 10 µL per well. PBMCs (Batch No.: P122101102C; Milecell Biotechnology Inc.) were counted and diluted to a cell concentration of about 1.3×10⁶ cells/mL, and then added to the aforementioned compound-containing 96-well plate at 150 µL per well. The system was mixed well and incubated in a 5% CO₂ incubator at 37°C for 24 h. Then 5 µL of LPS (final concentration: 10 ng/mL) and 5 µL of IL-1β (final concentration: 20 ng/mL) were added to each well, respectively, and the culture was continued for 20 h in a 5% CO₂ incubator at 37°C. After incubation, the 96-well plate was centrifuged at 2000 rpm for 4 min. The supernatant was collected and diluted 200-fold, then detected using an IL-6 ELISA kit. The OD₄₅₀ values were read, and converted to IL-6 concentrations based on a standard curve. The dose-response curves were fitted using GraphPad 8.0 to calculate the IC₅₀ values. The experimental results are shown in Table 24 below.

**Table 24 Inhibitory effect of the compounds on LPS/IL-1β-induced secretion of IL-6 in PBMC cells**

| Compound No. | IC₅₀ (nM) | Compound No. | IC₅₀ (nM) |
|---|---|---|---|
| Positive compound | 2.99 (mean result of two tests) | TM-85 | 8.41 |
| TM-1 | 5.86 | TM-90 | 1.44 |
| TM-2 | 5.07 | TM-106 | 2.52 |
| TM-3 | 0.39 | TM-110 | 21.59 |
| TM-4 | 0.27 | TM-128 | 36.59 |
| TM-5 | 0.26 | TM-130 | 5.12 |
| TM-6 | 1.15 | TM-131 | 5.07 |
| TM-7 | 0.18 | TM-132 | 3.52 |
| TM-8 | 0.84 | TM-133 | 3.55 |
| TM-9 | 2.03 | TM-134 | 2.59 |
| TM-10 | 1.34 | TM-135 | 2.66 |
| TM-15 | 4 | TM-136 | 6.63 |
| TM-16 | 4.59 | TM-137 | 2.4 |
| TM-17 | 3.37 | TM-138 | 6.96 |
| TM-18 | 3.72 | TM-139 | 4.35 |
| TM-26 | 0.54 | TM-141 | 4.49 |
| TM-27 | 1.49 | TM-142 | 0.88 |
| TM-29 | 0.88 | TM-143 | 11.89 |
| TM-30 | 1.05 | TM-144 | 38.36 |
| TM-31 | 3.29 | TM-145 | 2.62 |
| TM-32 | 1.21 | TM-146 | 44.13 |
| TM-33 | 0.87 | TM-148 | 11.59 |
| TM-41 | 3.89 | TM-149 | 2.19 |
| TM-42 | 1.65 | TM-150 | 6.94 |
| TM-43 | 8.92 | TM-151 | 7.13 |
| TM-44 | 10.24 | TM-152 | 9.43 |
| TM-45 | 8.81 | TM-153 | 6.79 |
| TM-46 | 7.46 | TM-154 | 1.45 |
| TM-47 | 3.06 | TM-155 | 1.00 |
| TM-48 | 1.61 | TM-156 | 1.17 |
| TM-49 | 1.33 | TM-157 | 1.46 |
| TM-50 | 4.65 | TM-158 | 14.59 |
| TM-51 | 3.14 | TM-166 | 0.89 |
| TM-52 | 18.69 | TM-174 | 1.49 |
| TM-53 | 6.14 | TM-175 | 13.25 |
| TM-54 | 3.06 | TM-205 | 2.47 |
| TM-55 | 1.61 | TM-207 | 40.87 |
| TM-56 | 8.81 | TM-221 | 1.88 |
| TM-57 | 10.24 | TM-233 | 0.83 |
| TM-59 | 10.87 | TM-234 | 8.165 |
| TM-60 | 1.94 | TM-237 | 16.54 |
| TM-61 | 1.15 | TM-239 | 7.309 |
| TM-62 | 2.48 | TM-240 | 1.092 |
| TM-63 | 8.91 | TM-241 | 6.349 |
| TM-65 | 2.26 | TM-243 | 3.96 |
| TM-66 | 19.62 | TM-244 | 4.953 |
| TM-67 | 4.01 | TM-245 | 2.576 |
| TM-74 | 0.86 | TM-249 | 3.084 |
| TM-75 | 13.25 | TM-251 | 2.19 |
| TM-83 | 44.13 | | |

### Test Example 3: Effect of the compounds on IRAK4 protein degradation in THP1 cells

This experiment was carried out in RPMI 1640 medium containing 10% FBS and 1% penicillin/streptomycin, with each of the compounds at an initial test concentration of 1 µM. THP-1 cells were seeded into a 96-well cell culture plate, with 2×10⁵ cells and 90 µL of culture medium per well. The cell culture plate was cultured in an incubator with 5% CO₂ at 37°C overnight. The compound was dissolved in DMSO to prepare a stock solution, which was serially diluted into a series of working solutions with a 5-fold concentration gradient using the culture medium and then added to the aforementioned 96-well plate at 10 µL per well. The cell culture plate was further incubated for 24 h in a 5% CO₂ incubator at 37°C. After incubation, the 96-well plate was centrifuged and the supernatant was discarded, followed by adding 40 µL of cell lysis buffer containing proteasome/phosphatase inhibitors to each well for sufficient lysis on an ice bath. Protein quantification was performed using the BCA protein quantification kit, followed by the preparation of Western blot samples. The sample was loaded at a rate of 10 µL per lane onto a Tris SDS-page gel containing 10 lanes and a gradient of 4% to 12% for electrophoresis. After electrophoresis, protein transfer was performed using a PVDF membrane under ice bath conditions for 1 h. After the transfer was completed, the PVDF membrane was blocked with gentle shaking in 5% skim milk at room temperature for 1 h. After blocking, the PVDF membrane was incubated with the primary antibody overnight with gentle shaking at 4°C. The PVDF membrane was washed 3 times with TBST, then the secondary antibody was added and incubated for 1 h with gentle shaking at room temperature, followed by washing of the PVDF membrane with TBST for 3 times. After the washes were completed, the chemiluminescent substrate was added; the PVDF membrane was scanned using a Clinx imager, semi-quantitative analysis was performed with ImageJ software, and the DC₅₀ was calculated using GraphPad Prism 8.0.

**Table 25 Effect of the compounds on IRAK4 protein degradation in THP1 cells**

| Compound No. | DC₅₀ (nM) | Compound No. | DC₅₀ (nM) |
|---|---|---|---|
| Positive compound | 6.54 | TM-147 | 11 |
| TM-003 | 2.76 | TM-151 | 1.66 |
| TM-004 | 3.03 | TM-152 | 1.09 |
| TM-005 | 4.62 | TM-153 | 0.79 |
| TM-006 | 0.70 | TM-154 | 1.27 |
| TM-007 | 3.73 | TM-155 | 0.31 |
| TM-008 | 3.58 | TM-156 | 0.32 |
| TM-009 | 5.41 | TM-157 | 1.12 |
| TM-010 | 4.00 | TM-158 | 1.41 |
| TM-015 | 1.41 | TM-166 | 1.58 |
| TM-016 | 0.41 | TM-167 | 1.17 |
| TM-017 | 1.19 | TM-168 | 1.78 |
| TM-018 | 1.20 | TM-174 | 1.24 |
| TM-027 | 4.27 | TM-182 | 3.82 |
| TM-029 | 1.13 | TM-185 | 3.22 |
| TM-030 | 1.33 | TM-186 | 0.98 |
| TM-031 | 4.06 | TM-196 | 2.84 |
| TM-032 | 1.40 | TM-197 | 3.29 |
| TM-033 | 2.86 | TM-198 | 0.86 |
| TM-041 | < 1 | TM-199 | 0.87 |
| TM-042 | 0.93 | TM-200 | 1.79 |
| TM-043 | 4.31 | TM-202 | 0.95 |
| TM-051 | 23.29 | TM-204 | 1.69 |
| TM-059 | 9.64 | TM-205 | 1.56 |
| TM-067 | 19.9 | TM-208 | 3.77 |
| TM-106 | 3.82 | TM-211 | 3.23 |
| TM-130 | 4.26 | TM-213 | 1.74 |
| TM-131 | 0.81 | TM-214 | 1.21 |
| TM-132 | 0.31 | TM-215 | 0.95 |
| TM-133 | 1.6 | TM-221 | 8.45 |
| TM-134 | 3 | TM-230 | 1.42 |
| TM-135 | 1.23 | TM-231 | < 1 |
| TM-136 | 1.71 | TM-240 | 2.61 |
| TM-137 | 9.41 | TM-241 | 11.65 |
| TM-139 | < 1 | TM-244 | 3.21 |
| TM-140 | 13.9 | TM-245 | 1.41 |
| TM-141 | 4.33 | TM-249 | 2.38 |
| TM-142 | 0.77 | TM-251 | 18.15 |
| TM-143 | 135.2 | | |

The above results show that: the compounds of the present invention have better degradation effects in THP1 cells.

### Test Example 4: Metabolic stability test

Clean incubation plates T60 and NCF60 were preheated at 37°C for 10 min. Liver microsomes were diluted to a concentration of 0.56 mg/mL with 100 mM phosphate buffer, and 445 µL of the microsome working solution (0.56 mg/mL) was transferred to the preheated incubation plates T60 and NCF60, followed by incubation of the plates T60 and NCF60 with continuous shaking at 37°C for 10 min. After incubation, 54 µL of liver microsomes were transferred to a blank plate, 6 µL of NAPDH cofactor was added to the blank plate, and then 180 µL of quenching solution was added to the blank plate. 5 µL of the compound working solution (100 µM) was added to the microsome-containing incubation plates T60 and NCF60, and mixed thoroughly 3 times. For plate NCF60, 50 µL of buffer was added and the system was mixed thoroughly 3 times before timing was started, and the plate was incubated with shaking at 37°C for 60 min. For the quenching plate, 180 µL of quenching solution and 6 µL of NADPH cofactor were added, and the plate was ensured to be cooled to prevent evaporation. For the T60 plate, the mixture was thoroughly mixed 3 times, and 54 µL of the mixture was immediately transferred to the quenching plate at the time point of 0 min. Then 44 µL of the NAPDH cofactor was added to the incubation plate (T60). Timing was started, and the plate was incubated with shaking at 37°C for 60 min. At the time points of 5, 15, 30, 45, and 60 min, 180 µL of the quenching solution was added to the quenching plate respectively, mixed once, and 60 µL of sample was continuously transferred from the plate T60 to the quenching plate at each time point. For plate NCF60, after mixing once, 60 µL of the sample was transferred from incubation plate NCF60 to the quenching plate containing quenching solution at the 60-min time point. All sampling plates were shaken for 10 min, then centrifuged at 4000 rpm at 4°C for 20 min. 80 µL of the supernatant was transferred to 240 µL of HPLC water, and mixed using a plate shaker for 10 min. Each bioanalytical plate was sealed and shaken for 10 min before LC-MS/MS analysis.

**Table 26 Metabolic stability of the compounds in human liver microsome**

| Compound | Human liver microsome (t_{1/2}, min) | Compound | Human liver microsome (t_{1/2}, min) |
|---|---|---|---|
| Positive compound | 39.4 | TM-137 | 92.3 |
| TM-3 | 145 | TM-138 | 91.0 |
| TM-4 | 145 | TM-152 | 103.8 |
| TM-6 | 124.3 | TM-233 | 138.1 |
| TM-8 | 145 | TM-240 | 98.6 |
| TM-132 | 93.1 | TM-241 | 105.8 |
| TM-133 | 106.9 | TM-244 | 79.5 |
| TM-134 | 114.2 | TM-245 | 87.4 |
| TM-135 | 65.0 | TM-249 | 139.1 |
| TM-136 | 100.8 | | |

The results show that the compounds of the present invention have better stability in liver microsome than the positive compound (positive drug).

### Test Example 5: In vivo metabolism test of the compounds of the present invention

Pharmacokinetic experiment on SD rats: in this experiment, two administration modes: oral intragastric administration and tail vein injection were selected. Oral administration was performed at a dose of 5 mg/kg using the compounds of the present invention. The administration volume was 10 mL/kg. Prior to use, an appropriate amount of the drug was accurately weighed and first dissolved in dimethyl sulfoxide (DMSO). An appropriate amount of Solutol and H₂O were then added in sequence at a final volume ratio of 5% DMSO, 10% Solutol and 85% H₂O. The mixture was sonicated and vortexed to homogeneity to prepare a clear drug solution with a concentration of 0.5 mg/mL. Intravenous administration was performed at a dose of 1 mg/kg using the compounds of the present invention. The administration volume was 10 mL/kg. Prior to use, an appropriate amount of each drug was accurately weighed separately, followed by the sequential addition of a suitable volume of PEG 400 and Saline at a final volume ratio of 20% PEG 400 to 80% Saline. The mixture was sonicated and vortexed to homogeneity to prepare a clear drug solution with a concentration of 0.1 mg/mL.

6 SD rats, each with body weight of 180-220 g, were randomly grouped into 2 groups. The animals were fasted overnight with free access to water. For the first group, intragastric administration was performed, and 200 µL of blood was collected from the orbital venous plexus into anticoagulant tubes at 15 min, 30 min, 1 h, 2 h, 4 h, 6 h, 8 h and 24 h post-administration, respectively. Within one hour, the blood samples were centrifuged at 10000 rpm for 20 min at 4°C (preserved on an ice box prior to centrifugation). The upper supernatant, i.e., plasma, was harvested and stored in a refrigerator at -20°C for subsequent LC-MS/MS analysis. For the second group, intravenous administration was conducted, and 200 µL of blood was collected from the orbital venous plexus into anticoagulant tubes at 5 min, 15 min, 30 min, 1 h, 2 h, 4 h, 8 h and 24 h post-administration, respectively. Within one hour, the blood samples were centrifuged at 10000 rpm for 20 min at 4°C (preserved on an ice box prior to centrifugation). The upper supernatant, i.e., plasma, was harvested and stored in a refrigerator at -20 °C for subsequent LC-MS/MS analysis.

**Table 27 Drug metabolism of the compounds in rats**

| Compound | Parameters Parameters | | Dose (mg/kg) | AUCₐₗₗ (ng*h/mL) | AUC_{inf} (ng*h/mL) | Peak plasma concentration *C*ₘₐₓ (ng/ml) | Oral bioavailability *F*_{obs} (%) |
|---|---|---|---|---|---|---|---|
| TM-4 | SD Rats | iv | 1.00 | 963.53±123.07 | 995.74±142.87 | 634.00±138.79 | |
| | | ig | 5.00 | 1353.75±515.03 | 1377.38±512.87 | 466.67±225.85 | 27.7 |
| TM-135 | SD Rats | iv | 1.00 | 1169.70±260.28 | 1220.33±332.94 | 790.00±65.87 | |
| | | ig | 5.00 | 1900.75±564.62 | 2025.27±528.61 | 433.00±197.82 | 33.2 |
| TM-174 | SD Rats | iv | 1.00 | 1481.89±167.24 | 1700.29±254.34 | 271.77±34.55 | |
| | | ig | 10.00 | 3631.55±1495.75 | 4160.20±144 4.41 | 355.57±201.32 | 24.5 |
| TM-240 | SD Rats | iv | 1.00 | 2626.63±171.73 | 2669.17±175 .00 | 652.33±31.97 | |
| | | ig | 10.00 | 7985.09±3137.10 | 7985.67±311 8.74 | 976.13±545.34 | 29.9 |
| TM-241 | SD Rats | iv | 1.00 | 4139.78±1285.37 | 4223.33 ±1296.29 | 919.98±123.88 | |
| | | ig | 10.00 | 14213.55±4656.22 | 14864.92±46 92.20 | 1648.97 ±640.56 | 35.20 |
| TM-244 | SD Rats | iv | 1.00 | 2014.17±360.84 | 2437.32 ±585.14 | 784.30±125.94 | |
| | | ig | 10.00 | 13010.04±2901.02 | 13209.96±29 56.88 | 1326.33±294.01 | 54.2 |
| TM-245 | SD Rats | iv | 1.00 | 2907.79±62.84 | 2984.42±113 .77 | 554.50±60.66 | |
| | | ig | 10.00 | 14486.50±1423.03 | 19014.62±50 56.76 | 1191.73±92.32 | 63.7 |
| TM-249 | SD Rats | iv | 1.00 | 2280.25±189.98 | 2400.61±353 .62 | 612.53±39.84 | |
| | | ig | 10.00 | 8610.23±4002.29 | 9307.62±468 4.55 | 814.30±186.72 | 38.8 |

The results are as follows: the compounds of the present invention have excellent oral bioavailability in rats.

### Test Example 6: Therapeutic effects of the compounds of the present invention on psoriasis model

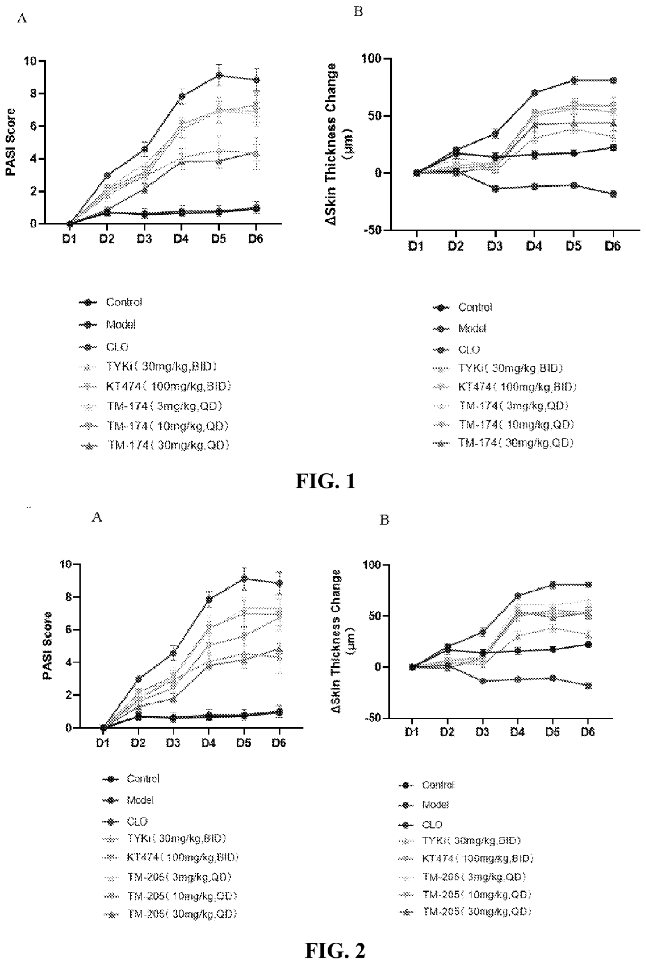

Male BALB/c mice, aged 6-8 weeks, were randomly divided into 9 groups after 1 week of acclimatization: blank control group, imiquimod model group (IMQ group, corresponding to the model groups in Figs. 1 and 2), clobetasol propionate cream group (CLO group), TYKI (BMS-986165) group, IRAK4 protein degrader KT-474 (from patent WO2020113233) group (100 mg/kg), and the compound of the present invention (at an appropriate dose), with 6-7 mice in each group. One day before modeling, the hair on the backs of the mice was removed with depilatory cream, with a depilated area of 2 × 2 cm². On the day of modeling, IMQ cream was used for modeling, and the corresponding drugs were administered intragastrically for treatment. The compound of the present invention (at an appropriate dose) was administered intragastrically once daily, and IMQ cream was applied to the hairless area on the back and left ear of mice 2 h after the first administration. The positive compound KT-474 (from patent WO2020113233) group had IMQ cream applied to the depilated area on the back and the left ear of mice at 4 h after the first administration. The blank group and the IMQ group were administered with vehicle intragastrically twice daily (in the morning and evening), with an 8-h interval between the two intragastric administrations. At 2 h after the first intragastric administration, vaseline and IMQ cream were applied topically to the depilated back and ears of the mice, respectively. The dosage of IMQ cream administered to the depilated area on the back and the left ear was 80 mg and 10 mg, respectively, and the mice in each group were administered the cream for 5 consecutive days.

Referrence was made to the Psoriasis Area and Severity Index (PASI) scoring criteria. The psoriasis, erythema, and thickness of skin lesions on the back were scored from 0 to 4 every morning from day 1 of modeling, with the scoring criteria as shown in Table 28. The 3 were added up to obtain a total score, i.e., a total PASI score. The ear thickness of the left ear of the mouse was measured using a digital vernier caliper three times every morning from day 1 of modeling, to obtain a mean value.

**Table 28 Psoriasis Area and Severity Index (PASI) scoring criteria**

| | 0 | 1 | 2 | 3 | 4 |
|---|---|---|---|---|---|
| Psoriasis | No psoriasis on the surface | Surface of some skin lesions covered with psoriasis | Surface of most skin lesions covered with psoriasis | Skin lesions almost completely covered with psoriasis | Skin lesions completely covered with psoriasis |
| Erythema | No erythema | Light red | Red | Dark red | Purple red |
| Thickness | Skin lesion flush with normal skin | Skin lesion slightly above normal skin surface | Skin lesion moderately raised | Skin lesions thickened and obviously raised | Skin lesions highly thickened and obviously raised |

The therapeutic effects of the compounds of the present invention on psoriasis model are shown in Fig. 1 and Fig. 2.

Fig. 1A is a statistical chart of the Psoriasis Area and Severity Index (PASI) scores in the treatment effect of the compound TM-174 of the present invention on the IMQ psoriasis model, and Fig. 1B is a statistical chart of the skin thickness test results in the treatment effect of the compound TM-174 of the present invention on the IMQ psoriasis model.

Fig. 2A is a statistical chart of the Psoriasis Area and Severity Index (PASI) scores showing the therapeutic effect of the compound TM-205 of the present invention on the IMQ psoriasis model. Fig. 2B is a statistical graph of the skin thickness test results of the compound TM-205 of the present invention.

The control group was the blank group, the model group was the imiquimod model group, the CLO group was the clobetasol propionate group, and the TYKI (30 mg/kg, BID) group was the BMS-986165 group.

Clobetasol propionate is an organic compound with the chemical formula C₂₅H₃₂ClFO₅. It appears as a white crystalline powder and is an anti-inflammatory corticosteroid. It is indicated for various skin diseases. For external use, it is suitable for pruritic and non-infectious inflammatory skin diseases responsive to topical glucocorticoid therapy, such as chronic eczema, neurodermatitis, psoriasis, palmoplantar pustulosis, lichen planus, and discoid lupus erythematosus.

BMS-986165 (also known as Deucravacitinib) is a novel, oral, selective TYK2 inhibitor.
KT-474 (100 mg/kg, QD) is an IRAK4 protein degrader KT-474 (from patent WO2020113233). The compound TM-174 of the present invention was administered intragastrically at different doses: TM-174 (3 mg/kg, QD), TM-174 (10 mg/kg, QD),
and TM-174 (30 mg/kg, QD).

Specific experimental data related to Figs. 1 and 2 are shown in Table 29.

**Table 29. Effects of the compound of the present invention on PASI score and ear thickness difference in the IMQ psoriasis model (Mean±SD, n=7)**

| Group (n=7) | Dose (mg/kg) | PASI Score | ΔSkin Thickness Change (µm) |
|---|---|---|---|
| | | d6 | d6 |
| Control | - | 0.93±0.25 | 22.38±6.10 |
| Model | - | 8.85±1.66 | 80.95±6.16 |
| CLO | 160mg/animal | 1.17±0.85**** | -18.10±6.33 **** |
| TYKi (30 mg/kg, BID) | 30 | 4.32±2.42**** | 53.57±15.87**** |
| KT474 (100 mg/kg, BID) | 100 | 6.93±2.36* | 31.90±9.90**** |
| TM-174 (3 mg/kg, QD) | 3 | 6.69±1.07** | 58.10±17.31*** |
| TM-174 (10 mg/kg, QD) | 10 | 7.31±1.87* | 59.29±18.23*** |
| TM-174 (30 mg/kg, QD) | 30 | 4.38±1.24**** | 43.81±16.66**** |
| TM-205 (3 mg/kg, QD) | 3 | 7.29±1.99* | 65.71±10.23** |
| TM-205 (10 mg/kg, QD) | 10 | 6.75±1.28** | 52.62±9.59**** |
| TM-205 (30 mg/kg, QD) | 30 | 4.84±0.80**** | 53.10±12.95**** |

| | | | |
|---|---|---|---|
| Note: vs Model, **p*<0.05, ***p<*0.01, ****p<*0.001, *****p*<0.0001. | | | |

The results show that the patented compounds exhibit excellent control effects on the IMQ-induced psoriasis model and have a distinct therapeutic effect on skin thickening.

### Test Example 7: Therapeutic effects of the compounds of the present invention on enteritis (DSS model)

The experimental mice were pre-acclimatized in the animal facility for one week; at the beginning of the experiment, the mice were given medication intragastrically daily according to their body weight. After the completion of intragastric administration in each group, mice had direct access to water bottles for free drinking. The blank group received normal water, while the other groups received pre-prepared DSS water. Each afternoon, the mice's abdomens were massaged to stimulate defecation, and one fecal pellet was collected onto a white porcelain plate. Fecal consistency was judged and recorded according to Table 30. The fecal occult blood qualitative test kit (Yuanye Biotechnology, #R24187-300T) was used to test for occult blood in feces and the results were interpreted according to Table 31.

**Table 30 DSS model scoring method**

| Scoring | Weight loss (%) | Stool consistency | Bloody stool |
|---|---|---|---|
| 0 | None | Normal | Negative |
| 1 | 1-5 | / | / |
| 2 | 6-10 | Semi-loose stools | Occult blood |
| 3 | 10-15 | / | / |
| 4 | >15 | Loose stools | Gross bloody stools |

**Table 31 Methods for judging fecal occult blood in the DSS model**

| Color change | Interpretation |
|---|---|
| Immediate purple color after reagent addition | ++++ |
| Purple color after reagent addition, gradually turning dark purple | +++ |
| Initial purple color after reagent addition, gradually turning distinct purple | ++ |
| Color changes from colorless to light purple or purple 10 s after reagent addition | + |
| No color development within 2 min after adding the reagent | Negative |

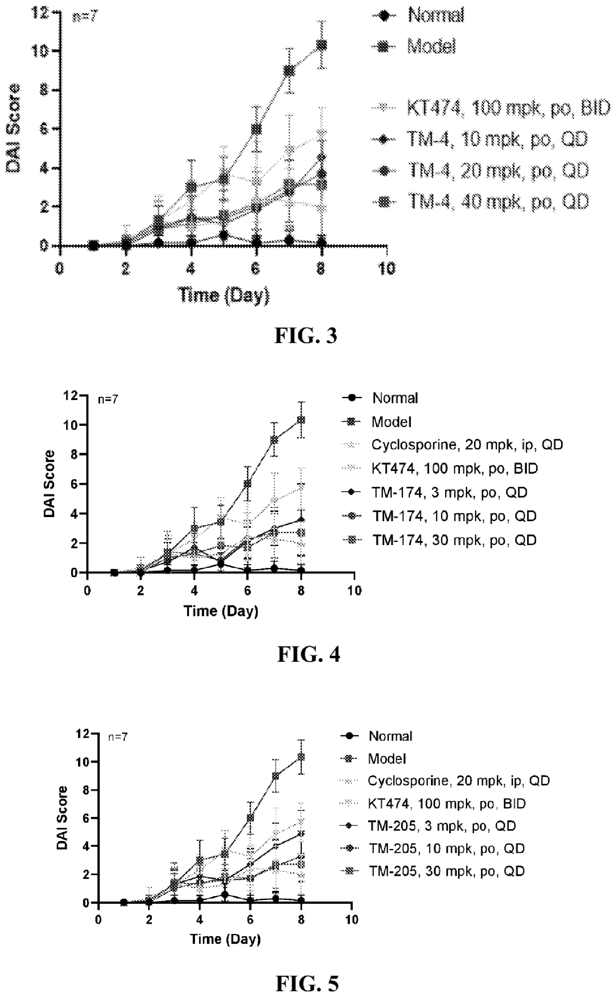

Fig. 3 is a statistical chart showing the therapeutic effect of the compound TM-4 of the present invention on the DSS-induced enteritis model.

Fig. 4 is a statistical chart showing the therapeutic effect of the compound TM-174 of the present invention on the DSS-induced enteritis model.

Fig. 5 is a statistical chart showing the therapeutic effect of the compound TM-205 of the present invention on the DSS-induced enteritis model.

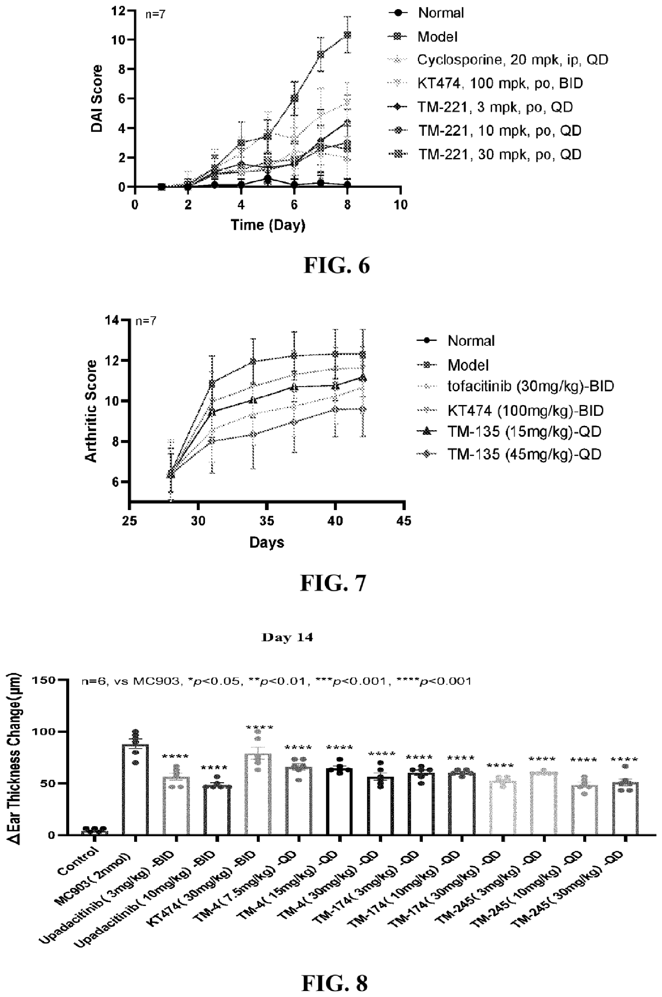

Fig. 6 is a statistical chart showing the therapeutic effect of the compound TM-221 of the present invention on the DSS-induced enteritis model.

Among others, the Normal group represents the normal control group, and the Model group represents the model group. Cyclosporine, 20mpk, ip, QD represents the cyclosporine group. Cyclosporine is a widely used immunosuppressant in clinical practice. It treats a variety of diseases by inhibiting the proliferation and function of T cells and B cells.

Specific experimental data related to Figs. 3-6 are shown in Table 32.

**Table 32 Effect of the compounds of the present invention on the DAI score of colitis mice (Mean±SD, n=7)**

| Group (n=7) | Dose (mpk) | DAI Score |
|---|---|---|
| | | d8 |
| Normal | - | 0.14±0.35 |
| Model | - | 10.33±1.11 |
| Cyclosporine, 20 mpk, ip, QD | 20 | 2.00±1.31**** |
| KT474, 100 mpk, po, BID | 100 | 5.71±1.28**** |
| TM-4, 10 mpk, po, QD | 10 | 4.57±1.18**** |
| TM-4, 20 mpk, po, QD | 20 | 3.71±1.58**** |
| TM-4, 40 mpk, po, QD | 40 | 3.14±2.53**** |
| TM-174, 3 mpk, po, QD | 3 | 3.57±2.26**** |
| TM-174, 10 mpk, po, QD | 10 | 2.71±1.39**** |
| TM-174, 30 mpk, po, QD | 30 | 2.71±0.70**** |
| TM-205, 3 mpk, po, QD | 3 | 4.86±1.55**** |
| TM-205, 10 mpk, po, QD | 10 | 3.29±1.67**** |
| TM-205, 30 mpk, po, QD | 30 | 2.71±0.70**** |
| TM-221, 3 mpk, po, QD | 3 | 4.43±1.68**** |
| TM-221, 10 mpk, po, QD | 10 | 3.00±1.07**** |
| TM-221, 30 mpk, po, QD | 30 | 2.57±2.50**** |

| | | |
|---|---|---|
| Note: vs Model, **p<*0.05, ***p*<0.01, ****p*<0.001*, ****p<*0.0001. | | |

The results show that the compounds of the present invention exhibit excellent therapeutic effects on DSS-induced enteritis model.

**Test Example 8:** Therapeutic effects of the compounds of the present invention on rheumatoid arthritis (CIA model)
1. DBA/1J mice, male, SPF grade, were pre-acclimatized in the animal facility for one week.
2. On day 1, for the primary immunization, 2 mg/mL bovine type II collagen was mixed thoroughly with an equal volume of complete Freund's adjuvant on ice using a homogenizer until fully emulsified. 100 µL of the collagen emulsion (final concentration 1 mg/mL) was intradermally injected at 2 cm from the base of the mouse tail. Medical iodophor was used to disinfect the tail after injection to prevent tail necrosis, which was conducted for 3 consecutive days.
3. On day 21, bovine type II collagen solution was thoroughly mixed and emulsified with an equal volume of Freund's incomplete adjuvant, using the same method as the primary immunization.
4. On day 26, LPS was administered intraperitoneally at a dose of 50 µg/animal to induce disease.
5. On day 28, the affected mice were scored according to Table 33 and the diameter of their ankle joints was measured. Mice were randomly grouped and treated with medication based on their scores and ankle diameter.

**Table 33 Arthritis Index (AI) scoring**

| Symptom | Score |
|---|---|
| No redness or swelling | 0 |
| Mild swelling of the little toe joint | 1 |
| Swelling of toe joints and toes | 2 |
| Swelling of the paw below the ankle joint | 3 |
| Swelling of all paw joints including the ankle joint | 4 |

Fig. 7 is a statistical chart showing the therapeutic effect of the compound TM-135 of the present invention on the CIA-induced rheumatoid arthritis model.

Among others, the Normal group represents the normal control group, and the Model group represents the model group. tofacitinib (30 mg/kg) - BID is the tofacitinib group. Tofacitinib is a JAK inhibitor developed by Pfizer that can effectively inhibit the activity of JAK1 and JAK3 and block the signal transduction of various inflammatory cytokines. Specific experimental data related to Fig. 7 are shown in Table 34.

**Table 34. Effect of the compound of the present invention on the AI score in CIA-induced rheumatoid arthritis model (Mean ± SD, n = 7)**

| Group (n=7) | Dose (mpk) | DAI Score |
|---|---|---|
| | | d42 |
| Normal | - | 0.00±0.00 |
| Model | - | 12.31±2.97 |
| tofacitinib (30 mg/kg) - BID | 30 | 10.67±3.13 |
| KT474 (100mg/kg)-BID | 100 | 11.63±2.55 |
| TM-135 (15mg/kg)-QD | 15 | 11.17±2.36 |
| TM-135 (45mg/kg)-QD | 45 | 9.60±3.33 |

The results show that the compounds of the present invention exhibit excellent therapeutic effects on CIA-induced rheumatoid arthritis model.

**Test Example 9:** Therapeutic effects of the compounds of the present invention on idiopathic dermatitis (AD model)
1. Balb/c mice, female, SPF grade, were pre-acclimatized in the animal facility for one week.
2. At the start of the experiment, body weight was measured and recorded, and the mice were administered intragastrically at a volume of 0.1 mL/10 g. Ear thickness of mice was measured daily (ear thickness was measured before administration on d1).
3. Two hours after intragastric administration, MC903 was applied topically. MC903 (2 nmol·ear⁻¹) dissolved in 95% ethanol was applied to the left ear of each mouse, while an equal volume of ethanol vehicle was applied to the right ear, with a volume of 10 µL per ear. The mice were treated daily from d1 to d14 continuously.

The therapeutic results of the compounds of the present invention on the MC903-induced atopic dermatitis model on day 14 are shown in Fig. 8.

Control group was the blank control group, MC903 (2 nmol) was the calcipotriol group, Upadacitinib (3 mg/kg)-BID was the utpatinib group, and Upadacitinib(10 mg/kg)-BID was the utpatinib group.

Calcipotriol (MC903) is a synthetic vitamin D3 analog that is widely used to treat psoriasis and can inhibit the proliferation and differentiation of leukemia cells.

Upadacitinib is a selective JAK inhibitor developed and manufactured by AbbVie in the United States, primarily used to treat a variety of inflammatory diseases.

The results indicate that the compound of the present invention exhibits a favorable therapeutic effect on atopic dermatitis.

To sum up, the present invention discloses a compound of formula I, which can effectively degrade IRAK4 or inhibit IRAK4 activity in other ways. It has very good prospects for use in treating IRAK4-mediated diseases including, e.g., immune diseases (such as psoriasis, hidradenitis suppurativa, atopic dermatitis, rheumatoid arthritis, systemic lupus erythematosus, alcoholic liver disease, autoimmune liver disease, or acne), tumors (such as multiple myeloma, lymphocytic leukemia, and lymphoma), Alzheimer's disease, and fibrotic disease, thus providing a new choice for clinical selection and/or preparation of a drug for treating a disease associated with the IRAK4 activity.

## Claims

1. A compound represented by formula I, a stereoisomer thereof, a deuterated compound thereof, or a pharmaceutically acceptable salt thereof: 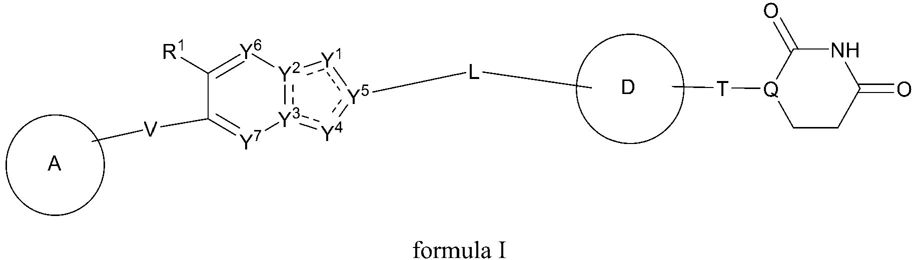 wherein
V is selected from -C(O)NH- or -NHC(O)-;
T is selected from -NH- or a chemical bond;
-̅ -̅ -̅ -̅ -̅ -̅ represents a single bond or a double bond;
Y² and Y³ are each independently selected from C or N;
Y¹ and Y⁴ are each independently selected from CR^{Y}, CR^{Y}R^{Y}, N, NR^{Y}, O, S, C(O), S(O), or S(O)₂;
Y⁵ is selected from C or N;
Y⁶ and Y⁷ are each independently selected from CR⁴ or N;
each R^{Y} is selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl;
Q is selected from CR^{Q} or N;
R^{Q} is selected from hydrogen, deuterium, halogen, cyano, C₁₋₆ alkyl, C₁₋₆ alkoxy, halogen-substituted C₁₋₆ alkyl, halogen-substituted C₁₋₆ alkoxy;
ring A is selected from 5- to 10-membered heteroaromatic rings; wherein the heteroaromatic ring is optionally substituted with 1, 2 or 3 R^{A1} groups:
each R^{A1} is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{A2}, -C₀₋₂ alkylene-NR^{A2}R^{A3}, -C₀₋₂ alkylene-3- to 10-membered carbocyclyl, -C₀₋₂ alkylene-4- to 10-membered heterocyclyl;
R^{A2} and R^{A3} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
R¹ and R⁴ are each independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR¹¹, -C₀₋₂ alkylene-NR¹¹R¹², -C₀₋₂ alkylene-NR¹¹C(O)R¹², -C₀₋₂ alkylene-C(O)R¹¹, -C₀₋₂ alkylene-C(O)NR¹¹R¹², -C₀₋₂ alkylene-3- to 10-membered carbocyclyl, -C₀₋₂ alkylene-4- to 10-membered heterocyclyl, -C₀₋₂ alkylene-4- to 10-membered bridged rings, -C₀₋₂ alkylene-4- to 10-membered bridged heterocycles, -C₀₋₂ alkylene-5- to 12-membered spiro rings, -C₀₋₂ alkylene-5- to 12-membered spiro heterocycles, -C₀₋₂ alkylene-6- to 10-membered aromatic rings, -C₀₋₂ alkylene-5- to 10-membered heteroaromatic ring; wherein the carbocyclyl, heterocyclyl, bridged ring, bridged heterocycle, spiro ring, spiro heterocycle, aromatic ring and heteroaromatic ring are optionally substituted with 1, 2 or 3 R¹³ groups;
R¹¹ and R¹² are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
each R¹³ is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR¹⁴, -C₀₋₂ alkylene--NR¹⁴R¹⁵, -C₀₋₂ alkylene-NR¹⁴C(O)R¹⁵, -C₀₋₂ alkylene-C(O)R¹⁴, -C₀₋₂ alkylene-C(O)NR¹⁴R¹⁵;
R¹⁴ and R¹⁵ are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
ring D is selected from 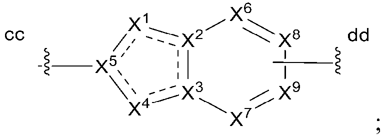 where the cc end in is attached to L and the dd end is attached to Q;
------ represents a single bond or a double bond;
X² and X³ are each independently selected from C or N;
X¹ and X⁴ are each independently selected from CR^{X}, CR^{X}RX^{X}, N, NR^{X}, O, S, C(O), S(O), or S(O)₂;
X⁵ is selected from C or N;
X⁶ and X⁷ are each independently selected from CR² or N;
X⁸ and X⁹ are each independently selected from C, CR² or N;
each R^{X} is selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl;
each R² is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR²¹, -C₀₋₂ alkylene-NR²¹R²², -C₀₋₂ alkylene-3- to 10-membered carbocyclyl, -C₀₋₂ alkylene-4- to 10-membered heterocyclyl;
R²¹ and R²² are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
L is 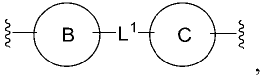 where ring B is attached to the nitrogen atom of the heteroaromatic ring at the R¹ end, and ring C is attached to ring D;
ring B is selected from 3- to 10-membered carbocyclyl, 4- to 10-membered heterocyclyl, 4- to 10-membered bridged rings, 4- to 10-membered bridged heterocycles, 5- to 12-membered spiro rings, 5- to 12-membered spiro heterocycles, 6- to 10-membered aromatic rings, and 5- to 10-membered heteroaromatic rings; wherein the carbocyclyl, heterocyclyl, bridged ring, bridged heterocycle, spiro ring, spiro heterocycle, aromatic ring and heteroaromatic ring are optionally substituted with 1, 2 or 3 R^{B} groups;
preferably, ring B is selected from 4-membered carbocyclyl, 5-membered carbocyclyl, 6-membered carbocyclyl, 7-membered carbocyclyl, 4-membered heterocyclyl, 5-membered heterocyclyl, 6-membered heterocyclyl, 7-membered heterocyclyl, 8-membered heterocyclyl, 5-membered bridged rings, 6-membered bridged rings, 7-membered bridged rings, 8-membered bridged rings, 5-membered bridged heterocycles, 6-membered bridged heterocycles, 7-membered bridged heterocycles, 8-membered bridged heterocycles, 7-membered spiro rings, 8-membered spiro rings, 9-membered spiro rings, 10-membered spiro rings, 11-membered spiro rings, 7-membered spiro heterocycles, 8-membered spiro heterocycles, 9-membered spiro heterocycles, 10-membered spiro heterocycles, and 11-membered spiro heterocycles; wherein the carbocyclyl, heterocyclyl, bridged ring, bridged heterocycle, spiro ring and spiro heterocycle are optionally substituted with 1, 2 or 3 R^{B} groups;
ring C is selected from 3- to 10-membered carbocyclyl, 4- to 10-membered heterocyclyl, 4- to 10-membered bridged rings, 4- to 10-membered bridged heterocycles, 5- to 12-membered spiro rings, 5- to 12-membered spiro heterocycles, 6- to 10-membered aromatic rings, and 5- to 10-membered heteroaromatic rings; wherein the carbocyclyl, heterocyclyl, bridged ring, bridged heterocycle, spiro ring, spiro heterocycle, aromatic ring and heteroaromatic ring are optionally substituted with 1, 2 or 3 R^{C} groups;
preferably, ring C is selected from 4-membered carbocyclyl, 5-membered carbocyclyl, 6-membered carbocyclyl, 7-membered carbocyclyl, 4-membered heterocyclyl, 5-membered heterocyclyl, 6-membered heterocyclyl, 7-membered heterocyclyl, 8-membered heterocyclyl, 5-membered bridged rings, 6-membered bridged rings, 7-membered bridged rings, 8-membered bridged rings, 5-membered bridged heterocycles, 6-membered bridged heterocycles, 7-membered bridged heterocycles, 8-membered bridged heterocycles, 7-membered spiro rings, 8-membered spiro rings, 9-membered spiro rings, 10-membered spiro rings, 11-membered spiro rings, 7-membered spiro heterocycles, 8-membered spiro heterocycles, 9-membered spiro heterocycles, 10-membered spiro heterocycles, and 11-membered spiro heterocycles; wherein the carbocyclyl, heterocyclyl, bridged ring, bridged heterocycle, spiro ring and spiro heterocycle are optionally substituted with 1, 2 or 3 R^{C} groups;
R^{B} and R^{C} are each independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{B1}, -C₀₋₂ alkylene-NR^{B1}R^{B2};
R^{B1} and R^{B2} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
L¹ is selected from a chemical bond, C₁₋₆ alkylene, C₂₋₆ alkenylene, and C₂₋₆ alkynylene; wherein the carbon atom in the alkylene, alkenylene, or alkynylene is optionally substituted with 1, 2, or 3 heteroatoms, and the alkylene, alkenylene, or alkynylene is optionally substituted with 1, 2, or 3 R^{L1} groups;
each R^{L1} is independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl.

2. The compound according to claim 1, the stereoisomer thereof, the deuterated compound thereof, or the pharmaceutically acceptable salt thereof, wherein:
Y¹ is selected from N, Y² is selected from C, Y³ is selected from C, Y⁴ is selected from CH, Y⁵ is selected from N, Y⁶ is selected from CH, Y⁷ is selected from CH; or Y¹ is selected from N, Y² is selected from C, Y³ is selected from N, Y⁴ is selected from CH, Y⁵ is selected from C, Y⁶ is selected from CH, Y⁷ is selected from CH; or Y¹ is selected from N, Y² is selected from C, Y³ is selected from N, Y⁴ is selected from N, Y⁵ is selected from C, Y⁶ is selected from CH, Y⁷ is selected from CH; or Y¹ is selected from N, Y² is selected from C, Y³ is selected from N, Y⁴ is selected from CH, Y⁵ is selected from C, Y⁶ is selected from CH, Y⁷ is selected from N;
or Y¹ is selected from N, Y² is selected from C, Y³ is selected from N, Y⁴ is selected from CH, Y⁵ is selected from C, Y⁶ is selected from N, Y⁷ is selected from CH; ring D is selected from

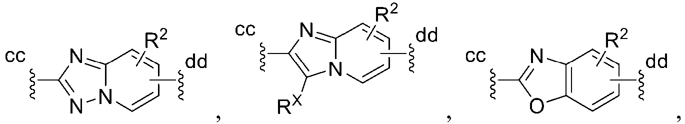

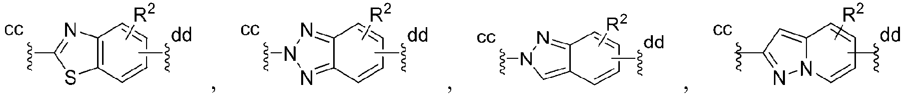

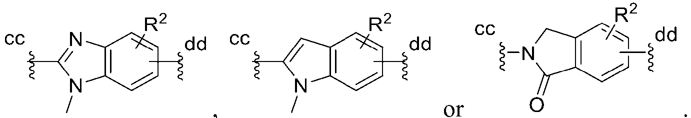

3. The compound according to claim 1, the stereoisomer thereof, the deuterated compound thereof, or the pharmaceutically acceptable salt thereof, wherein the compound represented by formula I is shown as follows: 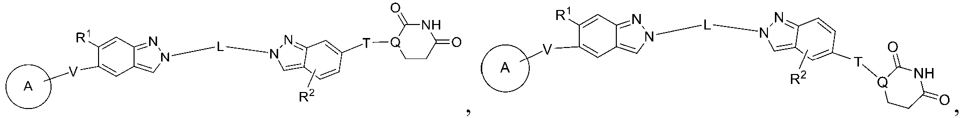 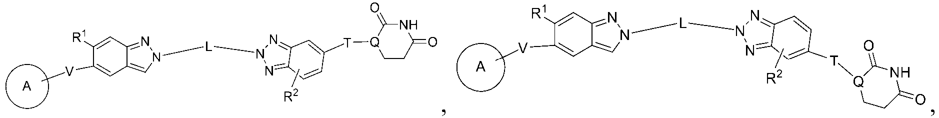 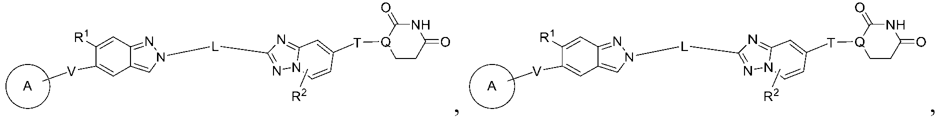 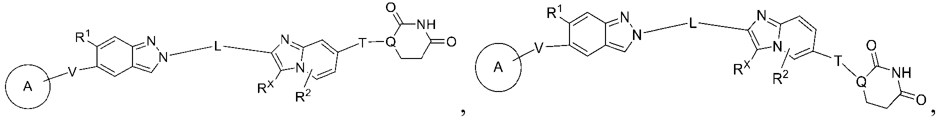 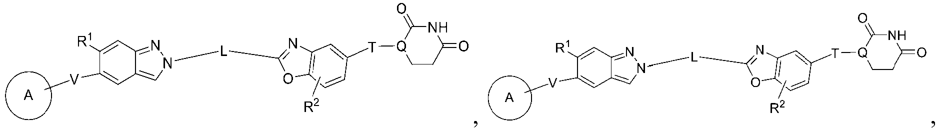 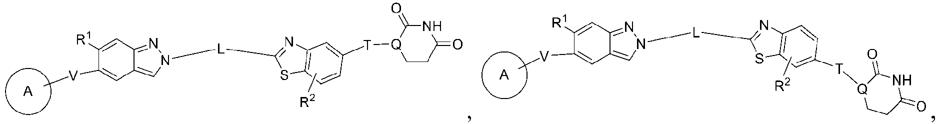 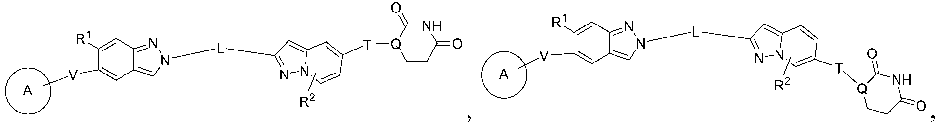 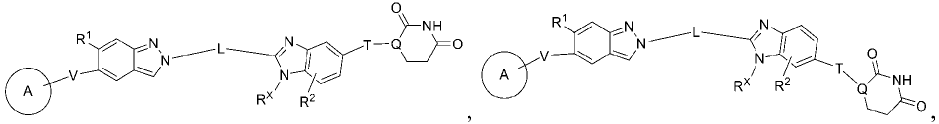 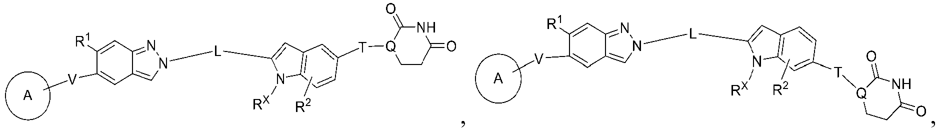 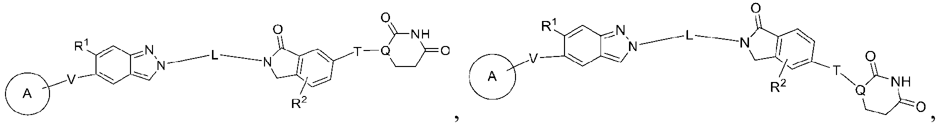 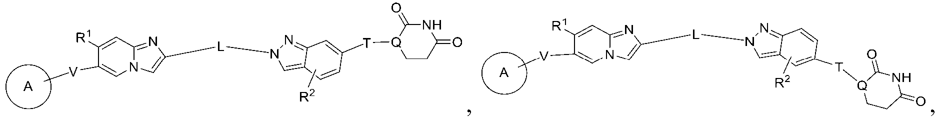 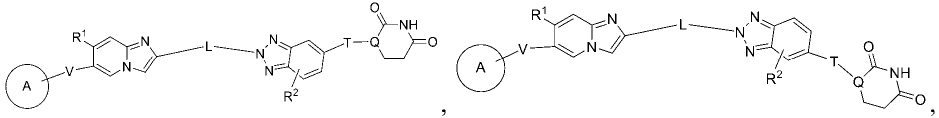 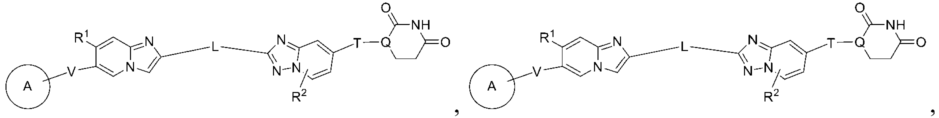 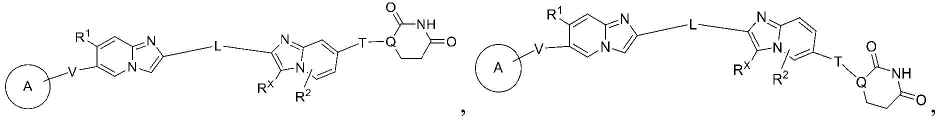 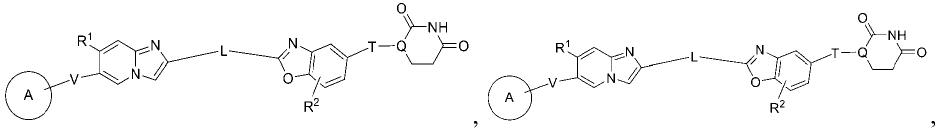 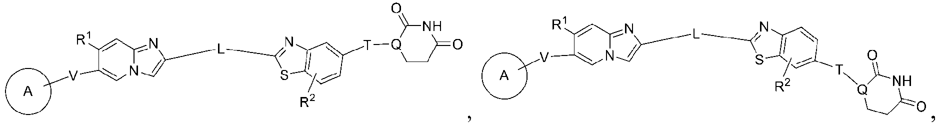 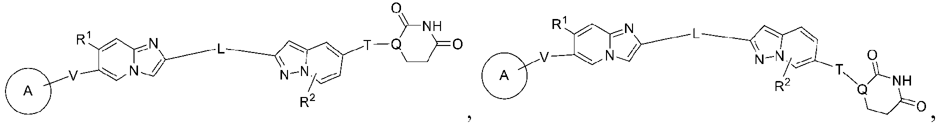 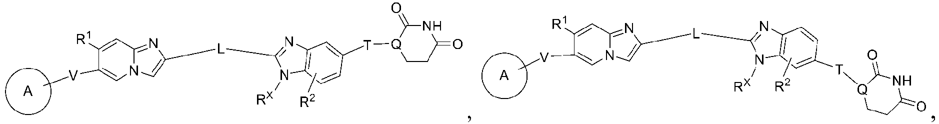 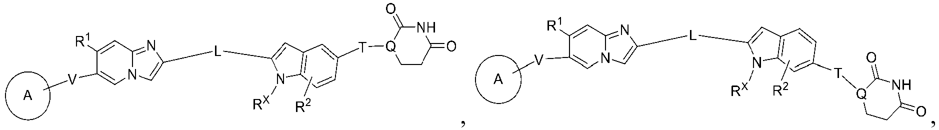 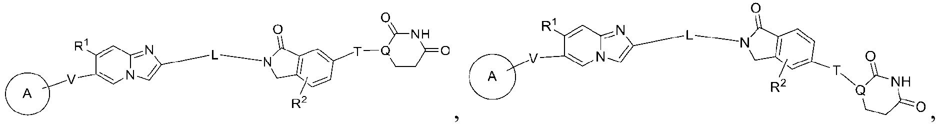 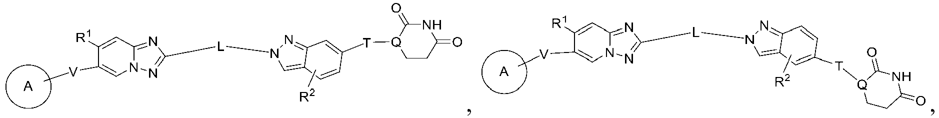 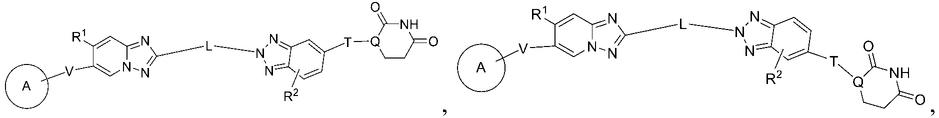 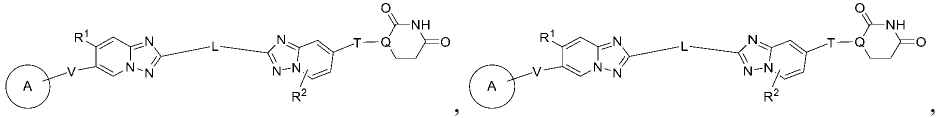 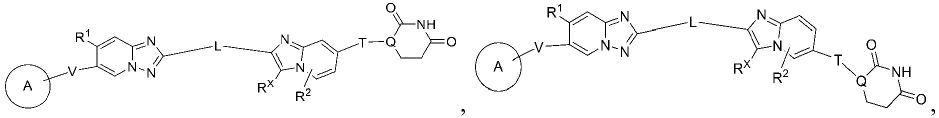 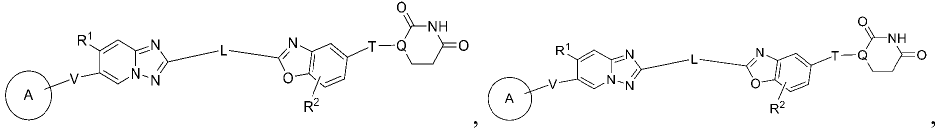 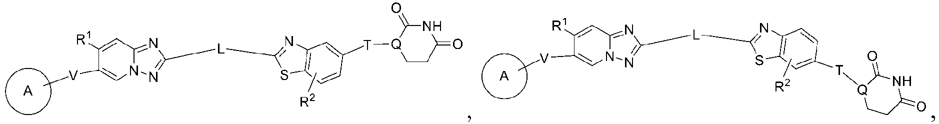 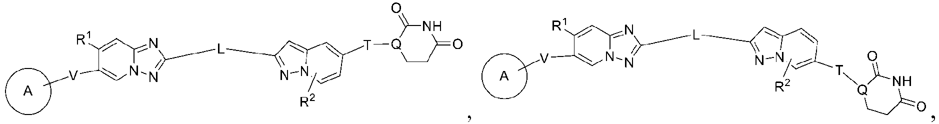 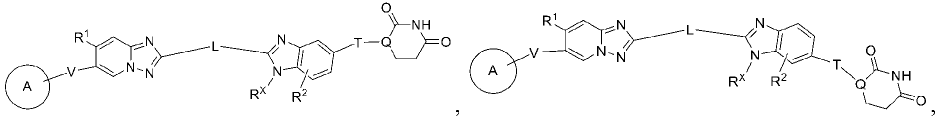 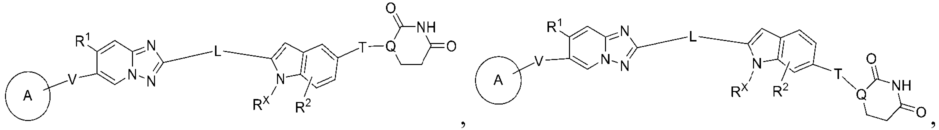 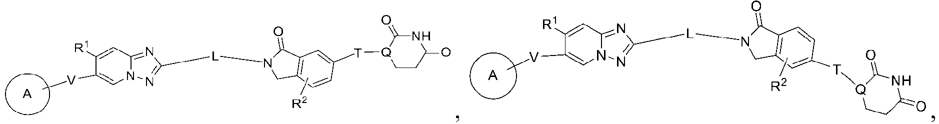 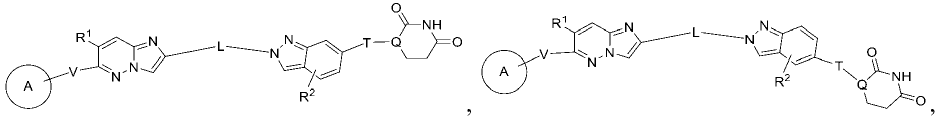 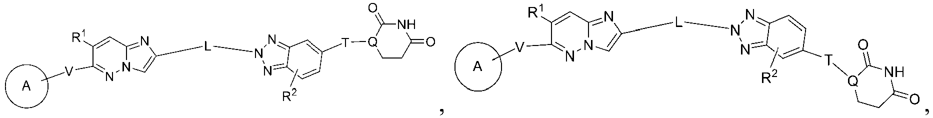 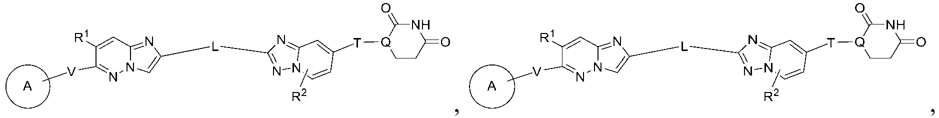 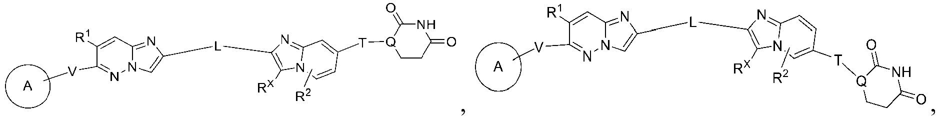 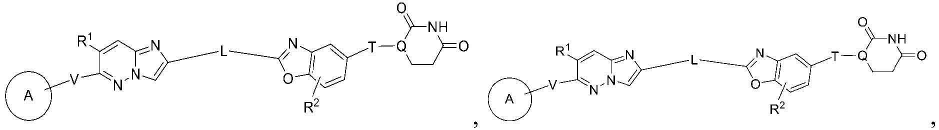 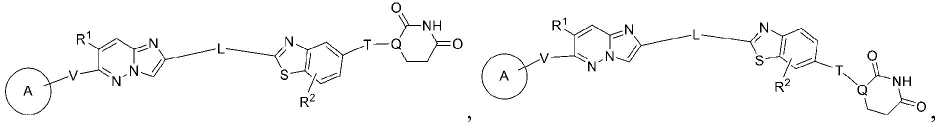 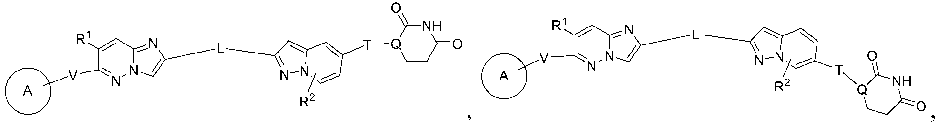 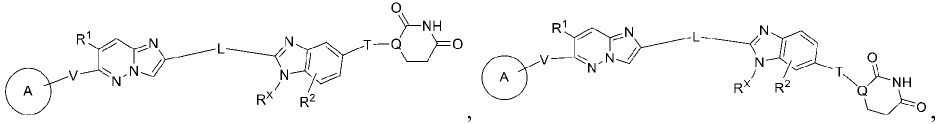 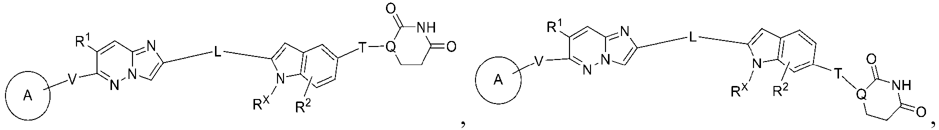 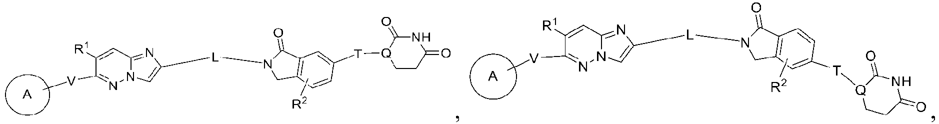 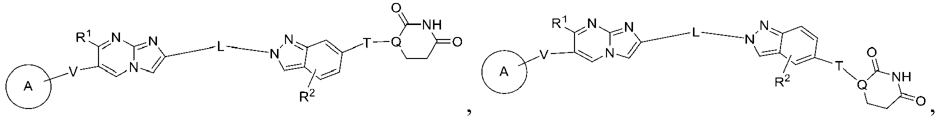 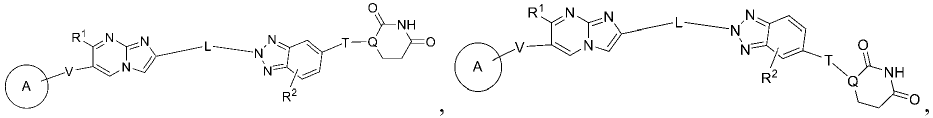 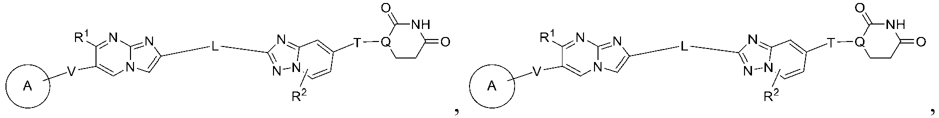 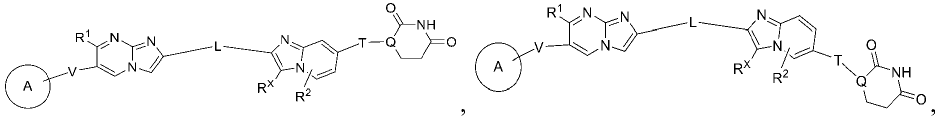 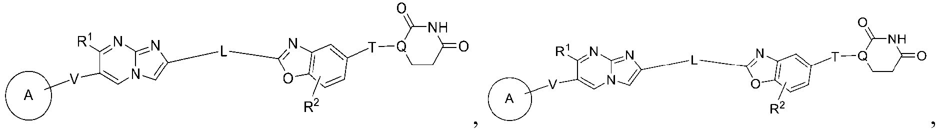 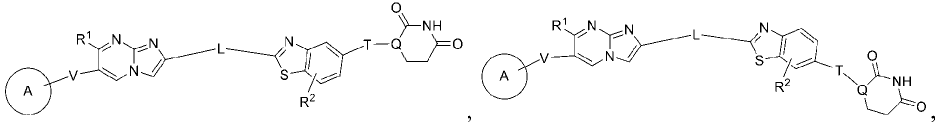 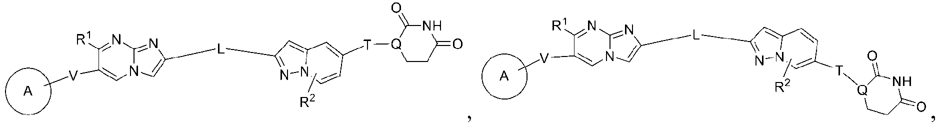 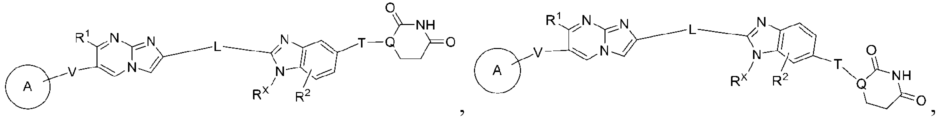 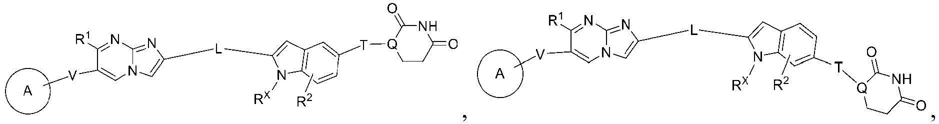 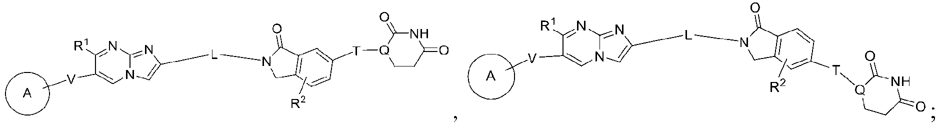 wherein V, T, Q, ring A, R¹, R², and L are as defined in claim 1.

4. The compound according to any one of claims 1-3, the stereoisomer thereof, the deuterated compound thereof, or the pharmaceutically acceptable salt thereof, wherein ring A is selected from: 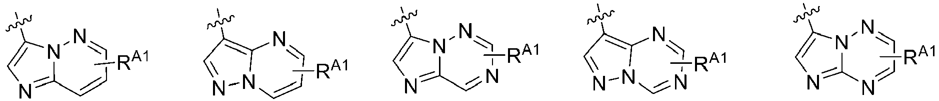 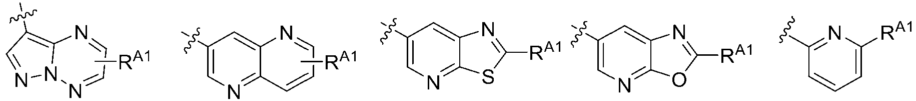 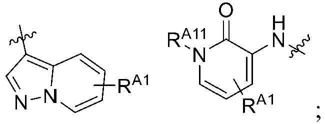
R^{A1} is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, monomethylamino or dimethylamino;
R^{A11} is selected from methyl, ethyl, propyl, cyclopropyl, and halogen-substituted methyl, ethyl, propyl, and cyclopropyl.

5. The compound according to any one of claims 1-3, the stereoisomer thereof, the deuterated compound thereof, or the pharmaceutically acceptable salt thereof, wherein:
R¹ is selected from hydrogen, deuterium, halogen, cyano, methyl, ethyl, propyl, cyclopropyl, cyclobutyl, cyclopentyl, hydroxymethyl, methoxy, ethoxy, trifluoromethyl, difluoromethyl, monofluoromethyl, methoxymethyl, ethoxymethyl, monomethylamino, dimethylamino, deuterated monomethylamino, and deuterated dimethylamino;
or, R¹ is selected from rings below:

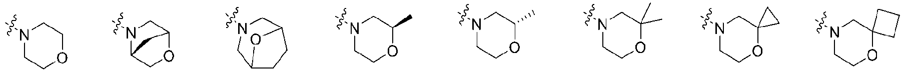

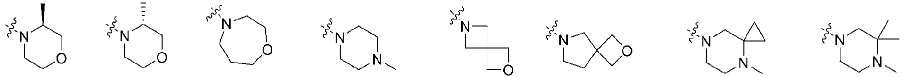

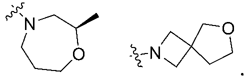

6. The compound according to any one of claims 1-3, the stereoisomer thereof, the deuterated compound thereof, or the pharmaceutically acceptable salt thereof, wherein:
R² is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, monomethylamino or dimethylamino.

7. The compound according to any one of claims 1-3, the stereoisomer thereof, the deuterated compound thereof, or the pharmaceutically acceptable salt thereof, wherein Q is selected from CH or N.

8. The compound according to any one of claims 1-3, the stereoisomer thereof, the deuterated compound thereof, or the pharmaceutically acceptable salt thereof, wherein:
ring B is selected from 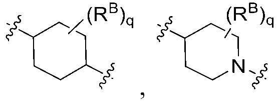 ; where q is 0, 1, 2 or 3;
each R^{B} is independently selected from hydrogen, deuterium, halogen, cyano, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, -C₀₋₂ alkylene-OR^{B1}, -C₀₋₂ alkylene-NR^{B1}R^{B2};
R^{B1} and R^{B2} are each independently selected from hydrogen, C₁₋₆ alkyl, C₂₋₆ alkenyl, C₂₋₆ alkynyl, halogen-substituted C₁₋₆ alkyl, halogen-substituted C₂₋₆ alkenyl, halogen-substituted C₂₋₆ alkynyl, deuterated C₁₋₆ alkyl, deuterated C₂₋₆ alkenyl, deuterated C₂₋₆ alkynyl;
ring C is selected from 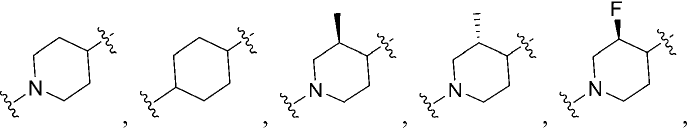 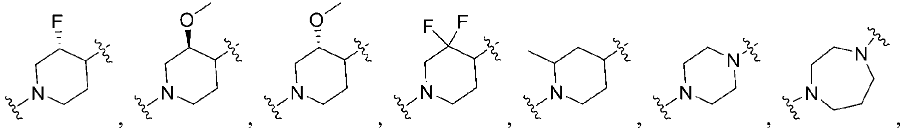 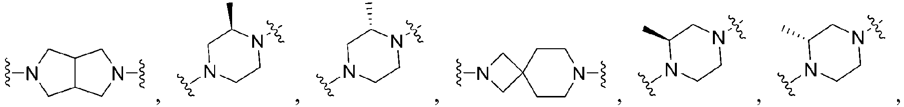 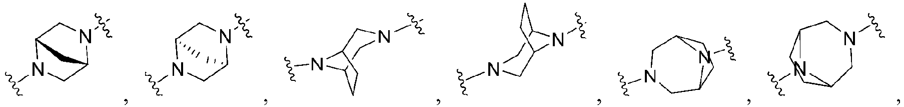 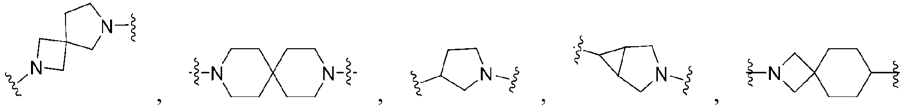 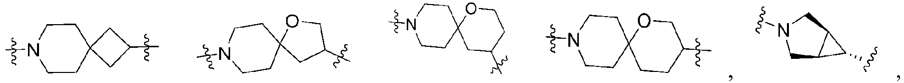 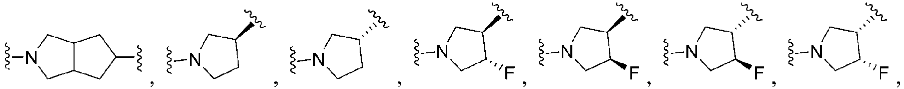 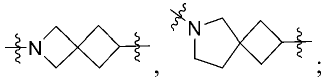 L¹ is selected from a chemical bond, methylene, and ethylene.

9. The compound according to claim 8, the stereoisomer thereof, the deuterated compound thereof, or the pharmaceutically acceptable salt thereof, wherein L is selected from structures below: 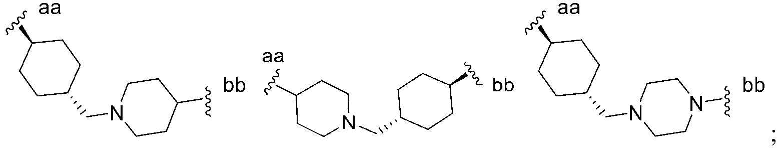 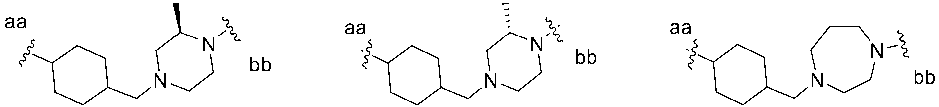 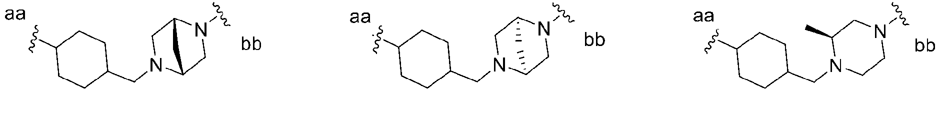 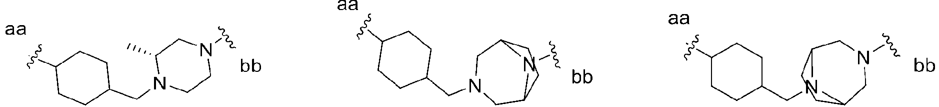 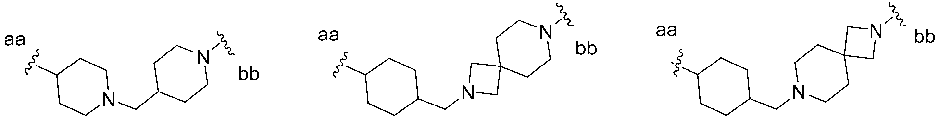 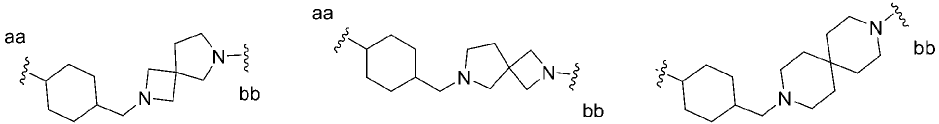 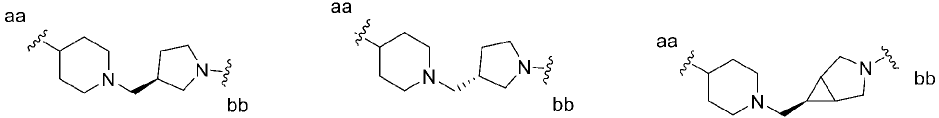 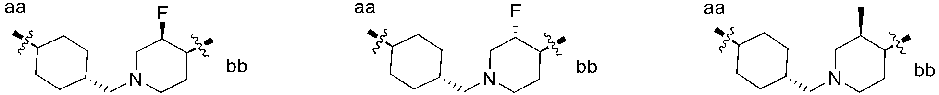 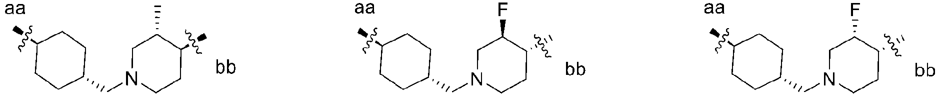 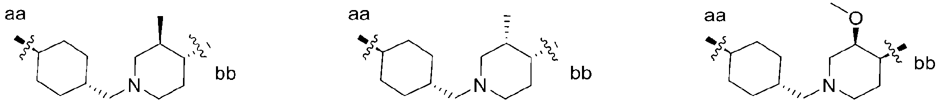 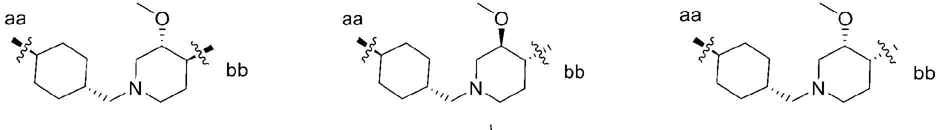 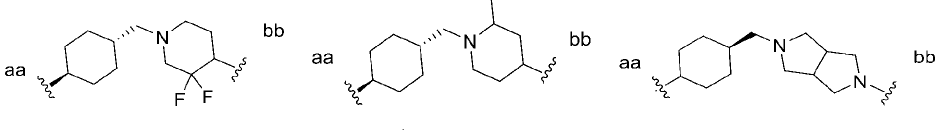 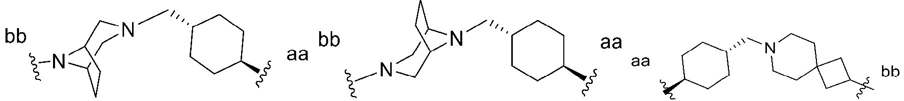 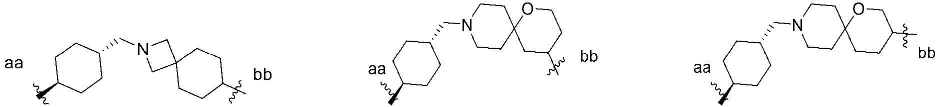 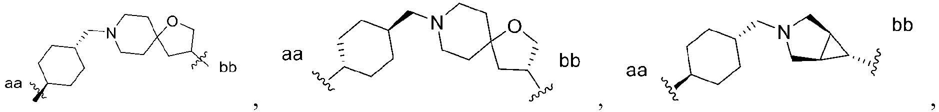 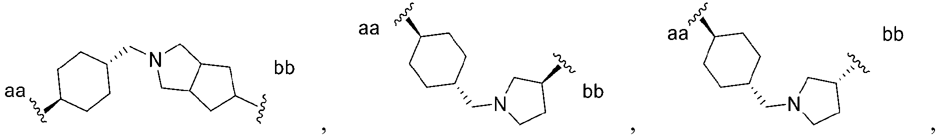 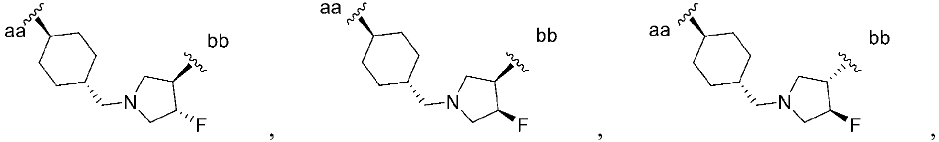 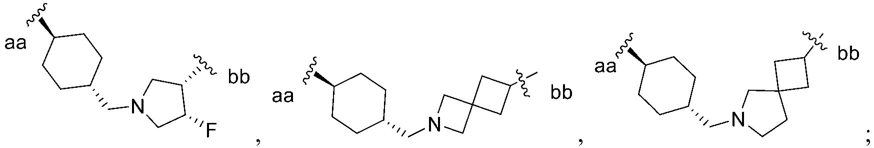 where the aa-end is attached to the nitrogen atom of the heteroaromatic ring at the R¹-end, and the bb-end is attached to ring D.

10. The compound according to any one of claims 1-9, the stereoisomer thereof, the deuterated compound thereof, or the pharmaceutically acceptable salt thereof, wherein the compound specifically is:

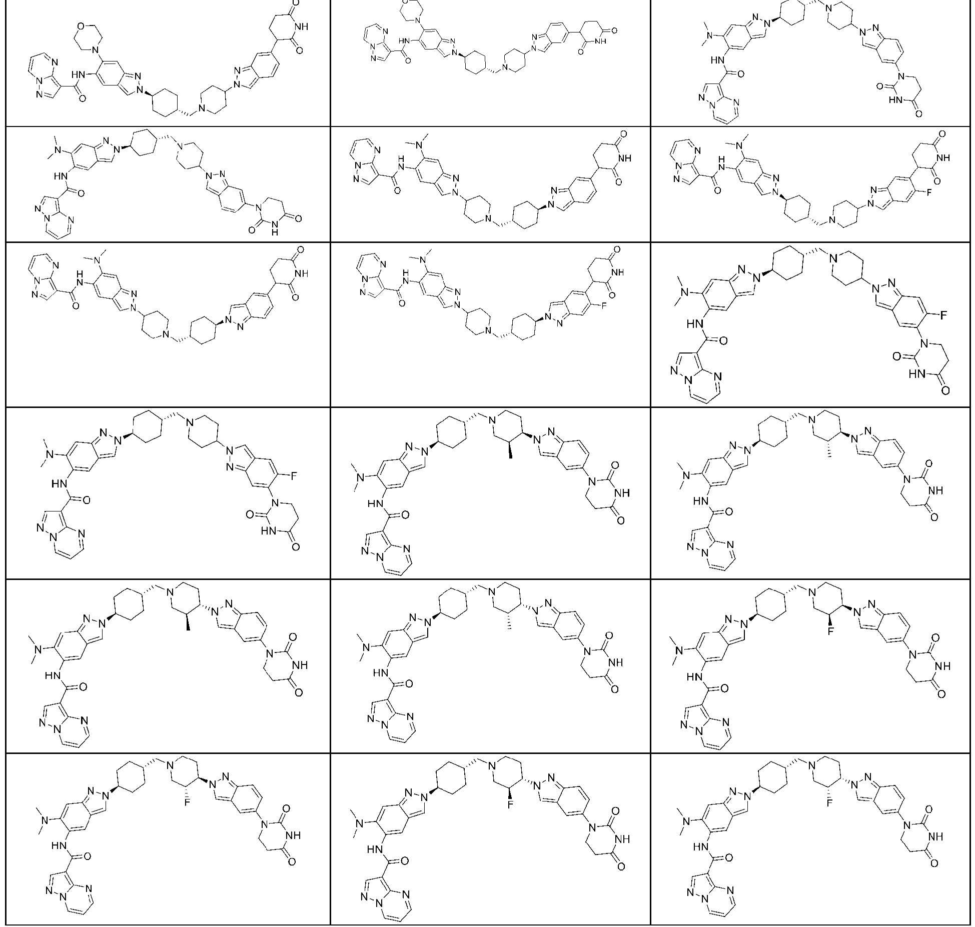

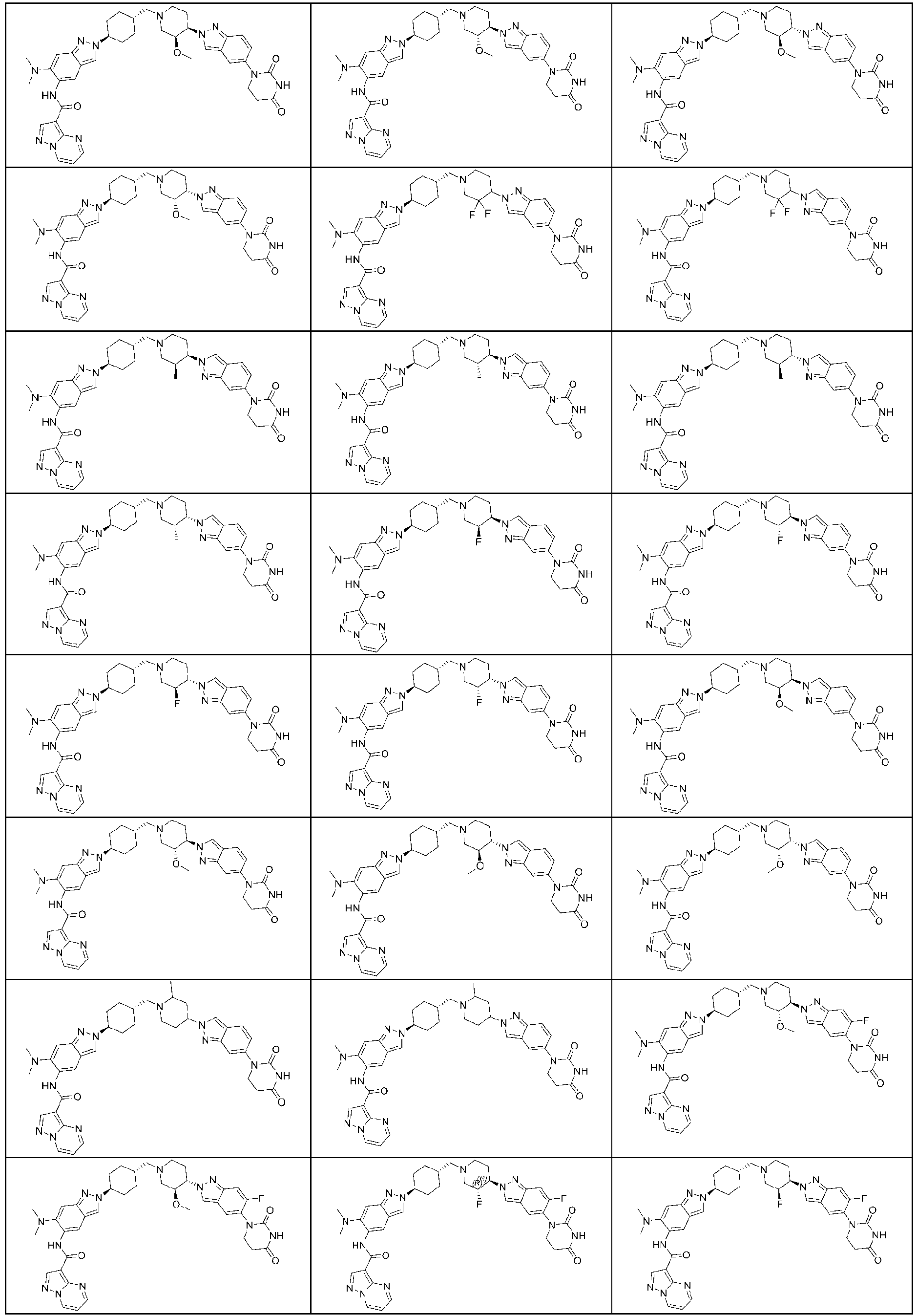

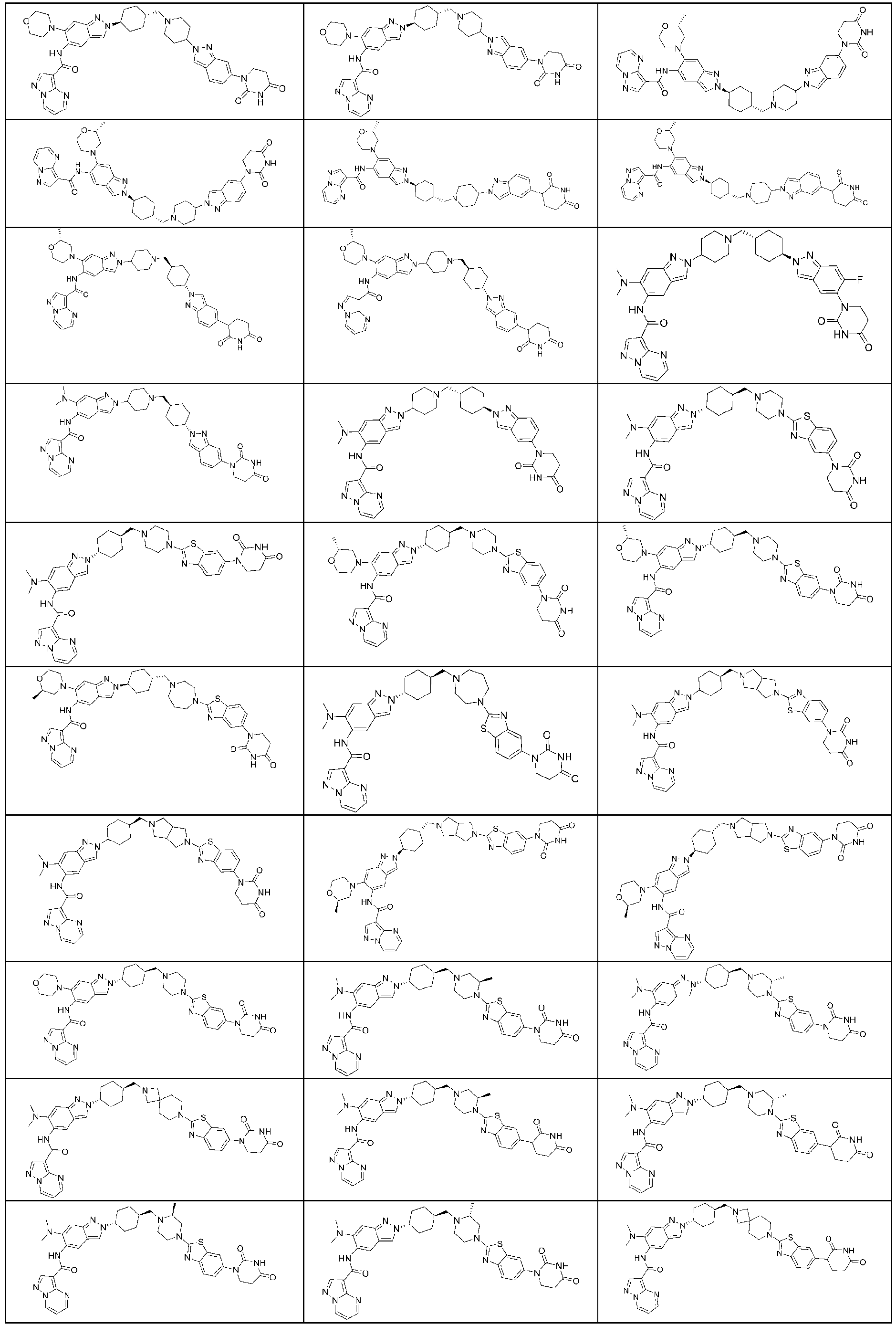

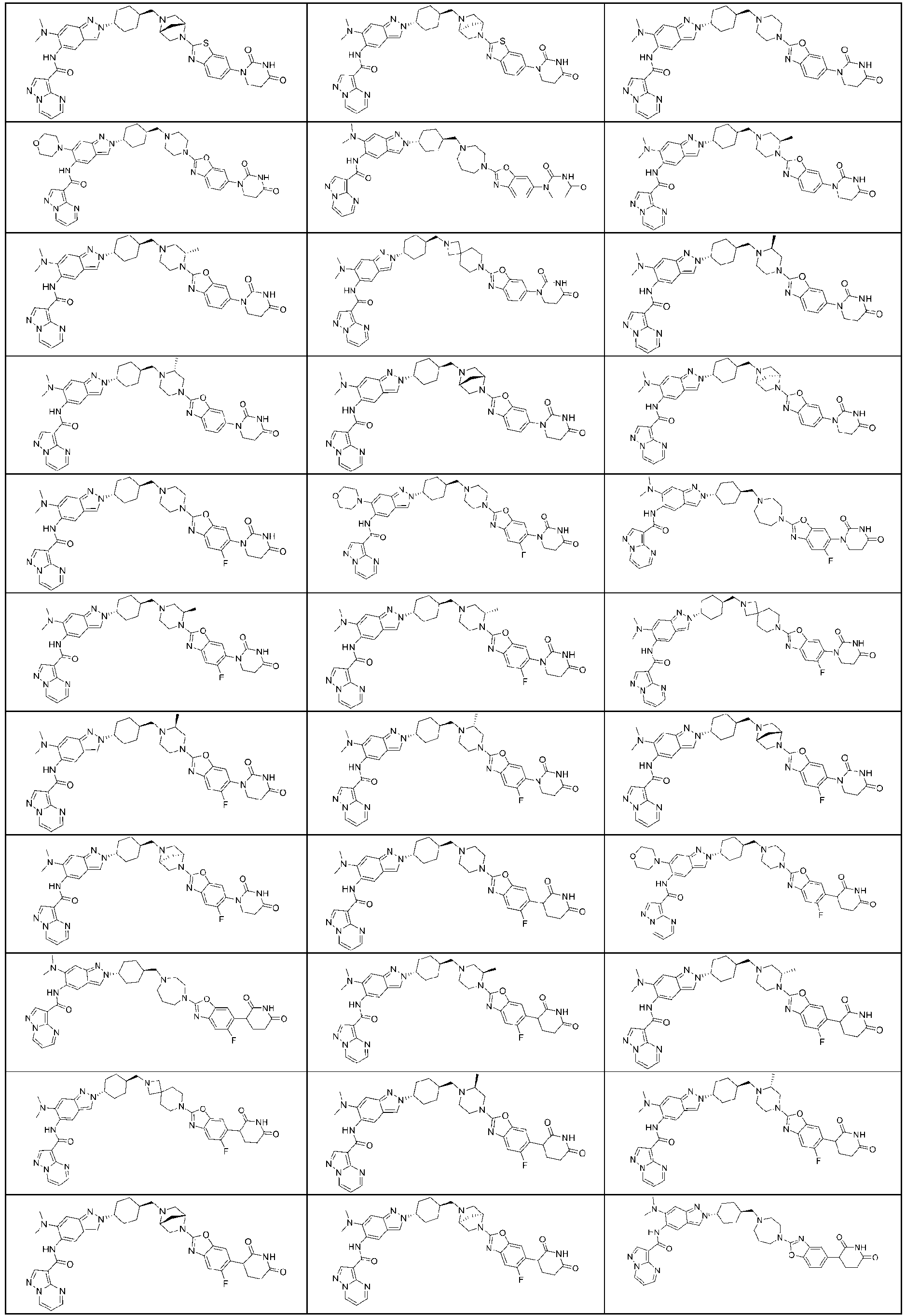

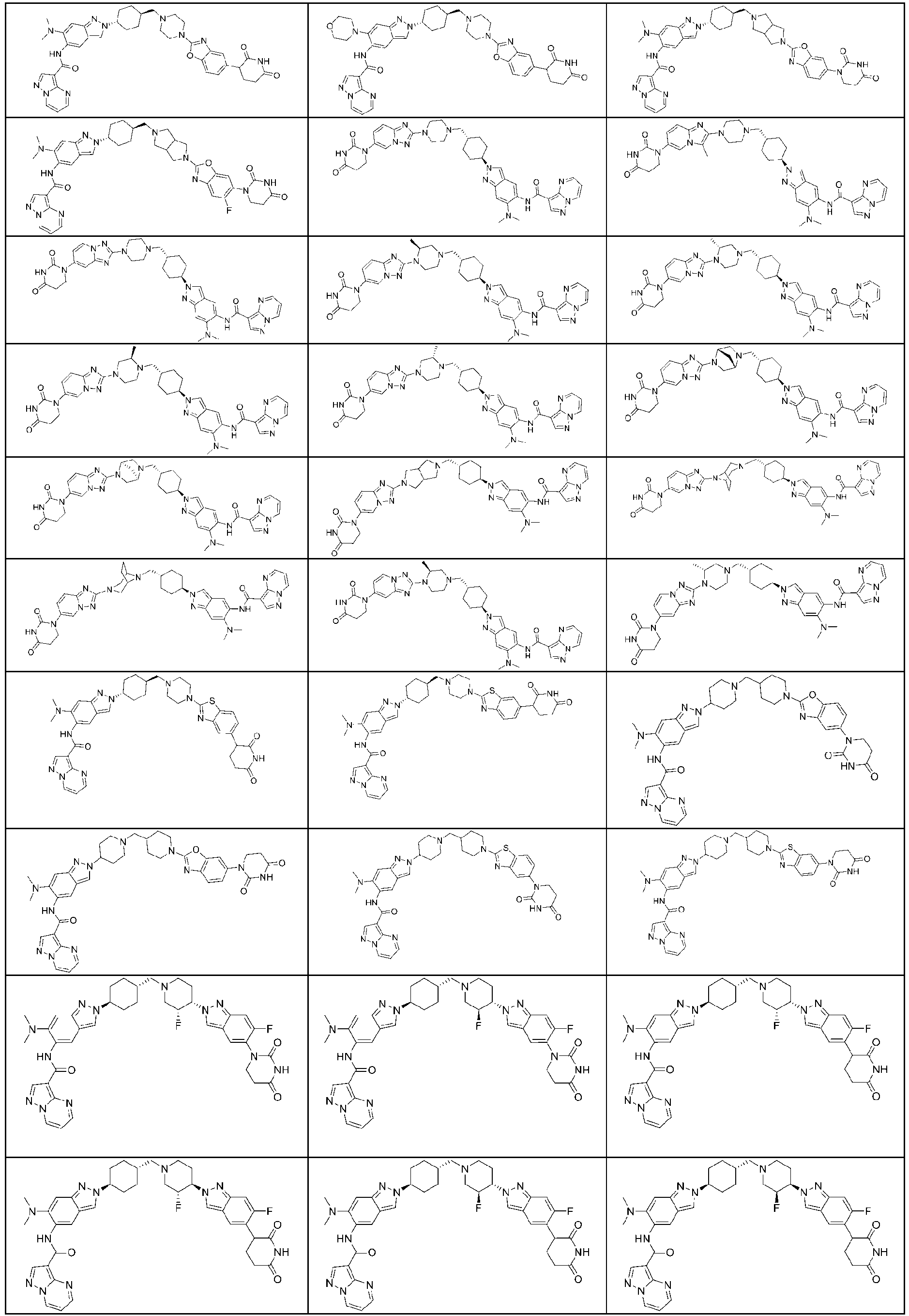

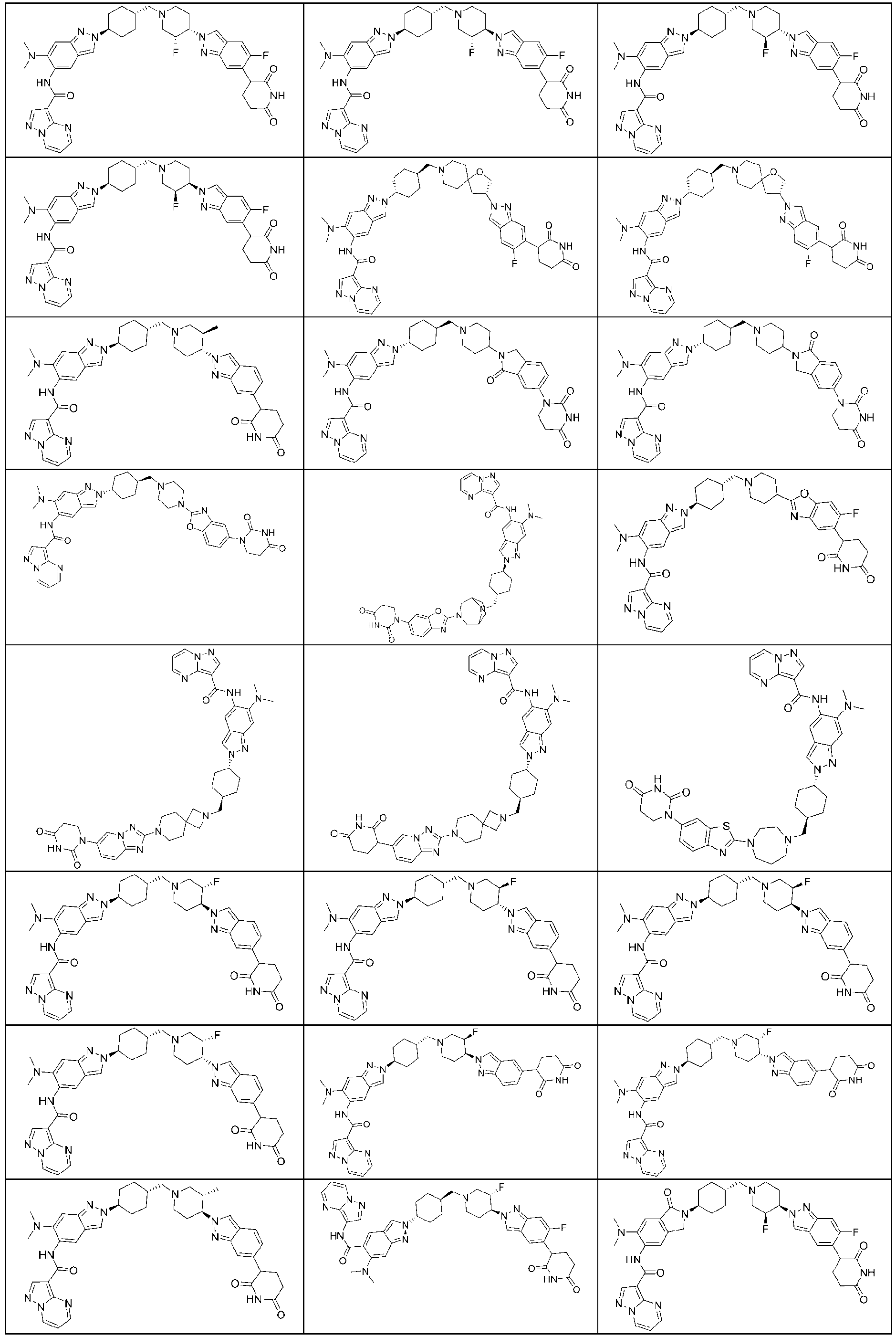

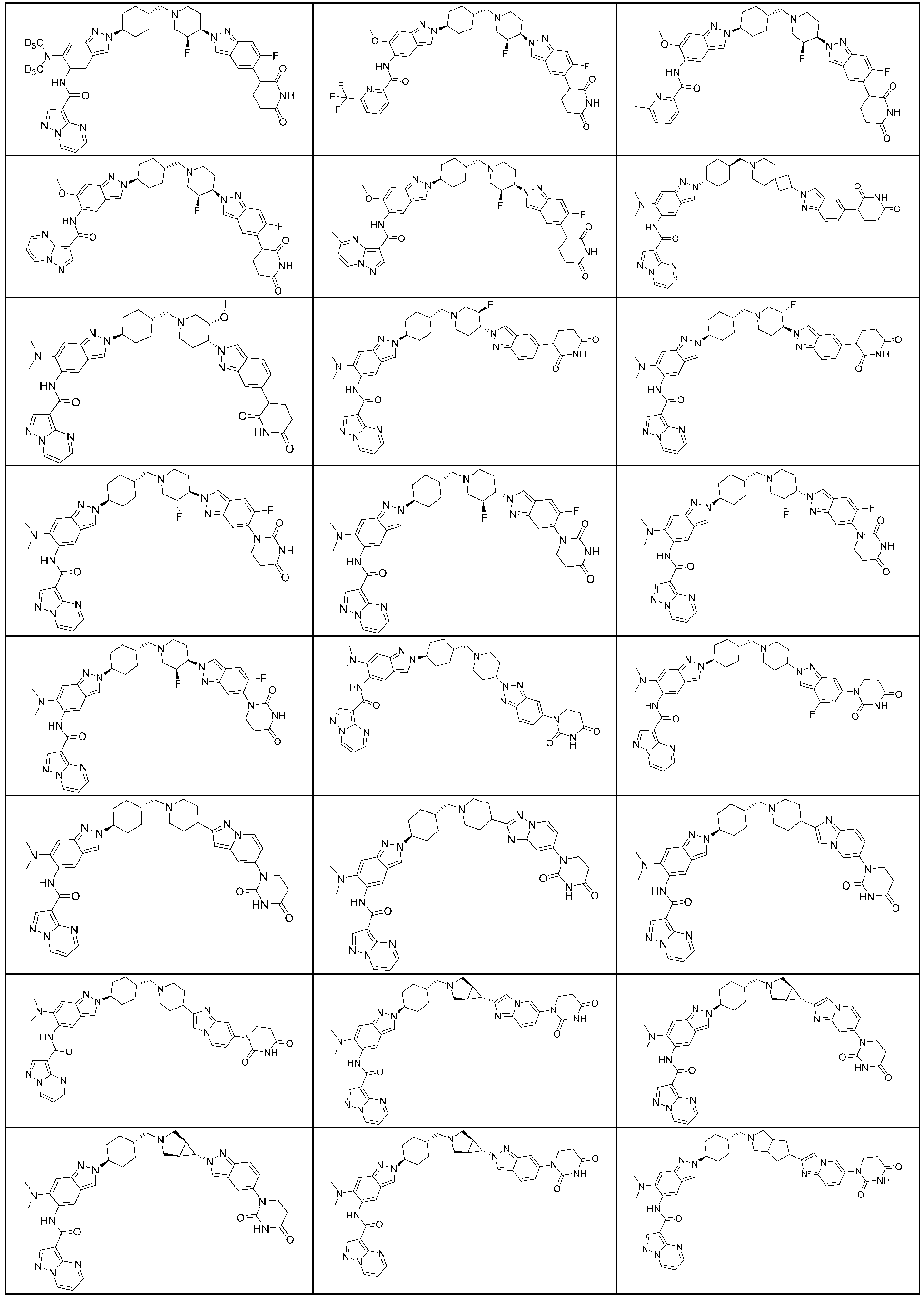

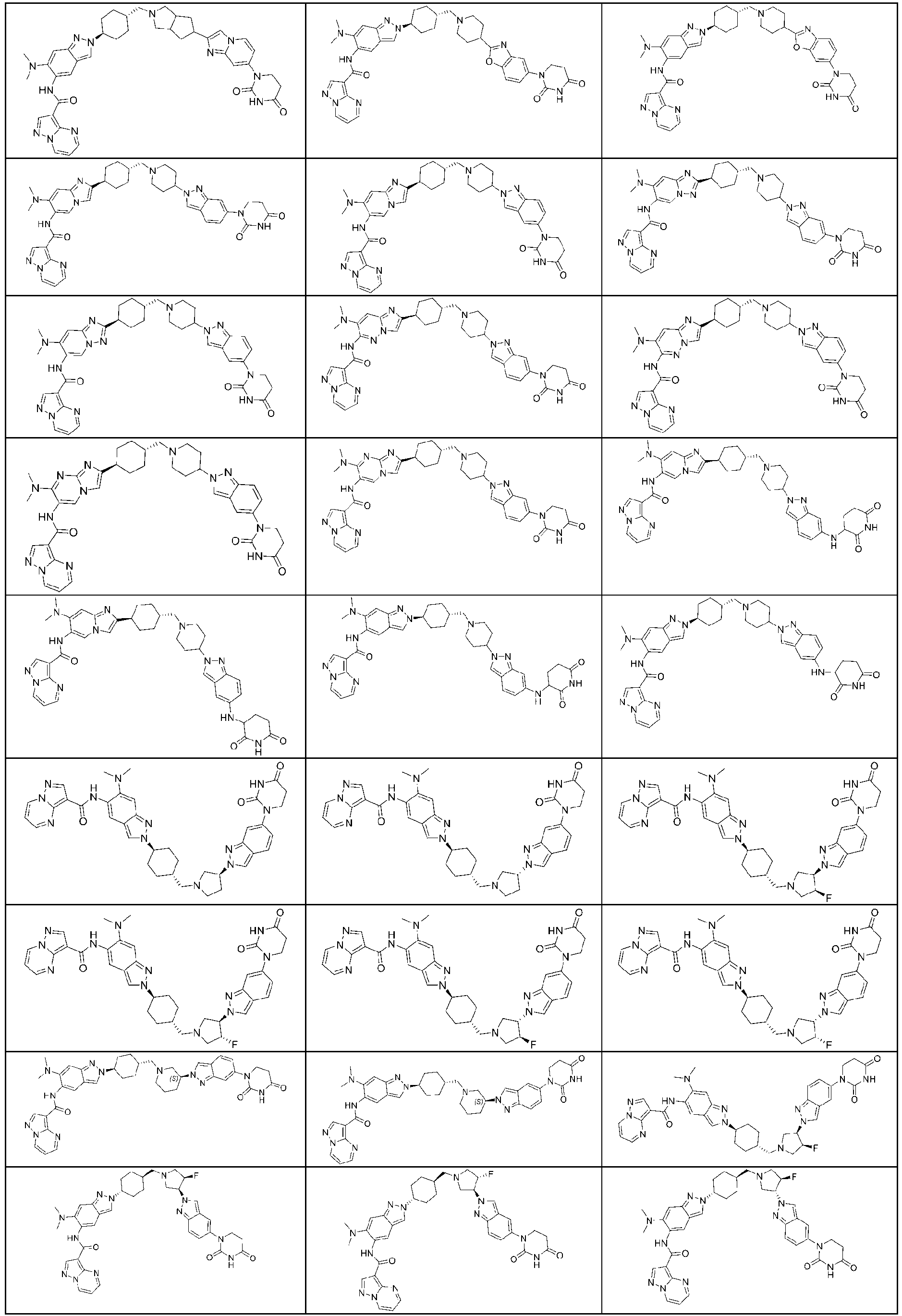

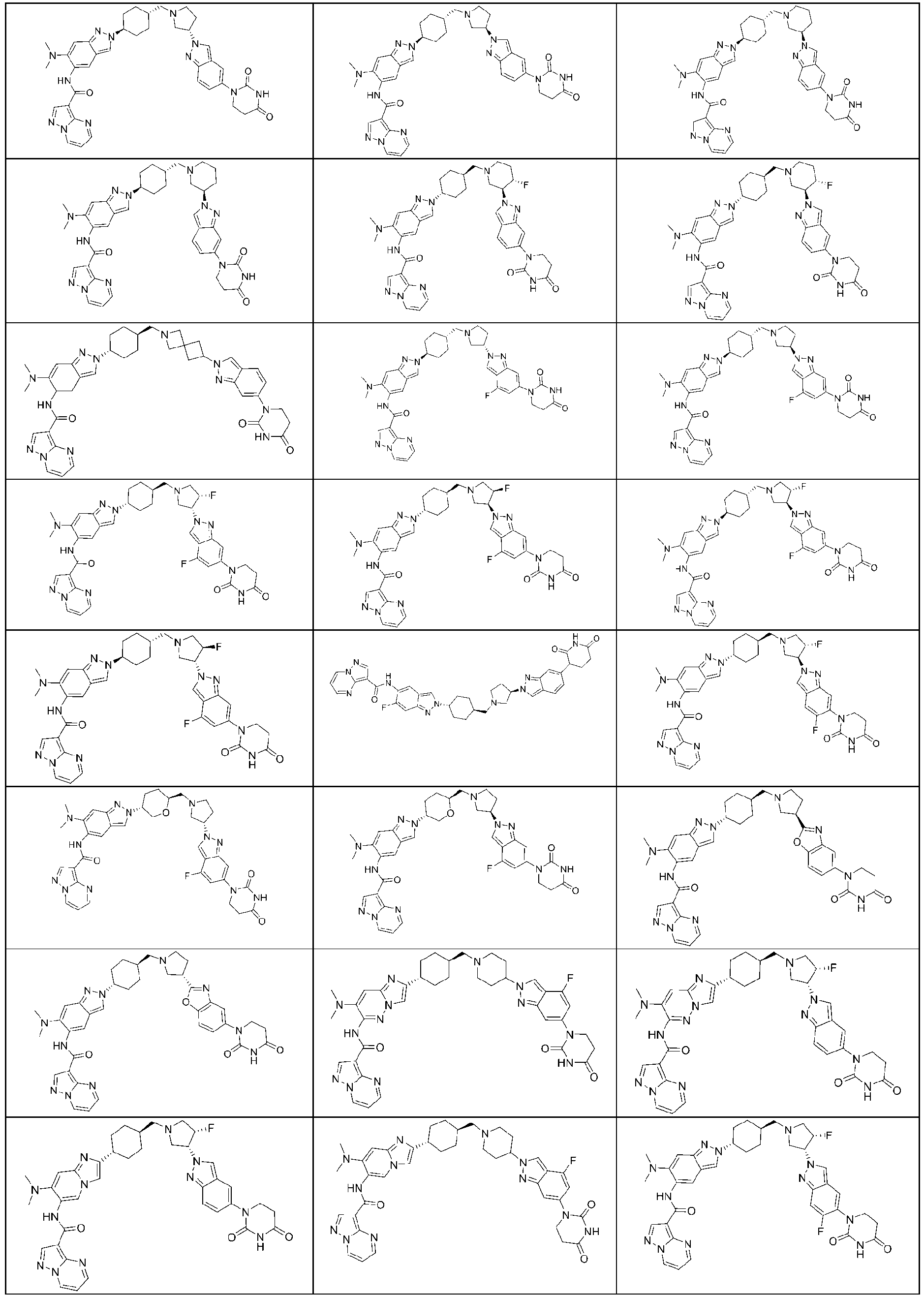

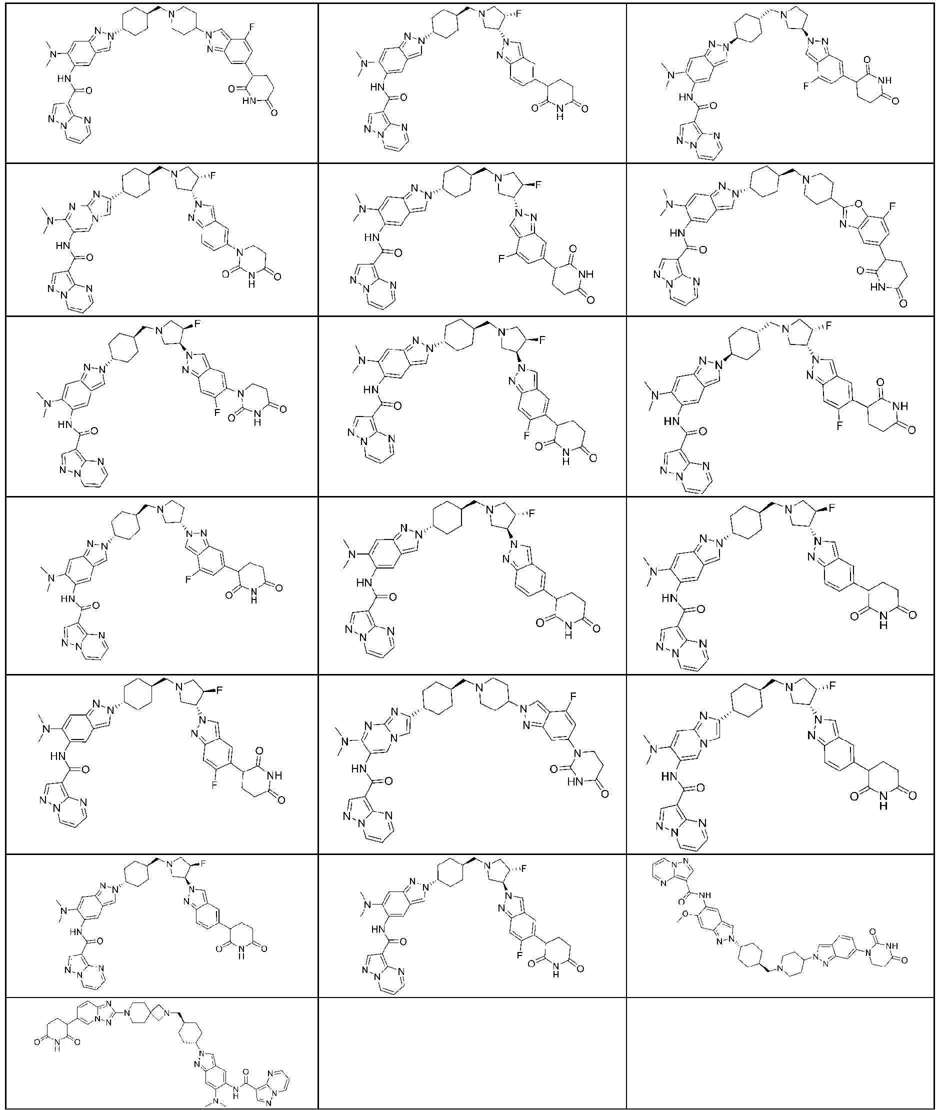

11. Use of the compound according to any one of claims 1-10, the stereoisomer thereof, the deuterated compound thereof, or the pharmaceutically acceptable salt thereof, in the preparation of a drug for the treatment and prevention of diseases associated with or mediated by one or more of the interleukin-1 receptor-associated kinase 4 (IRAK4) signaling pathway, interleukin-6 (IL-6) receptor, and tumor necrosis factor-α (TNFα).

12. The use according to claim 11, wherein the disease includes a cancer, a neurodegenerative disease, a viral disease, an autoimmune disease, an inflammatory disease, a genetic disease, a hormone-related disease, a metabolic disorder, an organ transplantation-related disease, an immunodeficiency disease, an osteoclastic disease, a proliferative disease, an infectious disease, thrombin-induced platelet aggregation, a liver disease, a lesion caused by T cell activation, and a cardiovascular disease.

13. A pharmaceutical composition, being a preparation prepared from the compound according to any one of claims 1-10, the stereoisomer thereof, the deuterated compound thereof, or the pharmaceutically acceptable salt thereof, together with a pharmaceutically acceptable excipient.
